# EUROPEAN PATENT APPLICATION

(11) **EP 3 739 060 A1**
(43) Date of publication of application: **18.11.2020**
(21) Application number: 20177718.2
(22) Date of filing: 19.11.2010
(51) Int. Cl.: C12Q 1/68

(54) **METHODS TO PREDICT CLINICAL OUTCOME OF CANCER**

(30) Priority: 23.11.2009 US 26376309 P
(62) Divisional of application: 19175741.8
(71) Applicant: Genomic Health, Inc., Redwood City, CA 94063 (US)
(72) Inventor: BAKER, Joffre B., Montara, CA 94037 (US); CRONIN, Maureen T., Los Altos, CA 94024 (US); COLLIN, Francois, Berkeley, CA 94702 (US); LIU, Mei-Lan, San Mateo, CA 94403 (US)
(74) Representative: Forresters IP LLP

(57) **Abstract**

The present invention provides methods to determine the prognosis and appropriate treatment for patients diagnosed with cancer, based on the expression levels of one or more biomarkers. More particularly, the invention relates to the identification of genes, or sets of genes, able to distinguish breast cancer patients with a good clinical prognosis from those with a bad clinical prognosis. The invention further provides methods for providing a personalized genomics report for a cancer patient. The inventions also relates to computer systems and software for data analysis using the prognostic and statistical methods disclosed herein.

## Description

### CROSS REFERENCE

This application claims the benefit of U.S. Provisional Patent Application No. 61/263,763, filed November 23, 2009, which application is incorporated herein by reference in its entirety.

### INTRODUCTION

Oncologists have a number of treatment options available to them, including different combinations of therapeutic regimens that are characterized as "standard of care." The absolute benefit from adjuvant treatment is larger for patients with poor prognostic features, and this has resulted in the policy to select only these so-called 'high-risk' patients for adjuvant chemotherapy. See, e.g., S. Paik, et al., J Clin Oncol. 24(23):3726-34 (2006). Therefore, the best likelihood of good treatment outcome requires that patients be assigned to optimal available cancer treatment, and that this assignment be made as quickly as possible following diagnosis.

Today our healthcare system is riddled with inefficiency and wasteful spending - one example of this is that the efficacy rate of many oncology therapeutics working only about 25% of the time. Many of those cancer patients are experiencing toxic side effects for costly therapies that may not be working. This imbalance between high treatment costs and low therapeutic efficacy is often a result of treating a specific diagnosis one way across a diverse patient population. But with the advent of gene profiling tools, genomic testing, and advanced diagnostics, this is beginning to change.

In particular, once a patient is diagnosed with breast cancer there is a strong need for methods that allow the physician to predict the expected course of disease, including the likelihood of cancer recurrence, long-term survival of the patient, and the like, and select the most appropriate treatment option accordingly. Accepted prognostic and predictive factors in breast cancer include age, tumor size, axillary lymph node status, histological tumor type, pathological grade and hormone receptor status. Molecular diagnostics, however, have been demonstrated to identify more patients with a low risk of breast cancer than was possible with standard prognostic indicators. S. Paik, The Oncologist 12(6):631-635 (2007).

Despite recent advances, the challenge of breast cancer treatment remains to target specific treatment regimens to pathogenically distinct tumor types, and ultimately personalize tumor treatment in order to maximize outcome. Accurate prediction of prognosis and clinical outcome would allow the oncologist to tailor the administration of adjuvant chemotherapy such that women with a higher risk of a recurrence or poor prognosis would receive more aggressive treatment. Furthermore, accurately stratifying patients based on risk would greatly advance the understanding of expected absolute benefit from treatment, thereby increasing success rates for clinical trials for new breast cancer therapies.

Currently, most diagnostic tests used in clinical practice are frequently not quantitative, relying on immunohistochemistry (IHC). This method often yields different results in different laboratories, in part because the reagents are not standardized, and in part because the interpretations are subjective and cannot be easily quantified. Other RNA-based molecular diagnostics require fresh-frozen tissues, which presents a myriad of challenges including incompatibilities with current clinical practices and sample transport regulations. Fixed paraffin-embedded tissue is more readily available and methods have been established to detect RNA in fixed tissue. However, these methods typically do not allow for the study of large numbers of genes (DNA or RNA) from small amounts of material. Thus, traditionally fixed tissue has been rarely used other than for IHC detection of proteins.

### SUMMARY

The present invention provides a set of genes, the expression levels of which are associated with a particular clinical outcome in cancer. For example, the clinical outcome could be a good or bad prognosis assuming the patient receives the standard of care. The clinical outcome may be defined by clinical endpoints, such as disease or recurrence free survival, metastasis free survival, overall survival, etc.

The present invention accommodates the use of archived paraffin-embedded biopsy material for assay of all markers in the set, and therefore is compatible with the most widely available type of biopsy material. It is also compatible with several different methods of tumor tissue harvest, for example, via core biopsy or fine needle aspiration. The tissue sample may comprise cancer cells.

In one aspect, the present invention concerns a method of predicting a clinical outcome of a cancer patient, comprising (a) obtaining an expression level of an expression product (e.g., an RNA transcript) of at least one prognostic gene listed in Tables 1-12 from a tissue sample obtained from a tumor of the patient; (b) normalizing the expression level of the expression product of the at least one prognostic gene, to obtain a normalized expression level; and (c) calculating a risk score based on the normalized expression value, wherein increased expression of prognostic genes in Tables 1, 3, 5, and 7 are positively correlated with good prognosis, and wherein increased expression of prognostic genes in Tables 2, 4, 6, and 8 are negatively associated with good prognosis. In some embodiments, the tumor is estrogen receptor-positive. In other embodiments, the tumor is estrogen receptor negative.

In one aspect, the present disclosure provides a method of predicting a clinical outcome of a cancer patient, comprising (a) obtaining an expression level of an expression product (e.g., an RNA transcript) of at least one prognostic gene from a tissue sample obtained from a tumor of the patient, where the at least one prognostic gene is selected from GSTM2, IL6ST, GSTM3, C8orf4, TNFRSF11B, NAT1, RUNX1, CSF1, ACTR2, LMNB1, TFRC, LAPTM4B, ENO1, CDC20, and IDH2; (b) normalizing the expression level of the expression product of the at least one prognostic gene, to obtain a normalized expression level; and (c) calculating a risk score based on the normalized expression value, wherein increased expression of a prognostic gene selected from GSTM2, IL6ST, GSTM3, C8orf4, TNFRSF11B, NAT1, RUNX1, and CSF1 is positively correlated with good prognosis, and wherein increased expression of a prognostic gene selected from ACTR2, LMNB1, TFRC, LAPTM4B, ENO1, CDC20, and IDH2 is negatively associated with good prognosis. In some embodiments, the tumor is estrogen receptor-positive. In other embodiments, the tumor is estrogen receptor negative.

In various embodiments, the normalized expression level of at least 2, or at least 5, or at least 10, or at least 15, or at least 20, or a least 25 prognostic genes (as determined by assaying a level of an expression product of the gene) is determined. In alternative embodiments, the normalized expression levels of at least one of the genes that co-expresses with prognostic genes in Tables 16-18 is obtained.

In another embodiment, the risk score is determined using normalized expression levels of at least one a stromal or transferrin receptor group gene, or a gene that co-expresses with a stromal or transferrin receptor group gene.

In another embodiment, the cancer is breast cancer. In another embodiment, the patient is a human patient.

In yet another embodiment, the cancer is ER-positive breast cancer.

In yet another embodiment, the cancer is ER-negative breast cancer.

In a further embodiment, the expression product comprises RNA. For example, the RNA could be exonic RNA, intronic RNA, or short RNA (e.g., microRNA, siRNA, promoter-associated small RNA, shRNA, etc.). In various embodiments, the RNA is fragmented RNA.

In a different aspect, the invention concerns an array comprising polynucleotides hybridizing to an RNA transcription of at least one of the prognostic genes listed in Tables 1-12.

In a still further aspect, the invention concerns a method of preparing a personalized genomics profile for a patient, comprising (a) obtaining an expression level of an expression product (e.g., an RNA transcript) of at least one prognostic gene listed in Tables 1-12 from a tissue sample obtained from a tumor of the patient; (b) normalizing the expression level of the expression product of the at least one prognostic gene to obtain a normalized expression level; and (c) calculating a risk score based on the normalized expression value, wherein increased expression of prognostic genes in Tables 1, 3, 5, and 7 are positively correlated with good prognosis, and wherein increased expression of prognostic genes in Tables 2, 4, 6, and 8 are negatively associated with good prognosis. In some embodiments, the tumor is estrogen receptor-positive, and in other embodiments the tumor is estrogen receptor negative.

In various embodiments, a subject method can further include providing a report. The report may include prediction of the likelihood of risk that said patient will have a particular clinical outcome.

The invention further provides a computer-implemented method for classifying a cancer patient based on risk of cancer recurrence, comprising (a) classifying, on a computer, said patient as having a good prognosis or a poor prognosis based on an expression profile comprising measurements of expression levels of expression products of a plurality of prognostic genes in a tumor tissue sample taken from the patient, said plurality of genes comprising at least three different prognostic genes listed in any of Tables 1-12, wherein a good prognosis predicts no recurrence or metastasis within a predetermined period after initial diagnosis, and wherein a poor prognosis predicts recurrence or metastasis within said predetermined period after initial diagnosis; and (b) calculating a risk score based on said expression levels.

### DETAILED DESCRIPTION

### DEFINITIONS

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

"Prognostic factors" are those variables related to the natural history of cancer, which influence the recurrence rates and outcome of patients once they have developed cancer. Clinical parameters that have been associated with a worse prognosis include, for example, lymph node involvement, and high grade tumors. Prognostic factors are frequently used to categorize patients into subgroups with different baseline relapse risks.

The term "prognosis" is used herein to refer to the prediction of the likelihood of cancer-attributable death or progression, including recurrence, metastatic spread, and drug resistance, of a neoplastic disease, such as breast cancer. The term "good prognosis" means a desired or "positive" clinical outcome. For example, in the context of breast cancer, a good prognosis may be an expectation of no recurrences or metastasis within two, three, four, five or more years of the initial diagnosis of breast cancer. The terms "bad prognosis" or "poor prognosis" are used herein interchangeably herein to mean an undesired clinical outcome. For example, in the context of breast cancer, a bad prognosis may be an expectation of a recurrence or metastasis within two, three, four, five or more years of the initial diagnosis of breast cancer.

The term "prognostic gene" is used herein to refer to a gene, the expression of which is correlated, positively or negatively, with a good prognosis for a cancer patient treated with the standard of care. A gene may be both a prognostic and predictive gene, depending on the correlation of the gene expression level with the corresponding endpoint. For example, using a Cox proportional hazards model, if a gene is only prognostic, its hazard ratio (HR) does not change when measured in patients treated with the standard of care or in patients treated with a new intervention.

The term "predictive gene" is used herein to refer to a gene, the expression of which is correlated, positively or negatively, with response to a beneficial response to treatment. For example, treatment could include chemotherapy.

The terms "risk score" or "risk classification" are used interchangeably herein to describe a level of risk (or likelihood) that a patient will experience a particular clinical outcome. A patient may be classified into a risk group or classified at a level of risk based on the methods of the present disclosure, e.g. high, medium, or low risk. A "risk group" is a group of subjects or individuals with a similar level of risk for a particular clinical outcome.

A clinical outcome can be defined using different endpoints. The term "long-term" survival is used herein to refer to survival for a particular time period, e.g., for at least 3 years, more preferably for at least 5 years. The term "Recurrence-Free Survival" (RFS) is used herein to refer to survival for a time period (usually in years) from randomization to first cancer recurrence or death due to recurrence of cancer. The term "Overall Survival" (OS) is used herein to refer to the time (in years) from randomization to death from any cause. The term "Disease-Free Survival" (DFS) is used herein to refer to survival for a time period (usually in years) from randomization to first cancer recurrence or death from any cause.

The calculation of the measures listed above in practice may vary from study to study depending on the definition of events to be either censored or not considered.

The term "biomarker" as used herein refers to a gene, the expression level of which, as measured using a gene product.

The term "microarray" refers to an ordered arrangement of hybridizable array elements, preferably polynucleotide probes, on a substrate.

As used herein, the term "normalized expression level" as applied to a gene refers to the normalized level of a gene product, e.g. the normalized value determined for the RNA expression level of a gene or for the polypeptide expression level of a gene.

The term "Cₜ" as used herein refers to threshold cycle, the cycle number in quantitative polymerase chain reaction (qPCR) at which the fluorescence generated within a reaction well exceeds the defined threshold, i.e. the point during the reaction at which a sufficient number of amplicons have accumulated to meet the defined threshold.

The term "gene product" or "expression product" are used herein to refer to the RNA transcription products (transcripts) of the gene, including mRNA, and the polypeptide translation products of such RNA transcripts. A gene product can be, for example, an unspliced RNA, an mRNA, a splice variant mRNA, a microRNA, a fragmented RNA, a polypeptide, a post-translationally modified polypeptide, a splice variant polypeptide, etc.

The term "RNA transcript" as used herein refers to the RNA transcription products of a gene, including, for example, mRNA, an unspliced RNA, a splice variant mRNA, a microRNA, and a fragmented RNA. "Fragmented RNA" as used herein refers to RNA a mixture of intact RNA and RNA that has been degraded as a result of the sample processing (e.g., fixation, slicing tissue blocks, etc.).

Unless indicated otherwise, each gene name used herein corresponds to the Official Symbol assigned to the gene and provided by Entrez Gene (URL: www.ncbi.nlm.nih.gov/sites/entrez) as of the filing date of this application.

The terms "correlated" and "associated" are used interchangeably herein to refer to a strength of association between two measurements (or measured entities). The disclosure provides genes and gene subsets, the expression levels of which are associated with a particular outcome measure. For example, the increased expression level of a gene may be positively correlated (positively associated) with an increased likelihood of good clinical outcome for the patient, such as an increased likelihood of long-term survival without recurrence of the cancer and/or metastasis-free survival. Such a positive correlation may be demonstrated statistically in various ways, e.g. by a low hazard ratio (e.g. HR < 1.0). In another example, the increased expression level of a gene may be negatively correlated (negatively associated) with an increased likelihood of good clinical outcome for the patient. In that case, for example, the patient may have a decreased likelihood of long-term survival without recurrence of the cancer and/or cancer metastasis, and the like. Such a negative correlation indicates that the patient likely has a poor prognosis, e.g., a high hazard ratio (e.g., HR > 1.0). "Correlated" is also used herein to refer to a strength of association between the expression levels of two different genes, such that expression level of a first gene can be substituted with an expression level of a second gene in a given algorithm in view of their correlation of expression. Such "correlated expression" of two genes that are substitutable in an algorithm usually gene expression levels that are positively correlated with one another, e.g., if increased expression of a first gene is positively correlated with an outcome (e.g., increased likelihood of good clinical outcome), then the second gene that is co-expressed and exhibits correlated expression with the first gene is also positively correlated with the same outcome

The term "recurrence," as used herein, refers to local or distant (metastasis) recurrence of cancer. For example, breast cancer can come back as a local recurrence (in the treated breast or near the tumor surgical site) or as a distant recurrence in the body. The most common sites of breast cancer recurrence include the lymph nodes, bones, liver, or lungs.

The term "polynucleotide," when used in singular or plural, generally refers to any polyribonucleotide or polydeoxribonucleotide, which may be unmodified RNA or

DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The phrase "amplification" refers to a process by which multiple copies of a gene or RNA transcript are formed in a particular sample or cell line. The duplicated region (a stretch of amplified polynucleotide) is often referred to as "amplicon." Usually, the amount of the messenger RNA (mRNA) produced, *i.e.,* the level of gene expression, also increases in the proportion of the number of copies made of the particular gene expressed.

The term "estrogen receptor (ER)" designates the estrogen receptor status of a cancer patient. A tumor is ER-positive if there is a significant number of estrogen receptors present in the cancer cells, while ER-negative indicates that the cells do not have a significant number of receptors present. The definition of "significant" varies amongst testing sites and methods (e.g., immunohistochemistry, PCR). The ER status of a cancer patient can be evaluated by various known means. For example, the ER level of breast cancer is determined by measuring an expression level of a gene encoding the estrogen receptor in a breast tumor sample obtained from a patient.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include, but are not limited to, breast cancer, ovarian cancer, colon cancer, lung cancer, prostate cancer, hepatocellular cancer, gastric cancer, pancreatic cancer, cervical cancer, liver cancer, bladder cancer, cancer of the urinary tract, thyroid cancer, renal cancer, carcinoma, melanoma, and brain cancer.

The gene subset identified herein as the "stromal group" includes genes that are synthesized predominantly by stromal cells and are involved in stromal response and genes that co-express with stromal group genes. "Stromal cells" are defined herein as connective tissue cells that make up the support structure of biological tissues. Stromal cells include fibroblasts, immune cells, pericytes, endothelial cells, and inflammatory cells. "Stromal response" refers to a desmoplastic response of the host tissues at the site of a primary tumor or invasion. See, e.g., E. Rubin, J. Farber, Pathology, 985-986 (2nd Ed. 1994). The stromal group includes, for example, CDH11, TAGLN, ITGA4, INHBA, COLIA1, COLIA2, FN1, CXCL14, TNFRSF1, CXCL12, C10ORF116, RUNX1, GSTM2, TGFB3, CAV1, DLC1, TNFRSF10, F3, and DICER1, and co-expressed genes identified in Tables 16-18.

The gene subset identified herein as the "metabolic group" includes genes that are associated with cellular metabolism, including genes associated with carrying proteins for transferring iron, the cellular iron homeostasis pathway, and homeostatic biochemical metabolic pathways, and genes that co-express with metabolic group genes. The metabolic group includes, for example, TFRC, ENO1, IDH2, ARF1, CLDN4, PRDX1, and GBP1, and co-expressed genes identified in Tables 16-18.

The gene subset identified herein as the "immune group" includes genes that are involved in cellular immunoregulatory functions, such as T and B cell trafficking, lymphocyte-associated or lymphocyte markers, and interferon regulation genes, and genes that co-express with immune group genes. The immune group includes, for example, CCL19 and IRF1, and co-expressed genes identified in Tables 16-18.

The gene subset identified herein as the "proliferation group" includes genes that are associated with cellular development and division, cell cycle and mitotic regulation, angiogenesis, cell replication, nuclear transport/stability, wnt signaling, apoptosis, and genes that co-express with proliferation group genes. The proliferation group includes, for example, PGF, SPC25, AURKA, BIRC5, BUB1, CCNB1, CENPA, KPNA, LMNB1, MCM2, MELK, NDC80, TPX2M, and WISP1, and co-expressed genes identified in Tables 16-18.

The term "co-expressed", as used herein, refers to a statistical correlation between the expression level of one gene and the expression level of another gene. Pairwise co-expression may be calculated by various methods known in the art, e.g., by calculating Pearson correlation coefficients or Spearman correlation coefficients. Co-expressed gene cliques may also be identified using a graph theory.

As used herein, the terms "gene clique" and "clique" refer to a subgraph of a graph in which every vertex is connected by an edge to every other vertex of the subgraph.

As used herein, a "maximal clique" is a clique in which no other vertex can be added and still be a clique.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

A "computer-based system" refers to a system of hardware, software, and data storage medium used to analyze information. The minimum hardware of a patient computer-based system comprises a central processing unit (CPU), and hardware for data input, data output (e.g., display), and data storage. An ordinarily skilled artisan can readily appreciate that any currently available computer-based systems and/or components thereof are suitable for use in connection with the methods of the present disclosure. The data storage medium may comprise any manufacture comprising a recording of the present information as described above, or a memory access device that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" or "computing means" references any hardware and/or software combination that will perform the functions required of it. For example, a suitable processor may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

As used herein, "graph theory" refers to a field of study in Computer Science and Mathematics in which situations are represented by a diagram containing a set of points and lines connecting some of those points. The diagram is referred to as a "graph", and the points and lines referred to as "vertices" and "edges" of the graph. In terms of gene co-expression analysis, a gene (or its equivalent identifier, e.g. an array probe) may be represented as a node or vertex in the graph. If the measures of similarity (e.g., correlation coefficient, mutual information, and alternating conditional expectation) between two genes are higher than a significant threshold, the two genes are said to be co-expressed and an edge will be drawn in the graph. When co-expressed edges for all possible gene pairs for a given study have been drawn, all maximal cliques are computed. The resulting maximal clique is defined as a gene clique. A gene clique is a computed co-expressed gene group that meets predefined criteria.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1 % Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

The term "node negative" cancer, such as "node negative" breast cancer, is used herein to refer to cancer that has not spread to the lymph nodes.

The terms "splicing" and "RNA splicing" are used interchangeably and refer to RNA processing that removes introns and joins exons to produce mature mRNA with continuous coding sequence that moves into the cytoplasm of a eukaryotic cell.

In theory, the term "exon" refers to any segment of an interrupted gene that is represented in the mature RNA product (B. Lewin. Genes IV Cell Press, Cambridge Mass. 1990). In theory the term "intron" refers to any segment of DNA that is transcribed but removed from within the transcript by splicing together the exons on either side of it. Operationally, exon sequences occur in the mRNA sequence of a gene as defined by Ref. SEQ ID numbers. Operationally, intron sequences are the intervening sequences within the genomic DNA of a gene, bracketed by exon sequences and having GT and AG splice consensus sequences at their 5' and 3' boundaries.

### GENE EXPRESSION ASSAY

The present disclosure provides methods that employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

### 1. Gene Expression Profiling

Methods of gene expression profiling include methods based on hybridization analysis of polynucleotides, methods based on sequencing of polynucleotides, and proteomics-based methods. The most commonly used methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes.

### 2. PCR-based Gene Expression Profiling Methods

### a. Reverse Transcriptase PCR (RT-PCR)

Of the techniques listed above, the most sensitive and most flexible quantitative method is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

In some cases, it may be appropriate to amplify RNA prior to initiating expression profiling. It is often the case that only very limited amounts of valuable clinical specimens are available for molecular analysis. This may be due to the fact that the tissues have already be used for other laboratory analyses or may be due to the fact that the original specimen is very small as in the case of needle biopsy or very small primary tumors. When tissue is limiting in quantity it is generally also the case that only small amounts of total RNA can be recovered from the specimen and as a result only a limited number of genomic markers can be analyzed in the specimen. RNA amplification compensates for this limitation by faithfully reproducing the original RNA sample as a much larger amount of RNA of the same relative composition. Using this amplified copy of the original RNA specimen, unlimited genomic analysis can be done to discovery biomarkers associated with the clinical characteristics of the original biological sample. This effectively immortalizes clinical study specimens for the purposes of genomic analysis and biomarker discovery.

As RNA cannot serve as a template for PCR, the first step in gene expression profiling by real-time RT-PCR (RT-PCR) is the reverse transcription of the RNA template into cDNA, followed by its exponential amplification in a PCR reaction. The two most commonly used reverse transcriptases are avian myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction. For further details see, e.g. Held et al., Genome Research 6:986-994 (1996).

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7900® Sequence Detection System™ (Perkin-Elmer-Applied Biosystems, Foster City, CA, USA), or LightCycler® 480 Real-Time PCR System (Roche Diagnostics, GmbH, Penzberg, Germany). In a preferred embodiment, the 5' nuclease procedure is run on a real-time quantitative PCR device such as the ABI PRISM 7900® Sequence Detection System™. The system consists of a thermocycler, laser, charge-coupled device (CCD), camera and computer. The system amplifies samples in a 384-well format on a thermocycler. During amplification, laser-induced fluorescent signal is collected in real-time through fiber optics cables for all 384 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Cₜ).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

The steps of a representative protocol for profiling gene expression using fixed, paraffin-embedded tissues as the RNA source, including mRNA isolation, purification, primer extension and amplification are given in various published journal articles. M. Cronin, Am J Pathol 164(1):35-42 (2004). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific primers followed by RT-PCR.

### b. Design of Intron-Based PCR Primers and Probes

PCR primers and probes can be designed based upon exon or intron sequences present in the mRNA transcript of the gene of interest. Prior to carrying out primer/probe design, it is necessary to map the target gene sequence to the human genome assembly in order to identify intron-exon boundaries and overall gene structure. This can be performed using publicly available software, such as Primer3 (Whitehead Inst.) and Primer Express® (Applied Biosystems).

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked intron and exon sequences can then be used to design primer and probe sequences for the desired target sites using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Rrawetz S, Misener S (eds) Bioinformatics Methods and Protocols: Methods in Molecular Biology. Humana Press, Totowa, NJ, pp 365-386).

Other factors that can influence PCR primer design include primer length, melting temperature (Tm), and G/C content, specificity, complementary primer sequences, and 3 '-end sequence. In general, optimal PCR primers are generally 17-30 bases in length, and contain about 20-80%, such as, for example, about 50-60% G+C bases, and exhibit Tm's between 50 and 80 ⁰C, e.g. about 50 to 70 ⁰C.

For further guidelines for PCR primer and probe design see, e.g. Dieffenbach, CW. et al, "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

Table A provides further information concerning the primer, probe, and amplicon sequences associated with the Examples disclosed herein.

### c. MassARRA Y System

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is preloaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### d. Other PCR-based Methods

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

### 3. Microarrays

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately twofold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Agilent's microarray technology.

The development of microarray methods for large-scale analysis of gene expression makes it possible to search systematically for molecular markers of cancer classification and outcome prediction in a variety of tumor types.

### 4. Gene Expression Analysis by Nucleic Acid Sequencing

Nucleic acid sequencing technologies are suitable methods for analysis of gene expression. The principle underlying these methods is that the number of times a cDNA sequence is detected in a sample is directly related to the relative expression of the mRNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

### 5. Isolating RNA from Body Fluids

Methods of isolating RNA for expression analysis from blood, plasma and serum (See for example, Tsui NB et al. (2002) 48,1647-53 and references cited therein) and from urine (See for example, Boom R et al. (1990) J Clin Microbiol. 28, 495-503 and reference cited therein) have been described.

### 6. Immunohistochemistry

Immunohistochemistry methods are also suitable for detecting the expression levels of the prognostic markers of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

### 7. Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

### 8. General Description of the mRNA Isolation, Purification, and Amplification

The steps of a representative protocol for profiling gene expression using fixed, paraffin-embedded tissues as the RNA source, including mRNA isolation, purification, primer extension and amplification are provided in various published journal articles (for example: T.E. Godfrey et al,. J. Molec. Diagnostics 2: 84-91 [2000]; K. Specht et al., Am. J. Pathol. 158: 419-29 [2001]). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific primers followed by RT-PCR. Finally, the data are analyzed to identify the best treatment option(s) available to the patient on the basis of the characteristic gene expression pattern identified in the tumor sample examined, dependent on the predicted likelihood of cancer recurrence.

### 9. Normalization

The expression data used in the methods disclosed herein can be normalized. Normalization refers to a process to correct for (normalize away), for example, differences in the amount of RNA assayed and variability in the quality of the RNA used, to remove unwanted sources of systematic variation in Cₜ measurements, and the like. With respect to RT-PCR experiments involving archived fixed paraffin embedded tissue samples, sources of systematic variation are known to include the degree of RNA degradation relative to the age of the patient sample and the type of fixative used to preserve the sample. Other sources of systematic variation are attributable to laboratory processing conditions.

Assays can provide for normalization by incorporating the expression of certain normalizing genes, which genes do not significantly differ in expression levels under the relevant conditions. Exemplary normalization genes include housekeeping genes such as PGK1 and UBB. (See, e.g., E. Eisenberg, et al., Trends in Genetics 19(7):362-365 (2003).) Normalization can be based on the mean or median signal (C_{T}) of all of the assayed genes or a large subset thereof (global normalization approach). In general, the normalizing genes, also referred to as reference genes should be genes that are known not to exhibit significantly different expression in colorectal cancer as compared to non-cancerous colorectal tissue, and are not significantly affected by various sample and process conditions, thus provide for normalizing away extraneous effects.

Unless noted otherwise, normalized expression levels for each mRNA/tested tumor/patient will be expressed as a percentage of the expression level measured in the reference set. A reference set of a sufficiently high number (e.g. 40) of tumors yields a distribution of normalized levels of each mRNA species. The level measured in a particular tumor sample to be analyzed falls at some percentile within this range, which can be determined by methods well known in the art.

In exemplary embodiments, one or more of the following genes are used as references by which the expression data is normalized: AAMP, ARF1, EEF1A1, ESD, GPS1, H3F3A, HNRPC, RPL13A, RPL41, RPS23, RPS27, SDHA, TCEA1, UBB, YWHAZ, B-actin, GUS, GAPDH, RPLPO, and TFRC. For example, the calibrated weighted average Ct measurements for each of the prognostic genes may be normalized relative to the mean of at least three reference genes, at least four reference genes, or at least five reference genes.

Those skilled in the art will recognize that normalization may be achieved in numerous ways, and the techniques described above are intended only to be exemplary, not exhaustive.

### REPORTING RESULTS

The methods of the present disclosure are suited for the preparation of reports summarizing the expected or predicted clinical outcome resulting from the methods of the present disclosure. A "report," as described herein, is an electronic or tangible document that includes report elements that provide information of interest relating to a likelihood assessment or a risk assessment and its results. A subject report includes at least a likelihood assessment or a risk assessment, e.g., an indication as to the risk of recurrence of breast cancer, including local recurrence and metastasis of breast cancer. A subject report can include an assessment or estimate of one or more of disease-free survival, recurrence-free survival, metastasis-free survival, and overall survival. A subject report can be completely or partially electronically generated, e.g., presented on an electronic display (e.g., computer monitor). A report can further include one or more of: 1) information regarding the testing facility; 2) service provider information; 3) patient data; 4) sample data; 5) an interpretive report, which can include various information including: a) indication; b) test data, where test data can include a normalized level of one or more genes of interest, and 6) other features.

The present disclosure thus provides for methods of creating reports and the reports resulting therefrom. The report may include a summary of the expression levels of the RNA transcripts, or the expression products of such RNA transcripts, for certain genes in the cells obtained from the patient's tumor. The report can include information relating to prognostic covariates of the patient. The report may include an estimate that the patient has an increased risk of recurrence. That estimate may be in the form of a score or patient stratifier scheme (e.g., low, intermediate, or high risk of recurrence). The report may include information relevant to assist with decisions about the appropriate surgery (e.g., partial or total mastectomy) or treatment for the patient.

Thus, in some embodiments, the methods of the present disclosure further include generating a report that includes information regarding the patient's likely clinical outcome, e.g. risk of recurrence. For example, the methods disclosed herein can further include a step of generating or outputting a report providing the results of a subject risk assessment, which report can be provided in the form of an electronic medium (e.g., an electronic display on a computer monitor), or in the form of a tangible medium (e.g., a report printed on paper or other tangible medium).

A report that includes information regarding the patient's likely prognosis (e.g., the likelihood that a patient having breast cancer will have a good prognosis or positive clinical outcome in response to surgery and/or treatment) is provided to a user. An assessment as to the likelihood is referred to below as a "risk report" or, simply, "risk score." A person or entity that prepares a report ("report generator") may also perform the likelihood assessment. The report generator may also perform one or more of sample gathering, sample processing, and data generation, e.g., the report generator may also perform one or more of: a) sample gathering; b) sample processing; c) measuring a level of a risk gene; d) measuring a level of a reference gene; and e) determining a normalized level of a risk gene. Alternatively, an entity other than the report generator can perform one or more sample gathering, sample processing, and data generation.

For clarity, it should be noted that the term "user," which is used interchangeably with "client," is meant to refer to a person or entity to whom a report is transmitted, and may be the same person or entity who does one or more of the following: a) collects a sample; b) processes a sample; c) provides a sample or a processed sample; and d) generates data (e.g., level of a risk gene; level of a reference gene product(s); normalized level of a risk gene ("prognosis gene") for use in the likelihood assessment. In some cases, the person(s) or entity(ies) who provides sample collection and/or sample processing and/or data generation, and the person who receives the results and/or report may be different persons, but are both referred to as "users" or "clients" herein to avoid confusion. In certain embodiments, e.g., where the methods are completely executed on a single computer, the user or client provides for data input and review of data output. A "user" can be a health professional (e.g., a clinician, a laboratory technician, a physician (e.g., an oncologist, surgeon, pathologist), etc.).

In embodiments where the user only executes a portion of the method, the individual who, after computerized data processing according to the methods of the present disclosure, reviews data output (e.g., results prior to release to provide a complete report, a complete, or reviews an "incomplete" report and provides for manual intervention and completion of an interpretive report) is referred to herein as a "reviewer." The reviewer may be located at a location remote to the user (e.g., at a service provided separate from a healthcare facility where a user may be located).

Where government regulations or other restrictions apply (e.g., requirements by health, malpractice, or liability insurance), all results, whether generated wholly or partially electronically, are subjected to a quality control routine prior to release to the user.

### CLINICAL UTILITY

The gene expression assay and information provided by the practice of the methods disclosed herein facilitates physicians in making more well-informed treatment decisions, and to customize the treatment of cancer to the needs of individual patients, thereby maximizing the benefit of treatment and minimizing the exposure of patients to unnecessary treatments which may provide little or no significant benefits and often carry serious risks due to toxic side-effects.

Single or multi-analyte gene expression tests can be used measure the expression level of one or more genes involved in each of several relevant physiologic processes or component cellular characteristics. The expression level(s) may be used to calculate such a quantitative score, and such score may be arranged in subgroups (e.g., tertiles) wherein all patients in a given range are classified as belonging to a risk category (e.g., low, intermediate, or high). The grouping of genes may be performed at least in part based on knowledge of the contribution of the genes according to physiologic functions or component cellular characteristics, such as in the groups discussed above.

The utility of a gene marker in predicting cancer may not be unique to that marker. An alternative marker having an expression pattern that is parallel to that of a selected marker gene may be substituted for, or used in addition to, a test marker. Due to the co-expression of such genes, substitution of expression level values should have little impact on the overall prognostic utility of the test. The closely similar expression patterns of two genes may result from involvement of both genes in the same process and/or being under common regulatory control in colon tumor cells. The present disclosure thus contemplates the use of such co-expressed genes or gene sets as substitutes for, or in addition to, prognostic methods of the present disclosure.

The molecular assay and associated information provided by the methods disclosed herein for predicting the clinical outcome in cancer, e.g. breast cancer, have utility in many areas, including in the development and appropriate use of drugs to treat cancer, to stratify cancer patients for inclusion in (or exclusion from) clinical studies, to assist patients and physicians in making treatment decisions, provide economic benefits by targeting treatment based on personalized genomic profile, and the like. For example, the recurrence score may be used on samples collected from patients in a clinical trial and the results of the test used in conjunction with patient outcomes in order to determine whether subgroups of patients are more or less likely to demonstrate an absolute benefit from a new drug than the whole group or other subgroups. Further, such methods can be used to identify from clinical data subsets of patients who are expected to benefit from adjuvant therapy. Additionally, a patient is more likely to be included in a clinical trial if the results of the test indicate a higher likelihood that the patient will have a poor clinical outcome if treated with surgery alone and a patient is less likely to be included in a clinical trial if the results of the test indicate a lower likelihood that the patient will have a poor clinical outcome if treated with surgery alone.

### STATISTICAL ANALYSIS OF GENE EXPRESSION LEVELS

One skilled in the art will recognize that there are many statistical methods that may be used to determine whether there is a significant relationship between an outcome of interest (e.g., likelihood of survival, likelihood of response to chemotherapy) and expression levels of a marker gene as described here. This relationship can be presented as a continuous recurrence score (RS), or patients may stratified into risk groups (e.g., low, intermediate, high). For example, a Cox proportional hazards regression model may fit to a particular clinical endpoint (e.g., RFS, DFS, OS). One assumption of the Cox proportional hazards regression model is the proportional hazards assumption, i.e. the assumption that effect parameters multiply the underlying hazard.

### Coexpression Analysis

The present disclosure provides genes that co-express with particular prognostic and/or predictive gene that has been identified as having a significant correlation to recurrence and/or treatment benefit. To perform particular biological processes, genes often work together in a concerted way, i.e. they are co-expressed. Co-expressed gene groups identified for a disease process like cancer can serve as biomarkers for disease progression and response to treatment. Such co-expressed genes can be assayed in lieu of, or in addition to, assaying of the prognostic and/or predictive gene with which they are co-expressed.

One skilled in the art will recognize that many co-expression analysis methods now known or later developed will fall within the scope and spirit of the present invention. These methods may incorporate, for example, correlation coefficients, co-expression network analysis, clique analysis, etc., and may be based on expression data from RT-PCR, microarrays, sequencing, and other similar technologies. For example, gene expression clusters can be identified using pair-wise analysis of correlation based on Pearson or Spearman correlation coefficients. (See, e.g., Pearson K. and Lee A., Biometrika 2, 357 (1902); C. Spearman, Amer. J. Psychol 15:72-101 (1904); J. Myers, A. Well, Research Design and Statistical Analysis, p. 508 (2nd Ed., 2003).) In general, a correlation coefficient of equal to or greater than 0.3 is considered to be statistically significant in a sample size of at least 20. (See, e.g., G. Norman, D. Streiner, Biostatistics: The Bare Essentials, 137-138 (3rd Ed. 2007).) In one embodiment disclosed herein, co-expressed genes were identified using a Spearman correlation value of at least 0.7.

### Computer program

The values from the assays described above, such as expression data, recurrence score, treatment score and/or benefit score, can be calculated and stored manually. Alternatively, the above-described steps can be completely or partially performed by a computer program product. The present invention thus provides a computer program product including a computer readable storage medium having a computer program stored on it. The program can, when read by a computer, execute relevant calculations based on values obtained from analysis of one or more biological sample from an individual (e.g., gene expression levels, normalization, thresholding, and conversion of values from assays to a score and/or graphical depiction of likelihood of recurrence/response to chemotherapy, gene co-expression or clique analysis, and the like). The computer program product has stored therein a computer program for performing the calculation.

The present disclosure provides systems for executing the program described above, which system generally includes: a) a central computing environment; b) an input device, operatively connected to the computing environment, to receive patient data, wherein the patient data can include, for example, expression level or other value obtained from an assay using a biological sample from the patient, or microarray data, as described in detail above; c) an output device, connected to the computing environment, to provide information to a user (e.g., medical personnel); and d) an algorithm executed by the central computing environment (e.g., a processor), where the algorithm is executed based on the data received by the input device, and wherein the algorithm calculates a, risk, risk score, or treatment group classification, gene co-expression analysis, thresholding, or other functions described herein. The methods provided by the present invention may also be automated in whole or in part.

### Manual and Computer-Assisted Methods and Products

The methods and systems described herein can be implemented in numerous ways. In one embodiment of particular interest, the methods involve use of a communications infrastructure, for example the Internet. Several embodiments are discussed below. It is also to be understood that the present disclosure may be implemented in various forms of hardware, software, firmware, processors, or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site associated (e.g., at a service provider's facility).

For example, during or after data input by the user, portions of the data processing can be performed in the user-side computing environment. For example, the user-side computing environment can be programmed to provide for defined test codes to denote a likelihood "risk score," where the score is transmitted as processed or partially processed responses to the reviewer's computing environment in the form of test code for subsequent execution of one or more algorithms to provide a results and/or generate a report in the reviewer's computing environment. The risk score can be a numerical score (representative of a numerical value, e.g. likelihood of recurrence based on validation study population) or a non-numerical score representative of a numerical value or range of numerical values (e.g., low, intermediate, or high).

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) that is executed via the operating system. In addition, various other peripheral devices may be connected to the computer platform such as an additional data storage device and a printing device.

As a computer system, the system generally includes a processor unit. The processor unit operates to receive information, which can include test data (e.g., level of a risk gene, level of a reference gene product(s); normalized level of a gene; and may also include other data such as patient data. This information received can be stored at least temporarily in a database, and data analyzed to generate a report as described above.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In an embodiment of particular interest, all or a portion of the input data and/or all or a portion of the output data (e.g., usually at least the final report) are maintained on a web server for access, preferably confidential access, with typical browsers. The data may be accessed or sent to health professionals as desired. The input and output data, including all or a portion of the final report, can be used to populate a patient's medical record which may exist in a confidential database at the healthcare facility.

A system for use in the methods described herein generally includes at least one computer processor (e.g., where the method is carried out in its entirety at a single site) or at least two networked computer processors (e.g., where data is to be input by a user (also referred to herein as a "client") and transmitted to a remote site to a second computer processor for analysis, where the first and second computer processors are connected by a network, e.g., via an intranet or internet). The system can also include a user component(s) for input; and a reviewer component(s) for review of data, generated reports, and manual intervention. Additional components of the system can include a server component(s); and a database(s) for storing data (e.g., as in a database of report elements, e.g., interpretive report elements, or a relational database (RDB) which can include data input by the user and data output. The computer processors can be processors that are typically found in personal desktop computers (e.g., IBM, Dell, Macintosh), portable computers, mainframes, minicomputers, or other computing devices.

The networked client/server architecture can be selected as desired, and can be, for example, a classic two or three tier client server model. A relational database management system (RDMS), either as part of an application server component or as a separate component (RDB machine) provides the interface to the database.

In one example, the architecture is provided as a database-centric client/server architecture, in which the client application generally requests services from the application server which makes requests to the database (or the database server) to populate the report with the various report elements as required, particularly the interpretive report elements, especially the interpretation text and alerts. The server(s) (e.g., either as part of the application server machine or a separate RDB/relational database machine) responds to the client's requests.

The input client components can be complete, stand-alone personal computers offering a full range of power and features to run applications. The client component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe and offers data management, information sharing between clients, network administration and security. The application and any databases used can be on the same or different servers.

Other computing arrangements for the client and server(s), including processing on a single machine such as a mainframe, a collection of machines, or other suitable configuration are contemplated. In general, the client and server machines work together to accomplish the processing of the present disclosure.

Where used, the database(s) is usually connected to the database server component and can be any device that will hold data. For example, the database can be a any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

### Computer-readable storage media

The present disclosure also contemplates a computer-readable storage medium (e.g. CD-ROM, memory key, flash memory card, diskette, etc.) having stored thereon a program which, when executed in a computing environment, provides for implementation of algorithms to carry out all or a portion of the results of a response likelihood assessment as described herein. Where the computer-readable medium contains a complete program for carrying out the methods described herein, the program includes program instructions for collecting, analyzing and generating output, and generally includes computer readable code devices for interacting with a user as described herein, processing that data in conjunction with analytical information, and generating unique printed or electronic media for that user.

Where the storage medium provides a program that provides for implementation of a portion of the methods described herein (e.g., the user-side aspect of the methods (e.g., data input, report receipt capabilities, etc.)), the program provides for transmission of data input by the user (e.g., via the internet, via an intranet, etc.) to a computing environment at a remote site. Processing or completion of processing of the data is carried out at the remote site to generate a report. After review of the report, and completion of any needed manual intervention, to provide a complete report, the complete report is then transmitted back to the user as an electronic document or printed document (e.g., fax or mailed paper report). The storage medium containing a program according to the present disclosure can be packaged with instructions (e.g., for program installation, use, etc.) recorded on a suitable substrate or a web address where such instructions may be obtained. The computer-readable storage medium can also be provided in combination with one or more reagents for carrying out response likelihood assessment (e.g., primers, probes, arrays, or other such kit components).

All aspects of the present invention may also be practiced such that a limited number of additional genes that are co-expressed with the disclosed genes, for example as evidenced by statistically meaningful Pearson and/or Spearman correlation coefficients, are included in a prognostic or predictive test in addition to and/or in place of disclosed genes.

Having described the invention, the same will be more readily understood through reference to the following Examples, which are provided by way of illustration, and are not intended to limit the invention in any way.

### EXAMPLE 1:

The study included breast cancer tumor samples obtained from 136 patients diagnosed with breast cancer ("Providence study"). Biostatistical modeling studies of prototypical data sets demonstrated that amplified RNA is a useful substrate for biomarker identification studies. This was verified in this study by including known breast cancer biomarkers along with candidate prognostic genesin the tissues samples. The known biomarkers were shown to be associated with clinical outcome in amplified RNA based on the criteria outlined in this protocol.

### Study design

Refer to the original Providence Phase II study protocol for biopsy specimen information. The study looked at the statistical association between clinical outcome and 384candidate biomarkers tested in amplified samples derived from 25 ng of mRNA that was extracted from fixed, paraffin-embedded tissue samples obtained from 136 of the original Providence Phase II study samples. The expression level of the candidate genes was normalized using reference genes. Several reference genes were analyzed in this study: AAMP, ARF1, EEF1A1, ESD, GPS1, H3F3A, HNRPC, RPL13A, RPL41, RPS23, RPS27, SDHA, TCEA1, UBB, YWHAZ, B-actin, GUS, GAPDH, RPLPO, and TFRC.

The 136 samples were split into 3 automated RT plates each with 2X 48 samples and 40 samples and 3 RT positive and negative controls. Quantitative PCR assays were performed in 384 wells without replicate using the QuantiTect Probe PCR Master Mix® (Qiagen). Plates were analyzed on the Light Cycler® 480 and, after data quality control, all samples from the RT plate 3 were repeated and new RT-PCR data was generated. The data was normalized by subtracting the median crossing point (Cp) (point at which detection rises above background signal) for five reference genes from the C_{P} value for each individual candidate gene. This normalization is performed on each sample resulting in final data that has been adjusted for differences in overall sample Cp. This data set was used for the final data analysis.

### Data Analysis

For each gene, a standard z test was run.. (S. Darby, J. Reissland, Journal of the Royal Statistical Society 144(3):298-331 (1981)). This returns a z score (measure of distance in standard deviations of a sample from the mean), p value, and residuals along with other statistics and parameters from the model. If the z score is negative, expression is positively correlated with a good prognosis; if positive, expression is negatively correlated to a good prognosis. Using the p values, a q value was created using a library q value. The poorly correlated and weakly expressed genes were excluded from the calculation of the distribution used for the q values. For each gene, Cox Proportional Hazard Model test was run checking survival time matched with the event vector against gene expression. This returned a hazard ratio (HR) estimating the effect of expression of each gene (individually) on the risk of a cancer-related event. The resulting data is provided in Tables 1-6. A HR < 1 indicates that expression of that gene is positively associated with a good prognosis, while a HR > 1 indicates that expression of that gene is negatively associated with a good prognosis.

### EXAMPLE 2:

### Study design

Amplified samples were derived from 25 ng of mRNA that was extracted from fixed, paraffin-embedded tissue samples obtained from 78 evaluable cases from a Phase II breast cancer study conducted at Rush University Medical Center. Three of the samples failed to provide sufficient amplified RNA at 25 ng, so amplification was repeated a second time with 50 ng of RNA. The study also analyzed several reference genes for use in normalization: AAMP, ARF1, EEF1A1, ESD, GPS1, H3F3A, HNRPC, RPL13A, RPL41, RPS23, RPS27, SDHA, TCEA1, UBB, YWHAZ, Beta-actin, RPLPO, TFRC, GUS, and GAPDH.

Assays were performed in 384 wells without replicate using the QuantiTect Probe PCR Master Mix. Plates were analyzed on the Light Cycler 480 instruments. This data set was used for the final data analysis. The data was normalized by subtracting the median C_{P} for five reference genes from the C_{P} value for each individual candidate gene. This normalization was performed on each sample resulting in final data that was adjusted for differences in overall sample C_{P}.

### Data analysis

There were 34 samples with average CP values above 35. However, none of the samples were excluded from analysis because they were deemed to have sufficient valuable information to remain in the study. Principal Component Analysis (PCA) was used to determine whether there was a plate effect causing variation across the different RT plates. The first principal component correlated well with the median expression values, indicating that expression level accounted for most of the variation between samples. Also, there were no unexpected variations between plates.

### Data for Other Variables

Group - The patients were divided into two groups (cancer/non-cancer). There was little difference between the two in overall gene expression as the difference between median CP value in each group was minimal (0.7).
Sample Age - The samples varied widely in their overall gene expression but there was a trend toward lower C_{P} values as they decreased in age.
Instrument - The overall sample gene expression from instrument to instrument was consistent. One instrument showed a slightly higher median C_{P} compared to the other three, but it was well within the acceptable variation.
RT Plate - The overall sample gene expression between RT plates was also very consistent. The median C_{P} for each of the 3 RT plates (2 automated RT plates and 1 manual plate containing repeated samples) were all within 1 C_{P} of each other.

### Univariate Analyses for Genes Significantly Different Between Study Groups

The genes were analyzed using the z-test and Cox Proportional Hazard Model, as described in Example 1. The resulting data can be seen in Tables 7-12.

### EXAMPLE 3:

The statistical correlations between clinical outcome and expression levels of the genes identified in Examples 1 and 2 were validated in breast cancer gene expression datasets maintained by the Swiss Institute of Bioinformatics (SIB). Further information concerning the SIB database, study datasets, and processing methods, is providing in P. Wirapati, et al., Breast Cancer Research 10(4):R65 (2008). Univariate Cox proportional hazards analyses were performed to confirm the relationship between clinical outcome (DFS, MFS, OS) of breast cancer patients and expression levels of the genes identified as significant in the amplified RNA studies described above. The meta-analysis included both fixed-effect and random-effect models, which are further described in L. Hedges and J. Vevea, Psychological Methods 3 (4): 486-504 (1998) and K. Sidik and J. Jonkman, Statistics in Medicine 26:1964-1981 (2006) (the contents of which are incorporated herein by reference). The results of the validation for all genes identified as having a stastistically significant association with breast cancer clinical outcome are described in Table 13. In those tables, "Est" designates an estimated coefficient of a covariate (gene expression); "SE" is standard error; "t" is the t-score for this estimate (i.e., Est/SE); and "fe" is the fixed estimate of effect from the meta analysis. Several of gene families with significant statistical association with clinical outcome (including metabolic, proliferation, immune, and stromal group genes) in breast cancer were confirmed using the SIB dataset. For example, Table 14 contains analysis of genes included in the metabolic group and Table 15 the stromal group.

### EXAMPLE 4:

A co-expression analysis was conducted using microarray data from six (6) breast cancer data sets. The "processed" expression values are taken from the GEO website, however, further processing was necessary. If the expression values are RMA, they are median normalized on the sample level. If the expression values are MAS5.0, they are: (1) changed to 10 if they are <10; (2) log base e transformed; and (3) median normalized on the sample level.

Generating Correlation Pairs: A rank matrix was generated by arranging the expression values for each sample in decreasing order. Then a correlation matrix was created by calculating the Spearman correlation values for every pair of probe IDs. Pairs of probes which had a Spearman value ≥ 0.7 were considered co-expressed. Redundant or overlapping correlation pairs in multiple datasets were identified. For each correlation matrix generated from an array dataset, pairs of significant probes that occur in >1 dataset were identified. This served to filter "non-significant" pairs from the analysis as well as provide extra evidence for "significant" pairs with their presence in multiple datasets. Depending on the number of datasets included in each tissue specific analysis, only pairs which occur in a minimum # or % of datasets were included.

Co-expression cliques were generated using the Bron-Kerbosch algorithm for maximal clique finding in an undirected graph. The algorithm generates three sets of nodes: *compsub, candidates,* and *not. Compsub* contains the set of nodes to be extended or shrunk by one depending on its traversal direction on the tree search. *Candidates* consists of all the nodes eligible to be added to *compsub. Not* contains the set of nodes that have been added to *compsub* and are now excluded from extension. The algorithm consists of five steps: selection of a candidate; adding the candidate node to *compsub;* creating new sets *candidates* and *not* from the old sets by removing all points not connected to the candidate node; recursively calling the extension operator on the new *candidates* and *not* sets; and upon return, remove the candidate node from *compsub* and place in the **old** *not* set.

There was a depth-first search with pruning, and the selection of candidate nodes had an effect on the run time of the algorithm. By selecting nodes in decreasing order of frequency in the pairs, the run time was optimized. Also, recursive algorithms generally cannot be implemented in a multi-threaded manner, but was multi-threaded the extension operator of the first recursive level. Since the data between the threads were independent because they were at the top-level of the recursive tree, they were run in parallel.

Clique Mapping and Normalization: Since the members of the co-expression pairs and cliques are at the probe level, one must map the probe IDs to genes (or Refseqs) before they can be analyzed. The Affymetrix gene map information was used to map every probe ID to a gene name. Probes may map to multiple genes, and genes may be represented by multiple probes. The data for each clique is validated by manually calculating the correlation values for each pair from a single clique.

The results of this co-expression analysis are set forth in Tables 16-18.

**TABLE A**

| Gene | Sequence ID | Official Symbol | F Primer Seq | SEQ ID NO: | R Primer Seq | SEQ ID NO: | Probe Seq | SEQ ID NO: | Target Seq Length | Amplicon Sequence | SEQ ID NO: |
|---|---|---|---|---|---|---|---|---|---|---|---|
| A-Catenin | NM_001903.1 | CTNNA1 | CGTTCCGAT CCTCTATAC TGCAT | 1 | AGGTCCCTG TTGGCCTTA TAGG | 385 | ATGCCTACAGCACCCTG ATGTCGCA | 769 | 78 | CGTTCCGATCCTCTATACTGCATCCCAG GCATGCCTACAGCACCCTGATGTCGCA GCCTATAAGGCCAACAGGGACCT | 1153 |
| AAMP | NM_001087.3 | AAMP | GTGTGGCA GGTGGACA CTAA | 2 | CTCCATCCA CTCCAGGTC TC | 386 | CGCTTCAAAGGACCAGA CCTCCTC | 770 | 66 | GTGTGGCAGGTGGACACTAAGGAGGAG GTCTGGTCCTTTGAAGCGGGAGACCTG GAGTGGATGGAG | 1154 |
| ABCB1 | NM_000927.2 | ABCB1 | AAACACCA CTGGAGCAT TGA | 3 | CAAGCCTGG AACCTATAG CC | 387 | CTCGCCAATGATGCTGCT CAAGTT | 771 | 77 | AAACACCACTGGAGCATTGACTACCAG GCTCGCCAATGATGCTGCTCAAGTTAA AGGGGCTATAGGTTCCAGGCTTG | 1155 |
| ABCC10 | NM_ 033450.2 | ABCC10 | ACCAGTGCC ACAATGCA G | 4 | ATAGCGCTG ACCACTGCC | 388 | CCATGAGCTGTAGCCGA ATGTCCA | 772 | 68 | ACCAGTGCCACAATGCAGTGGCTGGAC ATTCGGCTACAGCTCATGGGGGCGGCA GTGGTCAGCGCTAT | 1156 |
| ABCC5 | NM_005688.1 | ABCC5 | TGCAGACTG TACCATGCT GA | 5 | GGCCAGCAC CATAATCCT AT | 389 | CTGCACACGGTTCTAGG CTCCG | 773 | 76 | TGCAGACTGTACCATGCTGACCATTGC CCATCGCCTGCACACGGTTCTAGGCTCC GATAGGATTATGGTGCTGGCC | 1157 |
| ABR | NM_ 001092.3 | ABR | ACACGTCTG TCACCATGG AA | 6 | ACTAGGGTG CTCCGAGTG AC | 390 | TCTGCTCTACAAGCCCAT TGACCG | 774 | 67 | ACACGTCTGTCACCATGGAAGCTCTGC TCTACAAGCCCATTGACCGGGTCACTC GGAGCACCCTAGT | 1158 |
| ACTR2 | NM_ 005722.2 | ACTR2 | ATCCGCATT GAAGACCC A | 7 | ATCCGCTAG AACTGCACC AC | 391 | CCCGCAGAAAGCACATG GTATTCC | 775 | 66 | ATCCGCATTGAAGACCCACCCCGCAGA AAGCACATGGTATTCCTGGGTGGTGCA GTTCTAGCGGAT | 1159 |
| ACVR2B | NM_ 001106.2 | ACVR2B | GACTGTCTC GTTTCCCTG GT | 8 | TGGGCTTAG ATGCTTGAC TC | 392 | CTCTGTCACCAATGTGG ACCTGCC | 776 | 74 | GACTGTCTCGTTTCCCTGGTGACCTCTG TCACCAATGTGGACCTGCCCCCTAAAG AGTCAAGCATCTAAGCCCA | 1160 |
| AD024 | NM_ 020675.3 | SPC25 | TCAAAAGT ACGGACAC CTCCT | 9 | TGCAAATGC TTTGATGGA AT | 393 | TGTAGGTATCTCTTAGTC CCGCCATCTGA | 777 | 74 | TCAAAAGTACGGACACCTCCTGTCAGA TGGCGGGACTAAGAGATACCTACAAGG ATTCCATCAAAGCATTTGCA | 1161 |
| ADAM12 | NM_ 021641.2 | ADAM12 | GAGCATGC GTCTACTGC CT | 10 | CTGGTCACG GTCTCCATG T | 394 | CTGACACTCATCTGAGC CCTCCCA | 778 | 66 | GAGCATGCGTCTACTGCCTCACTGACA CTCATCTGAGCCCTCCCATGACATGGA GACCGTGACCAG | 1162 |
| ADAM17 | NM_003183.3 | ADAM17 | GAAGTGCC AGGAGGCG ATTA | 11 | CGGGCACTC ACTGCTATT ACC | 395 | TGCTACTTGCAAAGGCG TGTCCTACTGC | 779 | 73 | GAAGTGCCAGGAGGCGATTAATGCTAC TTGCAAAGGCGTGTCCTACTGCACAGG TAATAGCAGTGAGTGCCCG | 1163 |
| ADAM23 | NM_003812.1 | ADAM23 | CAAGGCCC CATCTGAAT CA | 12 | ACCCAGAAT CCAACAGTG CAA | 396 | CTGCGCTGGATGGACAC CGC | 780 | 62 | CAAGGCCCCATCTGAATCAGCTGCGCT GGATGGACACCGCCTTGCACTGTTGGA TTCTGGGT | 1164 |
| ADAMTS8 | NM_007037.2 | ADAMTS8 | GCGAGTTCA AAGTGTTCG AG | 13 | CACAGATGG CCAGTGTTT CT | 397 | CACACAGGGTGCCATCA ATCACCT | 781 | 72 | GCGAGTTCAAAGTGTTCGAGGCCAAGG TGATTGATGGCACCCTGTGTGGGCCAG AAACACTGGCCATCTGTG | 1165 |
| ADM | NM_ 001124.1 | ADM | TAAGCCAC AAGCACAC GG | 14 | TGGGCGCCT AAATCCTAA | 398 | CGAGTGGAAGTGCTCCC CACTTTC | 782 | 75 | TAAGCCACAAGCACACGGGGCTCCAGC CCCCCCGAGTGGAAGTGCTCCCCACTTT CTTTAGGATTTAGGCGCCCA | 1166 |
| AES | NM_ 001130.4 | AES | ACGAGATG TCCTACGGC TTGA | 15 | GGGCACAAA TCCCGTTCA G | 399 | CGATCTCAGCCTGTTTGT GCATCTCGAT | 783 | 78 | ACGAGATGTCCTACGGCTTGAACATCG AGATGCACAAACAGGCTGAGATCGTCA AAAGGCTGAACGGGATTTGTGCCC | 1167 |
| AGR2 | NM_006408.2 | AGR2 | AGCCAACA TGTGACTAA TTGGA | 16 | TCTGATCTC CATCTGCCT CA. | 400 | CAACACGTCACCACCCT TTGCTCT | 784 | 70 | AGCCAACATGTGACTAATTGGAAGAAG AGCAAAGGGTGGTGACGTGTTGATGAG GCAGATGGAGATCAGA | 1168 |
| AK055699 | NM 194317 | LYPD6 | CTGCATGTG ATTGAATAA GAAACAAG A | 17 | TGTGGACCT GATCCCTGT ACAC | 401 | TGACCACACCAAAGCCT CCCTGG | 785 | 78 | CTGCATGTGATTGAATAAGAAACAAGA AAGTGACCACACCAAAGCCTCCCTGGC TGGTGTACAGGGATCAGGTCCACA | 1169 |
| AKR7A3 | NM_012067.2 | AKR7A3 | GTGGAAAC GGAGCTCTT CC | 18 | CCAGAGGGT TGAAGGCAT AG | 402 | ACCTCAGTCCAAAGTGC CTGAGGC | 786 | 67 | GTGGAAACGGAGCTCTTCCCCTGCCTC AGGCACTTTGGACTGAGGTTCTATGCCT TCAACCCTCTGG | 1170 |
| AKT3 | NM_ 005465.1 | AKT3 | TTGTCTCTG CCTTGGACT ATCTACA ... | 19 | CCAGCATTA GATTCTCCA ACTTGA | 403 | TCACGGTACACAATCTTT CCGGA.............................. | 787 | 75 | TTGTCTCTGCCTTGGACTATCTACATTC CGGAAAGATTGTGTACCGTGATCTCAA GTTGGAGAATCTAATGCTGG | 1171 |
| ALCAM | NM_ 001627.1 | ALCAM | GAGGAATA TGGAATCCA AGGG | 20 | GTGGCGGAG ATCAAGAGG | 404 | CCAGTTCCTGCCGTCTGC TCTTCT.............................. | 788 | 66 | GAGGAATATGGAATCCAAGGGGGCCA GTTCCTGCCGTCTGCTCTTCTGCCTCTT GATCTCCGCCAC | 1172 |
| ALDH4 | NM_ 003748.2 | ALDH4A1 | GGACAGGG TAAGACCGT GAT...... | 21 | AACCGGAAG AAGTCGATG AG | 405 | CTGCAGCGTCAATCTCC GCTTG | 789 | 68 | GGACAGGGTAAGACCGTGATCCAAGCG GAGATTGACGCTGCAGCGGAACTCATC GACTTCTTCCGGTT | 1173 |
| ANGPT2 | NM_001147.1 | ANGPT2 | CCGTGAAA GCTGCTCTG TAA | 22 | TTGCAGTGG GAAGAACAG TC | 406 | AAGCTGACACAGCCCTC CCAAGTG | 790 | 69 | CCGTGAAAGCTGCTCTGTAAAAGCTGA CACAGCCCTCCCAAGTGAGCAGGACTG TTCTTCCCACTGCAA | 117-1 |
| ANXA2 | NM_004039.1 | ANXA2 | CAAGACAC TAAGGGCG ACTACCA | 23 | CGTGTCGGG CTTCAGTCA T | 407 | CCACCACACAGGTACAG CAGCGCT..... | 791 | 71 | CAAGACACTAAGGGCGACTACCAGAAA GCGCTGCTGTACCTGTGTGGTGGAGAT GACTGAAGCCCGACACG | 1175 |
| AP-1 (JUN official) | NM_ 002228.2 | JUN | GACTGCAA AGATGGAA ACGA | 24 | TAGCCATAA GGTCCGCTC TC | 408 | CTATGACGATGCCCTCA ACGCCTC | 792 | 81 | GACTGCAAAGATGGAAACGACCTTCTA TGACGATGCCCTCAACGCCTCGTTCCTC CCGTCCGAGAGCGGACCTTATGGCTA | 1176 |
| APEX-1 | NM_001641.2 | APEX1 | GATGAAGC CTTTCGCAA GTT | 25 | AGGTCTCCA CACAGCACA AG.......... | 409 | CTTTCGGGAAGCCAGGC CCTT... | 793 | 68 | GATGAAGCCTTTCGCAAGTTCCTGAAG GGCCTGGCTTCCCGAAAGCCCCTTGTG CTGTGTGGAGACCT | 1177 |
| APOD | NM_001647.1 | APOD | GTTTATGCC ATCGGCACC | 26 | GGAATACAC GAGGGCATA GTTC | 410 | ACTGGATCCTGGCCACC GACTATG | 794 | 67 | GTTTATGCCATCGGCACCGTACTGGATC CTGGCCACCGACTATGAGAACTATGCC CTCGTGTATTCC | 1178 |
| ARF1 | NM_ 001658.2 | ARF1 | CAGTAGAG ATCCCCGCA ACT | 27 | ACAAGCACA TGGCTATGG AA | 411 | CTTGTCCTTGGGTCACCC TGCA | 795 | 64 | CAGTAGAGATCCCCGCAACTCGCTTGT CCTTGGGTCACCCTGCATTCCATAGCCA TGTGCTTGT | 1179 |
| ARHI | NM_ 004675.1 | DIRAS3 | ATCAGAGA TTACCGCGT CGT | 28 | ACTTGTGCA GCAGCGTAC TT | 412 | ACACCAGCGGTGCCGAC TACC | 796 | 67 | ATCAGAGATTACCGCGTCGTGGTAGTC GGCACCGCTGGTGTGGGGAAAAGTACG CTGCTGCACAAGT | 1180 |
| ARNT2 | NM_014862.3 | ARNT2 | GACTGGGTC AGTGATGG CA | 29 | GGAGTGACG CATGGACAG A | 413 | CTAGAGCCATCCTTGGC CATCCTG | 797 | 68 | GACTGGGTCAGTGATGGCAACAGGATG GCCAAGGATGGCTCTAGAACACTCTGT CCATGCGTCACTCC | 1181 |
| ARSD | NM_ 001669.1 | ARSD | TCCCTGAGA ACGAAACC ACT | 30 | TGGTGCCAT TTTCCTATG AG | 414 | .............................. CAAGAATCTTGCAGCAG CATGGCT.......... | 798 | 79 | TCCCTGAGAACGAAACCACTTTTGCAA GAATCTTGCAGCAGCATGGCTATGCAA CCGGCCTCATAGGAAAATGGCACCA | 1182 |
| AURKB | NM_004217.1 | AURKB | AGCTGCAG AAGAGCTG CACAT | 31 | GCATCTGCC AACTCCTCC AT | 415 | TGACGAGCAGCGAACAG CCACG | 799 | 67 | AGCTGCAGAAGAGCTGCACATTTGACG AGCAGCGAACAGCCACGATCATGGAGG AGTTGGCAGATGC | 1183 |
| B-actin | NM_ 001101.2 | ACTB | CAGCAGAT GTGGATCA GCAAG | 32 | GCATTTGCG GTGGACGAT | 416 | AGGAGTATGACGAGTCC GGCCCC | 800 | 66 | CAGCAGATGTGGATCAGCAAGCAGGAG TATGACGAGTCCGGCCCCTCCATCGTCC ACCGCAAATGC | 1184 |
| B-Catenin | NM_001904.1 | CTNNB1 | GGCTCTTGT GCGTACTGT CCTT | 33 | TCAGATGAC GAAGAGCAC AGATG | 417 | AGGCTCAGTGATGTCTTC CCTGTCACCAG | 801 | 80 | GGCTCTTGTGCGTACTGTCCTTCGGGCT GGTGACAGGGAAGACATCACTGAGCCT GCCATCTGTGCTCTTCGTCATCTGA | 1185 |
| BAD | NM_032989.1 | BAD | GGGTCAGG TGCCTCGAG AT | 34 | CTGCTCACT CGGCTCAAA CTC | 418 | TGGGCCCAGAGCATGTT CCAGATC | 802 | 73 | GGGTCAGGTGCCTCGAGATCGGGCTTG GGCCCAGAGCATGTTCCAGATCCCAGA GTTTGAGCCGAGTGAGCAG | 1186 |
| BAG1 | NM_004323.2 | BAG1 | CGTTGTCAG CACTTGGAA TACAA | 35 | GTTCAACCT CTTCCTGTG GACTGT | 419 | CCCAATTAACATGACCC GGCAACCAT | 803 | 81 | CGTTGTCAGCACTTGGAATACAAGATG GTTGCCGGGTCATGTTAATTGGGAAAA AGAACAGTCCACAGGAAGAGGTTGAAC | 1187 |
| BAG4 | NM_ 004874.2 | BAG4 | CCTACGGCC GCTACTACG | 36 | GGGCGAAGA GGATATAAG GG.......... | 420 | AGATGTGCCGGTACACC CACCTC | 804 | 76 | CCTACGGCCGCTACTACGGGCCTGGGG GTGGAGATGTGCCGGTACACCCACCTC CACCCTTATATCCTCTTCGCCC | 1188 |
| BASE | NM_173859.1 | | GACTCCTCA GGGCAGAC TTTCTT | 37 | CGAAGGCAC TACTCAATG GTTTC | 421 | CCAGCCTGCAGACAACT GGCCTC.............. | 805 | 72 | GACTCCTCAGGGCAGACTTTCTTCCCAG CCTGCAGACAACTGGCCTCCAGAAACC ATTGAGTAGTGCCTTCG | 1189 |
| Bax | NM_004324.1 | BAX | CCGCCGTGG ACACAGAC T | 38 | TTGCCGTCA GAAAACATG TCA | 422 | TGCCACTCGGAAAAAGA CCTCTCGG | 806 | 70 | CCGCCGTGGACACAGACTCCCCCGAG AGGTCTTTTTCCGAGTGGCAGCTGACAT GTTTTCTGACGGCAA | 1190 |
| BBC3 | NM_014417.1 | BBC3 | CCTGGAGG GTCCTGTAC AAT | 39 | CTAATTGGG CTCCATCTC G | 423 | CATCATGGGACTCCTGC CCTTACC | 807 | 83 | CCTGGAGGGTCCTGTACAATCTCATCAT GGGACTCCTGCCCTTACCCAGGGGCCA CAGAGCCCCCGAGATGGAGCCCAATTA G | 1191 |
| BCAR1 | NM_014567.1 | BCAR1 | ACTGACAA GACCAGCA GCAT | 40 | TCCTGGGAG GTGAACTTA GG | 424 | AGTCACGACCCCTGCCC TCAC | 808 | 65 | ACTGACAAGACCAGCAGCATCCAGTCA CGACCCCTGCCCTCACCCCCTAAGTTCA CCTCCCAGGA | 1192 |
| BCAR3 | NM_ 003567.1 | BCAR3 | TGACTTCCT AGTTCGTGA CTCTCTGT | 41 | TGAGCGAGG TTCTTCCACT GA | 425 | CAGCCCTGGGAACTTTG TCCTGACC | 809 | 75 | TGACTTCCTAGTTCGTGACTCTCTGTCC AGCCCTGGGAACTTTGTCCTGACCTGTC AGTGGAAGAACCTCGCTCA | 1193 |
| BCAS1 | NM_003657.1 | BCAS1 | CCCCGAGA CAACGGAG ATAA | 42 | CTCGGGTTT GGCCTCTTT C | 426 | CTTTCCGTTGGCATCCGC AACAG | 810 | 73 | CCCCGAGACAACGGAGATAAGTGCTGT TGCGGATGCCAACGGAAAGAATCTTGG GAAAGAGGCCAAACCCGAG | 1194 |
| Bcl2 | NM_ 000633.1 | BCI.2 | CAGATGGA CCTAGTACC CACTGAGA | 43 | CCTATGATT TAAGGGCAT TTTTCC | 427 | TTCCACGCCGAAGGACA GCGAT | 811 | 73 | CAGATGGACCTAGTACCCACTGAGATT TCCACGCCGAAGGACAGCGATGGGAAA AATGCCCTTAAATCATAGG | 1195 |
| BCL2L12 | NM_ 138639.1 | BCL2L12 | AACCCACCC CTGTCTTGG | 44 | CTCAGCTGA CGGGAAAGG | 428 | TCCGGGTAGCTCTCAAA CTCGAGG | 812 | 73 | AACCCACCCCTGTCTTGGAGCTCCGGG TAGCTCTCAAACTCGAGGCTGCGCACC CCCTTTCCCGTCAGCTGAG | 1196 |
| BGN | NM_001711.3 | BGN | GAGCTCCGC AAGGATGA C | 45 | CTTGTTGTTC ACCAGGACG A | 429 | CAAGGGTCTCCAGCACC TCTACGC | 813 | 66 | GAGCTCCGCAAGGATGACTTCAAGGGT CTCCAGCACCTCTACGCCCTCGTCCTGG TGAACAACAAG | 1197 |
| BIK | NM_001197.3 | BIK | ATTCCTATG GCTCTGCAA TTGTC | 46 | GGCAGGAGT GAATGGCTC TTC | 430 | CCGGTTAACTGTGGCCT GTGCCC | 814 | 70 | ATTCCTATGGCTCTGCAATTGTCACCGG TTAACTGTGGCCTGTGCCCAGGAAGAG CCATTCACTCCTGCC | 1198 |
| BNIP3 | NM_004052.2 | BNIP3 | CTGGACGG AGTAGCTCC AAG | 47 | GGTATCTTG TGGTGTCTG CG | 431 | CTCTCACTGTGACAGCCC ACCTCG | 815 | 68 | CTGGACGGAGTAGCTCCAAGAGCTCTC ACTGTGACAGCCCACCTCGCTCGCAGA CACCACAAGATACC | 1199 |
| BSG | NM 001728.2 | BSG | AATTTTATG AGGGCCAC GG | 48 | GTGGCCAAG AGGTCAGAG TC | 432 | CTGTGTTCGACTCAGCCT CAGGGA | 816 | 66 | AATTTATGAGGGCCACGGGTCTGTGTT CGACTCAGCCTCAGGGACGACTCTGAC CTCTTGGCCAC | 1200 |
| BTRC | NM_ 033637.2 | BTRC | GTTGGGAC ACAGTTGGT CTG | 49 | TGAAGCAGT CAGTTGTGC TG | 433 | CAGTCGGCCCAGGACGG TCTACT | 817 | 63 | GTTGGGACACAGTTGGTCTGCAGTCGG CCCAGGACGGTCTACTCAGCACAACTG ACTGCTTCA | 1201 |
| BUB1 | NM_004336.1 | BUB1 | CCGAGGTTA ATCCAGCAC GTA | 50 | AAGACATGG CGCTCTCAG TTC | 434 | TGCTGGGAGCCTACACT TGGCCC | 818 | 68 | CCGAGGTTAATCCAGCACGTATGGGGC CAAGTGTAGGCTCCCAGCAGGAACTGA GAGCGCCATGTCTT | 1202 |
| BUB1B | NM_001211.3 | BUB1B | TCAACAGA AGGCTGAA CCACTAGA | 51 | CAACAGAGT TTGCCGAGA CACT | 435 | TACAGTCCCAGCACCGA CAATTCC | 819 | 82 | TCAACAGAAGGCTGAACCACTAGAAAG ACTACAGTCCCAGCACCGACAATTCCA AGCTCGAGTGTCTCGGCAAACTCTGTT G | 1203 |
| BUB3 | NM_004725.1 | BUB3 | CTGAAGCA GATGGTTCA TCATT........ | 52 | GCTGATTCC CAAGAGTCT AACC | 436 | CCTCGCTTTGTTTAACAG CCCAGG | 820 | 73 | CTGAAGCAGATGGTTCATCATTTCCTGG GCTGTTAAACAAAGCGAGGTTAAGGTT AGACTCTTGGGAATCAGC | 1204 |
| c-kit | NM_000222.1 | KIT | GAGGCAAC TGCTTATGG CTTAATTA | 53 | GGCACTCGG CTTGAGCAT | 437 | TTACAGCGACAGTCATG GCCGCAT | 821 | 75 | GAGGCAACTGCTTATGGCTTAATTAAG TCAGATGCGGCCATGACTGTCGCTGTA AAGATGCTCAAGCCGAGTGCC | 1205 |
| C10orf116 | NM_006829.2 | C10orf116 16 | CAAGAGCA GAGCCACC GT | 54 | TGAGACCGT TGGATTGGA TT | 438 | CCGGAGTCCTAGCCTCC CAAATTC | 822 | 67 | CAAGAGCAGAGCCACCGTAGCCGGAGT CCTAGCCTCCCAAATTCGGAAATCCAA TCCAACGGTCTCA | 1206 |
| C17orf37 | NM_ 032339.3 | C17orf37 | GTGACTGCA CAGGACTCT GG | 55 | AGGACCAAA GGGAGACCA A | 439 | CCTGCTCTGTTCTGGGGT CCAAAC | 823 | 67 | GTGACTGCACAGGACTCTGGGTTCCTG CTCTGTTCTGGGGTCCAAACCTTGGTCT CCCTTTGGTCCT | 1207 |
| C20orf1 | NM_ 012112 | TPX2 | TCAGCTGTG AGCTGCGG ATA | 56 | ACGGTCCTA GGTTTGAGG TTAAGA | 440 | CAGGTCCCATTGCCGGG CG | 824 | 65 | TCAGCTGTGAGCTGCGGATACCGCCCG GCAATGGGACCTGCTCTTAACCTCAAA CCTAGGACCGT | 1208 |
| C6orf66 | NM_14165.1 | NDUFAF4 | GCGGTATCA GGAATTTCA ACCT | 57 | GCGACAGAG GGCTTCATC TT | 441 | TGATTTCCCGTTCCGCTC GGTTCT .............. | 825 | 70 | GCGGTATCAGGAATTTCAACCTAGAGA ACCGAGCGGAACGGAAATCAGCAAG ATGAAGCCCTCTGTCGC | 1209 |
| C8ort4 | NM_020130.2 | C8orf4 | CTACGAGTC AGCCCATCC AT | 58 | TGCCCACGG CTTTCTTAC | 442 | CATGGCTACCACTTCGA CACAGCC | 826 | 67 | CTACGAGTCAGCCCATCCATCCATGGC TACCACTTCGACACAGCCTCTCGTAAG AAAGCCGTGGGCA | 1210 |
| CACNA2D 2 | NM_006030.1 | CACNA2D 2 | TGATGCTGC AGAGAACT TCC | 59 | CACGATGTC TTCCTCCTTG A | 443 | AAAGCACACCGCTGGCA GGAC | 827 | 67 | TGATGCTGCAGAGAACTTCCAGAAAGC ACACCGCTGGCAGGACAACATCAAGGA GGAAGACATCGTG | 1211 |
| CAT | NM_ 001752.1 | CAT | ATCCATTCG ATCTCACCA AGGT | 60 | TCCGGTTTA AGACCAGTT TACCA | 444 | TGGCCTCACAAGGACTA CCCTCTCATCC | 828 | 78 | ATCCATTCGATCTCACCAAGGTTTGGCC TCACAAGGACTACCCTCTCATCCCAGTT GGTAAACTGGTCTTAAACCGGA | 1212 |
| CAV1 | NM_001753.3 | CAV1 | GTGGCTCAA CATTGTGTT CC | 61 | CAATGGCCT CCATTTTTAC AG | 445 | ATTTCAGCTGATCAGTG GGCCTCC | 829 | 74 | GTGGCTCAACATTGTGTTCCCATTTCAG CTGATCAGTGGGCCTCCAAGGAGGGGC TGTAAAATGGAGGCCATTG | 1213 |
| CBX5 | NM_012117.1 | CBX5 | AGGGGATG GTCTCTGTC ATT.......... | 62 | AAAGGGGTG GGTAGAAAG GA | 446 | CATAATACATTCACCTCC CTGCCTCCTC | 830 | 78 | AGGGGATGGTCTCTGTCATTTCTCTTTG TACATAATACATTCACCTCCCTGCCTCC TCTCCTTTCTACCCACCCCTTT | 1214 |
| CCL19 | NM_006274.2 | CCL19 | GAACGCAT CATCCAGA GACTG | 63 | CCTCTGCAC GGTCATAGG TT | 447 | CGCTTCATCTTGGCTGAG GTCCTC | 831 | 78 | GAACGCATCATCCAGAGACTGCAGAGG ACCTCAGCCAAGATGAAGCGCCGCAGC AGTTAACCTATGACCGTGCAGAGG | 1215 |
| CCL3 | NM_003983.1 | CCL3 | AGCAGACA GTGGTCAGT CCTT | 64 | CTGCATGAT TCTGAGCAG GT | 448 | CTCTGCTGACACTCGAG CCCACAT.......... | 832 | 77 | AGCAGACAGTGGTCAGTCCTTTCTTGG CTCTGCTGACACTCGAGCCCACATTCCG TCACCTGCTCAGAATCATGCAG | 1216 |
| CCL5 | NM_002985.2 | CCL5 | AGGTTCTGA GCTCTGGCT TT | 65 | ATGCTGACT TCCTTCCTG GT | 449 | ACAGAGCCCTGGCAAAG CCAAG | 833 | 65 | AGGTTCTGAGCTCTGGCTTTGCCTTGGC TTTGCCAGGGCTCTGTGACCAGGAAGG AAGTCAGCAT | 1217 |
| CCNB1 | NM_ 031966.1 | CCNB1 | TTCAGGTTG TTGCAGGA GAC | 66 | CATCTTCTTG GGCACACAA T | 450 | TGTCTCCATTATTGATCG GTTCATGCA | 834 | 84 | TTCAGGTTGTTGCAGGAGACCATGTAC ATGACTGTCTCCATTATTGATCGGTTCA TGCAGAATAATTGTGTGCCCAAGAAGA TG | 1218 |
| CCND3 | NM_001760.2 | CCND3 | CCTCTGTGC TACAGATTA TACCTTTGC | 67 | CACTGCAGC CCCAATGCT | 451 | TACCCGCCATCCATGATC GCCA | 835 | 76 | CCTCTGTGCTACAGATTATACCTTTGCC ATGTACCCGCCATCCATGATCGCCACG GGCAGCATTGGGGCTGCAGTG | 1219 |
| CCNE2 variant 1 | NM_ 057749var 1 | CCNE2 | GGTCACCA AGAAACAT CAGTATGA A | 68 | TTCAATGAT AATGCAAGG ACTGATC | 452 | CCCAGATAATACAGGTG GCCAACAATTCCT | 836 | 85 | GGTCACCAAGAAACATCAGTATGAAAT TAGGAATTGTTGGCCACCTGTATTATCT GGGGGGATCAGTCCTTGCATTATCATT GAA | 1220 |
| CCR5 | NM_000579.1 | CCR5 | CAGACTGA ATGGGGGT GG | 69 | CTGGTTTGT CTGGAGAAG GC | 453 | TGGAATAAGTACCTAAG GCGCCCCC | 837 | 67 | CAGACTGAATGGGGGTGGGGGGGGCG CCTTAGGTACTTATTCCAGATGCCTTCT CCAGACAAACCAG | 1221 |
| CCR7 | NM_001838.2 | C'CR7 | GGATGACA TGCACTCAG CTC | 70 | CCTGACATT TCCCTTGTCC T | 454 | CTCCCATCCCAGTGGAG CCAA | 838 | 64 | GGATGACATGCACTCAGCTCTTGGCTC CACTGGGATGGGAGGAGAGGACAAGG GAAATGTCAGG | 1222 |
| CD1A | NM_ 001763.1 | CD1A | GGAGTGGA AGGAACTG GAAA | 71 | TCATGGGCG TATCTACGA AT | 455 | CGCACCATTCGGTCATTT GAGG | 839 | 78 | GGAGTGGAAGGAACTGGAAACATTATT CCGTATACGCACCATTCGGTCATTTGAG GGAATTCGTAGATACGCCCATGA 1223 | 1223 |
| CD24 | NM_013230.1 | CD24 | TCCAACTAA TGCCACCAC CAA | 72 | GAGAGAGTG AGACCACGA AGAGACT | 456 | CTGTTGACTGCAGGGCA CCACCA | 840 | 77 | TCCAACTAATGCCACCACCAAGGCGGC TGGTGGTGCCCTGCAGTCAACAGCCAG TCTCTTCGTGGTCTCACTCTCTC | 1224 |
| CD4 | NM_000616.2 | CD4 | GTGCTGGA GTCGGGACT AAC | 73 | TCCCTGCAT TCAAGAGGC | 457 | CAGGTCCCTTGTCCCAA GTTCCAC | 841 | 67 | GTGCTGGAGTCGGGACTAACCCAGGTC CCTTGTCCCAAGTTCCACTGCTGCCTCT TGAATGCAGGGA | 1225 |
| CD44E | X55150 | | ATCACCGAC AGCACAGA CA | 74 | ACCTGTGTT TGGATTTGC AG........ | 458 | CCCTGCTACCAATATGG ACTCCAGTCA | 842 | 90 | ATCACCGACAGCACAGACAGAATCCCT GCTACCAATATGGACTCCAGTCATAGT ACAACGCTTCAGCCTACTGCAAATCCA AACACAGGT | 1226 |
| CD44s | M59040.1 | | GACGAAGA CAGTCCCTG GAT | 75 | ACTGGGGTG GAATGTGTC TT | 459 | CACCGACAGCACAGACA GAATCCC | 843 | 78 | GACGAAGACAGTCCCTGGATCACCGAC AGCACAGACAGAATCCCTGCTACCAGA GACCAAGACACATTCCACCCCAGT | 1227 |
| CD44v6 | AJ251595v6 | | CTCATACCA GCCATCCAA TG | 76 | TTGGGTTGA AGAAATCAG TCC | 460 | CACCAAGCCCAGAGGAC AGTTCCT | 844 | 78 | CTCATACCAGCCATCCAATGCAAGGAA GGACAACACCAAGCCCAGAGGACAGTT CCTGGACTGATTTCTTCAACCCAA | 1228 |
| CD68 | NM_001251.1 | CD68 | TGGTTCCCA GCCCTGTGT | 77 | CTCCTCCAC CCTGGGTTG T | 461 | CTCCAAGCCCAGATTCA GATTCGAGTCA | 845 | 74 | TGGTTCCCAGCCCTGTGTCCACCTCCAA GCCCAGATTCAGATTCGAGTCATGTAC ACAACCCAGGGTGGAGGAG | 1229 |
| CD82 | NM_ 002231.2 | CD82 | GTGCAGGCT CAGGTGAA GTG | 78 | GACCTCAGG GCGATTCAT GA | 462 | TCAGCTTCTACAACTGG ACAGACAACGCTG | 846 | 84 | GTGCAGGCTCAGGTGAAGTGCTGCGGC TGGGTCAGCTTCTACAACTGGACAGAC AACGCTGAGCTCATGAATCGCCCTGAG GTC | 1230 |
| CDC20 | NM_001255.1 | CDC20 | TGGATTGGA GTTCTGGGA ATG | 79 | GCTTGCACT CCACAGGTA CACA | 463 | ACTGGCCGTGGCACTGG ACAACA | 847 | 68 | TGGATTGGAGTTCTGGGAATGTACTGG CCGTGGCACTGGACAACAGTGTGTACC TGTGGAGTGCAAGC | 1231 |
| cdc25A | NM_001789.1 | CDC25A | TCTTGCTGG CTACGCCTC TT | 80 | CTGCATTGT GGCACAGTT CTG | 464 | TGTCCCTGTTAGACGTCC TCCGTCCATA | 848 | 71 | TCTTGCTGGCTACGCCTCTTCTGTCCCT GTTAGACGTCCTCCGTCCATATCAGAA CTGTGCCACAATGCAG | 1232 |
| CDC25C | NM_001790.2 | CDC25C | GGTGAGCA GAAGTGGC CTAT | 81 | CTTCAGTCTT GGCCTGTTC A | 465 | CTCCCCGTCGATGCCAG AGAACT | 849 | 67 | GGTGAGCAGAAGTGGCCTATATCGCTC CCCGTCGATGCCAGAGAACTTGAACAG GCCAAGACTGAAG | 1233 |
| CDC4 | NM_018315.2 | FBXW7 | GCAGTCCGC TGTGTTCAA | 82 | GGATCCCAC ACCTTTTACC ATAA | 466 | TGCTCCACTAACAACCCT CCTGCC | 850 | 77 | GCAGTCCGCTGTGTTCAATATGATGGC AGGAGGGTTGTTAGTGGAGCATATGAT TTTATGGTAAAGGTGTGGGATCC | 1234 |
| CDC42BP A | NM_003607.2 | CDC42BPA | GAGCTGAA AGACGCAC ACTG | 83 | GCCGCTCAT TGATCTCCA | 467 | AATTCCTGCATGGCCAG TTTCCTC | 851 | 67 | GAGCTGAAAGACGCACACTGTCAGAGG AAACTGGCCATGCAGGAATTCATGGAG ATCAATGAGCGGC | 1235 |
| CDC42EP4 | NM_012121.4 | CDC42EP4 | CGGAGAAG GGCACCAG TA | 84 | CCGTCATTG GCCTTCTTC | 468 | CTGCCCAAGAGCCTGTC ATCCAG | 852 | 67 | CGGAGAAGGGCACCAGTAAGCTGCCCA AGAGCCTGTCATCCAGCCCCGTGAAGA AGGCCAATGACGG | 1236 |
| CDH11 | NM_ 001797.2 | CDH11 | GTCGGCAG AAGCAGGA CT | 85 | CTACTCATG GGCGGGATG | 469 | CCTTCTGCCCATAGTGAT CAGCGA .......................... | 853 | 70 | GTCGGCAGAAGCAGGACTTGTACCTTC TGCCCATAGTGATCAGCGATGGCGGCA TCCCGCCCATGAGTAG | 1237 |
| CDH3 | NM_001793.3 | CDH3 | ACCCATGTA CCGTCCTCG | 86 | CCGCCTTCA GGTTCTCAA T | 470 | CCAACCCAGATGAAATC GGCAACT | 854 | 71 | ACCCATGTACCGTCCTCGGCCAGCCAA CCCAGATGAAATCGGCAACTTTATAAT TGAGAACCTGAAGGCGG | 1238 |
| CDK4 | NM_000075.2 | CDK4 | CCTTCCCAT CAGCACAG TTC | 87 | TTGGGATGC TCAAAAGCC | 471 | CCAGTCGCCTCAGTAAA GCCACCT | 855 | 66 | CCTTCCCATCAGCACAGTTCGTGAGGT GGCTTTACTGAGGCGACTGGAGGCTTT TGAGCATCCCAA | 1239 |
| CDK5 | NM_004935.2 | CDK5 | AAGCCCTAT CCGATGTAC CC | 88 | CTGTGGCAT TGAGTTTGG G | 472 | CACAACATCCCTGGTGA ACGTCGT | 856 | 67 | AAGCCCTATCCGATGTACCCGGCCACA ACATCCCTGGTGAACGTCGTGCCCAAA CTCAATGCCACAG | 1240 |
| CDKN3 | NM_005193.2 | CDKN3 | TGGATCTCT ACCAGCAA TGTG........ | 89 | ATGTCAGGA GTCCCTCCA TC | 473 | ATCACCCATCATCATCCA ATCGCA | 857 | 70 | TGGATCTCTACCAGCAATGTGGAATTA TCACCCATCATCATCCAATCGCAGATG GAGGGACTCCTGACAT | 1241 |
| CEACAM1 | NM_001712.2 | CEACAM1 | ACTTGCCTG TTCAGAGCA CTCA | 90 | TGGCAAATC CGAATTAGA GTGA | 474 | TCCTTCCCACCCCCAGTC CTGTC | 858 | 71 | ACTTGCCTGTTCAGAGCACTCATTCCTT CCCACCCCCAGTCCTGTCCTATCACTCT AATTCGGATTTGCCA | 1242 |
| CEBPA | NM_004364.2 | CEBPA | TTGGTTTTG CTCGGATAC TTG | 91 | GTCTCAGAC CCTTCCCCC | 475 | AAAATGAGACTCTCCGT CGGCAGC | 859 | 66 | TTGGTTTTGCTCGGATACTTGCCAAAAT GAGACTCTCCGTCGGCAGCTGGGGGAA GGGTCTGAGAC | 1243 |
| CEGP1 | NM_ 020974.1 | SCUBE2 | TGACAATCA GCACACCTG CAT | 92 | TGTGACTAC AGCCGTGAT CCTTA........ | 476 | CAGGCCCTCTTCCGAGC GGT | 860 | 77 | TGACAATCAGCACACCTGCATTCACCG CTCGGAAGAGGGCCTGAGCTGCATGAA TAAGGATCACGGCTGTAGTCACA | 1244 |
| CENPA | NM_ 001809.2 | CENPA | TAAATTCAC TCGTGGTGT GGA | 93 | GCCTCTTGT AGGGCCAAT AG | 477 | CTTCAATTGGCAAGCCC AGGC | 861 | 63 | TAAATTCACTCGTGGTGTGGACTTCAAT TGGCAAGCCCAGGCCCTATTGGCCCTA CAAGAGGC | 1245 |
| CGA (CHGA official) | NM_001275.2 | CHGA | CTGAAGGA GCTCCAAG ACCT | 94 | CAAAACCGC TGTGTTTCTT C | 478 | TGCTGATGTGCCCTCTCC TTGG | 862 | 76 | CTGAAGGAGCTCCAAGACCTCGCTCTC CAAGGCGCCAAGGAGAGGGCACATCA GCAGAAGAAACACAGCGGTTTTG | 1246 |
| CGalpha | NM_000735.2 | CGA | CCAGAATG CACGCTACA GGAA | 95 | GCCCATGCA CTGAAGTAT TGG | 479 | ACCCATTCTTCTCCCAGC CGGG | 863 | 69 | CCAGAATGCACGCTACAGGAAAACCCA TTCTTCTCCCAGCCGGGTGCCCCAATAC TTCAGTGCATGGGC | 1247 |
| CGB | NM_000737.2 | CGB | CCACCATAG GCAGAGGC A | 96 | AGTCGTCGA GTGCTAGGG AC | 480 | ACACCCTACTCCCTGTGC CTCCAG | 864 | 80 | CCACCATAGGCAGAGGCAGGCCTTCCT ACACCCTACTCCCTGTGCCTCCAGCCTC GACTAGTCCCTAGCACTCGACGACT | 1248 |
| CHAF1B | NM_ 005441.1 | CHAF1B | GAGGCCAG TGGTGGAA ACAG | 97 | TCCGAGGCC ACAGCAAAC | 481 | AGCTGATGAGTCTGCCC TACCGCCTG .......... | 865 | 72 | GAGGCCAGTGGTGGAAACAGGTGTGGA GCTGATGAGTCTGCCCTACCGCCTGGT GTTTGCTGTGGCCTCGGA | 1249 |
| CHER | NM_018323.1 | CHFR | AAGGAAGT GGTCCCTCT GTG | 98 | GACGCAGTC TTTCTGTCTG G | 482 | TGAAGTCTCCAGCTTTGC CTCAGC | 866 | 76 | AAGGAAGTGGTCCCTCTGTGGCAAGTG ATGAAGTCTCCAGCTTTGCCTCAGCTCT CCCAGACAGAAAGACTGCGTC | 1250 |
| CHI3L1 | NM_ 001276.1 | CHI3L1 | AGAATGGG TGTGAAGG CG | 99 | TGCAGAGCA GCACTGGAG | 483 | CACCAGGACCACAAAGC CTGTTTG | 867 | 66 | AGAATGGGTGTGAAGGCGTCTCAAACA GGCTTTGTGGTCCTGGTGCTGCTCCAGT GCTGCTCTGCA | 1251 |
| CKS2 | NM_ 001827.1 | CKS2 | GGCTGGAC GTGGTTTTG TCT | 100 | CGCTGCAGA AAATGAAAC GA | 484 | CTGCGCCCGCTCTTCGCG | 868 | 62 | GGCTGGACGTGGTTTTGTCTGCTGCGCC CGCTCTTCGCGCTCTCGTTTCATTTTCT GCAGCG | 1252 |
| Claudin 4 | NM_ 001305.2 | CLDN4 | GGCTGCTTT GCTGCAACT G | 101 | CAGAGCGGG CAGCAGAAT A ........ | 485 | CGCACAGACAAGCCTTA CTCCGCC | 869 | 72 | GGCTGCTTTGCTGCAACTGTCCACCCCG CACAGACAAGCCTTACTCCGCCAAGTA TTCTGCTGCCCGCTCTG | 1253 |
| CLIC1 | NM_001288.3 | CLIC1 | CGGTACTTG AGCAATGC CTA | 102 | TCGATCTCC TCATCATCT GG.......... | 486 | CGGGAAGAATTCGCTTC CACCTG | 870 | 68 | CGGTACTTGAGCAATGCCTACGCCCGG GAAGAATTCGCTTCCACCTGTCCAGAT GATGAGGAGATCGA | 1254 |
| CLU | NM_001831.1 | CLU | CCCCAGGAT ACCTACCAC TACCT | 103 | TGCGGGACT TGGGAAAGA | 487 | CCCTTCAGCCTGCCCCAC CG | 871 | 76 | CCCCAGGATACCTACCACTACCTGCCCT TCAGCCTGCCCCACCGGAGGCCTCACT TCTTCTTTCCCAAGTCCCGCA | 1255 |
| CNOT2 | NM_014515.3 | CNOT2 | AAATCGCA GCTTATCAC AAGG | 104 | TGTTGGTAC CCCTGTTGTT G | 488 | ACTCAGTTACCGAGCCA CGTCACG | 872 | 67 | AAATCGCAGCTTATCACAAGGCACTCA GTTACCGAGCCACGTCACGCCAACAAC AGGGGTACCAACA | 1256 |
| COL1A1 | NM_000088.2 | COL1A1 | GTGGCCATC CAGCTGACC | 105 | CAGTGGTAG GTGATGTTC TGGGA | 489 | TCCTGCGCCTGATGTCCA CCG | 873 | 68 | GTGGCCATCCAGCTGACCTTCCTGCGCC TGATGTCCACCGAGGCCTCCCAGAACA TCACCTACCACTG | 1257 |
| COL1 A2 | NM_000089.2 | COL1A2 | CAGCCAAG AACTGGTAT AGGAGCT | 106 | AAACTGGCT GCCAGCATT G | 490 | TCTCCTAGCCAGACGTGT TTCTTGTCCTTG | 874 | 80 | CAGCCAAGAACTGGTATAGGAGCTCCA AGGACAAGAAACACGTCTGGCTAGGAG AAACTATCAATGCTGGCAGCCAGTTT | 1258 |
| COMT | NM_ 000754.2 | COMT | CCTTATCGG CTGGAACG AGTT | 107 | CTCCTTGGT GTCACCCAT GAG | 491 | CCTGCAGCCCATCCACA ACCT | 875 | 67 | CCTTATCGGCTGGAACGAGTTCATCCTG CAGCCCATCCACAACCTGCTCATGGGT GACACCAAGGAG | 1259 |
| Contig 51037 | NM_198477 | CXCL17 | CGACAGTTG CGATGAAA GTTCTAA | 108 | GGCTGCTAG AGACCATGG ACAT | 492 | CCTCCTCCTGTTGCTGCC ACTAATGCT | 876 | 81 | CGACAGTTGCGATGAAAGTTCTAATCT CTTCCCTCCTCCTGTTGCTGCCACTAAT GCTGATGTCCATGGTCTCTAGCAGCC | 1260 |
| COPS3 | NM_003653.2 | COPS3 | ATGCCCAGT GTTCCTGAC TT | 109 | CTCCCCATT ACAAGTGCT GA | 493 | CGAAACGCTATTCTCAC AGGTTCAGC | 877 | 72 | ATGCCCAGTGTTCCTGACTTCGAAACG CTATTCTCACAGGTTCAGCTCTTCATCA GCACTTGTAATGGGGAG | 1261 |
| CRYAB | NM_ 001885.1 | CRYAB | GATGTGATT GAGGTGCA TGG | 110 | GAACTCCCT GGAGATGAA ACC | 494 | TGTTCATCCTGGCGCTCT TCATGT | 878 | 69 | GATGTGATTGAGGTGCATGGAAAACAT GAAGAGCGCCAGGATGAACATGGTTTC ATCTCCAGGGAGTTC | 1262 |
| CRYZ | NM_ 001889.2 | CRYZ | AAGTCCTGA AATTGCGAT CA | 111 | CACATGCAT GGACCTTGA TT | 495 | CCGATTCCAAAAGACCA TCAGGTTCT | 879 | 78 | AAGTCCTGAAATTGCGATCAGATATTG CAGTACCGATTCCAAAAGACCATCAGG TTCTAATCAAGGTCCATGCATGTG | 1263 |
| CSF1 isoC | NM_172211.1 | CSF1 | CAGCAAGA ACTGCAAC AACA | 112 | ATCCCTCGG ACTGCCTCT | 496 | TTTGCTGAATGCTCCAGC CAAGG | 880 | 68 | CAGCAAGAACTGCAACAACAGCTTTGC TGAATGCTCCAGCCAAGGCCATGAGAG GCAGTCCGAGGGAT | 1264 |
| CSF1 | NM_000757.3 | CSF1 | TGCAGCGG CTGATTGAC A | 113 | CAACTGTTC CTGGTCTAC AAACTCA | 497 | TCAGATGGAGACCTCGT GCCAAATTACA | 881 | 74 | TGCAGCGGCTGATTGACAGTCAGATGG AGACCTCGTGCCAAATTACATTTGAGTT TGTAGACCAGGAACAGTTG | 1265 |
| CSF1R | NM_ 005211.1 | CSFIR | GAGCACAA CCAAACCTA CGA | 114 | CCTGCAGAG ATGGGTATG AA | 498 | AGCCACTCCCCACGCTG TTGT....................... | 882 | 80 | GAGCACAACCAAACCTACGAGTGCAGG GCCCACAACAGCGTGGGGAGTGGCTCC TGGGCCTTCATACCCATCTCTGCAGG | 1266 |
| CSF2RA | NM_ 006140.3 | CSF2RA | TACCACACC CAGCATTCC TC | 115 | CTAGAGGCT GGTGCCACT GT | 499 | CGCAGATCCGATTTCTCT GGGATC | 883 | 67 | TACCACACCCAGCATTCCTCCTGATCCC AGAGAAATCGGATCTGCGAACAGTGGC ACCAGCCTCTAG | 1267 |
| CSK (SRC) | NM_004383.1 | CSK | CCTGAACAT GAAGGAGC TGA | 116 | CATCACGTC TCCGAACTC C | 500 | TCCCGATGGTCTGCAGC AGCT | 884 | 64 | CCTGAACATGAAGGAGCTGAAGCTGCT GCAGACCATCGGGAAGGGGGAGTTCGG AGACGTGATG | 1268 |
| CTGF | NM_001901.1 | CTGF | GAGTTCAA GTGCCCTGA CG | 117 | AGTTGTAAT GGCAGGCAC AG | 501 | AACATCATGTTCTTCTTC ATGACCTCGC | 885 | 76 | GAGTTCAAGTGCCCTGACGGCGAGGTC ATGAAGAAGAACATGATGTTCATCAAG ACCTGTGCCTGCCATTACAACT | 1269 |
| CTHRC1 | NM_ 138455.2 | CTHRC1 | GCTCACTTC GGCTAAAA TGC | 118 | TCAGCTCCA TTGAATGTG AAA | 502 | ACCAACGCTGACAGCAT GCATTTC | 886 | 67 | GCTCACTTCGGCTAAAATGCAGAAATG CATGCTGTCAGCGTTGGTATTTCACATT CAATGGAGCTGA | 1270 |
| CTSD | NM_ 001909.1 | CTSD | GTACATGAT CCCCTGTGA GAAGGT | 119 | GGGACAGCT TGTAGCCTT TGC | 503 | ACCCTGCCCGCGATCAC ACTGA | 887 | 80 | GTACATGATCCCCTGTGAGAAGGTGTC CACCCTGCCCGCGATCACACTGAAGCT GGGAGGCAAAGGCTACAAGCTGTCCC | 1271 |
| CTSL2 | NM_001333.2 | CTSL2 | TGTCTCACT GAGCGAGC AGAA | 120 | ACCATTGCA GCCCTGATT G | 504 | CTTGAGGACGCGAACAG TCCACCA | 888 | 67 | TGTCTCACTGAGCGAGCAGAATCTGGT GGACTGTTCGCGTCCTCAAGGCAATCA GGGCTGCAATGGT | 1272 |
| CTSL2int2 | NM_ 001333.2in t2 | | ACCAGGCA ATAACCTAA CAGC | 121 | CTGTTCTCC AAGCCAAGA CA | 505 | AGGTGCAATATGGCAT ATATCTCCATTG | 889 | 79 | ACCAGGCAATAACCTAACAGCACCCAT TATAGGTGCAATATGGGCATATATCTC CATTGTGTCTTGGCTTGGAGAACAG | 1273 |
| CXCL10 | NM_001565.1 | CXCL10 | GGAGCAAA ATCGATGCA GT | 122 | TAGGGAAGT GATGGGAGA GG | 506 | TCTGTGTGGTCCATCCTT GGAAGC | 890 | 68 | GGAGCAAAATCGATGCAGTGCTTCCAA GGATGGACCACACAGAGGCTGCCTCTC CCATCACTTCCCTA | 1274 |
| CXCL12 | NM_ 000609.3 | CXCL12 | GAGCTACA GATGCCCAT GC ...... | 123 | TTTGAGATG CTTGACGTT GG | 507 | TTCTTCGAAAGCCATGTT GCCAGA | 891 | 67 | GAGCTACAGATGCCCATGCCGATTCTT CGAAAGCCATGTTGCCAGAGCCAACGT CAAGCATCTCAAA | 1275 |
| CXCL14 | NM_004887.3 | CXCL14 | TGCGCCCTT TCCTCTGTA | 124 | CAATGCGGC ATATACTGG G | 508 | TACCCTTAAGAACGCCC CCTCCAC | 892 | 74 | TGCGCCCTTTCCTCTGTACATATACCCT TAAGAACGCCCCCTCCACACACTGCCC CCCAGTATATGCCGCATTG | 1276 |
| CXCR4 | NM_003467.1 | CXCR4 | TGACCGCTT CTACCCCAA TG | 125 | AGGATAAGG CCAACCATG ATGT | 509 | CTGAAACTGGAACACAA CCACCCACAAG | 893 | 72 | TGACCGCTTCTACCCCAATGACTTGTGG GTGGTTGTGTTCCAGTTTCAGCACATCA TGGTTGGCCTTATCCT | 1277 |
| CYP17A1 | NM_ 000102.2 | CYP17A1 | CCGGAGTG ACTCTATCA CCA | 126 | GCCAGCATT GCCATTATC T | 510 | TGGACACACTGATGCAA GCCAAGA | 894 | 76 | CCGGAGTGACTCTATCACCAACATGCT GGACACACTGATGCAAGCCAAGATGAA CTCAGATAATGGCAATGCTGGC | 1278 |
| CYP19A1 | NM_000103.2 | CYP19A1 | TCCTTATAG GTACTTTCA GCCATTTG | 127 | CACCATGGC GATGTACTT TCC | 511 | CACAGCCACGGGCCCA AA | 895 | 70 | TCCTTATAGGTACTTTCAGCCATTTGGC TTTGGGCCCCGTGGCTGTGCAGGAAAG TACATCGCCATGGTG | 1279 |
| CYP1B1 | NM_ 000104.2 | CYP1B1 | CCAGCTTTG TGCCTGTCA CTAT... | 128 | GGGAATGTG GTAGCCCAA GA | 512 | CTCATGCCACCACTGCC AACACCTC | 896 | 71 | CCAGCTTTGTGCCTGTCACTATTCCTCA TGCCACCACTGCCAACACCTCTGTCTTG GGCTACCACATTCCC | 1280 |
| CYR61 | NM_001554.3 | CYR61 | TGCTCATTC TTGAGGAG CAT | 129 | GTGGCTGCA TTAGTGTCC AT | 513 | CAGCACCCTTGGCAGTTT CGAAAT | 897 | 76 | TGCTCATTCTTGAGGAGCATTAAGGTAT TTCGAAACTGCCAAGGGTGCTGGTGCG GATGGACACTAATGCAGCCAC | 1281 |
| DAB2 | NM_001343.1 | DAB2 | TGGTGGGTC TAGGTGGTG TA | 130 | ACCAAAGAT GCTGTGTTC CA | 514 | CTGTCACACTCCCTCAGG CAGGAC | 898 | 67 | TGGTGGGTCTAGGTGGTGTAACTGTCA CACTCCCTCAGGCAGGACCATGGAACA CAGCATCTTTGGT | 1282 |
| DCC | NM_005215.1 | DCC | AAATGTCCT CCTCGACTG CT | 131 | TGAATGCCA TCTTTCTTCC A | 515 | ATCACTGGAACTCCTCG GTCGGAC | 899 | 75 | AAATGTCCTCCTCGACTGCTCCGCGGA GTCCGACCGAGGAGTTCCAGTGATCAA GTGGAAGAAAGATGGCATTCA | 1283 |
| DCC_exons 18-23 | X76132_ 18-23 | | GGTCACCGT TGGTGTCAT CA | 132 | GAGCGTCGG GTGCAAATC | 516 | CAGCCACGATGACCACT ACCAGCACT | 900 | 66 | GGTCACCGTTGGTGTCATCACAGTGCT GGTAGTGGTCATCGTGGCTGTGATTTGC ACCCGACGCTC | 1284 |
| DCC_exons 6-7 | X76132_6-7 | | ATGGAGAT GTGGTCATT CCTAGTG | 133 | CACCACCCC AAGTATCCG TAAG | 517 | TGCTTCCTCCCACTATCT GAAAATAA | 901 | 74 | ATGGAGATGTGGTCATTCCTAGTGATT ATTTTCAGATAGTGGGAGGAAGCAACT TACGGATACTTGGGGTGGTG | 1285 |
| DCK | NM_000788.1 | DCK | GCCGCCAC AAGACTAA GGAAT | 134 | CGATGTTCC CTTCGATGG AG | 518 | AGCTGCCCGTCTTTCTCA GCCAGC | 902 | 110 | GCCGCCACAAGACTAAGGAATGGCCAC CCCGCCCAAGAGAAGCTGCCCGTCTTT CTCAGCCAGCTCTGAGGGGACCCGCAT CAAGAAAATCTCCATCGAAGGGAACAT CG | 1286 |
| DICER1 | NM_177438.1 | DICER1 | TCCAATTCC AGCATCACT GT | 135 | GGCAGTGAA GGCGATAAA GT | 519 | AGAAAAGCTGTTTGTCT CCCCAGCA | 903 | 68 | TCCAATTCCAGCATCACTGTGGAGAAA AGCTGTTTGTCTCCCCAGCATACTTTAT CGCCTTCACTGCC | 1287 |
| DLC1 | NM_ 006094.3 | DLC1 | GATTCAGAC GAGGATGA GCC | 136 | CACCTCTTG CTGTCCCTTT G | 520 | AAAGTCCATTTGCCACT GATGGCA | 904 | 68 | GATTCAGACGAGGATGAGCCTTGTGCC ATCAGTGGCAAATGGACTTTCCAAAGG GACAGCAAGAGGTG | 1288 |
| DLL4 | NM_ 019074.2 | DLL4 | CACGGAGG TATAAGGC AGGAG | 137 | AGAAGGAAG GTCCAGCCG | 521 | CTACCTGGACATCCCTGC TCAGCC | 905 | 67 | CACGGAGGTATAAGGCAGGAGCCTACC TGGACATCCCTGCTCAGCCCCGCGGCT GGACCTTCCTTCT | 1289 |
| DR5 | NM _003842.2 | TNFRSF10 B | CTCTGAGAC AGTGCTTCG ATGACT........ | 138 | CCATGAGGC CCAACTTCC T | 522 | CAGACTTGGTGCCCTTTG ACTCC | 906 | 84 | CTCTGAGACAGTGCTTCGATGACTTTGC AGACTTGGTGCCCTTTGACTCCTGGGA GCCGCTCATGAGGAAGTTGGGCCTCAT GG | 1290 |
| DSP | NM_004415.1 | DSP | TGGCACTAC TGCATGATT GACA | 139 | CCTGCCGCA TTGTTTTCAG | 523 | CAGGGCCATGACAATCG CCAA | 907 | 73 | TGGCACTACTGCATGATTGACATAGAG AAGATCAGGGCCATGACAATCGCCAAG CTGAAAACAATGCGGCAGG | 1291 |
| DTYMK | NM_ 012145.1 | DTYMK | AAATCGCTG GGAACAAG TG | 140 | AATGCGTAT CTGTCCACG AC | 524 | CGCCCTGGCTCAACTTTT CCTTAA | 908 | 78 | AAATCGCTGGGAACAAGTGCCGTTAAT TAAGGAAAAGTTGAGCCAGGGCGTGAC CCTCGTCGTGGACAGATACGCATT | 1292 |
| DUSP1 | NM_ 004417.2 | DUSP1 | AGACATCA GCTCCTGGT TCA | 141 | GACAAACAC CCTTCCTCC AG | 525 | CGAGGCCATTGACTTCA TAGACTCCA | 909 | 76 | AGACATCAGCTCCTGGTTCAACGAGGC CATTGACTTCATAGACTCCATCAAGAA TGCTGGAGGAAGGGTGTTTGTC | 1293 |
| DUSP4 | NM_ 001394.4 | DUSP4 | TGGTGACG ATGGAGGA GC | 142 | CTCGTCCCG GTTCATCAG | 526 | TTGAGCACACTGCAGTC CATCTCC | 910 | 68 | TGGTGACGATGGAGGAGCTGCGGGAGA TGGACTGCAGTGTGCTCAAAGGCTGA TGAACCGGGACGAG | 1294 |
| E2F1 | NM_ 005225.1 | E2F1 | ACTCCCTCT ACCCTTGAG CA | 143 | CAGGCCTCA GTTCCTTCA GT ...... | 527 | CAGAAGAACAGCTCAGG GACCCCT | 911 | 75 | ACTCCCTCTACCCTTGAGCAAGGGCAG GGGTCCCTGAGCTGTTCTTCTGCCCCAT ACTGAAGGAACTGAGGCCTG | 1295 |
| EBRP | AF243433.1 | | CTGCTGGAT GACCTTCCT C | 144 | CCAACAGTA CAGCCAGTT GC | 528 | CTCACCAGAAGCCCCAA CCTCAAC.......... | 912 | 76 | CTGCTGGATGACCTTCCTCCCAGAGTG GCTCACCAGAAGCCCCAACCTCAACAC CAGCAACTGGCTGTACTGTTGG | 1296 |
| EDN1 endothelin | NM_001955.1 | EDN1 | TGCCACCTG GACATCATT TG | 145 | TGGACCTAG GGCTTCCAA GTC | 529 | CACTCCCGAGCACGTTG TTCCGT | 913 | 73 | TGCCACCTGGACATCATTTGGGTCAAC ACTCCCGAGCACGTTGTTCCGTATGGA CTTGGAAGCCCTAGGTCCA | 1297 |
| EDN2 | NM_001956.2 | EDN2 | CGACAAGG AGTGCGTCT ACTTCT | 146 | CAGGCCGTA AGGAGCTGT CT | 530 | CCACTTGGACATCATCTG GGTGAACACTC.......... | 914 | 79 | CGACAAGGAGTGCGTCTACTTCTGCCA CTTGGACATCATCTGGGTGAACACTCCT GAACAGACAGCTCCTTACGGCCTG | 1298 |
| EDNRA | NM_001957.1 | EDNRA | TTTCCTCAA ATTTGCCTC AAG | 147 | TTACACATC CAACCAGTG CC | 531 | CCTTTGCCTCAGGGCATC CTTTT | 915 | 76 | TTTCCTCAAATTTGCCTCAAGATGGAAA CCCTTTGCCTCAGGGCATCCTTTTGGCT GGCACTGGTTGGATGTGTAA | 1299 |
| EDNRB | NM_000115.1 | EDNRB | ACTGTGAAC TGCCTGGTG C | 148 | ACCACAGCA TGGGTGAGA G | 532 | TGCTACCTGCCCCTTTGT CATGTG.............................. | 916 | 72 | ACTGTGAACTGCCTGGTGCAGTGTCCA CATGACAAAGGGGCAGGTAGCACCCTC TCTCACCCATGCTGTGGT | 1300 |
| EEF1A1 | NM_001402.5 | EEF1A1 | CGAGTGGA GACTGGTGT TCTC | 149 | CCGTTGTAA CGTTGACTG GA...... | 533 | CAAAGGTGACCACCATA CCGGGTT | 917 | 67 | CGAGTGGAGACTGGTGTTCTCAAACCC GGTATGGTGGTCACCTTTGCTCCAGTCA ACGTTACAACGG | 1301 |
| EEF1A2 | NM_001958.2 | EEF1A2 | ATGGACTCC ACAGAGCC G | 150 | GGCGCTGAC TTCCTTGAC | 534 | CTCGTCGTAGCGCTTCTC GCTGTA | 918 | 66 | ATGGACTCCACAGAGCCGGCCTACAGC GAGAAGCGCTACGACGAGATCGTCAAG GAAGTCAGCGCC | 1302 |
| EFP | NM_005082.2 | TRIM25 | TTGAACAG AGCCTGACC AAG | 151 | TGTTGAGAT TCCTCGCAG TT | 535 | TGATGCTTTCTCCAGAAA CTCGAACTCA | 919 | 74 | TTGAACAGAGCCTGACCAAGAGGGATG AGTTCGAGTTTCTGGAGAAAGCATCAA AACTGCGAGGAATCTCAACA | 1303 |
| EGR1 | NM_ 001964.2 | EGR1 | GTCCCCGCT GCAGATCTC T | 152 | CTCCAGCTT AGGGTAGTT GTCCAT | 536 | CGGATCCTTTCCTCACTC GCCCA | 920 | 76 | GTCCCCGCTGCAGATCTCTGACCCGTTC GGATCCTTTCCTCACTCGCCCACCATGG ACAACTACCCTAAGCTGGAG | 1304 |
| EGR3 | NM_ 004430.2 | EGR3 | CCATGTGGA TGAATGAG GTG | 153 | TGCCTGAGÄ AGAGGTGAG GT | 537 | ACCCAGTCTCACCTTCTC CCCACC | 921 | 78 | CCATGTGGATGAATGAGGTGTCTCCTTT CCATACCCAGTCTCACCTTCTCCCCACC CTACCTCACCTCTTCTCAGGCA | 1305 |
| EIF4EBP1 | NM_004095.2 | EIF4EBP1 | GGCGGTGA AGAGTCAC AGT | 154 | TTGGTAGTG CTCCACACG AT | 538 | TGAGATGGACATTTAAA GCACCAGCC | 922 | 66 | GGCGGTGAAGAGTCACAGTTTGAGATG GACATTTAAAGCACCAGCCATCGTGTG GAGCACTACCAA | 1306 |
| ELF3 | NM_ 004433.2 | ELF3 | TCGAGGGC AAGAAGAG CAA | 155 | GATGAGGAT GTCCCGGAT GA | 539 | CGCCCAGAGGCACCCAC CTG | 923 | 71 | TCGAGGGCAAGAAGAGCAAGCACGCG CCCAGAGGCACCCACCTGTGGGAGTTC ATCCGGGACATCCTCATC | 1307 |
| EMP1 | NM_001423.1 | EMP1 | GCTAGTACT TTGATGCTC CCTTGAT | 156 | GAACAGCTG GAGGCCAAG TC | 540 | CCAGAGAGCCTCCCTGC AGCCA | 924 | 75 | GCTAGTACTTTGATGCTCCCTTGATGGG GTCCAGAGAGCCTCCCTGCAGCCACCA GACTTGGCCTCCAGCTGTTC | 1308 |
| ENO1 | NM_001428.2 | ENO1 | CAAGGCCG TGAACGAG AAGT | 157 | CGGTCACGG AGCCAATCT | 541 | CTGCAACTGCCTCCTGCT CAAAGTCA | 925 | 68 | CAAGGCCGTGAACGAGAAGTCCTGCAA CTGCCTCCTGCTCAAAGTCAACCAGATT GGCTCCGTGACCG | 1309 |
| EP300 | NM_001429.1 | EP300 | AGCCCCAG CAACTACA GTCT | 158 | TGTTCAAAG GTTGACCAT GC | 542 | CACTGACATCATGGCTG GCCTTG.............. | 926 | 75 | AGCCCCAGCAACTACAGTCTGGGATGC CAAGGCCAGCCATGATGTCAGTGGCCC AGCATGGTCAACCTTTGAACA | 1310 |
| EpCAM | NM_002354.1 | EPCAM | GGGCCCTCC AGAACAAT GAT...... | 159 | TGCACTGCT TGGCCTTAA AGA ..... | 543 | CCGCTCTCATCGCAGTCA GGATCAT | 927 | 75 | GGGCCCTCCAGAACAATGATGGGCTTT ATGATCCTGACTGCGATGAGAGCGGGC TCTTTAAGGCCAAGCAGTGCA | 1311 |
| EPHA2 | NM_004431.2 | EPHA2 | CGCCTGTTC ACCAAGATT GAC | 160 | GTGGCGTGC CTCGAAGTC | 544 | TGCGCCCGATGAGATCA CCG | 928 | 72 | CGCCTGTTCACCAAGATTGACACCATT GCGCCCGATGAGATCACCGTCAGCAGC GACTTCGAGGCACGCCAC | 1312 |
| EPHB2 | NM_ 004442.4 | EPHB2 | CAACCAGG CAGCTCCAT C | 161 | GTAATGCTG TCCACGGTG C | 545 | CACCTGATGCATGATGG ACACTGC | 929 | 66 | CAACCAGGCAGCTCCATCGGCAGTGTC CATCATGCATCAGGTGAGCCGCACCGT GGACAGCATTAC | 1313 |
| EPHB4 | NM_004444.3 | EPHB4 | TGAACGGG GTATCCTCC TTA | 162 | AGGTACCTC TCGGTCAGT GG | 546 | CGTCCCATTTGAGCCTGT CAATGT .................. | 930 | 77 | TGAACGGGGTATCCTCCTTAGCCACGG GGCCCGTCCCATTTGAGCCTGTCAATGT CACCACTGACCGAGAGGTACCT | 1314 |
| ER2 | NM_001437.1 | ESR2 | TGGTCCATC GCCAGTTAT CA | 163 | TGTTCTAGC GATCTTGCT TCACA | 547 | ATCTGTATGCGGAACCT CAAAAGAGTCCCT | 931 | 76 | TGGTCCATCGCCAGTTATCACATCTGTA TGCGGAACCTCAAAAGAGTCCCTGGTG TGAAGCAAGATCGCTAGAACA | 1315 |
| ERBB4 | NM_005235.1 | ERBB4 | TGGCTCTTA ATCAGTTTC GTTACCT | 164 | CAAGGCATA TCGATCCTC ATAAAGT | 548 | TGTCCCACGAATAATGC GTAAATTCTCCAG | 932 | 86 | TGGCTCTTAATCAGTTTCGTTACCTGCC TCTGGAGAATTTACGCATTATTCGTGGG ACAAAACTTTATGAGGATCGATATGCC TTG | 1316 |
| ERCC1 | NM_ 001983.1 | ERCC1 | GTCCAGGTG GATGTGAA AGA | 165 | CGGCCAGGA TACACATCT TA | 549 | CAGCAGGCCCTCAAGGA GCTG | 933 | 67 | GTCCAGGTGGATGTGAAAGATCCCCAG CAGGCCCTCAAGGAGCTGGCTAAGATG TGTATCCTGGCCG | 1317 |
| ERG | NM_ 004449.3 | ERG | CCAACACTA GGCTCCCCA | 166 | CCTCCGCCA GGTCTTTAG T | 550 | AGCCATATGCCTTCTCAT CTGGGC | 934 | 70 | CCAACACTAGGCTCCCACCAGCCATA TGCCTTCTCATCTGGGCACTTACTACTA AAGACCTGGCGGAGG | 1318 |
| ERRa | NM_ 004451.3 | ESRRA | GGCATTGA GCCTCTCTA CATGA | 167 | TCTCCGAGG AACCCTTTG G | 551 | AGAGCCGGCCAGCCCTG ACAG | 935 | 67 | GGCATTGAGCCTCTCTACATCAAGGCA GAGCCGGCCAGCCCTGACAGTCCAAAG GGTTCCTCGGAGA | 1319 |
| ESD | NM_001984.1 | ESD | GTCACTCCG CCACCGTAG | 168 | CTGTCCAAT TGCTGATTG CTT | 552 | TCGCCTACCATTTGGTGC AAGCAA | 936 | 66 | GTCACTCCGCCACCGTAGAATCGCCTA CCATTTGGTGCAAGCAAAAAGCAATCA GCAATTGGACAG | 1320 |
| ESPL1 | NM_012291.1 | ESPL1 | ACCCCCAG ACCGGATC AG | 169 | TGTAGGGCA GACTTCCTC AAACA | 553 | CTGGCCCTCATGTCCCCT TCACG | 937 | 70 | ACCCCCAGACCGGATCAGGCAAGCTGG CCCTCATGTCCCCTTCACGGTGTTTGAG GAAGTCTGCCCTACA | 1321 |
| ESRRG | NM_001438.1 | ESRRG | CCAGCACC ATTGTTGAA GAT | 170 | AGTCTCTTG GGCATCGAG TT | 554 | CCCCAGACCAAGTGTGA ATACATGCT | 938 | 67 | CCAGCACCATTGTTGAAGATCCCCAGA CCAAGTGTGAATACATGCTCAACTCGA TGCCCAAGAGACT | 1322 |
| EstR1 | NM_ 000125.1 | ESR1 | CGTGGTGCC CCTCTATGA C | 171 | GGCTAGTGG GCGCATGTA G | 555 | CTGGAGATGCTGGACGC CC | 939 | 68 | CGTGGTGCCCCTCTATGACCTGCTGCTG GAGATGCTGGACGCCCACCGCCTACAT GCGCCCACTAGCC | 1323 |
| ETV5 | NM_ 004454.1 | ETV5 | ACCATGTAT CGAGAGGG GC | 172 | TGACCAGGA ACTGCCACA G | 556 | TTACCAGAGGCGAGGTT CCCTTCA | 940 | 67 | ACCATGTATCGAGAGGGGCCCCCTTAC CAGAGGCGAGGTTCCCTTCAGCTGTGG CAGTTCCTGGTCA | 1324 |
| EZH2 | NM_004456.3 | EZH2 | TGGAAACA GCGAAGGA TACA | 173 | CACCGAACA CTCCCTAGT CC | 557 | TCCTGACTTCTGTGAGCT CATTGCG | 941 | 78 | TGGAAACAGCGAAGGATACAGCCTGTG CACATCCTGACTTCTGTGAGCTCATTGC GCGGGACTAGGGAGTGTTCGGTG | 1325 |
| F3 | NM_001993.2 | F3 | GTGAAGGA TGTGAAGC AGACGTA | 174 | AACCGGTGC TCTCCACAT TC | 558 | TGGCACGGGTCTTCTCCT ACC | 942 | 73 | GTGAAGGATGTGAAGCAGACGTACTTG GCACGGGTCTTCTCCTACCCGGCAGGG AATGTGGAGAGCACCGGTT | 1326 |
| FAR | NM004460.2 | FAP | CTGACCAG AACCACGG CT | 175 | GGAAGTGGG TCATGTGGG | 559 | CGGCCTGTCCACGAACC ACTTATA | 943 | 66 | CTGACCAGAACCACGGCTTATCCGGCC TGTCCACGAACCACTTATACACCCACA TGACCCACTTCC | 1327 |
| FASN | NM_004104.4 | FASN | GCCTCTTCC TGTTCGACG | 176 | GCTTTGCCC GGTAGCTCT | 560 | TCGCCCACCTACGTACTG GCCTAC | 944 | 66 | GCCTCTTCCTGTTCGACGGCTCGCCCAC CTACGTACTGGCCTACACCCAGAGCTA CCGGGCAAAGC | 1328 |
| FGFR2 isoform 1 | NM_000141.2 | FGFR2 | GAGGGACT GTTGGCATG CA | 177 | GAGTGAGAA TTCGATCCA AGTCTTC | 561 | TCCCAGAGACCAACGTT CAAGCAGTTG | 945 | 80 | GAGGGACTGTTGGCATGCAGTGCCCTC CCAGAGACCAACGTTCAAGCAGTTGGT AGAAGACTTGGATCGAATTCTCACTC | 1329 |
| FGFR4 | NM_002011.3 | FGFR4 | CTGGCTTAA GGATGGAC AGG | 178 | ACGAGACTC CAGTGCTGA TG | 562 | CCTTTCATGGGGAGAAC CGCATT | 946 | 81 | CTGGCTTAAGGATGGACAGGCCTTTCA TGGGGAGAACCGCATTGGAGGCATTCG GCTGCGCCATCAGCACTGGAGTCTCGT | 1330 |
| FHIT | NM_002012.1 | FHIT | CCAGTGGA GCGCTTCCA T | 179 | CTCTCTGGG TCGTCTGAA ACAA | 563 | TCGGCCACTTCATCAGG ACGCAG | 947 | 67 | CCAGTGGAGCGCTTCCATGACCTGCGT CCTGATGAAGTGGCCGATTTGTTTCAG ACGACCCAGAGAG | 1331 |
| FLOT2 | NM_ 004475.1 | FLOT2 | GACATCTGC GCTCCATCC | 180 | CAAACTGGT CCCGGTCCT | 564 | AATCTGCTCCACTGTCAG GGTCCC | 948 | 66 | GACATCTGCGCTCCATCCTCGGACCCT GACAGTGGAGCAGATTTATCAGGACCG GGACCAGTTTG | 1332 |
| FN1 | NM_002026.2 | FN1 | GGAAGTGA CAGACGTG AAGGT | 181 | ACACGGTAG CCGGTCACT | 565 | ACTCTCAGGCGGTGTCC ACATGAT | 949 | 69 | GGAAGTGACAGACGTGAAGGTCACCAT CATGTGGACACCGCCTGAGAGTGCAGT GACCGGCTACCGTGT | 1333 |
| FOS | NM_ 005252.2 | FOS | CGAGCCCTT TGATGACTT CCT | 182 | GGAGCGGGC TGTCTCAGA | 566 | TCCCAGCATCATCCAGG CCCAG | 950 | 67 | CGAGCCCTTTGATGACTTCCTGTTCCCA GCATCATCCAGGCCCAGTGGCTCTGAG ACAGCCCGCTCC | 1334 |
| FOXC2 | NM_005251.1 | FOXC2 | GAGAACAA GCAGGGCT GG | 183 | CTTGACGAA GCACTCGTT GA | 567 | AGAACAGCATCCGCCAC AACCTCT | 951 | 66 | GAGAACAAGCAGGGCTGGCAGAACAG CATCCGCCACAACCTCTCGCTCAACGA GTGCTTCGTCAAG | 1335 |
| FOXO3A | NM_ 001455.1 | FOXO3 | TGAAGTCCA GGACGATG ATG | 184 | ACGGCTTGC TTACTGAAG GT | 568 | CTCTACAGCAGCTCAGC CAGCCTG | 952 | 83 | TGAAGTCCAGGACGATGATGCGCCTCT CTCGCCCATGCTCTACAGCAGCTCAGC CAGCCTGTCACCTTCAGTAAGCAAGCC GT | 1336 |
| FOXP1 | NM_ 032682.3 | FOXP1 | CGACAGAG CTTGTGCAC CT | 185 | GGTCGTCCA TTGGAATCC T | 569 | CAGACCAAGCCTTTGCC CAGAATT | 953 | 70 | CGACAGAGCTTGTGCACCTAAGCTGCA GACCAAGCCTTTGCCCAGAATTTAAGG ATTCCAATGGACGACC | 1337 |
| FOXP3 | NM_ 014009.2 | FOXP3 | CTGTTTGCT GTCCGGAG G | 186 | GTGGAGGAA CTCTGGGAA TG | 570 | TGTTTCCATGGCTACCCC ACAGGT.................. | 954 | 66 | CTGTTTGCTGTCCGGAGGCACCTGTGG GGTAGCCATGGAAACAGCACATTCCCA GAGTTCCTCCAC | 1338 |
| FSCN1 | NM_003088.1 | FSCN1 | CCAGCTGCT ACTTTGACA TCGA | 187 | GGTCACAAA CTTGCCATT GGA | 571 | TGACCGGCGCATCACAC TGAGG | 955 | 74 | CCAGCTGCTACTTTGACATCGAGTGGC GTGACCGGCGCATCACACTGAGGGCGT CCAATGGCAAGTTTGTGACC | 1339 |
| FUS | NM_004960.1 | FUS | GGATAATTC AGACAACA ACACCATCT | 188 | TGAAGTAAT CAGCCACAG ACTCAAT | 572 | TCAATTGTAACATTCTCA CCCAGGCCTTG | 956 | 80 | GGATAATTCAGACAACAACACCATCTT TGTGCAAGGCCTGGGTGAGAATGTTAC AATTGAGTCTGTGGCTGATTACTTCA | 1340 |
| FYN | NM_002037.3 | FYN | GAAGCGCA GATCATGA AGAA | 189 | CTCCTCAGA CACCACTGC AT | 573 | CTGAAGCACGACAAGCT GGTCCAG | 957 | 69 | GAAGCGCAGATCATGAAGAAGCTGAA GCACGACAAGCTGGTCCAGCTCTATGC AGTGGTGTCTGAGGAG | 1341 |
| G-Catenin | NM_002230.1 | JUP | TCAGCAGC AAGGGCAT CAT | 190 | GGTGGTTTT CTTGAGCGT GTACT | 574 | CGCCCGCAGGCCTCATC CT | 958 | 68 | TCAGCAGCAAGGGCATCATGGAGGAGG ATGAGGCCTGCGGGCGCCAGTACACGC TCAAGAAAACCACC | 1342 |
| GAB2 | NM_ 012296.2 | GAB2 | TGTTTGGAG GGAAGGGC T | 191 | GAAGATAGC TGAGGGCTG TGAC | 575 | TGAGCCAGATTCCACAC CTCACGT........ | 959 | 74 | TGTTTGGAGGGAAGGGCTGGGGCTCTG AGCCAGATTCCACACCTCACGTTCAGT CACAGCCCTCAGCTATCTTC | 1343 |
| GADD45 | NM_001924.2 | GADD45A | GTGCTGGTG ACGAATCC A | 192 | CCCGGCAAA AACAAATAA GT | 576 | TTCATCTCAATGGAAGG ATCCTGCC.................. | 960 | 73 | GTGCTGGTGACGAATCCACATTCATCTC AATGGAAGGATCCTGCCTTAAGTCAAC TTATTTGTTTTTGCCGGG | 1344 |
| GADD45B | NM_015675.1 | GADD45B | ACCCTCGAC AAGACCAC ACT.......... | 193 | TGGGAGTTC ATGGGTACA GA | 577 | AACTTCAGCCCCAGCTC CCAAGTC | 961 | 70 | ACCCTCGACAAGACCACACTTTGGGAC TTGGGAGCTGGGGCTGAAGTTGCTCTG TACCCATGAACTCCCA | 1345 |
| GAPDH | NM_ 002046.2 | GAPDH | ATTCCACCC ATGGCAAA TTC.......... | 194 | GATGGGATT TCCATTGAT GACA | 578 | CCGTTCTCAGCCTTGACG GTGC.......... | 962 | 74 | ATTCCACCCATGGCAAATTCCATGGCA CCGTCAAGGCTGAGAACGGGAAGCTTG TCATCAATGGAAATCCCATC | 1346 |
| GATA3 | NM_00205 1.1 | GATA3 | CAAAGGAG CTCACTGTG GTGTCT | 195 | GAGTCAGAA TGGCTTATT CACAGATG | 579 | TGTTCCAACCACTGAATC TGGACC | 963 | 75 | CAAAGGAGCTCACTGTGGTGTCTGTGT TCCAACCACTGAATCTGGACCCCATCT GTGAATAAGCCATTCTGACTC | 1347 |
| GBP1 | NM_002053.1 | GBP1 | TTGGGAAAT ATTTGGGCA TT | 196 | AGAAGCTAG GGTGGTTGT CC. | 580 | TTGGGACATTGTAGACTT GGCCAGAC | 964 | 73 | TTGGGAAATATTTGGGCATTGGTCTGG CCAAGTCTACAATGTCCCAATATCAAG GACAACCACCCTAGCTTCT | 1348 |
| GBP2 | NM_ 004120.2 | GBP2 | GCATGGGA ACCATCAAC CA | 197 | TGAGGAGTT TGCCTTGAT TCG | 581 | CCATGGACCAACTTCAC TATGTGACAGAGC | 965 | 83 | GCATGGGAACCATCAACCAGCAGGCCA TGGACCAACTTCACTATGTGACAGAGC TGACAGATCGAATCAAGGCAAACTCCT CA | 1349 |
| GCLM | NM_ 002061.1 | GCLM | TGTAGAATC AAACTCTTC ATCATCAAC TAG | 198 | CACAGAATC CAGCTGTGC AACT | 582 | TGCAGTTGACATGGCCT GTTCAGTCC | 966 | 85 | TGTAGAATCAAACTCTTCATCATCAACT AGAAGTGCAGTTGACATGGCCTGTTCA GTCCTTGGAGTTGCACAGCTGGATTCTG TG | 1350 |
| GDF15 | NM_ 004864.1 | GDF15 | CGCTCCAGA CCTATGATG ACT | 199 | ACAGTGGAA GGACCAGGA CT | 583 | TGTTAGCCAAAGACTGC CACTGCA | 967 | 72 | CGCTCCAGACCTATGATGACTTGTTAGC CAAAGACTGCCACTGCATATGAGCAGT CCTGGTCCTTCCACTGT | 1351 |
| GH1 | NM_000515.3 | GH1 | GATCCCAA GGCCCAACT C | 200 | AGCCATTGC AGCTAGGTG AG | 584 | TGTCCACAGGACCCTGA GTGGTTC | 968 | 66 | GATCCCAAGGCCCAACTCCCCGAACCA CTCAGGGTCCTGTGGACAGCTCACCTA GCTGCAATGGCT | 1352 |
| GJA1 | NM_ 000165.2 | GJA1 | GTTCACTGG GGGTGTATG G | 201 | AAATACCAA CATGCACCT CTCTT | 585 | ATCCCCTCCCTCTCCACC CATCTA | 969 | 68 | GTTCACTGGGGGTGTATGGGGTAGATG GGTGGAGAGGGAGGGGATAAGAGAGG TGCATGTTGGTATTT | 1353 |
| GJB2 | NM_004004.3 | GJB2 | TGTCATGTA CGACGGCTT CT | 202 | AGTCCACAG TGTTGGGAC AA | 586 | AGGCGTTGCACTTCACC AGCC | 970 | 74 | TGTCATGTACGACGGCTTCTCCATGCAG CGGCTGGTGAAGTGCAACGCCTGGCCT TGTCCCAACACTGTGGACT | 1354 |
| GMNN | NM_015895.3 | GMNN | GTTCGCTAC GAGGATTG AGC | 203 | TGCGTACCC ACTTCCTGC | 587 | CCTCTTGCCCACTTACTG GGTGGA | 971 | 67 | GTTCGCTACGAGGATTGAGCGTCTCCA CCCAGTAAGTGGGCAAGAGGCGGCAG GAAGTGGGTACGCA | 1355 |
| GNAZ | NM_ 002073.2 | GNAZ | TTCTGGACC TGGGACCTT AG | 204 | AAAGAGCTG TGAGTGGCT GG | 588 | CCGGGTGACAGCACTAA CCAGACC | 972 | 68 | TTCTGGACCTGGGACCTTAGGAGCCGG GTGACAGCACTAACCAGACCTCCAGCC ACTCACAGCTCTTT | 1356 |
| GPR30 | NM_ 001505.1 | GPER | CGTGCCTCT ACACCATCT TC | 205 | ATGTTCACC ACCAGGATC AG | 589 | CTCTTCCCCATCGGCTTT GTGG | 973 | 70 | CGTGCCTCTACACCATCTTCCTCTTCCC CATCGGCTTTGTGGGCAACATCCTGATC CTGGTGGTGAACAT | 1357 |
| GPS1 | NM_004127.4 | GPS1 | AGTACAAG CAGGCTGCC AAG | 206 | GCAGCTCAG GGAAGTCAC A | 590 | CCTCCTGCTGGCTTCCTT TGATCA | 974 | 66 | AGTACAAGCAGGCTGCCAAGTGCCTCC TGCTGGCTTCCTTTGATCACTGTGACTT CCCTGAGCTGC | 1358 |
| GPX1 | NM_000581.2 | GPX1 | GCTTATGAC CGACCCCA A | 207 | AAAGTTCCA GGCAACATC GT | 591 | CTCATCACCTGGTCTCCG GTGTGT | 975 | 67 | GCTTATGACCGACCCCAAGCTCATCAC CTGGTCTCCGGTGTGTCGCAACGATGTT GCCTGGAACTTT | 1359 |
| GPX2 | NM_ 002083.1 | GPX2 | CACACAGA TCTCCTACT CCATCCA | 208 | GGTCCAGCA GTGTCTCCT GAA | 592 | CATGCTGCATCCTAAGG CTCCTCAGG | 976 | 75 | CACACAGATCTCCTACTCCATCCAGTCC TGAGGAGCCTTAGGATGCAGCATGCCT TCAGGAGACACTGCTGGACC | 1360 |
| GPX4 | NM_002085.1 | GPX4 | CTGAGTGTG GTTTGCGGA T | 209 | TACTCCCTG GCTCCTGCT T | 593 | CTGGCCTTCCCGTGTAAC CAGTTC | 977 | 66 | CTGAGTGTGGTTTGCGGATCCTGGCCTT CCCGTGTAACCAGTTCGGGAAGCAGGA GCCAGGGAGTA | 1361 |
| GRB7 | NM_005310.1 | GRB7 | CCATCTGCA TCCATCTTG TT | 210 | GGCCACCAG GGTATTATC TG | 594 | CTCCCCACCCTTGAGAA GTGCCT | 978 | 67 | CCATCTGCATCCATCTTGTTTGGGCTCC CCACCCTTGAGAAGTGCCTCAGATAAT ACCCTGGTGGCC | 1362 |
| GREB1 variant a | NM_014668.2 | GREB1 | CAGATGAC AATGGCCA CAAT | 211 | GAAGCCTTT CTTTCCACA GC | 595 | CACAATTCCCAGAGAAA CCAAGAAGAGC | 979 | 71 | CAGATGACAATGGCCACAATGCTCTTC TTGGTTTCTCTGGGAATTGTGTTGGCTG TGGAAAGAAAGGCTTC | 1363 |
| GREB1 variant b | NM_033090.1 | GREB1 | TGCTTAGGT GCGGTAAA ACCA | 212 | CAAGAGCCT GAATGCGTC AGT | 596 | ACCACGCGAACGGTGCA TCG............................... | 980 | 73 | TGCTTAGGTGCGGTAAAACCAGCGCTT GTCCGATGCACCGTTCGCGTGGTAAAC TGACGCATTCAGGCTCTTG | 1364 |
| GREB1 variant c | NM_148903.1 | GREB1 | CCCCAGGC ACCAGCTTT A | 213 | ACTTCGGCT GTGTGTTAT ATGCA | 597 | TCCCCGAGCCCAGCAGG ACA .............................. | 981 | 64 | CCCCAGGCACCAGCTTTACTCCCCGAG CCCAGCAGGACATCTGCATATAACACA CAGCCGAAGT | 1365 |
| GRN | NM_002087.1 | GRN | TGCCCCCAA GACACTGTG T | 214 | GAGGTCCGT GGTAGCGTT CTC | 598 | TGACCTGATCCAGAGTA AGTGCCTCTCCA | 982 | 72 | TGCCCCCAAGACACTGTGTGTGACCTG ATCCAGAGTAAGTGCCTCTCCAAGGAG AACGCTACCACGGACCTC | 1366 |
| GSTM1 | NM_000561.1 | GSTM1 | AAGCTATG AGGAAAAG AAGTACAC GAT | 215 | GGCCCAGCT TGAATTTTTC A | 599 | TCAGCCACTGGCTTCTGT CATAATCAGGAG | 983 | 86 | AAGCTATGAGGAAAAGAAGTACACGAT GGGGGACGCTCCTGATTATGACAGAAG CCAGTGGCTGAATGAAAAATTCAAGCT GGGCC | 1367 |
| GSTM2 gene | NM_000848gen e | | CTGGGCTGT GAGGCTGA GA........ | 216 | GCGAATCTG CTCCTTTTCT GA | 600 | CCCGCCTACCCTCGTAA AGCAGATTCA | 984 | 71 | CTGGGCTGTGAGGCTGAGAGTGAATCT GCTTTACGAGGGTAGGCGGGGAATCAG AAAAGGAGCAGATTCGC | 1368 |
| GSTM2 | NM_ 000848.2 | GSTM2 | CTGCAGGC ACTCCCTGA AAT | 217 | CCAAGAAAC CATGGCTGC TT | 601 | CTGAAGCTCTACTCACA GTTTCTGGG | 985 | 68 | CTGCAGGCACTCCCTGAAATGCTGAAG CTCTACTCACAGTTTCTGGGGAAGCAG CCATGGTTTCTTGG | 1369 |
| GSTM3 | NM_000849.3 | GSTM3 | CAATGCCAT CTTGCGCTA CAT | 218 | GTCCACTCG AATCTTTTCT TCTTCA | 602 | CTCGCAAGCACAACATG TGTGGTGAGA | 986 | 76 | CAATGCCATCTTGCGCTACATCGCTCGC AAGCACAACATGTGTGGTGAGACTGAA GAAGAAAAGATTCGAGTGGAC | 1370 |
| GSTT1 | NM_000853.1 | GSTT1 | CACCATCCC CACCCTGTC T | 219 | GGCCTCAGT GTGCATCAT TCT | 603 | CACAGCCGCCTGAAAGC CACAAT | 987 | 66 | CACCATCCCCACCCTGTCTTCCACAGCC GCCTGAAAGCCACAATGAGAATGATGC ACACTGAGGCC | 1371 |
| GUS | NM_000181.1 | GUSB | CCCACTCAG TAGCCAAGT CA | 220 | CACGCAGGT GGTATCAGT CT | 604 | TCAAGTAAACGGGCTGT TTTCCAAACA | 988 | 73 | CCCACTCAGTAGCCAAGTCACAATGTT TGGAAAACAGCCCGTTTACTTGAGCAA GACTGATACCACCTGCGTG | 1372 |
| H3F3A | NM_002107.3 | H3F3A | CCAAACGT GTAACAATT ATGCC | 221 | TCTTAAGCA CGTTCTCCA CG | 605 | AAAGACATCCAGCTAGC ACGCCG | 989 | 70 | CCAAACGTGTAACAATTATGCCAAAAG ACATCCAGCTAGCACGCCGCATACGTG GAGAACGTGCTTAAGA | 1373 |
| HDAC1 | NM_004964.2 | HDAC1 | CAAGTACC ACAGCGAT GACTACATT AA | 222 | GCTTGCTGT ACTCCGACA TGTT | 606 | TTCTTGCGCTCCATCCGT CCAGA | 990 | 74 | CAAGTACCACAGCGATGACTACATTAA ATTCTTGCGCTCCATCCGTCCAGATAAC ATGTCGGAGTACAGCAAGC | 1374 |
| HDAC6 | NM_006044.2 | HDAC6 | TCCTGTGCT CTGGAAGC C | 223 | CTCCACGGT CTCAGTTGA TCT | 607 | CAAGAACCTCCCAGAAG GGCTCAA | 991 | 66 | TCCTGTGCTCTGGAAGCCCTTGAGCCCT TCTGGGAGGTTCTTGTGAGATCAACTG AGACCGTGGAG | 1375 |
| HER2 | NM_004448.1 | ERBB2 | CGGTGTGA GAAGTGCA GCAA | 224 | CCTCTCGCA AGTGCTCCA T | 608 | CCAGACCATAGCACACT CGGGCAC | 992 | 70 | CGGTGTGAGAAGTGCAGCAAGCCCTGT GCCCGAGTGTGCTATGGTCTGGGCATG GAGCACTTGCGAGAGG | 1376 |
| HES1 | NM_ 005524.2 | HES1 | GAAAGATA GCTCGCGGC A | 225 | GGAGGTGCT TCACTGTCA TTT | 609 | CAGAATGTCCGCCTTCTC CAGCTT | 993 | 68 | GAAAGATAGCTCGCGGCATTCCAAGCT GGAGAAGGCGGACATTCTGGAAATGAC AGTGAAGCACCTCC | 1377 |
| HGFAC | NM_001528.2 | HGFAC | CAGGACAC AAGTGCCA GATT | 226 | GCAGGGAGC TGGAGTAGC | 610 | CGCTCACGTTCTCATCCA AGTGG | 994 | 72 | CAGGACACAAGTGCCAGATTGCGGGCT GGGGCCACTTGGATGAGAACGTGAGCG GCTACTCCAGCTCCCTGC | 1378 |
| HLA-DPB1 | NM_002121.4 | HLA-DPB1 | TCCATGATG GTTCTGCAG GTT | 227 | TGAGCAGCA CCATCAGTA ACG | 611 | CCCCGGACAGTGGCTCT GACG | 995 | 73 | TCCATGATGGTTCTGCAGGTTTCTGCGG CCCCCCGGACAGTGGCTCTGACGGCGT TACTGATGGTGCTGCTCA | 1379 |
| HMGB1 | NM_002128.3 | HMGB1 | TGGCCTGTC CATTGGTGA T | 228 | GCTTGTCAT CTGCAGCAG TGTT | 612 | TTCCACATCTCTCCCAGT TTCTTCGCAA | 996 | 71 | TGGCCTGTCCATTGGTGATGTTGCGAA GAAACTGGGAGAGATGTGGAATAACAC TGCTGCAGATGACAAGC | 1380 |
| HNF3A | NM_004496.1 | FOXA1 | TCCAGGATG TTAGGAACT GTGAAG | 229 | GCGTGTCTG CGTAGTAGC TGTT | 613 | AGTCGCTGGTTTCATGCC CTTCCA...... | 997 | 73 | TCCAGGATGTTAGGAACTGTGAAGATG GAAGGGCATGAAACCAGCGACTGGAA CAGCTACTACGCAGACACGC | 1381 |
| HNRPAB | NM_ 004499.3 | HNRNPAB | AGCAGGAG CGACCAACT GA | 230 | GTTTGCCAA GTTAAATTT GGTACATAA T | 614 | CTCCATATCCAAACAAA GCATGTGTGCG | 998 | 84 | AGCAGGAGCGACCAACTGATCGCACAC ATGCTTTGTTTGGATATGGAGTGAACA CAATTATGTACCAAATTTAACTTGGCA AAC | 1382 |
| HNRPC | NM_ 004500.3 | HNRNPC | GCAGCAGT CGGCTTCTC T | 231 | GGGAGGGAG AAGAGATTC GAT | 615 | AGTCTCCTACTCCCGGGT TCTGCG | 999 | 68 | GCAGCAGTCGGCTTCTCTACGCAGAAC CCGGGAGTAGGAGACTCAGAATCGAAT CTCTTCTCCCTCCC | 1383 |
| HoxA1 | NM_005522.3 | HOXA1 | AGTGACAG ATGGACAA TGCAAGA | 232 | CCGAGTCGC CACTGCTAA GT | 616 | TGAACTCCTTCCTGGAAT ACCCCA | 1000 | 69 | AGTGACAGATGGACAATGCAAGAATGA ACTCCTTCCTGGAATACCCCATACTTAG CAGTGGCGACTCGG | 1384 |
| HoxA5 | NM_019102.2 | HOXA5 | TCCCTTGTG TTCCTTCTG TGAA | 233 | GGCAATAAA CAGGCTCAT GATTAA | 617 | AGCCCTGTTCTCGTTGCC CTAATTCATC | 1001 | 78 | TCCCTTGTGTTCCTTCTGTGAAGAAGCC CTGTTCTCGTTGCCCTAATTCATCTTTT AATCATGAGCCTGTTTATTGCC | 1385 |
| HOXB13 | NM_ 006361.2 | IIOXB13 | CGTGCCTTA TGGTTACTT TGG | 234 | CACAGGGTT TCAGCGAGC | 618 | ACACTCGGCAGGAGTAG TACCCGC | 1002 | 71 | CGTGCCTTATGGTTACTTTGGAGGCGG GTACTACTCCTGCCGAGTGTCCCGGAG CTCGCTGAAACCCTGTG | 1386 |
| HOXB7 | NM_004502.2 | HOXB7 | CAGCCTCAA GTTCGGTTT TC.......... | 235 | GTTGGAAGC AAACGCACA | 619 | ACCGGAGCCTTCCCAGA ACAAACT | 1003 | 68 | CAGCCTCAAGTTCGGTTTTCGCTACCGG AGCCTTCCCAGAACAAACTTCTTGTGC GTTTGCTTCCAAC | 1387 |
| HSD17B1 | NM_ 000413.1 | HSD17B1 | CTGGACCGC ACGGACAT C.......... | 236 | CGCCTCGCG AAAGACTTG | 620 | ACCGCTTCTACCAATACC TCGCCCA | 1004 | 78 | CTGGACCGCACGGACATCCACACCTTC CACCGCTTCTACCAATACCTCGCCCACA GCAAGCAAGTCTTTCGCGAGGCG | 1388 |
| HSD17B2 | NM_ 002153.1 | HSD17B2 | GCTTTCCAA GTGGGGAA TTA.......... | 237 | TGCCTGCGA TATTTGTTA GG | 621 | AGTTGCTTCCATCCAACC TGGAGG | 1005 | 68 | GCTTTCCAAGTGGGGAATTAAAGTTGC TTCCATCCAACCTGGAGGCTTCCTAACA AATATCGCAGGCA | 1389 |
| HSHIN1 | NM_ 017493.3 | OTUD4 | CAGTCTCGC CATGTTGAA GT | 238 | ATAAACGCT TCAAATTTC TCTCTG | 622 | CAGAATGGCCTGTATTC ACTATCTTCGAGA | 1006 | 77 | CAGTCTCGCCATGTTGAAGTCAGAATG GCCTGTATTCACTATCTTCGAGAGAAC AGAGAGAAATTTGAAGCGTTTAT | 1390 |
| HSPA1A | NM_005345.4 | HSPA1A | CTGCTGCGA CAGTCCACT A | 239 | CAGGTTCGC TCTGGGAAG | 623 | AGAGTGACTCCCGTTGT CCCAAGG | 1007 | 70 | CTGCTGCGACAGTCCACTACCTTTTTCG AGAGTGACTCCCGTTGTCCCAAGGCTT CCCAGAGCGAACCTG | 1391 |
| HSPA1B | NM_ 005346.3 | HSPA1B | GGTCCGCTT CGTCTTTCG A | 240 | GCACAGGTT CGCTCTGGA A | 624 | TGACTCCCGCGGTCCCA AGG | 1008 | 63 | GGTCCGCTTCGTCTTTCGAGAGTGACTC CCGCGGTCCCAAGGCTTTCCAGAGCGA ACCTGTGC | 1392 |
| HSPA4 | NM_002154.3 | HSPA4 | TTCAGTGTG TCCAGTGCA TC | 241 | ATCTGTTTCC ATTGGCTCC T | 625 | CATTTTCCTCAGACTTGT GAACCTCCACT | 1009 | 72 | TTCAGTGTGTCCAGTGCATCTTTAGTGG AGGTTCACAAGTCTGAGGAAAATGAGG AGCCAATGGAAACAGAT | 1393 |
| HSPA5 | NM_005347.2 | HSPA5 | GGCTAGTA GAACTGGA TCCCAACA | 242 | GGTCTGCCC AAATGCTTT TC | 626 | TAATTAGACCTAGGCCT CAGCTGCACTGCC | 1010 | 84 | GGCTAGTAGAACTGGATCCCAACACCA AACTCTTAATTAGACCTAGGCCTCAGCT GCACTGCCCGAAAAGCATTTGGGCAGA CC | 1394 |
| HSPA8 | NM_006597.3 | IISPA8 | CCTCCCTCT GGTGGTGCT T.......... | 243 | GCTACATCT ACACTTGGT TGGCTTAA | 627 | CTCAGGGCCCACCATTG AAGAGGTTG | 1011 | 73 | CCTCCCTCTGGTGGTGCTTCCTCAGGGC CCACCATTGAAGAGGTTGATTAAGCCA ACCAAGTGTAGATGTAGC | 1395 |
| HSPB1 | NM_ 001540.2 | HSPB1 | CCGACTGG AGGAGCAT AAA | 244 | ATGCTGGCT GACTCTGCT C | 628 | CGCACTTTTCTGAGCAG ACGTCCA | 1012 | 84 | CCGACTGGAGGAGCATAAAAGCGCAGC CGAGCCCAGCGCCCCGCACTTTTCTGA GCAGACGTCCAGAGCAGAGTCAGCCAG CAT | 1396 |
| IBSP | NM_ 004967.2 | IBSP | GAATACCA CACTTTCTG CTACAACAC T | 245 | GGATTGCAG CTAACCCTG TATACC | 629 | CCAGGCGTGGCGTCCTC TCCATA.............................. | 1013 | 83 | GAATACCACACTTTCTGCTACAACACT GGGCTATGGAGAGGACGCCACGCCTGG CACAGGGTATACAGGGTTAGCTGCAAT CC | 1397 |
| ICAM1 | NM_000201.1 | ICAM1 | GCAGACAG TGACCATCT ACAGCTT | 246 | CTTCTGAGA CCTCTGGCT TCGT | 630 | CCGGCGCCCAACGTGAT TCT | 1014 | 68 | GCAGACAGTGACCATCTACAGCTTTCC GGCGCCCAACGTGATTCTGACGAAGCC AGAGGTCTCAGAAG | 1398 |
| ID1 | NM_002165.1 | ID1 | AGAACCGC AAGGTGAG CAA | 247 | TCCAACTGA AGGTCCCTG ATG | 631 | TGGAGATTCTCCAGCAC GTCATCGAC | 1015 | 70 | AGAACCGCAAGGTGAGCAAGGTGGAG ATTCTCCAGCACGTCATCGACTACATCA GGGACCTTCAGTTGGA | 1399 |
| ID4 | NM_ 001546.2 | ID4 | TGGCCTGGC TCTTAATTT G | 248 | TGCAATCAT GCAAGACCA C | 632 | CTTTTGTTTTGCCCAGTA TAGACTCGGAAG | 1016 | 83 | TGGCCTGGCTCTTAATTTGCTTTTGTTTT GCCCAGTATAGACTCGGAAGTAACAGT TATAGCTAGTGGTCTTGCATGATTGCA | 1400 |
| IDH2 | NM_ 002168.2 | IDH2 | GGTGGAGA GTGGAGCC ATGA | 249 | GCTCGTTCA GCTTCACAT TGC | 633 | CCGTGAATGCAGCCCGC CAG | 1017 | 74 | GGTGGAGAGTGGAGCCATGACCAAGG ACCTGGCGGGCTGCATTCACGGCCTCA GCAATGTGAAGCTGAACGAGC | 1401 |
| IGF1R | NM_ 000875.2 | IGF1R | GCATGGTA GCCGAAGA TTTCA | 250 | TTTCCGGTA ATAGTCTGT CTCATAGAT ATC | 634 | CGCGTCATACCAAAATC TCCGATTTTGA | 1018 | 83 | GCATGGTAGCCGAAGATTTCACAGTCA AAATCGGAGATTTTGGTATGACGCGAG ATATCTATGAGACAGACTATTACCGGA AA | 1402 |
| IGF2 | NM_000612.2 | IGF2 | CCGTGCTTC CGGACAAC TT | 251 | TGGACTGCT TCCAGGTGT CA | 635 | TACCCCGTGGGCAAGTT CTTCCAA | 1019 | 72 | CCGTGCTTCCGGACAACTTCCCCAGAT ACCCCGTGGGCAAGTTCTTCCAATATG ACACCTGGAAGCAGTCCA | 1403 |
| IGFBP6 | NM_003178.1 | IGFBP6 | TGAACCGC AGAGACCA ACAG | 252 | GTCTTGGAC ACCCGCAGA AT | 636 | ATCCAGGCACCTCTACC ACGCCCTC | 1020 | 77 | TGAACCGCAGAGACCAACAGAGGAATC CAGGCACCTCTACCACGCCCTCCCAGC CCAATTCTGCGGGTGTCCAAGAC | 1404 |
| IGFBP7 | NM_ 001553.1 | IGEBP7 | GGGTCACTA TGGAGTTCA AAGGA | 253 | GGGTCTGAA TGGCCAGGT T | 637 | CCCGGTCACCAGGCAGG AGTTCT | 1021 | 68 | GGGTCACTATGGAGTTCAAAGGACAGA ACTCCTGCCTGGTGACCGGGACAACCT GGCCATTCAGACCC | 1405 |
| IKBKE | NM_ 014002.2 | IKBKE | GCCTCCCAT AGCTCCTTA CC | 254 | CAGAGCTCT TGCATGTGG AG | 638 | CAGCCCTACACGAAAGG ACCTGCT | 1022 | 66 | GCCTCCCATAGCTCCTTACCCCAGCCCT ACACGAAAGGACCTGCTTCTCCACATG CAAGAGCTCTG | 1406 |
| IL-8 | NM_000584.2 | IL8 | AAGGAACC ATCTCACTG TGTGTAAAC | 255 | ATCAGGAAG GCTGCCAAG AG | 639 | TGACTTCCAAGCTGGCC GTGGC | 1023 | 70 | AAGGAACCATCTCACTGTGTGTAAACA TGACTTCCAAGCTGGCCGTGGCTCTCTT GGCAGCCTTCCTGAT | 1407 |
| IL10 | NM_000572.1 | IL10 | GGCGCTGTC ATCGATTTC TT | 256 | TGGAGCTTA TTAAAGGCA TTCTTCA | 640 | CTGCTCCACGGCCTTGCT CTTG | 1024 | 79 | GGCGCTGTCATCGATTTCTTCCCTGTGA AAACAAGAGCAAGGCCGTGGAGCAGG TGAAGAATGCCTTTAATAAGCTCCA | 1408 |
| IL11 | NM_000641.2 | IL11 | TGGAAGGTT CCACAAGTC AC | 257 | TCTTGACCTT GCAGCTTTG T | 641 | CCTGTGATCAACAGTAC CCGTATGGG | 1025 | 66 | TGGAAGGTTCCACAAGTCACCCTGTGA TCAACAGTACCCGTATGGGACAAAGCT GCAAGGTCAAGA | 1409 |
| IL17RB | NM_ 018725.2 | IL17RB | ACCCTCTGG TGGTAAATG GA | 258 | GGCCCCAAT GAAATAGAC TG | 642 | TCGGCTTCCCTGTAGAGC TGAACA | 1026 | 76 | ACCCTCTGGTGGTAAATGGACATTTTCC TACATCGGCTTCCCTGTAGAGCTGAAC ACAGTCTATTTCATTGGGGCC | 1410 |
| IL6ST | NM_002184.2 | IL6ST | GGCCTAATG TTCCAGATC CT | 259 | AAAATTGTG CCTTGGAGG AG | 643 | CATATTGCCCAGTGGTC ACCTCACA | 1027 | 74 | GGCCTAATGTTCCAGATCCTTCAAAGA GTCATATTGCCCAGTGGTCACCTCACAC TCCTCCAAGGCACAATTTT | 1411 |
| ING1 | NM_ 005537.2 | ING1 | ACTTTCCTG CGAGGTCA GTC | 260 | AACTCCGAG TGGTGATCC A | 644 | ATTCAAAACAGAGCCCC CAAAGCC | 1028 | 66 | ACTTTCCTGCGAGGTCAGTCAAGGCTTT GGGGGCTCTGTTTTGAATGTGGATCAC CACTCGGAGTT | 1412 |
| INHBA | NM_002192.1 | INHBA | GTGCCCGA GCCATATAG CA | 261 | CGGTAGTGG TTGATGACT GTTGA | 645 | ACGTCCGGGTCCTCACT GTCCTTCC | 1029 | 72 | GTGCCCGAGCCATATAGCAGGCACGTC CGGGTCCTCACTGTCCTTCCACTCAACA GTCATCAACCACTACCG | 1413 |
| IRF1 | NM_ 002198.1 | IRF1 | AGTCCAGCC GAGATGCT AAG | 262 | AGAAGGTAT CAGGGCTGG AA | 646 | CCCACATGACTTCCTCTT GGCCTT | 1030 | 69 | AGTCCAGCCGAGATGCTAAGAGCAAGG CCAAGAGGAAGTCATGTGGGGATTCCA GCCCTGATACCTTCT | 1414 |
| IRS1 | NM_ 005544.1 | IRS 1 | CCACAGCTC ACCTTCTGT CA | 263 | CCTCAGTGC CAGTCTCTT CC | 647 | TCCATCCCAGCTCCAGCC AG | 1031 | 74 | CCACAGCTCACCTTCTGTCAGGTGTCCA TCCCAGCTCCAGCCAGCTCCCAGAGAG GAAGAGACTGGCACTGAGG | 1415 |
| ITGA3 | NM_002204.1 | ITGA3 | CCATGATCC TCACTCTGC TG | 264 | GAAGCTTTG TAGCCGGTG AT | 648 | CACTCCAGACCTCGCTTA GCATGG | 1032 | 77 | CCATGATCCTCACTCTGCTGGTGGACTA TACACTCCAGACCTCGCTTAGCATGGT AAATCACCGGCTACAAAGCTTC | 1416 |
| ITGA4 | NM_000885.2 | ITGA4 | CAACGCTTC AGTGATCA ATCC | 265 | GTCTGGCCG GGATTCTTT | 649 | CGATCCTGCATCTGTAA ATCGCCC | 1033 | 66 | CAACGCTTCAGTGATCAATCCCGGGGC GATTTACAGATGCAGGATCGGAAAGAA TCCCGGCCAGAC | 1417 |
| ITGA5 | NM_002205.1 | ITGA5 | AGGCCAGC CCTACATTA TCA | 266 | GTCTTCTCC ACAGTCCAG CA | 650 | TCTGAGCCTTGTCCTCTA TCCGGC .................. | 1034 | 75 | AGGCCAGCCCTACATTATCAGAGCAAG AGCCGGATAGAGGACAAGGCTCAGATC TTGCTGGACTGTGGAGAAGAC | 1418 |
| ITGA6 | NM_000210.1 | ITGA6 | CAGTGACA AACAGCCCT TCC | 267 | GTTTAGCCT CATGGGCGT C | 651 | TCGCCATCTTTTGTGGGA TTCCTT .................. | 1035 | 69 | CAGTGACAAACAGCCCTTCCAACCCAA GGAATCCCACAAAAGATGGCGATGACG CCCATGAGGCTAAAC | 1419 |
| ITGAV | NM_002210.2 | ITGAV | ACTCGGACT GCACAAGC TATT | 268 | TGCCATCAC CATTGAAAT CT | 652 | CCGACAGCCACAGAATA ACCCAAA | 1036 | 79 | ACTCGGACTGCACAAGCTATTTTTGATG ACAGCTATTTGGGTTATTCTGTGGCTGT CGGAGATTTCAATGGTGATGGCA | 1420 |
| ITGB1 | NM_002211.2 | ITGB1 | TCAGAATTG GATTTGGCT CA | 269 | CCTGAGCTT AGCTGGTGT TG | 653 | TGCTAATGTAAGGCATC ACAGTCTTTTCCA | 1037 | 74 | TCAGAATTGGATTTGGCTCATTTGTGGA AAAGACTGTGATGCCTTACATTAGCAC AACACCAGCTAAGCTCAGG | 1421 |
| ITGB3 | NM_000212.2 | ITGB3 | ACCGGGGA GCCCTACAT GA | 270 | CCTTAAGCT CTTTCACTG ACTCAATCT | 654 | AAATACCTGCAACCGTT ACTGCCGTGAC | 1038 | 78 | ACCGGGGAGCCCTACATGACGAAAATA CCTGCAACCGTTACTGCCGTGACGAGA TTGAGTCAGTGAAAGAGCTTAAGG | 1422 |
| ITGB4 | NM_ 000213.2 | ITGB4 | CAAGGTGC CCTCAGTGG A | 271 | GCGCACACC TTCATCTCAT | 655 | CACCAACCTGTACCCGT ATTGCGA | 1039 | 66 | CAAGGTGCCCTCAGTGGAGCTCACCAA CCTGTACCCGTATTGCGACTATGAGAT GAAGGTGTGCGC | 1423 |
| ITGB5 | NM_ 002213.3 | ITGB5 | TCGTGAAA GATGACCA GGAG | 272 | GGTGAACAT CATGACGCA GT | 656 | TGCTATGTTTCTACAAAA CCGCCAAGG | 1040 | 71 | TCGTGAAAGATGACCAGGAGGCTGTGC TATGTTTCTACAAAACCGCCAAGGACT GCGTCATGATGTTCACC | 1424 |
| JAG1 | NM_ 000214.1 | JAG1 | TGGCTTACA CTGGCAATG G | 273 | GCATAGCTG TGAGATGCG G | 657 | ACTCGATTTCCCAGCCA ACCACAG | 1041 | 69 | TGGCTTACACTGGCAATGGTAGTTTCTG TGGTTGGCTGGGAAATCGAGTGCCGCA TCTCACAGCTATGC | 1425 |
| JUNB | NM_ 002229.2 | JUNB | CTGTCAGCT GCTGCTTGG | 274 | AGGGGGTGT CCGTAAAGG | 658 | CAAGGGACACGCCTTCT GAACGT....... | 1042 | 70 | CTGTCAGCTGCTGCTTGGGGTCAAGGG ACACGCCTTCTGAACGTCCCCTGCCCCT TTACGGACACCCCCT | 1426 |
| Ki-67 | NM_ 002417.1 | MKI67 | CGGACTTTG GGTGCGACT T | 275 | TTACAACTC TTCCACTGG GACGAT....... | 659 | CCACTTGTCGAACCACC GCTCGT | 1043 | 80 | CGGACTTTGGGTGCGACTTGACGAGCG GTGGTTCGACAAGTGGCCTTGCGGGCC GGATCGTCCCAGTGGAAGAGTTGTAA | 1427 |
| KIAA0555 | NM_ 014790.3 | JAKMIP2 | AAGCCCGA GGCACTCAT T | 276 | TGTCTGTGA GCTTGGTCC TG | 660 | CCCTTCAAGCTGCCAAT GAAGACC | 1044 | 67 | AAGCCCGAGGCACTCATTGTTGCCCTTC AAGCTGCCAATGAAGACCTCAGGACCA AGCTCACAGACA | 1428 |
| KIAA1199 | NM_018689.1 | KIAA1199 | GCTGGGAG GCAGGACTT C | 277 | GAAGCAGGT CAGAGTGAG CC | 661 | CTTCAAGGCCATGCTGA CCATCAG | 1045 | 66 | GCTGGGAGGCAGGACTTCCTCTTCAAG GCCATGCTGACCATCAGCTGGCTCACT CTGACCTGCTTC | 1429 |
| KIF14 | NM_014875.1 | KIF14 | GAGCTCCAT GGCTCATCC | 278 | TCACACCCA CTGAATCCT ACTG | 662 | TGCATTCCTCTGAGCTCA CTGCTG | 1046 | 69 | GAGCTCCATGGCTCATCCCCAGCAGTG AGCTCAGAGGAATGCACACCCAGTAGG ATTCAGTGGGTGTGA | 1430 |
| KIF20A | NM_005733.1 | KIF20A | TCTCTTGCA GGAAGCCA GA | 279 | CCGTAGGGC CAATTCAGA C | 663 | AGTCAGTGGCCCATCAG CAATCAG | 1047 | 67 | TCTCTTGCAGGAAGCCAGACAACAGTC AGTGGCCCATCAGCAATCAGGGTCTGA ATTGGCCCTACGG | 1431 |
| KIF2C | NM_ 006845.2 | KIF2C | AATTCCTGC TCCAAAAG AAAGTCTT | 280 | CGTGATGCG AAGCTCTGA GA | 664 | AAGCCGCTCCACTCGCA TGTCC | 1048 | 73 | AATTCCTGCTCCAAAAGAAAGTCTTCG AAGCCGCTCCACTCGCATGTCCACTGTC TCAGAGCTTCGCATCACG | 1432 |
| KLK11 | NM_ 006853.1 | KLK11 | CACCCCGGC TTCAACAAC | 281 | CATCTTCAC CAGCATGAT GTCA | 665 | CCTCCCCAACAAAGACC ACCGCA | 1049 | 66 | CACCCCGGCTTCAACAACAGCCTCCCC AACAAAGACCACCGCAATGACATCATG CTGGTGAAGATG | 1433 |
| KLK6 | NM_002774.2 | KLK6 | GACGTGAG GGTCCTGAT TCT | 282 | TCCTCACTC ATCACGTCC TC | 666 | TTACCCCAGCTCCATCCT TGCATC | 1050 | 78 | GACGTGAGGGTCCTGATTCTCCCTGGTT TTACCCCAGCTCCATCCTTGCATCACTG GGGAGGACGTGATGAGTGAGGA | 1434 |
| KLRC1 | NM_0022S9.3 | KLRC1 | CATCCTCAT GGATTGGTG TG | 283 | GCCAAACCA TTCATTGTC AC | 667 | TTCGTAACAGCAGTCAT CATCCATGG | 1051 | 67 | CATCCTCATGGATTGGTGTGTTTCGTAA CAGCAGTCATCATCCATGGGTGACAAT GAATGGTTTGGC | 1435 |
| KNSL2 | BC000712.1 | | CCACCTCGC CATGATTTT TC | 284 | GCAATCTCT TCAAACACT TCATCCT.... | 668 | TTTGACCGGGTATTCCCA CCAGGAA | 1052 | 77 | CCACCTCGCCATGATTTTTCCTTTGACC GGGTATTCCCACCAGGAAGTGGACAGG ATGAAGTGTTTGAAGAGATTGC | 1436 |
| KNTC2 | NM_ 006101.1 | NDC80 | ATGTGCCAG TGAGCTTGA GT | 285 | TGAGCCCCT GGTTAACAG TA | 669 | CCTTGGAGAAACACAAG CACCTGC | 1053 | 71 | ATGTGCCAGTGAGCTTGAGTCCTTGGA GAAACACAAGCACCTGCTAGAAAGTAC TGTTAACCAGGGGCTCA | 1437 |
| KPNA2 | NM_002266.1 | KPNA2 | TGATGGTCC AAATGAAC GAA | 286 | AAGCTTCAC AAGTTGGGG C | 670 | ACTCCTGTTTTCACCACC ATGCCA | 1054 | 67 | TGATGGTCCAAATGAACGAATTGGCAT GGTGGTGAAAACAGGAGTTGTGCCCCA ACTTGTGAAGCTT | 1438 |
| L1CAM | NM_000425.2 | L1CAM | CTTGCTGGC CAATGCCTA | 287 | TGATTGTCC GCAGTCAGG | 671 | ATCTACGTTGTCCAGCTG CCAGCC | 1055 | 66 | CTTGCTGGCCAATGCCTACATCTACGTT GTCCAGCTGCCAGCCAAGATCCTGACT GCGGACAATCA | 1439 |
| LAMA3 | NM_ 000227.2 | LAMA3 | CAGATGAG GCACATGG AGAC | 288 | TTGAAATGG CAGAACGGT AG | 672 | CTGATTCCTCAGGTCCTT GGCCTG | 1056 | 73 | CAGATGAGGCACATGGAGACCCAGGCC AAGGACCTGAGGAATCAGTTGCTCAAC TACCGTTCTGCCATTTCAA | 1440 |
| LAMA5 | NM_005560.3 | LAMA5 | CTCCTGGCC AACAGCAC T | 289 | ACACAAGGC CCAGCCTCT | 673 | CTGTTCCTGGAGCATGG CCTCTTC | 1057 | 67 | CTCCTGGCCAACAGCACTGCACTAGAA GAGGCCATGCTCCAGGAACAGCAGAGG CTGGGCCTTGTGT | 1441 |
| LAMB1 1 | NM_002291.1 | LAMB1 | CAAGGAGA CTGGGAGG TGTC | 290 | CGGCAGAAC TGACAGTGT TC | 674 | CAAGTGCCTGTACCACA CGGAAGG | 1058 | 66 | CAAGGAGACTGGGAGGTGTCTCAAGTG CCTGTACCACACGGAAGGGGAACACTG TCAGTTCTGCCG | 1442 |
| LAMB3 | NM_000228.1 | LAMB3 | ACTGACCA AGCCTGAG ACCT...... | 291 | GTCACACTT GCAGCATTT CA | 675 | CCACTCGCCATACTGGG TGCAGT | 1059 | 67 | ACTGACCAAGCCTGAGACCTACTGCAC CCAGTATGGCGAGTGGCAGATGAAATG CTGCAAGTGTGAC | 1443 |
| LAMC2 | NM_ 005562.1 | LAMC2 | ACTCAAGC GGAAATTG AAGCA | 292 | ACTCCCTGA AGCCGAGAC ACT | 676 | AGGTCTTATCAGCACAG TCTCCGCCTCC | 1060 | 80 | ACTCAAGCGGAAATTGAAGCAGATAGG TCTTATCAGCACAGTCTCCGCCTCCTGG ATTCAGTGTCTCGGCTTCAGGGAGT | 1444 |
| LAPTM4B | NM_018407.4 | LAPTM4B | AGCGATGA AGATGGTC GC | 293 | GACATGGCA GCACAAGCA | 677 | CTGGACGCGGTTCTACTC CAACAG | 1061 | 67 | AGCGATGAAGATGGTCGCGCCCTGGAC GCGGTTCTACTCCAACAGCTGCTGCTTG TGCTGCCATGTC | 1445 |
| LGALS3 | NM_ 002306.1 | LGALS3 | AGCGGAAA ATGGCAGA CAAT | 294 | CTTGAGGGT TTGGGTTTC CA | 678 | ACCCAGATAACGCATCA TGGAGCGA | 1062 | 69 | AGCGGAAAATGGCAGACAATTTTTCGC TCCATGATGCGTTATCTGGGTCTGGAA ACCCAAACCCTCAAG | 1446 |
| LIMK1 | NM_ 016735.1 | | GCTTCAGGT GTTGTGACT GC | 295 | AAGAGCTGC CCATCCTTCT C | 679 | TGCCTCCCTGTCGCACCA GTACTA | 1063 | 67 | GCTTCAGGTGTTGTGACTGCAGTGCCTC CCTGTCGCACCAGTACTATGAGAAGGA TGGGCAGCTCTT | 1447 |
| LIMS1 | NM_004987.3 | LIMS1 | TGAACAGT AATGGGGA GCTG | 296 | TTCTGGGAA CTGCTGGAA G | 680 | ACTGAGCGCACACGAAA CACTGCT | 1064 | 71 | TGAACAGTAATGGGGAGCTGTACCATG AGCAGTGTTTCGTGTGCGCTCAGTGCTT CCAGCAGTTCCCAGAA | 1448 |
| LMNB 1 | NM_005573.1 | LMNB1 | TGCAAACG CTGGTGTCA CA | 297 | CCCCACGAG TTCTGGTTCT TC | 681 | CAGCCCCCAACTGACC TCATC | 1065 | 66 | TGCAAACGCTGGTGTCACAGCCAGCCC CCCAACTGACCTCATCTGGAAGAACCA GAACTCGTGGGG | 1449 |
| LOX | NM_002317.3 | LOX | CCAATGGG AGAACAAC GG | 298 | CGCTGAGGC TGGTACTGT G | 682 | CAGGCTCAGCAAGCTGA ACACCTG | 1066 | 66 | CCAATGGGAGAACAACGGGCAGGTGTT CAGCTTGCTGAGCCTGGGCTCACAGTA CCAGCCTCAGCG | 1450 |
| LRIG1 | NM_ 015541.1 | | CTGCAACAC CGAAGTGG AC | 299 | GTCTCTGGA CACAGGCTG G | 683 | TTACTCCAGGGACAAG CCTTCCA | 1067 | 67 | CTGCAACACCGAAGTGGACTGTTACTC CAGGGGACAAGCCTTCCACCCCCAGCC TGTGTCCAGAGAC | 1451 |
| LSM1 | NM_014462.1 | LSM1 | AGACCAAG CTGGAAGC AGAG | 300 | GAGGAATGG AAAGACCTC GG | 684 | CCTTCAGGGCCTGCACTT TCAACT | 1068 | 66 | AGACCAAGCTGGAAGCAGAGAAGTTG AAAGTGCAGGCCCTGAAGGACCGAGGT CTTTCCATTCCTC | 1452 |
| LTBP1 | NM_ 206943.1 | LTBP1 | ACATCCAG GGCTCTGTG G | 301 | GCAGACACA ATGGAAAGA ACC | 685 | CTGTGTTTAGGCACTCCC CTTGCG | 1069 | 67 | ACATCCAGGGCTCTGTGGTCCGCAAGG GGAGTGCCTAAACACAGAGGGTTCTTT CCATTGTGTCTGC | 1453 |
| LYRIC | NM_ 178812.2 | MTDH | GACCTGGCC TTGCTGAAG | 302 | CGGACAGTT TCTTCCGGTT | 686 | TTCTTCTTCTGTTCCTCG CTCCGG | 1070 | 67 | GACCTGGCCTTGCTGAAGAATCTCCGG AGCGAGGAACAGAAGAAGAAGAACCG GAAGAAACTGTCCG | 1454 |
| MAD1L1 | NM_003550.1 | MAD1L1 | AGAAGCTG TCCCTGCAA GAG | 303 | AGCCGTACC AGCTCAGAC TT | 687 | CATGTTCTTCACAATCGC TGCATCC | 1071 | 67 | AGAAGCTGTCCCTGCAAGAGCAGGATG CAGCGATTGTGAAGAACATGAAGTCTG AGCTGGTACGGCT | 1455 |
| MCM2 | NM_ 004526.1 | MCM2 | GACTTTTGC CCGCTACCT TTC | 304 | GCCACTAAC TGCTTCAGT ATGAAGAG | 688 | ACAGCTCATTGTTGTCAC GCCGGA | 1072 | 75 | GACTTTTGCCCGCTACCTTTCATTCCGG CGTGACAACAATGAGCTGTTGCTCTTC ATACTGAAGCAGTTAGTGGC | 1456 |
| MELK | NM_014791.1 | MELK | AGGATCGC CTGTCAGAA GAG | 305 | TGCACATAA GCAACAGCA GA | 689 | CCCGGGTTGTCTTCCGTC AGATAG | 1073 | 70 | AGGATCGCCTGTCAGAAGAGGAGACCC GGGTTGTCTTCCGTCAGATAGTATCTGC TGTTGCTTATGTGCA | 1457 |
| MGMT | NM_ 002412.1 | MGMT | GTGAAATG AAACGCAC CACA | 306 | GACCCTGCT CACAACCAG AC | 690 | CAGCCCTTTGGGGAAGC TGG | 1074 | 69 | GTGAAATGAAACGCACCACACTGGACA GCCCTTTGGGGAAGCTGGAGCTGTCTG GTTGTGAGCAGGGTC | 1458 |
| mGST1 | NM_020300.2 | MGST1 | ACGGATCTA CCACACCAT TGC | 307 | TCCATATCC AACAAAAAA ACTCAAAG | 691 | TTTGACACCCCTTCCCCA GCCA .............................. | 1075 | 79 | ACGGATCTACCACACCATTGCATATTTG ACACCCCTTCCCCAGCCAAATAGAGCT TTGAGTTTTTTTGTTGGATATGGA | 1459 |
| MMP1 | NM_ 002421.2 | MMP1 | GGGAGATC ATCGGGAC AACTC | 308 | GGGCCTGGT TGAAAAGCA T | 692 | AGCAAGATTTCCTCCAG GTCCATCAAAAGG | 1076 | 72 | GGGAGATCATCGGGACAACTCTCCTTT TGATGGACCTGGAGGAAATCTTGCTCA TGCTTTTCAACCAGGCCC | 1460 |
| MMP12 | NM_002426.1 | MMP12 | CCAACGCTT GCCAAATCC T | 309 | ACGGTAGTG ACAGCATCA AAACTC | 693 | AACCAGCTCTCTGTGAC CCCAATT | 1077 | 78 | CCAACGCTTGCCAAATCCTGACAATTC AGAACCAGCTCTCTGTGACCCCAATTT GAGTTTTGATGCTGTCACTACCGT | 1461 |
| MMP2 | NM_004530.1 | MMP2 | CCATGATGG AGAGGCAG ACA | 310 | GGAGTCCGT CCTTACCGT CAA | 694 | CTGGGAGCATGGCGATG GATACCC | 1078 | 86 | CCATGATGGAGAGGCAGACATCATGAT CAACTTTGGCCGCTGGGAGCATGGCGA TGGATACCCCTTTGACGGTAAGGACGG ACTCC | 1462 |
| MMP7 | NM_ 002423.2 | MMP7 | GGATGGTA GCAGTCTAG GGATTAACT | 311 | GGAATGTCC CATACCCAA AGAA | 695 | CCTGTATGCTGCAACTCA TGAACTTGGC | 1079 | 79 | GGATGGTAGCAGTCTAGGGATTAACTT CCTGTATGCTGCAACTCATGAACTTGGC CATTCTTTGGGTATGGGACATTCC | 1463 |
| MMP8 | NM_ 002424.1 | MMP8 | TCACCTCTC ATCTTCACC AGGAT | 312 | TGTCACCGT GATCTCTTT GGTAA | 696 | AAGCAATGTTGATATCT GCCTCTCCCTGTG | 1080 | 79 | TCACCTCTCATCTTCACCAGGATCTCAC AGGGAGAGGCAGATATCAACATTGCTT TTTACCAAAGAGATCACGGTGACA | 1464 |
| MMTV-like env | AF346816.1 | | CCATACGTG CTGCTACCT GT | 313 | CCTAAAGGT TTGAATGGC AGA | 697 | TCATCAAACCATGGTTC ATCACCAATATC | 1081 | 72 | CCATACGTGCTGCTACCTGTAGATATTG GTGATGAACCATGGTTTGATGATTCTGC CATTCAAACCTTTAGG | 1465 |
| MNAT1 | NM_002431.1 | MNAT1 | CGAGAGTCT GTAGGAGG GAAACC | 314 | GGTTCCGAT ATTTGGTGG TCTTAC | 698 | CGAGGGCAACCCTGATC GTCCA | 1082 | 75 | CGAGAGTCTGTAGGAGGGAAACCGCCA TGGACGATCAGGGTTGCCCTCGGTGTA AGACCACCAAATATCGGAACC | 1466 |
| MRP1 | NM _004996.2 | ABCC1 | TCATGGTGC CCGTCAATG | 315 | CGATTGTCT TTGCTCTTCA TGTG | 699 | ACCTGATACGTCTTGGTC TTCATCGCCAT | 1083 | 79 | TCATGGTGCCCGTCAATGCTGTGATGG CGATGAAGACCAAGACGTATCAGGTGG CCCACATGAAGAGCAAAGACAATCG | 1467 |
| MRP3 | NM_ 003786.2 | ABCC3 | TCATCCTGG CGATCTACT TCCT | 316 | CCGTTGAGT GGAATCAGC AA | 700 | TCTGTCCTGGCTGGAGTC GCTTTCAT | 1084 | 91 | TCATCCTGGCGATCTACTTCCTCTGGCA GAACCTAGGTCCCTCTGTCCTGGCTGG AGTCGCTTTCATGGTCTTGCTGATTCCA CTCAACGG | 1468 |
| MS4A1 | NM_ 021950.2 | MS4A1 | TGAGAAAC AAACTGCA CCCA | 317 | CAAGGCCTC AAATCTCAA GG | 701 | TGAACTCCGCAGCTAGC ATCCAAA | 1085 | 70 | TGAGAAACAAACTGCACCCACTGAACT CCGCAGCTAGCATCCAAATCAGCCCTT GAGATTTGAGGCCTTG | 1469 |
| MSH2 | NM_000251.1 | MSH2 | GATGCAGA ATTGAGGC AGAC | 318 | TCTTGGCAA GTCGGTTAA GA | 702 | CAAGAAGATTTACTTCG TCGATTCCCAGA | 1086 | 73 | GATGCAGAATTGAGGCAGACTTTACAA GAAGATTTACTTCGTCGATTCCCAGATC TTAACCGACTTGCCAAGA | 1470 |
| MTA3 | XM_038567 | | GCTCGTGGT TCTGTAGTC CA | 319 | ACAAAGGGA GAGCGTGAA GT | 703 | TCAGTCAACATCACCCTC CTAGGATGA | 1087 | 69 | GCTCGTGGTTCTGTAGTCCAGTCATCCT AGGAGGGTGATGTTGACTGAGACTTCA CGCTCTCCCTTTGT | 1471 |
| MX1 | NM_002462.2 | MX1 | GAAGGAAT GGGAATCA GTCATGA | 320 | GTCTATTAG AGTCAGATC CGGGACAT | 704 | TCACCCTGGAGATCAGC TCCCGA | 1088 | 78 | GAAGGAATGGGAATCAGTCATGAGCTA ATCACCCTGGAGATCAGCTCCCGAGAT GTCCCGGATCTGACTCTAATAGAC | 1472 |
| MYBL2 | NM_ 002466.1 | MYBL2 | GCCGAGAT CGCCAAGA TG | 321 | CTTTTGATG GTAGAGTTC CAGTGATTC | 705 | CAGCATTGTCTGTCCTCC CTGGCA | 1089 | 74 | GCCGAGATCGCCAAGATGTTGCCAGGG AGGACAGACAATGCTGTGAAGAATCAC TGGAACTCTACCATCAAAAG | 1473 |
| NAT1 | NM_000662.4 | NAT1 | TGGTTTTGA GACCACGA TGT | 322 | TGAATCATG CCAGTGCTG TA | 706 | TGGAGTGCTGTAAACAT ACCCTCCCA | 1090 | 75 | TGGTTTTGAGACCACGATGTTGGGAGG GTATGTTTACAGCACTCCAGCCAAAAA ATACAGCACTGGCATGATTCA | 1474 |
| NAT2 | NM_000015.1 | NAT2 | TAACTGACA TTCTTGAGC ACCAGAT | 323 | ATGGCTTGC CCACAATGC | 707 | CGGGCTGTTCCCTTTGAG AACCTTAACA | 1091 | 73 | TAACTGACATTCTTGAGCACCAGATCC GGGCTGTTCCCTTTGAGAACCTTAACAT GCATTGTGGGCAAGCCAT | 1475 |
| NRG 1 | NM_013957.1 | NRG1 | CGAGACTCT CCTCATAGT GAAAGGTA T | 324 | CTTGGCGTG TGGAAATCT ACAG | 708 | ATGACCACCCCGGCTCG TATGTCA | 1092 | 83 | CGAGACTCTCCTCATAGTGAAAGGTAT GTGTCAGCCATGACCACCCCGGCTCGT ATGTCACCTGTAGATTTCCACACGCCA AG | 1476 |
| OPN, osteopontin | NM_ 000582.1 | SPP1 | CAACCGAA GTTTTCACT CCAGTT | 325 | CCTCAGTCC ATAAACCAC ACTATCA | 709 | TCCCCACAGTAGACACA TATGATGGCCG .......... | 1093 | 80 | CAACCGAAGTTTTCACTCCAGTTGTCCC CACAGTAGACACATATGATGGCCGAGG TGATAGTGTGGTTTATGGACTGAGG | 1477 |
| p16-INK4 | L27211.1 | | GCGGAAGG TCCCTCAGA CA | 326 | TGATGATCT AAGTTTCCC GAGGTT | 710 | CTCAGAGCCTCTCTGGTT CTTTCAATCGG | 1094 | 76 | GCGGAAGGTCCCTCAGACATCCCCGAT TGAAAGAACCAGAGAGGCTCTGAGAA ACCTCGGGAAACTTAGATCATCA | 1478 |
| PAI1 | NM_000603.1 | SERPINE1 | CCGCAACGT GGTTTTCTC A | 327 | TGCTGGGTT TCTCCTCCTG TT | 711 | CTCGGTGTTGGCCATGCT CCAG .............................. | 1095 | 81 | CCGCAACGTGGTTTTCTCACCCTATGGG GTGGCCTCGGTGTTGGCCATGCTCCAG CTGACAACAGGAGGAGAAACCCAGCA | 1479 |
| PGF | NM_002632.4 | PGF | GTGGTTTTC CCTCGGAGC | 328 | AGCAAGGGA ACAGCCTCA T | 712 | ATCTTCTCAGACGTCCCG AGCCAG | 1096 | 71 | GTGGTTTTCCCTCGGAGCCCCCTGGCTC GGGACGTCTGAGAAGATGCCGGTCATG AGGCTGTTCCCTTGCT | 1480 |
| PR | NM_000926.2 | PGR | GCATCAGG CTGTCATTA TGG | 329 | AGTAGTTGT GCTGCCCTT CC | 713 | TGTCCTTACCTGTGGGAG CTGTAAGGTC | 1097 | 85 | GCATCAGGCTGTCATTATGGTGTCCTTA CCTGTGGGAGCTGTAAGGTCTTCTTTAA GAGGGCAATGGAAGGGCAGCACAACT ACT | 1481 |
| PRDX1 | NM_002574.2 | PRDX1 | AGGACTGG GACCCATG AAC | 330 | CCCATAATC CTGAGCAAT GG | 714 | TCCTTTGGTATCAGACCC GAAGCG | 1098 | 67 | AGGACTGGGACCCATGAACATTCCTTT GGTATCAGACCCGAAGCGCACCATTGC TCAGGATTATGGG | 1482 |
| PTEN | NM_000314.1 | PTEN | TGGCTAAGT GAAGATGA CAATCATG | 331 | TGCACATAT CATTACACC AGTTCGT | 715 | CCTTTCCAGCTTTACAGT GAATTGCTGCA | 1099 | 81 | TGGCTAAGTGAAGATGACAATCATGTT GCAGCAATTCACTGTAAAGCTGGAAAG GGACGAACTGGTGTAATGATATGTGCA | 1483 |
| PTP4A3 | NM_007079.2 | PTP4A3 | AATATTTGT GCGGGGTA TGG | 332 | AACGAGATC CCTGTGCTT GT | 716 | CCAAGAGAAACGAGATT TAAAAACCCACC | 1100 | 70 | AATATTTGTGCGGGTATGGGGGTGGG TTTTTAAATCTCGTTTCTCTTGGACAAG CACAGGGATCTCGTT | 1484 |
| RhoB | NM_ 004040.2 | RHOB | AAGCATGA ACAGGACTT GACC | 333 | CCTCCCCAA GTCAGTTGC | 717 | CTTTCCAACCCCTGGGG AAGACAT | 1101 | 67 | AAGCATGAACAGGACTTGACCATCTTT CCAACCCCTGGGGAAGACATTTGCAAC TGACTTGGGGAGG | 1485 |
| RPL13A | NM_012423.2 | RPL13A | GCAAGGAA AGGGTCTTA GTCAC | 334 | ACACCTGCA CAATTCTCC G | 718 | CCTCCCGAAGTTGCTTGA AAGCAC | 1102 | 68 | GCAAGGAAAGGGTCTTAGTCACTGCCT CCCGAAGTTGCTTGAAAGCACTCGGAG AATTGTGCAGGTGT | 1486 |
| RPL41 | NM_021104.1 | RPL41 | GAAACCTCT GCGCCATG A | 335 | TTCTTTTGCG CTTCAGCC | 719 | CATTCGCTTCTTCCTCCA CTTGGC | 1103 | 66 | GAAACCTCTGCGCCATGAGAGCCAAGT GGAGGAAGAAGCGAATGCGCAGGCTG AAGCGCAAAAGAA | 1487 |
| RPLPO | NM_001002.2 | RPLP0 | CCATTCTAT CATCAACG GGTACAA | 336 | TCAGCAAGT GGGAAGGTG TAATC | 720 | TCTCCACAGACAAGGCC AGGACTCG | 1104 | 75 | CCATTCTATCATCAACGGGTACAAACG AGTCCTGGCCTTGTCTGTGGAGACGGA TTACACCTTCCCACTTGCTGA | 1488 |
| RPS23 | NM_001025.1 | RPS23 | GTTCTGGTT GCTGGATTT GG | 337 | CCTTAAAGC GGACTCCAG G | 721 | ATCACCAACAGCATGAC CTTTGCG .............. | 1105 | 67 | GTTCTGGTTGCTGGATTTGGTCGCAAAG GTCATGCTGTTGGTGATATTCCTGGAGT CCGCTTTAAGG | 1489 |
| RPS27 | NM_001030.3 | RPS27 | TCACCACGG TCTTTAGCC A | 338 | TCCTCCTGT AGGCTGGCA | 722 | AGGACAGTGGAGCAGCC AACACAC | 1106 | 80 | TCACCACGGTCTTTAGCCATGCACAAA CGGTAGTTTTGTGTGTTGGCTGCTCCAC TGTCCTCTGCCAGCCTACAGGAGGA | 1490 |
| RRM1 | NM_001033.1 | RRM1 | GGGCTACTG GCAGCTAC ATT | 339 | CTCTCAGCA TCGGTACAA GG | 723 | CATTGGAATTGCCATTA GTCCCAGC | 1107 | 66 | GGGCTACTGGCAGCTACATTGCTGGGA CTAATGGCAATTCCAATGGCCTTGTACC GATGCTGAGAG | 1491 |
| RRM2 | NM_001034.1 | RRM2 | CAGCGGGA TTAAACAGT CCT | 340 | ATCTGCGTT GAAGCAGTG AG | 724 | CCAGCACAGCCAGTTAA AAGATGCA | 1108 | 71 | CAGCGGGATTAAACAGTCCTTTAACCA GCACAGCCAGTTAAAAGATGCAGCCTC ACTGCTTCAACGCAGAT | 1492 |
| RUNX1 | NM_001754.2 | RUNX1 | AACAGAGA CATTGCCAA CCA | 341 | GTGATTTGC CCAGGAAAG TTT | 725 | TTGGATCTGCTTGCTGTC CAAACC | 1109 | 69 | AACAGAGACATTGCCAACCATATTGGA TCTGCTTGCTGTCCAAACCAGCAAACTT CCTGGGCAAATCAC | 1493 |
| S100A10 | NM_ 002966.1 | S100A10 | ACACCAAA ATGCCATCT CAA | 342 | TTTATCCCC AGCGAATTT GT | 726 | CACGCCATGGAAACCAT GATGTTT.......... | 1110 | 77 | ACACCAAAATGCCATCTCAAATGGAAC ACGCCATGGAAACCATGATGTTTACAT TTCACAAATTCGCTGGGGATAAA | 1494 |
| S100A2 | NM_005978.2 | S100A2 | TGGCTGTGC TGGTCACTA CCT | 343 | TCCCCCTTA CTCAGCTTG AACT | 727 | CACAAGTACTCCTGCCA AGAGGGCGAC | 1111 | 73 | TGGCTGTGCTGGTCACTACCTTCCACAA GTACTCCTGCCAAGAGGGCGACAAGTT CAAGCTGAGTAAGGGGGA | 1495 |
| S100A4 | NM_002961.2 | S100A4 | GACTGCTGT CATGGCGTG | 344 | CGAGTACTT GTGGAAGGT GGAC | 728 | ATCACATCCAGGGCCTT CTCCAGA | 1112 | 70 | GACTGCTGTCATGGCGTGCCCTCTGGA GAAGGCCCTGGATGTGATGGTGTCCAC CTTCCACAAGTACTCG | 1496 |
| S100A7 | NM_002963.2 | S100A7 | CCTGCTGAC GATGATGA AGGA | 345 | GCGAGGTAA TTTGTGCCCT TT | 729 | TTCCCCAACTTCCTTAGT GCCTGTGACA | 1113 | 75 | CCTGCTGACGATGATGAAGGAGAACTT CCCCAACTTCCTTAGTGCCTGTGACAAA AAGGGCACAAATTACCTCGC | 1497 |
| S100A8 | NM_ 002964.3 | S100A8 | ACTCCCTGA TAAAGGGG AATTT | 346 | TGAGGACAC TCGGTCTCT AGC | 730 | CATGCCGTCTACAGGGA TGACCTG | 1114 | 76 | ACTCCCTGATAAAGGGGAATTTCCATG CCGTCTACAGGGATGACCTGAAGAAAT TGCTAGAGACCGAGTGTCCTCA | 1498 |
| S100A9 | NM_ 002965.3 | S100A9 | CACCCTGCC TCTACCCAA C | 347 | CTAGCCCCA CAGCCAAGA | 731 | CCCGGGGCCTGTTATGTC AAACT | 1115 | 67 | CACCCTGCCTCTACCCAACCAGGGCCC CGGGGCCTGTTATGTCAAACTGTCTTGG CTGTGGGGCTAG | 1499 |
| S100B | NM_ 006272.1 | S100B | CATGGCCGT GTAGACCCT AA | 348 | AGTTTTAAG GGTGCCCCG | 732 | CCGGAGGGAACCCTGAC TACAGAA | 1116 | 70 | CATGGCCGTGTAGACCCTAACCCGGAG GGAACCCTGACTACAGAAATTACCCCG GGGCACCCTTAAAACT | 1500 |
| S100G | NM_004057.2 | S100G | ACCCTGAGC ACTGGAGG AA | 349 | GAGACTTTG GGGGATTCC A | 733 | AGGATAAGACCACAGCA CAGGCGC.............................. | 1117 | 67 | ACCCTGAGCACTGGAGGAAGAGCGCCT GTGCTGTGGTCTTATCCTATGTGGAATC CCCCAAAGTCTC | 1501 |
| S100P | NM_ 005980.2 | S100P | AGACAAGG ATGCCGTGG ATAA | 350 | GAAGTCCAC CTGGGCATC TC | 734 | TTGCTCAAGGACCTGGA CGCCAA | 1118 | 67 | AGACAAGGATGCCGTGGATAAATTGCT CAAGGACCTGGACGCCAATGGAGATGC CCAGGTGGACTTC | 1502 |
| SDHA | NM_ 004168.1 | SDHA | GCAGAACT GAAGATGG GAAGAT | 351 | CCCTTTCCA AACTTGAGG C | 735 | CTGTCCACCAAATGCAC GCTGATA | 1119 | 67 | GCAGAACTGAAGATGGGAAGATTTATC AGCGTGCATTTGGTGGACAGAGCCTCA AGTTTGGAAAGGG | 1503 |
| SEMA3F | NM_004186.1 | SEMA3F | CGCGAGCC CCTCATTAT ACA | 352 | CACTCGCCG TTGACATCC T | 736 | CTCCCCACAGCGCATCG AGGAA | 1120 | 86 | CGCGAGCCCCTCATTATACACTGGGCA GCCTCCCCACAGCGCATCGAGGAATGC GTGCTCTCAGGCAAGGATGTCAACGGC GAGTG | 1504 |
| SFRP2 | NM_003013.2 | SFRP2 | CAAGCTGA ACGGTGTGT CC | 353 | TGCAAGCTG TCTTTGAGC C | 737 | CAGCACCGATTTCTTCAG GTCCCT | 1121 | 66 | CAAGCTGAACGGTGTGTCCGAAAGGGA CCTGAAGAAATCGGTGCTGTGGCTCAA AGACAGCTTGCA | 1505 |
| SIR2 | NM_012238.3 | SIRT1 | AGCTGGGG TGTCTGTTT CAT | 354 | ACAGCAAGG CGAGCATAA AT | 738 | CCTGACTTCAGGTCAAG GGATGG | 1122 | 72 | AGCTGGGGTGTCTGTTTCATGTGGAAT ACCTGACTTCAGGTCAAGGGATGGTAT TTATGCTCGCCTTGCTGT | 1506 |
| SKIL | NM_ 005414.2 | SKIL | AGAGGCTG AATATGCA GGACA | 355 | CTATCGGCC TCAGCATGG | 739 | CCAATCTCTGCCTCAGTT CTGCCA | 1123 | 66 | AGAGGCTGAATATGCAGGACAGTTGGC AGAACTGAGGCAGAGATTGGACCATGC TGAGGCCGATAG | 1507 |
| SKP2 | NM_ 005983.2 | SKP2 | AGTTGCAG AATCTAAGC CTGGAA | 356 | TGAGTTTTTT GCGAGAGTA TTGACA | 740 | CCTGCGGCTTTCGGATCC CA | 1124 | 71 | AGTTGCAGAATCTAAGCCTGGAAGGCC TGCGGCTTTCGGATCCCATTGTCAATAC TCTCGCAAAAAACTCA | 1508 |
| SLPI | NM_003064.2 | SLPI | ATGGCCAAT GTTTGATGC T | 357 | ACACTTCAA GTCACGCTT GC | 741 | TGGCCATCCATCTCACA GAAATTGG | 1125 | 74 | ATGGCCAATGTTTGATGCTTAACCCCCC CAATTTCTGTGAGATGGATGGCCAGTG CAAGCGTGACTTGAAGTGT | 1509 |
| SNAI1 | NM_005985.2 | SNAI1 | CCCAATCGG AAGCCTAA CTA | 358 | GTAGGGCTG CTGGAAGGT AA | 742 | TCTGGATTAGAGTCCTGC AGCTCGC | 1126 | 69 | CCCAATCGGAAGCCTAACTACAGCGAG CTGCAGGACTCTAATCCAGAGTTTACCT TCCAGCAGCCCTAC | 1510 |
| STK15 | NM_003600.1 | AURKA | CATCTTCCA GGAGGACC ACT | 359 | TCCGACCTT CAATCATTT CA | 743 | CTCTGTGGCACCCTGGA CTACCTG | 1127 | 69 | CATCTTCCAGGAGGACCACTCTCTGTG GCACCCCTGGACTACCTGCCCCCTGAAA TGATTGAAGGTCGGA | 1511 |
| STMN1 | NM_ 005563.2 | STMN1 | AATACCCA ACGCACAA ATGA | 360 | GGAGACAAT GCAAACCAC AC | 744 | CACGTTCTCTGCCCCGTT TCTTG | 1128 | 71 | AATACCCAACGCACAAATGACCGCACG TTCTCTGCCCCGTTTCTTGCCCCAGTGT GGTTTGCATTGTCTCC | 1512 |
| STMY3 | NM_005940.2 | MMP11 | CCTGGAGG CTGCAACAT ACC | 361 | TACAATGGC TTTGGAGGA TAGCA | 745 | ATCCTCCTGAAGCCCTTT TCGCAGC | 1129 | 90 | CCTGGAGGCTGCAACATACCTCAATCC TGTCCCAGGCCGGATCCTCCTGAAGCC CTTTTCGCAGCACTGCTATCCTCCAAAG CCATTGTA | 1513 |
| STTRV | NM_ 001168.1 | BIRC5 | TGTTTTGAT TCCCGGGCT TA | 362 | CAAAGCTGT CAGCTCTAG CAAAAG[?] | 746 | TGCCTTCTTCCTCCCTCA CTTCTCACCT | 1130 | 80 | TGTTTTGATTCCCGGGCTTACCAGGTGA GAAGTGAGGGAGGAAGAAGGCAGTGT CCCTTTTGCTAGAGCTGACAGCTTTG | 1514 |
| SYK | NM_003177.1 | SYK | TCTCCAGCA AAAGCGAT GTCT | 363 | TTCATCCCTC GATATGGCT TCT | 747 | CCATAGGAGAATGCTTC CCACATCAACACT | 1131 | 85 | TCTCCAGCAAAAGCGATGTCTGGAGCT TTGGAGTGTTGATGTGGGAAGCATTCT CCTATGGGCAGAAGCCATATCGAGGGA TGAA | 1515 |
| TAGLN | NM_003186.2 | TAGLN | GATGGAGC AGGTGGCTC AGT | 364 | AGTCTGGAA CATGTCAGT CTTGATG | 748 | CCCATAGTCCTCAGCCG CCTTCAG | 1132 | 73 | GATGGAGCAGGTGGCTCAGTTCCTGAA GGCGGCTGAGGACTCTGGGGTCATCAA GACTGACATGTTCCAGACT | 1516 |
| TCEA1 | NM_ 201437.1 | TCEA1 | CAGCCCTGA GGCAAGAG A | 365 | CGAGCATTT GTCTCATCC TTT | 749 | CTTCCAGCGGCAATGTA AGCAACA | 1133 | 72 | CAGCCCTGAGGCAAGAGAAGAAAGTA CTTCCAGCGGCAATGTAAGCAACAGAA AGGATGAGACAAATGCTCG | 1517 |
| TFRC | NM_ 003234.1 | TFRC | GCCAACTGC TTTCATTTG TG | 366 | ACTCAGGCC CATTTCCTTT A.......... | 750 | AGGGATCTGAACCAATA CAGAGCAGACA | 1134 | 68 | GCCAACTGCTTTCATTTGTGAGGGATCT GAACCAATACAGAGCAGACATAAAGG AAATGGGCCTGAGT | 1518 |
| TGFB2 | NM_003238.1 | TGFB2 | ACCAGTCCC CCAGAAGA CTA | 367 | CCTGGTGCT GTTGTAGAT GG | 751 | TCCTGAGCCCGAGGAAG TCCC | 1135 | 75 | ACCAGTCCCCCAGAAGACTATCCTGAG CCCGAGGAAGTCCCCCCGGAGGTGATT TCCATCTACAACAGCACCAGG | 1519 |
| TGFB3 | NM_ 003239.1 | TGFB3 | GGATCGAG CTCTTCCAG ATCCT | 368 | GCCACCGAT ATAGCGCTG TT | 752 | CGGCCAGATGAGCACAT TGCC | 1136 | 65 | GGATCGAGCTCTTCCAGATCCTTCGGCC AGATGAGCACATTGCCAAACAGCGCTA TATCGGTGGC | 1520 |
| TGFBR2 | NM_003242.2 | TGFBR2 | AACACCAA TGGGTTCCA TCT.......... | 369 | CCTCTTCATC AGGCCAAAC T | 753 | TTCTGGGCTCCTGATTGC TCAAGC | 1137 | 66 | AACACCAATGGGTTCCATCTTTCTGGGC TCCTGATTGCTCAAGCACAGTTTGGCCT GATGAAGAGG | 1521 |
| TIMP3 | NM_ 000362.2 | TIMP3 | CTACCTGCC TTGCTTTGT GA | 370 | ACCGAAATT GGAGAGCAT GT | 754 | CCAAGAACGAGTGTCTC TGGACCG | 1138 | 67 | CTACCTGCCTTGCTTTGTGACTTCCAAG AACGAGTGTCTCTGGACCGACATGCTC TCCAATTTCGGT | 1522 |
| TNFRSF11 A | NM_003839.2 | TNFRSF11 A | CCAGCCCAC AGACCAGTT A | 371 | TTCAGAGAA AGGAGGTGT GGA | 755 | TGTTCCTCACTGAGCCTG GAAGCA | 1139 | 67 | CCAGCCCACAGACCAGTTACTGTTCCTC ACTGAGCCTGGAAGCAAATCCACACCT CCTTTCTCTGAA | 1523 |
| TNFRSF11 B | NM_ 002546.2 | TNFRSF11 B | TGGCGACC AAGACACC TT | 372 | GGGAAAGTG GTACGTCTT TGAG.......... | 756 | AGGGCCTAATGCACGCA CTAAAGC | 1140 | 67 | TGGCGACCAAGACACCTTGAAGGGCCT AATGCACGCACTAAAGCACTCAAAGAC GTACCACTTTCCC | 1524 |
| TNFSF11 | NM_ 003701.2 | TNFSF11 | CATATCGTT GGATCACA GCAC | 373 | TTGGCCAGA TCTAACCAT GA | 757 | TCCACCATCGCTTTCTCT GCTCTG | 1141 | 71 | CATATCGTTGGATCACAGCACATCAGA GCAGAGAAAGCGATGGTGGATGGCTCA TGGTTAGATCTGGCCAA | 1525 |
| TWIST1 | NM_000474.2 | TWIST1 | GCGCTGCG GAAGATCA TC | 374 | GCTTGAGGG TCTGAATCT TGCT .......... | 758 | CCACGCTGCCCTCGGAC AAGC | 1142 | 64 | GCGCTGCGGAAGATCATCCCCACGCTG CCCTCGGACAAGCTGAGCAAGATTCAG ACCCTCAAGC | 1526 |
| UBB | NM_ 018955.1 | UBB | GAGTCGAC CCTGCACCT G | 375 | GCGAATGCC ATGACTGAA | 759 | AATTAACAGCCACCCCT CAGGCG | 1143 | 522 | GAGTCGACCCTGCACCTGGTCCTGCGT CTGAGAGGTGGTATGCAGATCTTCGTG AAGACCCTGACCGGCAAGACCATCACC CTGGAAGTGGAGCCCAGTGACACCATC GAAAATGTGAAGGCCAAGATCCAGGAT AAAGAAGGCATCCCTCCCGACCAGCAG AGGCTCATCTTTGCAGGCAAGCAGCTG GAAGATGGCCGCACTCTTTCTGACTAC AACATCCAGAAGGAGTCGACCCTGCAC CTGGTCCTGCGTCTGAGAGGTGGTATG CAGATCTTCGTGAAGACCCTGACCGGC AAGACCATCACTCTGGAAGTGGAGCCC AGTGACACCATCGAAAATGTGAAGGCC AAGATCCAAGATAAAGAAGGCATCCCT CCCGACCAGCAGAGGCTCATCTTTGCA GGCAAGCAGCTGGAAGATGGCCGCACT CTTTCTGACTACAACATCCAGAAGGAG TCGACCCTGCACCTGGTCCTGCGCCTGA GGGGTGGCTGTTAATTCTTCAGTCATGG CATTCGC | 1527 |
| VCAM1 | NM_ 001078.2 | VCAM1 | TGGCTTCAG GAGCTGAA TACC | 376 | TGCTGTCGT GATGAGAAA ATAGTG | 760 | CAGGCACACACAGGTGG GACACAAAT | 1144 | 89 | TGGCTTCAGGAGCTGAATACCCTCCCA GGCACACACAGGTGGGACACAAATAA GGGTTTTGGAACCACTATTTTCTCATCA CGACAGCA | 1528 |
| VIM | NM_003380.1 | VIM | TGCCCTTAA AGGAACCA ATGA.......... | 377 | GCTTCAACG GCAAAGTTC TCTT | 761 | ATTTCACGCATCTGGCGT TCCA | 1145 | 72 | TGCCCTTAAAGGAACCAATGAGTCCCT GGAACGCCAGATGCGTGAAATGGAAG AGAACTTTGCCGTTGAAGC | 1529 |
| VTN | NM_ 000638.2 | VTN | AGTCAATCT TCGCACACG G | 378 | GTACTGAGC GATGGAGCG T | 762 | TGGACACTGTGGACCCT CCCTACC | 1146 | 67 | AGTCAATCTTCGCACACGGCGAGTGGA CACTGTGGACCCTCCCTACCCACGCTCC ATCGCTCAGTAC | 1530 |
| WAVE3 | NM_006646.4 | WASE3 | CTCTCCAGT GTGGGCAC C | 379 | GCGGTGTAG CTCCCAGAG T | 763 | CCAGAACAGATGCGAGC AGTCCAT | 1147 | 68 | CTCTCCAGTGTGGGCACCAGCCGGCCA GAACAGATGCGAGCAGTCCATGACTCT GGGAGCTACACCGC | 1531 |
| WISP1 | NM_ 003882.2 | WISP1 | AGAGGCAT CCATGAACT TCACA | 380 | CAAACTCCA CAGTACTTG GGTTGA | 764 | CGGGCTGCATCAGCACA CGC | 1148 | 75 | AGAGGCATCCATGAACTTCACACTTGC GGGCTGCATCAGCACACGCTCCTATCA ACCCAAGTACTGTGGAGTTTG | 1532 |
| Wnt-5a | NM_ 003392.2 | WNT5A | GTATCAGG ACCACATGC AGTACATC | 381 | TGTCGGAAT TGATACTGG CATT | 765 | TTGATGCCTGTCTTCGCG CCTTCT | 1149 | 75 | GTATCAGGACCACATGCAGTACATCGG AGAAGGCGCGAAGACAGGCATCAAAG AATGCCAGTATCAATTCCGACA | 1533 |
| Wnt-5b | NM_ 032642.2 | WNT5B | TGTCTTCAG GGTCTTGTC CA | 382 | GTGCACGTG GATGAAAGA GT | 766 | TTCCGTAAGAGGCCTGG TGCTCTC | 1150 | 79 | TGTCTTCAGGGTCTTGTCCAGAATGTAG ATGGGTTCCGTAAGAGGCCTGGTGCTC TCTTACTCTTTCATCCACGTGCAC | 1534 |
| WWOX | NM_016373.1 | WWOX | ATCGCAGCT GGTGGGTGT AC | 383 | AGCTCCCTG TTGCATGGA CTT | 767 | CTGCTGTTTACCTTGGCG AGGCCTTTC | 1151 | 74 | ATCGCAGCTGGTGGGTGTACACACTGC TGTTTACCTTGGCGAGGCCTTTCACCAA GTCCATGCAACAGGGAGCT | 1535 |
| YWHAZ | NM_ 003406.2 | YWHAZ | GTGGACATC GGATACCC AAG | 384 | GCAGACAAA AGTTGGAAG GC | 768 | CCCCTCCTTCTCCTGCTT CAGCTT | 1152 | 81 | GTGGACATCGGATACCCAAGGAGACGA AGCTGAAGCAGGAGAAGGAGGGGAAA ATTAACCGGCCTTCCAACTTTTGTCTGC | 1536 |

**Table 1: Cox proportional hazards for Prognostic Genes that are positively associated with good prognosis for breast cancer (Providence study)**

| **Table 1** | | | |
|---|---|---|---|
| **Gene_all** | **z (Coef)** | **HR** | **p (Wald)** |
| GSTM2 | -4.306 | 0.525 | 0.000 |
| IL6ST | -3.730 | 0.522 | 0.000 |
| CEGP1 | -3.712 | 0.756 | 0.000 |
| Bcl2 | -3.664 | 0.555 | 0.000 |
| GSTM1 | -3.573 | 0.679 | 0.000 |
| ERBB4 | -3.504 | 0.767 | 0.000 |
| GADD45 | -3.495 | 0.601 | 0.000 |
| PR | -3.474 | 0.759 | 0.001 |
| GPR30 | -3.348 | 0.660 | 0.001 |
| CAV1 | -3.344 | 0.649 | 0.001 |
| C10orf116 | -3.194 | 0.681 | 0.001 |
| DR5 | -3.102 | 0.543 | 0.002 |
| DICER1 | -3.097 | 0.296 | 0.002 |
| EstR1 | -2.983 | 0.825 | 0.003 |
| BTRC | -2.976 | 0.639 | 0.003 |
| GSTM3 | -2.931 | 0.722 | 0.003 |
| GATA3 | -2.874 | 0.745 | 0.004 |
| DLC 1 | -2.858 | 0.564 | 0.004 |
| CXCL14 | -2.804 | 0.693 | 0.005 |
| IL17RB | -2.796 | 0.744 | 0.005 |
| C8orf4 | -2.786 | 0.699 | 0.005 |
| FOXO3A | -2.786 | 0.617 | 0.005 |
| TNFRSF11B | -2.690 | 0.739 | 0.007 |
| BAG1 | -2.675 | 0.451 | 0.008 |
| SNAI1 | -2.632 | 0.692 | 0.009 |
| TGFB3 | -2.617 | 0.623 | 0.009 |
| NAT1 | -2.576 | 0.820 | 0.010 |
| FUS | -2.543 | 0.376 | 0.011 |
| F3 | -2.527 | 0.705 | 0.012 |
| GSTM2 gene | -2.461 | 0.668 | 0.014 |
| EPHB2 | -2.451 | 0.708 | 0.014 |
| LAMA3 | -2.448 | 0.778 | 0.014 |
| BAD | -2.425 | 0.506 | 0.015 |
| IGF1R | -2.378 | 0.712 | 0.017 |
| RUNX1 | -2.356 | 0.511 | 0.018 |
| ESRRG | -2.289 | 0.825 | 0.022 |
| HSHIN1 | -2.275 | 0.371 | 0.023 |
| CXCL12 | -2.151 | 0.623 | 0.031 |
| IGFBP7 | -2.137 | 0.489 | 0.033 |
| SKIL | -2.121 | 0.593 | 0.034 |
| PTEN | -2.110 | 0.449 | 0.035 |
| AKT3 | -2.104 | 0.665 | 0.035 |
| MGMT | -2.060 | 0.571 | 0.039 |
| LRIG1 | -2.054 | 0.649 | 0.040 |
| S100B | -2.024 | 0.798 | 0.043 |
| GREB1 variant a | -1.996 | 0.833 | 0.046 |
| CSF1 | -1.976 | 0.624 | 0.048 |
| ABR | -1.973 | 0.575 | 0.048 |
| AK055699 | -1.972 | 0.790 | 0.049 |

**Table 2: Cox proportional hazards for Prognostic Genes that are negatively associated with good prognosis for breast cancer (Providence study)**

| **Table 2** | | | |
|---|---|---|---|
| **Gene_all** | **z (Coef)** | **HR** | **p (Wald)** |
| S100A7 | 1.965 | 1.100 | 0.049 |
| MCM2 | 1.999 | 1.424 | 0.046 |
| Contig 51037 | 2.063 | 1.185 | 0.039 |
| S100P | 2.066 | 1.170 | 0.039 |
| ACTR2 | 2.119 | 2.553 | 0.034 |
| MYBL2 | 2.158 | 1.295 | 0.031 |
| DUSP1 | 2.166 | 1.330 | 0.030 |
| HOXB13 | 2.192 | 1.206 | 0.028 |
| SURV | 2.216 | 1.329 | 0.027 |
| MELK | 2.234 | 1.336 | 0.026 |
| HSPA8 | 2.240 | 2.651 | 0.025 |
| cdc25A | 2.314 | 1.478 | 0.021 |
| C20_orf1 | 2.336 | 1.497 | 0.019 |
| LMNB1 | 2.387 | 1.682 | 0.017 |
| S100A9 | 2.412 | 1.185 | 0.016 |
| CENPA | 2.419 | 1.366 | 0.016 |
| CDC25C | 2.437 | 1.384 | 0.015 |
| GAPDH | 2.498 | 1.936 | 0.012 |
| KNTC2 | 2.512 | 1.450 | 0.012 |
| PRDX1 | 2.540 | 2.131 | 0.011 |
| RRM2 | 2.547 | 1.439 | 0.011 |
| ADM | 2.590 | 1.445 | 0.010 |
| ARF1 | 2.634 | 2.973 | 0.008 |
| E2F1 | 2.716 | 1.486 | 0.007 |
| TFRC | 2.720 | 1.915 | 0.007 |
| STK15 | 2.870 | 1.860 | 0.004 |
| LAPTM4B | 2.880 | 1.538 | 0.004 |
| EpCAM | 2.909 | 1.919 | 0.004 |
| ENO1 | 2.958 | 2.232 | 0.003 |
| CCNB1 | 3.003 | 1.738 | 0.003 |
| BUB1 | 3.018 | 1.590 | 0.003 |
| Claudin 4 | 3.034 | 2.151 | 0.002 |
| CDC20 | 3.056 | 1.555 | 0.002 |
| Ki-67 | 3.329 | 1.717 | 0.001 |
| KPNA2 | 3.523 | 1.722 | 0.000 |
| IDH2 | 3.994 | 1.638 | 0.000 |

**Table 3: Cox proportional hazards for Prognostic Genes that are positively associated with good prognosis for ER-negative (ER0) breast cancer (Providence study)**

| **Gene_ER0** | **HR** | **z (Coef)** | **p (Wald)** |
|---|---|---|---|
| SYK | 0.185 | -2.991 | 0.003 |
| Wnt-5a | 0.443 | -2.842 | 0.005 |
| WISP1 | 0.455 | -2.659 | 0.008 |
| CYR61 | 0.405 | -2.484 | 0.013 |
| GADD45 | 0.520 | -2.474 | 0.013 |
| TAGLN | 0.364 | -2.376 | 0.018 |
| TGFB3 | 0.465 | -2.356 | 0.018 |
| INHBA | 0.610 | -2.255 | 0.024 |
| CDH11 | 0.584 | -2.253 | 0.024 |
| CHAF1B | 0.551 | -2.113 | 0.035 |
| ITGAV | 0.192 | -2.101 | 0.036 |
| SNAI1 | 0.655 | -2.077 | 0.038 |
| IL11 | 0.624 | -2.026 | 0.043 |
| KIAA1199 | 0.692 | -2.005 | 0.045 |
| TNFRSF 11B | 0.659 | -1.989 | 0.047 |

**Table 4: Cox proportional hazards for Prognostic Genes that are negatively associated with good prognosis for ER-negative (ER0) breast cancer (Providence study)**

| **Gene_ER0** | **HR** | **z (Coef)** | **p (Wald)** |
|---|---|---|---|
| RPL41 | 3.547 | 2.062 | 0.039 |
| Claudin 4 | 2.883 | 2.117 | 0.034 |
| LYRIC | 4.029 | 2.364 | 0.018 |
| TFRC | 3.223 | 2.596 | 0.009 |
| VTN | 2.484 | 3.205 | 0.001 |

**Table 5: Cox proportional hazards for Prognostic Genes that are positively associated with good prognosis for ER-positive (ER1) breast cancer (Providence study)**

| **Gene-ER1** | **HR** | **z (Coef)** | **p (Wald)** |
|---|---|---|---|
| DR5 | 0.428 | -3.478 | 0.001 |
| GSTM2 | 0.526 | -3.173 | 0.002 |
| HSHIN1 | 0.175 | -3.031 | 0.002 |
| ESRRG | 0.736 | -3.028 | 0.003 |
| VTN | 0.622 | -2.935 | 0.003 |
| Bcl2 | 0.469 | -2.833 | 0.005 |
| ERBB4 | 0.705 | -2.802 | 0.005 |
| GPR30 | 0.625 | -2.794 | 0.005 |
| BAG1 | 0.339 | -2.733 | 0.006 |
| CAV1 | 0.635 | -2.644 | 0.008 |
| IL6ST | 0.503 | -2.551 | 0.011 |
| C10orf116 | 0.679 | -2.497 | 0.013 |
| FOXO3A | 0.607 | -2.473 | 0.013 |
| DICER 1 | 0.311 | -2.354 | 0.019 |
| GADD45 | 0.645 | -2.338 | 0.019 |
| CSF1 | 0.500 | -2.312 | 0.021 |
| F3 | 0.677 | -2.300 | 0.021 |
| GBP2 | 0.604 | -2.294 | 0.022 |
| APEX-1 | 0.234 | -2.253 | 0.024 |
| FUS | 0.322 | -2.252 | 0.024 |
| BBC3 | 0.581 | -2.248 | 0.025 |
| GSTM3 | 0.737 | -2.203 | 0.028 |
| ITGA4 | 0.620 | -2.161 | 0.031 |
| EPHB2 | 0.685 | -2.128 | 0.033 |
| IRF1 | 0.708 | -2.105 | 0.035 |
| CRYZ | 0.593 | -2.103 | 0.035 |
| CCL19 | 0.773 | -2.076 | 0.038 |
| SKIL | 0.540 | -2.019 | 0.043 |
| MRP1 | 0.515 | -1.964 | 0.050 |

**Table 6: Cox proportional hazards for Prognostic Genes that are negatively associated with good prognosis for ER-positive (ER1) breast cancer (Providence study)**

| **Gene_ER1** | **HR** | **z (Coef)** | **p (Wald)** |
|---|---|---|---|
| CTHRC1 | 2.083 | 1.958 | 0.050 |
| RRM2 | 1.450 | 1.978 | 0.048 |
| BUB1 | 1.467 | 1.988 | 0.047 |
| LMNB 1 | 1.764 | 2.009 | 0.045 |
| SURV | 1.380 | 2.013 | 0.044 |
| EpCAM | 1.966 | 2.076 | 0.038 |
| CDC20 | 1.504 | 2.081 | 0.037 |
| GAPDH | 2.405 | 2.126 | 0.033 |
| STK15 | 1.796 | 2.178 | 0.029 |
| HSPA8 | 3.095 | 2.215 | 0.027 |
| LAPTM4B | 1.503 | 2.278 | 0.023 |
| MCM2 | 1.872 | 2.370 | 0.018 |
| CDC25C | 1.485 | 2.423 | 0.015 |
| ADM | 1.695 | 2.486 | 0.013 |
| MMP 1 | 1.365 | 2.522 | 0.012 |
| CCNB1 | 1.893 | 2.646 | 0.008 |
| Ki-67 | 1.697 | 2.649 | 0.008 |
| E2F1 | 1.662 | 2.689 | 0.007 |
| KPNA2 | 1.683 | 2.701 | 0.007 |
| DUSP1 | 1.573 | 2.824 | 0.005 |
| GDF15 | 1.440 | 2.896 | 0.004 |

**Table 7: Cox proportional hazards for Prognostic Genes that are positively associated with good prognosis for breast cancer (Rush study)**

| **Gene_all** | **z (Coef)** | **HR** | **p (Wald)** |
|---|---|---|---|
| GSTM2 | -3.275 | 0.752 | 0.001 |
| GSTM1 | -2.946 | 0.772 | 0.003 |
| C8orf4 | -2.639 | 0.793 | 0.008 |
| ELF3 | -2.478 | 0.769 | 0.013 |
| RUNX 1 | -2.388 | 0.609 | 0.017 |
| IL6ST | -2.350 | 0.738 | 0.019 |
| AAMP | -2.325 | 0.715 | 0.020 |
| PR | -2.266 | 0.887 | 0.023 |
| FHIT | -2.193 | 0.790 | 0.028 |
| CD44v6 | -2.191 | 0.754 | 0.028 |
| GREB1 variant c | -2.120 | 0.874 | 0.034 |
| ADAM17 | -2.101 | 0.686 | 0.036 |
| EstR1 | -2.084 | 0.919 | 0.037 |
| NAT1 | -2.081 | 0.878 | 0.037 |
| TNFRSF11B | -2.074 | 0.843 | 0.038 |
| ITGB4 | -2.006 | 0.740 | 0.045 |
| CSF1 | -1.963 | 0.750 | 0.050 |

**Table 8: Cox proportional hazards for Prognostic Genes that are negatively associated with good prognosis for breast cancer (Rush study)**

| **Gene_all** | **z (Coef)** | **HR** | **p (Wald)** |
|---|---|---|---|
| STK15 | 1.968 | 1.298 | 0.049 |
| TFRC | 2.049 | 1.399 | 0.040 |
| ITGB 1 | 2.071 | 1.812 | 0.038 |
| ITGAV | 2.081 | 1.922 | 0.037 |
| MYBL2 | 2.089 | 1.205 | 0.037 |
| MRP3 | 2.092 | 1.165 | 0.036 |
| SKP2 | 2.143 | 1.379 | 0.032 |
| LMNB 1 | 2.155 | 1.357 | 0.031 |
| ALCAM | 2.234 | 1.282 | 0.025 |
| COMT | 2.271 | 1.412 | 0.023 |
| CDC20 | 2.300 | 1.253 | 0.021 |
| GAPDH | 2.307 | 1.572 | 0.021 |
| GRB7 | 2.340 | 1.205 | 0.019 |
| S100A9 | 2.374 | 1.120 | 0.018 |
| S100A7 | 2.374 | 1.092 | 0.018 |
| HER2 | 2.425 | 1.210 | 0.015 |
| ACTR2 | 2.499 | 1.788 | 0.012 |
| S100A8 | 2.745 | 1.144 | 0.006 |
| ENO1 | 2.752 | 1.687 | 0.006 |
| MMP 1 | 2.758 | 1.212 | 0.006 |
| LAPTM4B | 2.775 | 1.375 | 0.006 |
| FGFR4 | 3.005 | 1.215 | 0.003 |
| C17orf37 | 3.260 | 1.387 | 0.001 |

**Table 9: Cox proportional hazards for Prognostic Genes that are positively associated with good prognosis for ER-negative (ER0) breast cancer (Rush study)**

| **Gene_ER0** | **z (Coef)** | **HR** | **p (Wald)** |
|---|---|---|---|
| SEMA3F | -2.465 | 0.503 | 0.014 |
| LAMA3 | -2.461 | 0.519 | 0.014 |
| CD44E | -2.418 | 0.719 | 0.016 |
| AD024 | -2.256 | 0.617 | 0.024 |
| LAMB3 | -2.237 | 0.690 | 0.025 |
| Ki-67 | -2.209 | 0.650 | 0.027 |
| MMP7 | -2.208 | 0.768 | 0.027 |
| GREB1 variant c | -2.019 | 0.693 | 0.044 |
| ITGB4 | -1.996 | 0.657 | 0.046 |
| CRYZ | -1.976 | 0.662 | 0.048 |
| CD44s | -1.967 | 0.650 | 0.049 |

**Table 10: Cox proportional hazards for Prognostic Genes that are negatively associated with good prognosis for ER-negative (ER0) breast cancer (Rush study)**

| **Gene_ER0** | **z (Coef)** | **HR** | **p (Wald)** |
|---|---|---|---|
| S100A8 | 1.972 | 1.212 | 0.049 |
| EEF1A2 | 2.031 | 1.195 | 0.042 |
| TAGLN | 2.072 | 2.027 | 0.038 |
| GRB7 | 2.086 | 1.231 | 0.037 |
| HER2 | 2.124 | 1.232 | 0.034 |
| ITGAV | 2.217 | 3.258 | 0.027 |
| CDH11 | 2.237 | 2.728 | 0.025 |
| COL1A1 | 2.279 | 2.141 | 0.023 |
| C17orf37 | 2.319 | 1.329 | 0.020 |
| COL1A2 | 2.336 | 2.577 | 0.020 |
| ITGB5 | 2.375 | 3.236 | 0.018 |
| ITGA5 | 2.422 | 2.680 | 0.015 |
| RPL41 | 2.428 | 6.665 | 0.015 |
| ALCAM | 2.470 | 1.414 | 0.013 |
| CTHRC1 | 2.687 | 3.454 | 0.007 |
| PTEN | 2.692 | 8.706 | 0.007 |
| FN 1 | 2.833 | 2.206 | 0.005 |

**Table 11: Cox proportional hazards for Prognostic Genes that are positively associated with good prognosis for ER-positive (ER1) breast cancer (Rush study)**

| **Gene_ ER1** | **z (Coef)** | **HR** | **p (Wald)** |
|---|---|---|---|
| GSTM1 | -3.938 | 0.628 | 0.000 |
| HNF3A | -3.220 | 0.500 | 0.001 |
| EstR1 | -3.165 | 0.643 | 0.002 |
| Bcl2 | -2.964 | 0.583 | 0.003 |
| GATA3 | -2.641 | 0.624 | 0.008 |
| ELF3 | -2.579 | 0.741 | 0.010 |
| C8orf4 | -2.451 | 0.730 | 0.014 |
| GSTM2 | -2.416 | 0.774 | 0.016 |
| PR | -2.416 | 0.833 | 0.016 |
| RUNX1 | -2.355 | 0.537 | 0.019 |
| CSF1 | -2.261 | 0.662 | 0.024 |
| IL6ST | -2.239 | 0.627 | 0.025 |
| AAMP | -2.046 | 0.704 | 0.041 |
| TNFRSF11B | -2.028 | 0.806 | 0.043 |
| NAT1 | -2.025 | 0.833 | 0.043 |
| ADAM17 | -1.981 | 0.642 | 0.048 |

**Table 12: Cox proportional hazards for Prognostic Genes that are negatively associated with good prognosis for ER-positive (ER1) breast cancer (Rush study)**

| **Gene_ER1** | **z (Coef)** | **HR** | **p (Wald)** |
|---|---|---|---|
| HSPA1B | 1.966 | 1.382 | 0.049 |
| AD024 | 1.967 | 1.266 | 0.049 |
| FGFR4 | 1.991 | 1.175 | 0.047 |
| CDK4 | 2.014 | 1.576 | 0.044 |
| ITGB 1 | 2.021 | 2.163 | 0.043 |
| EPHB2 | 2.121 | 1.342 | 0.034 |
| LYRIC | 2.139 | 1.583 | 0.032 |
| MYBL2 | 2.174 | 1.273 | 0.030 |
| PGF | 2.176 | 1.439 | 0.030 |
| EZH2 | 2.199 | 1.390 | 0.028 |
| HSPA1A | 2.209 | 1.452 | 0.027 |
| RPLPO | 2.273 | 2.824 | 0.023 |
| LMNB 1 | 2.322 | 1.529 | 0.020 |
| IL-8 | 2.404 | 1.166 | 0.016 |
| C6orf66 | 2.468 | 1.803 | 0.014 |
| GAPDH | 2.489 | 1.950 | 0.013 |
| P16-INK4 | 2.490 | 1.541 | 0.013 |
| CLIC1 | 2.557 | 2.745 | 0.011 |
| ENO1 | 2.719 | 2.455 | 0.007 |
| ACTR2 | 2.878 | 2.543 | 0.004 |
| CDC20 | 2.931 | 1.452 | 0.003 |
| SKP2 | 2.952 | 1.916 | 0.003 |
| LAPTM4B | 3.124 | 1.558 | 0.002 |

**Table 13: Validation of Prognostic Genes in SIB data sets.**

| **Table 13** Official Symbol | EMC2 ∼Est | EMC2∼SE | EMC2∼t | JRH1∼Est | JRH1∼SE | JRH1∼t | JRH2∼Est | JRH2∼SE | JRH2∼t | MGH∼Est | MGH∼SE | MGH∼t | NCH∼Est | NCH∼SE | NCH∼t | NKI∼Est | NKI∼SE | NKI∼t |
|---|---|---|---|---|---|---|---|---|---|---|---|---|---|---|---|---|---|---|
| AAMP | NA | NA | NA | -0.05212 | 0.50645 | -0.10291 | 0.105615 | 1.01216 | 0.104346 | -0.26943 | 0.620209 | -0.43441 | 0.088826 | 0.283082 | 0.313782 | 0.312939 | 0.228446 | 1.36986 |
| ABCC1 | NA | NA | NA | NA | NA | NA | 2.36153 | 0.76485 | 3.087573 | 0.253516 | 0.284341 | 0.891591 | 0.213191 | 0.154486 | 1.390002 | 0.094607 | 0.259279 | 0.366298 |
| ABCC3 | NA | NA | NA | 0.386945 | 0.504324 | 0.767255 | 0.305901 | 0.544322 | 0.561985 | 0.126882 | 0.221759 | 0.572162 | -0.00756 | 0.167393 | -0.04517 | 0.06613 | 0.096544 | 0.684974 |
| ABR | NA | NA | NA | 0.431151 | 0.817818 | 0.527197 | 0.758422 | 1.0123 | 0.749207 | NA | NA | NA | NA | NA | NA | -0.06114 | 0.095839 | -0.63795 |
| ACTR2 | NA | NA | NA | NA | NA | NA | -0.26297 | 0.4774 | -055084 | 0071853 | 0.205648 | 0.349398 | 0.131215 | 0.267434 | 0.490644 | 0.539449 | 0.254409 | 2.120401 |
| ADAM 17 | NA | NA | NA | 0.078212 | 0.564555 | 0.138538 | -0.20948 | 1.06045 | -0.19754 | 0.29698 | 0.435924 | 0.681266 | -0.18523 | 0.407965 | -0.45402 | 0.068689 | 0.12741 | 0.539115 |
| ADM | NA | NA | NA | NA | NA | NA | 0.320052 | 0.201407 | 1.589081 | 0.225324 | 0.142364 | 1.582732 | 0.314064 | 0.201161 | 1.561257 | 0.264131 | 0.06376 | 4.142582 |
| LYPD6 | NA | NA | NA | NA | NA | NA | NA | NA | NA | -0.38423 | 0.120883 | -3.17855 | -0.23802 | 0.209786 | -1.1346 | -04485 | 0.106865 | -4.19691 |
| AKT3 | NA | NA | NA | NA | NA | NA | -2.10931 | 1.58606 | -1.32991 | -1.43148 | 0.576851 | -2.48154 | 0.181912 | 0.147743 | 1.231273 | 0.149731 | 0.140716 | 1.064065 |
| ALCAM | NA | NA | NA | -0.17112 | 0.224449 | -0.7624 | 0.120168 | 0.212325 | 0.565963 | -0.36428 | 0.239833 | -1.51888 | 0.002712 | 0.084499 | 0.032094 | -0.3019 | 0.094459 | -3.19609 |
| APEX1 | NA | NA | NA | 0.068917 | 0.410873 | 0.167732 | -0.02247 | 0.790107 | -0.02843 | -0.07674 | 0.181782 | -0.42215 | -0.00097 | 0.268651 | -0.00361 | -0.13398 | 0.232019 | -0.57746 |
| ARF1 | NA | NA | NA | 0.839013 | 0.346692 | 2.420053 | 0.369609 | 0.40789 | 0.906149 | 2.03958 | 0.804729 | 2.534493 | -0.15337 | 0.204529 | -0.74984 | 0.944168 | 0.204641 | 4.613777 |
| AURKA | NA | NA | NA | 0.488329 | 0.248241 | 1.967157 | 0.285095 | 0.243026 | 1.173105 | 0.270093 | 0.169472 | 1.593732 | -0.07663 | 0.213247 | -0.35934 | 0.643963 | 0.101097 | 6.369754 |
| BAD | NA | NA | NA | 0.027049 | 0.547028 | 0.049446 | 0.121904 | 0.587599 | 0.207461 | NA | NA | NA | 0.38364 | 0.389915 | 0.983907 | 0.149641 | 0.221188 | 0.676533 |
| BAG1 | NA | NA | NA | 0.505074 | 0.709869 | 0.711503 | -0.13983 | 0.36181 | -0.38648 | -0.36295 | 0.282963 | -1.28267 | -0.11976 | 0.203911 | -0.58733 | -0.41603 | 0.138093 | -3.01265 |
| BBC3 | NA | NA | NA | NA | NA | NA | 0.182425 | 0.78708 | 0.231774 | NA | NA | NA | 0.056993 | 0.249671 | 0.228274 | -0.5633 | 0.158825 | -3.54669 |
| BCAR3 | NA | NA | NA | NA | NA | NA | -0.29238 | 0.522706 | -0.55935 | -0.41595 | 0.216837 | -1.91825 | 0.072246 | 0.304443 | 0.237306 | -0.26067 | 0.114992 | -2.26685 |
| BCL2 | NA | NA | NA | -1.10678 | 0.544697 | -2.03192 | 0.124104 | 0.228026 | 0.544254 | -2.47368 | 1.23296 | -2.00629 | NA | NA | NA | -0.30738 | 0.079518 | -3.86557 |
| BIRC5 | NA | NA | NA | -0.40529 | 0.608667 | -066586 | 0.319899 | 0.242736 | 1.317889 | NA | NA | NA | 0.268836 | 0.122325 | 2197719 | 0.390779 | 0.069127 | 5.6531 |
| BTRC | NA | NA | NA | NA | NA | NA | 0.017988 | 0.648834 | 0.027723 | NA | NA | NA | - 0.63958 | 0.485936 | 1.31618 | -0.52394 | 0.139699 | -3.75051 |
| BUB1 | NA | NA | NA | 0.84036 | 0.319874 | 2.627159 | 0.565139 | 0.322406 | 1.75288 | 0.206656 | 0.268687 | 0.769133 | 0.104644 | 0.142318 | 0.735283 | 0.426611 | 0.094852 | 4.49763 |
| C10orf116 | NA | NA | NA | -0.1418 | 0.261554 | -0.54216 | 0.036378 | 0.182183 | 0.19968 | NA | NA | NA | 0.064337 | 0.14087 | 0.456713 | -0.22589 | 0.082836 | -2.72696 |
| C17orf37 | NA | NA | NA | NA | NA | NA | NA | NA | NA | NA | NA | NA | 0.1532 | 0.294177 | 0.520775 | NA | NA | NA |
| TPX2 | NA | NA | NA | NA | NA | NA | 0.311175 | 0.271756 | 1.145053 | NA | NA | NA | -0.01014 | 0.317222 | -0.03198 | 0.536914 | 0.116472 | 4.609812 |
| C8orf4 | NA | NA | NA | NA | NA | NA | -0.06402 | 0.197663 | -0.32386 | -0.07043 | 0.106335 | -0.66236 | -0.03221 | 0.189009 | -0.1704 | -0.3396 | 0.083273 | -4.07813 |
| CAV1 | NA | NA | NA | -0.20701 | 0.254401 | -0.81372 | -0.19588 | 0.289251 | -0.67721 | -0.06896 | 0.2269 | -0.30391 | 0.078825 | 0.340843 | 0.231265 | -0.30885 | 0.133788 | -2.30848 |
| CCL19 | NA | NA | NA | 0.101779 | 0.483649 | 0.21044 | -0.45509 | 0.26597 | -1.71104 | 0.246585 | 0.153468 | 1.606752 | 0.024132 | 0.130045 | 0.185564 | -0.08897 | 0.087102 | -1.02143 |
| CCNB1 | NA | NA | NA | 0.14169 | 0.276165 | 0.513063 | 0.587021 | 0.249935 | 2.348695 | NA | NA | NA | -0.02016 | 0.230327 | -0.08751 | 0.495483 | 0.10424 | 4.75329 |
| CDC20 | NA | NA | NA | -0.82502 | 0.360648 | -2.2876 | 0.075789 | 0.208662 | 0.363213 | 0.095023 | 0.198727 | 0.478159 | 0.482934 | 0.216025 | 2.235547 | 0.35587 | 0.125008 | 2.846778 |
| CDC25A | NA | NA | NA | -0.15046 | 0.724766 | -0.2076 | 0.358589 | 0.638958 | 0.561209 | 0.257084 | 0.227966 | 1.12773 | 0.078265 | 0.111013 | 0.705008 | 0.48387 | 0.105238 | 4.597864 |
| CDC25C | NA | NA | NA | 0.047781 | 0.511454 | 0.093422 | 1.07486 | 0.456637 | 2.353861 | 0.340882 | 0.240266 | 1.418769 | -0.22371 | 0.269481 | -0.83013 | 0.287063 | 0.136568 | 2.101979 |
| CDH11 | NA | NA | NA | -0.55211 | 0.469473 | -1.17601 | 0.072308 | 0.265898 | 0.27194 | 0.028252 | 0.199053 | 0.141931 | -0.0883 | 0.124418 | -0.70971 | 0.13223 | 0.097541 | -1.35564 |
| CDK4 | NA | NA | NA | NA | NA | NA | 0.759572 | 0.757398 | 1.00287 | 0.18468 | 0.129757 | 1.423276 | 0.304045 | 0.17456 | 1.741779 | 0.267465 | 0.148641 | 1.799403 |

| **Table 13** Official Symbol | EMC2 ∼Est | EMC2∼SE | HMC2∼t | JRH1∼Est | JRH1∼SE | JRH1∼t | JRH2∼Est | JRH2∼SE | JRH2∼t | MGH∼Est | MGH∼SE | MGH∼t | NCH∼Est | NCH∼SE | NCH∼t | NKI∼Est | NKI∼SE | NKI∼t |
|---|---|---|---|---|---|---|---|---|---|---|---|---|---|---|---|---|---|---|
| SCUBE2 | NA | NA | NA | NA | NA | NA | -0.0454 | 0.120869 | -0.37564 | NA | NA | NA | -0.01783 | 0.063429 | -0.28108 | -0.24635 | 0.048622 . | -5.0667 |
| CENPA | NA | NA | NA | NA | NA | NA | 0.296857 | 0.253493 | 1.171066 | NA | NA | NA | 0.225878 | 0.249928 | 0.903772 | 0.467131 | 0.081581 | 5.726013 |
| CHAF1B | NA | NA | NA | 0.591417 | 0.58528 | 1.010486 | 0.284056 | 0.637446 | 0.445616 | 0.47534 | 0.323193 | 1.470762 | 0.233081 | 0.291389 | 0.799896 | 0.519868 | 0.125204 | 4.152168 |
| CLDN4 | NA | NA | NA | -0.54144 | 0.470758 | -1.15014 | 0.33033 | 0.351865 | 0.938798 | 0.185116 | 0.314723 | 0.588187 | -0.23129 | 0.426627 | -0.54213 | 0.564756 | 0.210595 | 2.681716 |
| CLIC1 | NA | NA | NA | 0.678131 | 0.359483 | 1.886406 | 0.764626 | 0.767633 | 0.996083 | 0.171995 | 0.821392 | 0.209395 | -0.05548 | 0.414451 | -0.13385 | 0.383134 | 0.165674 | 2.312578 |
| COL1A1 | NA | NA | NA | NA | NA | NA | 0.273073 | 0.249247 | 1.095592 | NA | NA | NA | 0.004033 | 0.146511 | 0.027527 | NA | NA | NA |
| COL1A2 | NA | NA | NA | NA | NA | NA | 0.216939 | 0.367138 | 0.590892 | 0.157848 | 0.123812 | 1.274901 | 0.057815 | 0.163831 | 0.352894 | -0.00235 | 0.064353 | -0.03653 |
| COMT | NA | NA | NA | 0.749278 | 0.356566 | 2.101373 | -0.05068 | 0.448567 | -0.11298 | -245771 | 1.02805 | -2.39065 | 0.526063 | 0.226489 | 2.322687 | -0.00764 | 0.129967 | -0.05878 |
| CRYZ | NA | NA | NA | NA | NA | NA | -0.31201 | 0.303615 | -1.02766 | -0.53751 | 0.214408 | -2.50696 | -0.32472 | 0.253244 | -1.28224 | -0.25514 | 0.124909 | -2.04264 |
| CSF1 | NA | NA | NA | NA | NA | NA | -1.40833 | 1.21432 | -1.15977 | NA | NA | NA | -0.14894 | 0.352724 | -0.42226 | -0.11194 | 0.240555 | -0.46532 |
| CTHRC1 | NA | NA | NA | NA | NA | NA | NA | NA | NA | 0.574897 | 0.535382 | 1.073807 | -0.08389 | 0.137325 | -0.6109 | 0.024002 | 0.097692 | 0.245691 |
| CXCL12 | NA | NA | NA | -0.36476 | 0.372499 | -0.97921 | -0.4566 | 0.219587 | -2.07935 | NA | NA | NA | -0.08863 | 0.138097 | -0.64183 | -0.36944 | 0.138735 | -2.66293 |
| CXCL14 | NA | NA | NA | -0.23692 | 0.333761 | -0.70985 | 0.361375 | 0.159544 | 2.265049 | NA | NA | NA | -0.06592 | 0.093353 | -0.70609 | -0.16877 | 0.054117 | -3.11866 |
| CYR61 | NA | NA | NA | 0.310818 | 0.515557 | 0.602878 | -0.24435 | 0.252867 | -0.9663 | 0.571476 | 0.323144 | 1.768487 | -0.11281 | 0.164296 | -0.68663 | 0.087147 | 0.082372 | 1.057965 |
| DICER1 | NA | NA | NA | NA | NA | NA | -0.33943 | 0.39364 | -0.8623 | 0.038811 | 0.409835 | 0.0947 | 0.086141 | 0.143687 | 0.599504 | -046887 | 0.150367 | -3.11814 |
| DLC1 | NA | NA | NA | 0.13581 | 0.37927 | 0358083 | -0.4102 | 0.387258 | -1.05923 | -0.09793 | 0.247069 | -0.39638 | -0.03473 | 0.238947 | -0.14533 | -0.35001 | 0.130472 | -2.68262 |
| TNFRSF10B | NA | NA | NA | -0.09001 | 0.619057 | -0.1454 | 0.80742 | 0.544479 | 1.482922 | 0.159018 | 0.456205 | 0.348567 | -0.19927 | 0.160381 | -124248 | 0.053214 | 0.164091 | 0.324294 |
| DUSP1 | NA | NA | NA | -0.20229 | 0.200782 | -1.00753 | -0.02736 | 0.224043 | -0.12212 | NA | NA | NA | -0.03006 | 0.152909 | -0.19657 | -0.0472 | 0.09086 | -0.51952 |
| E2F1 | NA | NA | NA | NA | NA | NA | 0.845576 | 0.685556 | 1.233416 | -1.06849 | 0.824212 | -1.29638 | 0.356102 | 0.38254 | 0.930888 | 0.617258 | 0.121385 | 5.085126 |
| EEF1A2 | 0.26278 | 0.091435 | 2.873951 | NA | NA | NA | 0.362569 | 0.17103 | 2.119915 | NA | NA | NA | -0.0028 | 0.233293 | -0.01199 | -0.01585 | 0.06608 | -0.23987 |
| ELF3 | NA | NA | NA | 1.34589 | 0.628064 | 2.142919 | 0.569231 | 0.430739 | 1.321522 | 0.209853 | 0.239225 | 0.87722 | 0.026264 | 0.109569 | 0.2397 | 0.165848 | 0.143091 | 1.159039 |
| ENO1 | NA | NA | NA | NA | NA | NA | 0.179739 | 0.312848 | 0.574525 | NA | NA | NA | -0.01727 | 0.097939 | -0.17629 | 0.3682 | 0.094778 | 3.884888 |
| EPHB2 | NA | NA | NA | 0.155831 | 0.717587 | 0.21716 | -0.19469 | 0.90381 | -0.21541 | 1.38257 | 0.444196 | 3.112522 | -0.46953 | 0.395102 | -1.18837 | 0.318437 | 0.123672 | 2.574851 |
| ERBB2 | NA | NA | NA | -0.32795 | 0.215691 | -1.52044 | 0.065275 | 0.189094 | 0.3452 | 0.314084 | 0.126321 | 2.486396 | 0.23616 | 0.121533 | 1.943176 | 0.08469 | 0.056744 | 1.492504 |
| ERBB4 | NA | NA | NA | NA | NA | NA | -0.12516 | 0.182846 | -0.68451 | -0.13567 | 0.114364 | -1.18626 | 0.191218 | 0.114326 | 1.672568 | -0.28508 | 0.066294 | -4.30028 |
| ESRRG | NA | NA | NA | NA | NA | NA | 0.122595 | 0.204322 | 0.600009 | 0.356845 | 0.216506 | 1.648199 | 0.023341 | 0.078378 | 0.297795 | -0.16542 | 0.093598 | -1.76733 |
| ESR1 | NA | NA | NA | -0.14448 | 0.127214 | -1.13569 | 0.009283 | 0.107091 | 0.086687 | -0.12127 | 0.111184 | -1.09075 | 0.127143 | 0.109672 | 1.159302 | -0.16933 | 0.044665 | -3.79121 |
| EZH2 | NA | NA | NA | NA | NA | NA | 0.36213 | 0.244107 | 1.483489 | NA | NA | NA | 0.008861 | 0.200897 | 0.044106 | 0.478266 | 0.107424 | 4.452134 |
| F3 | NA | NA | NA | 0.719395 | 0.524742 | 1.37095 | -0.21237 | 0.363632 | -0.58402 | -0.00167 | 0.448211 | -0.00372 | -0.13187 | 0.134218 | -0.98248 | -0.29217 | 0.093753 | -3.11637 |
| FGFR4 | NA | NA | NA | 0.864262 | 0.479596 | 1.802063 | 0.451249 | 0.296065 | 1.524155 | 0.230309 | 0.229234 | 1.00469 | -0.15142 | 0.109674 | -1.3806 | -0.04922 | 0.146198 | -0.33666 |
| FHIT | NA | NA | NA | 1.00058 | 0.938809 | 1.065797 | -1.58314 | 0.766553 | -2.06527 | 0.087228 | 0.322399 | 0.270559 | -0.08366 | 0.344886 | -0.24256 | -0.1378 | 0.121745 | -1.13183 |
| FN1 | NA | NA | NA | 0.056943 | 0.154068 | 0.369595 | 0.282152 | 0.407361 | 0.692634 | 0.417442 | 0.859619 | 0.485613 | -0.05187 | 0.111777 | -0.46402 | 0.112875 | 0.066759 | 1.690796 |
| FOXA1 | NA | NA | NA | NA | NA | NA | 0.054619 | 0.1941 | 0.281398 | NA | NA | NA | -0.04211 | 0.103534 | -0.40677 | -0.08953 | 0.043624 | -2.05225 |
| FUS | NA | NA | NA | NA | NA | NA | 2.73816 | 1.95693 | 1.399212 | -0.18397 | 0.269637 | -0.68227 | 0.119801 | 0.199389 | 0.600841 | 0.115971 | 0.188545 | 0.615084 |

| **Table 13** Official Symbol | EMC2 ∼Est | EMC2∼SE | EMC2∼t | JRH1∼Est | JRH1∼SE | JRH1∼t | JRH2∼Est | JRH2∼SE | JRH2∼t | MGH∼Est | MGH∼SE | MGH∼t | NCH∼Est | NCH∼SE | NCH∼t | NKI∼Est | NKI∼SE | NKI∼t |
|---|---|---|---|---|---|---|---|---|---|---|---|---|---|---|---|---|---|---|
| GADD45A | NA | NA | NA | NA | NA | NA | -0.09194 | 0.324263 | -0.28352 | -0.33447 | 0.236846 | -1.41219 | -0.43753 | 0.333292 | -1.31276 | -0.15889 | 0.115794 | -1.37217 |
| GAPDH | -0.00386 | 0.125637 | -0.03075 | 0.869317 | 0.274798 | 3.163476 | 0.728889 | 0.497848 | 1.464079 | NA | NA | NA | 0.396067 | 0.169944 | 2.330574 | 0.286211 | 0.073946 | 3.870541 |
| GATA3 | NA | NA | NA | -0.33431 | 0.127225 | -2.62767 | -0.00759 | 0.145072 | -0.05233 | 0.190453 | 0.170135 | 1.119423 | 0.058244 | 0.115942 | 0.502355 | -0.13285 | 0.054984 | -2.41625 |
| GBP2 | NA | NA | NA | 0.120416 | 0.247997 | 0.485554 | -0.49134 | 0.289525 | -1.69704 | 0.517501 | 0.299148 | 1.729916 | 0.082647 | 0.173301 | 0.4769 | -0.19825 | 0.1358 | -1.45985 |
| GDF15 | NA | NA | NA | 0.219861 | 0.231613 | 0.94926 | 0.317951 | 0.183188 | 1.735654 | NA | NA | NA | 0.200247 | 0.14325 | 1.397885 | 0.052347 | 0.063101 | 0.829563 |
| GRB7 | NA | NA | NA | -0.46505 | 0.485227 | -0.95842 | 0.143585 | 0.218034 | 0.658544 | NA | NA | NA | 0.027699 | 0.459937 | 0.060224 | 0126284 | 0.054856 | 2.302117 |
| GSTM1 | NA | NA | NA | NA | NA | NA | NA | NA | NA | NA | NA | NA | NA | NA | NA | -0.18141 | 0.14912 | -1.21652 |
| GSTM2 | NA | NA | NA | NA | NA | NA | NA | NA | NA | NA | NA | NA | NA | NA | NA | -0.15655 | 0.118111 | -1.32547 |
| GSTM3 | NA | NA | NA | -1.19919 | 0.478486 | -2.50622 | -0.08173 | 0.176832 | -0.46219 | NA | NA | NA | -0.09058 | 0.129247 | -0.70086 | -0.336 | 0.086817 | -3.87028 |
| HOXB13 | NA | NA | NA | NA | NA | NA | 0.780988 | 0.524959 | 1.487712 | 0.461343 | 0.122399 | 3.769173 | 0.453876 | 0.324863 | 1.39713 | 0.161713 | 0.053047 | 3.048485 |
| OTUD4 | NA | NA | NA | NA | NA | NA | -0.54088 | 1.59038 | -0.34009 | 0.154269 | 0.633438 | 0.243542 | 0.150174 | 0.149267 | 1.006076 | -0.08847 | 0.130112 | -0.67992 |
| HSPA1A | NA | NA | NA | 0.199478 | 0.304533 | 0.655029 | 0.56215 | 0.592113 | 0.949396 | NA | NA | NA | 0.187486 | 0.231047 | 0.811463 | 0.174571 | 0.117296 | 1.488295 |
| HSPA1B | NA | NA | NA | NA | NA | NA | 0.60089 | 0.32867 | 1.828247 | NA | NA | NA | NA | NA | NA | 0.249602 | 0.129038 | 1.934329 |
| HSPA8 | NA | NA | NA | 0.88406 | 0.420719 | 2.101308 | 1.13504 | 0.667937 | 1.699322 | 0.647034 | 0.346081 | 1.869603 | 0.208652 | 0.225656 | 0.924646 | 0.054243 | 0.178314 | 0.304198 |
| IDH2 | NA | NA | NA | -0.0525 | 0.232201 | -0.22611 | 0.151299 | 0.327466 | 0.46203 | NA | NA | NA | 0.265828 | 0.105592 | 2.517501 | 0.284862 | 0.089145 | 3.195498 |
| IGF1R | NA | NA | NA | -0.62963 | 0.509985 | -1.23461 | -0.05773 | 0.176259 | -0.32753 | -0.11077 | 0.162941 | -0.67982 | -0.37931 | 0.371019 | -1.02236 | -0.13655 | 0.08362 | -1.63299 |
| IGFBP7 | NA | NA | NA | NA | NA | NA | 0.047112 | 0.479943 | 0.098162 | NA | NA | NA | 0.163138 | 0.200674 | 0.81295 | 0.06541 | 0.10077 | 0.649097 |
| IL11 | NA | NA | NA | NA | NA | NA | 1.191111 | 1.41017 | 0.844678 | NA | NA | NA | -0.17423 | 0.144228 | -1.20804 | -0.048 | 0.126254 | -0.38015 |
| IL17RB | NA | NA | NA | NA | NA | NA | 0.143131 | 0.294647 | 0.485771 | -0.44343 | 0.132744 | -3.3405 | NA | NA | NA | -0.01632 | 0.122679 | -0.13305 |
| IL6ST | NA | NA | NA | -0.08851 | 0.151324 | -058488 | -0.00958 | 0.287723 | -0.03329 | -0.76052 | 0.386504 | -1.96769 | -0.4336 | 0.319875 | -1.35553 | -0.41477 | 0.111102 | -3.73322 |
| IL8 | NA | NA | NA | 0.222258 | 0.235694 | 0.942994 | 0.262285 | 0.346572 | 0.756798 | -0.12567 | 0.154036 | -0.81583 | -1.28729 | 0.493461 | - 2.6087 | 0.171912 | 0.07248 | 2.371858 |
| INHBA | NA | NA | NA | 0.095254 | 0.476446 | 0.199927 | 0.342597 | 0.27142 | 1.262239 | NA | NA | NA | -0.12767 | 0.132531 | -0.96331 | 0.133895 | 0.111083 | 1.20536 |
| IRF1 | NA | NA | NA | 0.87337 | 0.941443 | 0.927693 | -0.39282 | 0.392589 | -1.00059 | 0.474132 | 0.503423 | 0.941816 | -0.2456 | 0.294202 | -0.8348 | -0.08017 | 0.171067 | -0.46864 |
| ITGA4 | NA | NA | NA | NA | NA | NA | -0.91318 | 0.542311 | -1.68388 | NA | NA | NA | 0.034844 | 0.074049 | 0.470549 | -0.05101 | 0.133497 | -0.38211 |
| ITGA5 | NA | NA | NA | 1.44044 | 0.636806 | 2.261976 | 0.97846 | 0.67341 | 1.452993 | 0.206218 | 0.263291 | 0.783232 | 0.367111 | 0.333768 | 1.099899 | 0.500604 | 0.163986 | 3.052724 |
| ITGAV | NA | NA | NA | 0.14845 | 0.345246 | 0.429983 | 0.383127 | 0.60722 | 0.630953 | -0.23212 | 0.278464 | -0.83358 | -0.14166 | 0.222286 | -0.6373 | -0.21993 | 0.158945 | -1.38371 |
| ITGB1 | NA | NA | NA | 1.22836 | 0.683544 | 1.797046 | -0.0587 | 1.73799 | -0.03378 | -0.13651 | 0.121624 | -1.12236 | -0.52799 | 0.346298 | -1.52468 | 0.150333 | 0.133426 | 1.126714 |
| ITGB4 | NA | NA | NA | 0.548277 | 0.334628 | 1.638467 | 0.252015 | 0.365768 | 0.689002 | -0.12971 | 0.168517 | -0.76973 | 0.189568 | 0.163609 | 1.158665 | 0.166748 | 0.175308 | 0.951172 |
| ITGB5 | NA | NA | NA | -0.17231 | 0.250618 | -0.68752 | 0.037961 | 0.401861 | 0.094464 | 0.682674 | 0.74847 | 0.912093 | -0.04952 | 0.16668 | -0.29707 | 0.010302 | 0.104545 | 0.098544 |
| MK167 | NA | NA | NA | -0.43304 | 0.708832 | -0.61092 | 0.482583 | 0.321739 | 1.499921 | NA | NA | NA | 0.128582 | 0.129422 | 0.99351 | 0.397232 | 0.176102 | 2.255693 |
| KIAA1199 | NA | NA | NA | NA | NA | NA | -0.02195 | 0.382802 | -0.05735 | 0.081394 | 0.121221 | 0.671448 | NA | NA | NA | 0.238809 | 0.113748 | 2.099457 |
| KPNA2 | 0.301662 | 0.171052 | 1.763569 | -0.5507 | 0.55364 | -0.99468 | 0.21269 | 0.256724 | 0.828477 | -1.6447 | 1.00101 | -1.64304 | 0.213725 | 0.196767 | 1.086183 | 0.422135 | 0.089135 | 4.735922 |
| LAMA3 | NA | NA | NA | -0.74591 | 0.563373 | -1.32401 | -0.21092 | 0.29604 | -0.71245 | NA | NA | NA | -0.03143 | 0.133752 | -0.23497 | -0.30023 | 0.122124 | -2.45838 |
| LAMB3 | NA | NA | NA | NA | NA | NA | 0.345497 | 0.263827 | 1.309559 | 0.03108 | 0.139904 | 0.222154 | 0.106874 | 0.139587 | 0.765644 | -0.03167 | 0.069644 | -0.45477 |

| **Table 13** Official Symbol | EMC2 ∼Est | HMC2∼SE | EMC2∼t | JRH1∼Est | JRH1∼SE | JRH1∼t | JRH2∼Est | JRH2∼SE | JRH2∼t | MOH∼Est | MGH∼SE | MGH∼t | NCH∼Est | NCH∼SE | NCH∼t | NKI∼Est | NKI∼SE | NKI∼t |
|---|---|---|---|---|---|---|---|---|---|---|---|---|---|---|---|---|---|---|
| LAPTM4B | NA | NA | NA | NA | NA | NA | -0.04029 | 0.234986 | -0.17148 | 0.352765 | 0.40304 | 0.875261 | 0.156358 | 0.140366 | 1.113931 | 0.334588 | 0.083358 | 4.013853 |
| LMNB1 | NA | NA | NA | 0.648703 | 0.285233 | 2.274292 | 0.621431 | 0.389912 | 1.593772 | NA | NA | NA | -0.1517 | 0.242463 | -0.62567 | 0.461325 | 0.98382 | 4.689115 |
| LRIG1 | NA | NA | NA | NA | NA | NA | -0.00217 | 0.260339 | -0.00832 | 061468 | 0.216033 | -2.84532 | -0.24368 | 0.172969 | -1.40878 | -0.50209 | 0.1119 | -4.48694 |
| MTDH | NA | NA | NA | NA | NA | NA | -0.10827 | 0.493025 | -0.21961 | 0.084824 | 0.292285 | 0.290209 | 0.039288 | 0.233351 | 0.168365 | 0.430557 | 0.145357 | 2.962066 |
| MCM2 | NA | NA | NA | 0.875004 | 0.492588 | 1.77634 | 0.77667 | 0.376275 | 2.064102 | 0.118904 | 0.288369 | 0.412333 | 0.586577 | 0.252123 | 2.326551 | 0.504911 | 0.154078 | 3.276983 |
| MELK | NA | NA | NA | 0.850914 | 0.313784 | 2.711783 | 0.16347 | 0.256575 | 0.637124 | NA | NA | NA | 0.216763 | 0.1352 | 1.603277 | 0.471343 | 0.103644 | 4.547711 |
| MGMT | NA | NA | NA | NA | NA | NA | 0.151967 | 0.583459 | 0.260459 | 0.267185 | 0.295678 | 0.903635 | -0.37332 | 0.507157 | -0.73611 | -0.14716 | 0.165874 | -0.88716 |
| MMP1 | NA | NA | NA | 0.43277 | 0.16023 | 2.70093 | -0.02427 | 0.158939 | -0.15272 | 0.180359 | 0.078781 | 2.289386 | 0.559716 | 0.331212 | 1.689903 | 0.167053 | 0.064595 | 2.586172 |
| MMP7 | NA | NA | NA | 0.198055 | 0.143 | 1.385 | 0.106475 | 0.193338 | 0.550719 | -1.06791 | 1.30502 | -0.81831 | 0.012294 | 0.101346 | 0.121311 | NA | NA | NA |
| MYBL2 | NA | NA | NA | 0.731162 | 0.267911 | 2.729123 | 0.098974 | 0.600361 | 0.164857 | 0.612646 | 0.509356 | 1.202785 | 0.396938 | 0.171503 | 2.314467 | 0.751827 | 0.151477 | 4.963308 |
| NAT1 | NA | NA | NA | -0.57746 | 15.1186 | -0.0382 | -0.01397 | 0.117033 | -0.11939 | -0.05035 | 0.105736 | -0.47614 | -0.15619 | 0.139368 | -1.12073 | -0.20435 | 0.058054 | -3.52 |
| PGF | NA | NA | NA | 0.901309 | 0.501058 | 1.798812 | 1.43389 | 1.27617 | 1.123589 | NA | NA | NA | 0.05255 | 0.14245 | 0.368898 | 0.055127 | 0.36118 | 0.152631 |
| PGR | NA | NA | NA | NA | NA | NA | -0.33243 | 0.276025 | -1.20435 | -0.95852 | 0.593621 | -1.61469 | -0.01033 | 0.08386 | -0.12312 | -0.30421 | 0.073055 | -4.1.6405 |
| PRDX1 | NA | NA | NA | NA | NA | NA | -0.41082 | 0.47383 | -0.86703 | NA | NA | NA | 0.253047 | 0.182621 | 1.38564 | 0.231612 | 0.161791 | 1.431551 |
| PTEN | NA | NA | NA | -0.17429 | 0.629039 | -0.27708 | -0.15599 | 0.541475 | -0.28808 | -0.10814 | 0.287261 | -0.37645 | 0.113229 | 0.228164 | 0.496261 | -0.3204 | 0.149745 | -2.13962 |
| RPI41 | NA | NA | NA | NA | NA | NA | 1.02038 | 1.83528 | 0.555981 | 0.213155 | 0.288282 | 0.739398 | 0.030854 | 0.188269 | 0.163881 | -0.08602 | 0.122667 | -0.70126 |
| RPLP0 | NA | NA | NA | 0.398754 | 0.282913 | 1.409458 | 0.246775 | 1.2163 | 0.20289 | 0.488909 | 0.174981 | 2.794069 | 0.004595 | 0.198497 | 0.023148 | 0.0081014 | 0.079365 | 0.102105 |
| RRM2 | NA | NA | NA | NA | NA | NA | 0.196643 | 0.262745 | 0.748418 | NA | NA | NA | 0.229458 | 0.11665 | 1.967064 | 0.434693 | 0.152104 | 2.857867 |
| RUNX1 | NA | NA | NA | -0.22834 | 0.318666 | -0.71656 | 0.302803 | 0.420043 | 0.720886 | 0.277566 | 0.267511 | 1.037587 | 0.124568 | 0.088457 | 1.408231 | -0.18878 | 0.138365 | -1.36435 |
| S100A8 | NA | NA | NA | NA | NA | NA | 0.066629 | 0.11857 | 0.561939 | NA | NA | NA | 0.142073 | 0.080349 | 1.768194 | 0.094631 | 0.041656 | 2.271738 |
| S100A9 | NA | NA | NA | NA | NA | NA | 0.111103 | 0.13176 | 0.843223 | NA | NA | NA | 0.090314 | 0.058415 | 1.546083 | 0.111093 | 0.045472 | 2.443086 |
| S100B | NA | NA | NA | 0.097319 | 0.589664 | 0.165041 | -0.2365 | 0.349444 | -0.67678 | NA | NA | NA | 0.239753 | 0.145105 | 1.652272 | 0.195383 | 0.295751 | 0.660633 |
| S100P | NA | NA | NA | 0.378047 | 0.120687 | 3.132458 | 0.302607 | 0.133752 | 2.262448 | NA | NA | NA | 0.202856 | 0.092114 | 2.202218 | 0.103276 | 0.04811 | 2.146677 |
| SEMA3F | NA | NA | NA | -0.27556 | 0.615782 | -0.4475 | 0.498631 | 0.616195 | 0.80921 | 0.107802 | 0.274191 | 0.393164 | -0.17978 | 0.185166 | -0.97092 | NA | NA | NA |
| SKIL | NA | NA | NA | NA | NA | NA | 0.026279 | 0.587743 | 0.044712 | NA | NA | NA | 0.143484 | 0.103564 | 1.385462 | 0.124124 | 0.120741 | 1.028019 |
| SKP2 | NA | NA | NA | NA | NA | NA | 0.2502 | 0.469372 | 0.533053 | 0.470759 | 0.2802 | 1.680082 | -0.71691 | 0.354699 | -2.02117 | 0.056728 | 0.128585 | 0.441174 |
| SNAI1 | NA | NA | NA | NA | NA | NA | 0.165897 | 1.09586 | 0.151385 | 0.163855 | 0.228308 | 0.717693 | 0.04601 | 0.259767 | -0.17711 | 0.057651 | 0.124454 | 0.463235 |
| SYK | NA | NA | NA | -0.26425 | 0.588491 | -0.44903 | -0.22515 | 0.492582 | -0.45707 | NA | NA | NA | 1.30716 | 0.591071 | -2.21151 | 0.178238 | 0.168423 | 1.058276 |
| TAGLN | NA | NA | NA | NA | NA | NA | 0.042223 | 0.251268 | 0.168039 | 0.010727 | 0.098919 | 0.108442 | 0.194543 | 0.115463 | 1.684895 | 0.077881 | 0.119491 | 0.651776 |
| TFRC | NA | NA | NA | -0.91 825 | 0.636275 | -1.44317 | 0.162921 | 0.352486 | 0.462206 | 0.029015 | 0.193689 | 0.149803 | 0.056174 | 0.166875 | 0.336622 | 0.157216 | 0.10845 | 1.449663 |
| TGFB3 | NA | NA | NA | -1.0219 | 0.358953 | -2.84689 | -0.39774 | 0.470041 | -0.84619 | 0.046498 | 0.2296 | 0.202518 | -0.30473 | 0.247338 | -1.23202 | -0.36531 | 0.09592 | -3.80851 |
| TNFRSH1B | NA | NA | NA | NA | NA | NA | -0.10399 | 0.440721 | -0.23595 | -1.15976 | 0.400921 | -2.89274 | -0.2492 | 0.289075 | -0.86207 | -0.22072 | 0.10171 | -2.17005 |
| VTN | NA | NA | NA | -0.18721 | 0.475541 | -0.39367 | -2.39601 | 1.83129 | -1.30837 | NA | NA | NA | 0.048066 | 0.34143 | 0.140779 | -0.05675 | 0.116352 | -0.48774 |
| WISP1 | NA | NA | NA | NA | NA | NA | 0.437936 | 0.592058 | 0.739684 | -0.03674 | 0.212861 | -0.1726 | NA | NA | NA | -0.36317 | 0.153002 | -2.3736 |

| **Table 13** Official Symbol | EMC2 ∼Est | EMC2∼SE | EMC2∼t | JRH1∼Est | JRH1∼SE | JRH1∼t | JRH2∼Est | JRH2∼SE | JRH2∼t | MGH∼Est | MGH∼SE | MGH∼t | NCH∼Est | NCH∼SE | NCH∼t | NKI∼Est | NKI∼SE | NKI∼t |
|---|---|---|---|---|---|---|---|---|---|---|---|---|---|---|---|---|---|---|
| WNT5A | NA | NA | NA | NA | NA | NA | 0.180255 | 0.286462 | 0.629246 | 0.06984 | 0.223411 | 0.312605 | -0.14987 | 0.146576 | -1.02248 | -0.29433 | 0.084559 | -3.48081 |
| C6orf66 | NA | NA | NA | NA | NA | NA | 0.35565 | 0.504627 | 0.704778 | 0.179742 | 0.364806 | 0.492706 | -0.53606 | 0.448343 | -1.19564 | 0.296686 | 0.199046 | 1.49054 |
| FOXO3A | NA | NA | NA | NA | NA | NA | -0.04428 | 0.39855 | -0.1111 | 0.176454 | 0.221502 | 0.796625 | 0.059822 | 0.171485 | 0.348846 | -0.2855 | 0.194121 | -1.47074 |
| GPR30 | NA | NA | NA | 0.01829 | 0.925976 | 0.019752 | -0.298 | 0.747388 | -0.39872 | -0.03208 | 0.1214 | -0.26427 | 0.157898 | 0.174583 | 0.904429 | 0.080079 | 0.104254 | 0.768115 |
| KNTC2 | NA | NA | NA | NA | NA | NA | -0.02315 | 0.289403 | -0.07999 | -0.14241 | 0.246904 | -0.57677 | 0.274706 | 0.14532 | 1.890352 | 0.432186 | 0.120356 | 3.590897 |

| **Table 13** Official Symbol | STNO ∼Est | STNO ∼SE | STNO∼t | STOCK ∼Est | STUCK ∼SE | STOCK ∼t | TRAN S BIG∼Est | TRANS BIG∼SE | TRANS BIG∼t | UCSF ∼Est | UCSF ∼SE | UCSF∼t | UPP∼Est | UPP∼SE | UPP∼t | fe | sefe | |
|---|---|---|---|---|---|---|---|---|---|---|---|---|---|---|---|---|---|---|
| AAMP | 0.189376 | 0.309087 | 0.612695 | 0.836415 | 0.549695 | 1.521598 | 0.051406 | 0.111586 | 0.460681 | 0.770516 | 0.762039 | 1.011124 | 1.25423 | 0.577991 | 2.169982 | 0.146929 | 0.085151 | |
| ABCC1 | NA | NA | NA | 0.640672 | 0.375725 | 1.705162 | NA | NA | NA | NA | NA | NA | 0.274551 | 0.271361 | 1.011756 | 0.281451 | 0.104466 | |
| ABCC3 | 0.311364 | 0.100031 | 3.112675 | 0.166453 | 0.159249 | 1.045237 | NA | NA | NA | 0.381707 | 0.250896 | 1.521375 | 0.178451 | 0.097237 | 1.835219 | 0.172778 | 0.048133 | |
| ABR | 0.095087 | 0.266216 | 0.357181 | 0.08129 | 0.196104 | 0.414525 | NA | NA | NA | -0.17319 | 0.728313 | -0.23779 | -0.16409 | 0.120793 | -1.35847 | -0.06034 | 0.067134 | |
| ACTR2 | NA | NA | NA | 0.302753 | 0.39656 | 0.763448 | NA | NA | NA | NA | NA | NA | 0.21463 | 0.353554 | 0.607064 | 0.199885 | 0.117995 | |
| ADAM17 | NA | NA | NA | 0.437069 | 0.276977 | 1.577997 | NA | NA | NA | 0.35888 | 0.433785 | 0.827322 | 0.131246 | 0.194946 | 0.673243 | 0.129961 | 0.090699 | |
| ADM | NA | NA | NA | 0.555634 | 0.242705 | 2.289339 | 0.025583 | 0.038218 | 0.669405 | NA | NA | NA | 0.361033 | 0.203349 | 1.775435 | 0.119028 | 0.030564 | |
| LYPD6 | NA | NA | NA | -0.42358 | 0.145799 | -2.90525 | -0.06178 | 0.031054 | -1.98944 | NA | NA | NA | -0.1544 | 0.073668 | -2.09587 | -0.12675 | 0.026288 | |
| AKT3 | NA | NA | NA | 0.12232 | 0.182253 | 0.671155 | NA | NA | NA | NA | NA | NA | -0.06832 | 0.125172 | -0.5458 | 0.05204 | 0.071861 | |
| ALCAM | -0.14634 | 0.126842 | -1.15369 | -0.41301 | 0.190485 | -2.16822 | NA | NA | NA | -0.25661 | 0.251874 | -1.01879 | -0.1468 | 0.143998 | -1.01942 | -0.15502 | 0.046361 | |
| APEX1 | 0.005151 | 0.257871 | 0.019976 | 0.739037 | 0.539346 | 1.370247 | NA | NA | NA | -0.96465 | 0.704753 | -1.36878 | 1.23743 | 0.466987 | 2.649817 | 0.019915 | 0.10244 | |
| ARF1 | 0 | 0.107397 | 0 | 0.862387 | 0.279535 | 3.085077 | NA | NA | NA | 0.304097 | 0.58718 | 0.517894 | 0.751279 | 0.361093 | 2.080569 | 0.281544 | 0.07587 | |
| AURKA | 0.38795 | 0.127032 | 3.053955 | 0.688845 | 0.210275 | 3.275924 | 0.020041 | 0.064473 | 0.310835 | -0.0146 | 0.28312 | -0.05156 | 0.427382 | 0.126638 | 3.374832 | 0.262652 | 0.041246 | |
| BAD | -0.30035 | 0.250277 | -120006 | 0.228387 | 0.543493 | 0.420221 | NA | NA | NA | -0.43933 | 0.659711 | -0.66594 | 0.351434 | 0.360157 | 0.97578 | 0.059151 | 0.126378 | |
| BAG1 | NA | NA | NA | -0.39593 | 0.380547 | -1.04043 | NA | NA | NA | 0.516764 | 0.524112 | 0.98598 | 0.380154 | 0.211079 | 1.801003 | -0.16426 | 0.087173 | |
| BBC3 | NA | NA | NA | -0.26155 | 0.219839 | -1.18974 | -0.04709 | 0.086372 | -0.5452 | 0.263477 | 0.606256 | 0.434597 | -0.13039 | 0.141473 | -0.92165 | -0.14598 | 0.061462 | |
| BCAR3 | NA | NA | NA | -0.49692 | 0.265837 | -1.86927 | NA | NA | NA | NA | NA | NA | -0.29435 | 0.182614 | -1.61186 | -028755 | 0.080198 | |
| BCL2 | -0.38181 | 0.112494 | -3.39408 | -0.73699 | 0.228055 | -3.23 162 | NA | NA | NA | -0.3453 | 0.410691 | -0.84078 | -0.11988 | 0.174734 | -0.68605 | -0.32009 | 0.056047 | |
| BIRC5 | 0.190534 | 0.126151 | 1.510365 | 0.582957 | 0.159354 | 3.658251 | 0.007906 | 0.045316 | 0.174454 | 0.357332 | 0.286621 | 1.246706 | 0.43455 | 0.110681 | 3.926148 | 0.186649 | 0.031964 | |
| BTRC | NA | NA | NA | -0.92763 | 0.307218 | -3.01944 | NA | NA | NA | NA | NA | NA | -0.0225 | 0.1807 | -0.12451 | -0.40405 | 0.100468 | |
| BUB1 | 0.357653 | 0.101235 | 3.532899 | 1.09451 | 0.258044 | 4.241563 | 0.014276 | 0.040135 | 0.355694 | 0.376719 | 0.340175 | 1.107427 | 0.469009 | 0.162539 | 2.885517 | 0.154368 | 0.032048 | |
| C10orf11 6 | -0.09621 | 0.085948 | -1.11936 | -0.34745 | 0.112777 | -3.08087 | NA | NA | NA | 0.013111 | 156.117 | 8.40E-05 | -0.00923 | 0.100902 | -0.09148 | -0.13 | 0.042521 | |
| C17orf37 | NA | NA | NA | 0.382862 | 0.185356 | 2.06555 | NA | NA | NA | NA | NA | NA | 0.385651 | 0.113625 | 3.394068 | 0.362223 | 0.092012 | |
| TPX2 | NA | NA | NA | 0800822 | 0.195737 | 4.091316 | NA | NA | NA | 0.213479 | 0.284008 | 0.751665 | 0.44053 | 0.139377 | 3.160708 | 0.480408 | 0.073094 | |
| C8orf4 | NA | NA | NA | -0.36113 | 0.130038 | -2.77713 | NA | NA | NA | NA | NA | NA | 0.0037 | 0.109064 | 0.033921 | -0.18346 | 0.048256 | |
| CAV1 | 0.135002 | 0.093948 | 1.436991 | -0.65852 | 0.275751 | -2.38811 | NA | NA | NA | -0.54391 | 0.428883 | -1.2682 | -0.31503 | 0.150431 | -2.09415 | -0.11726 | 0.058989 | |
| CCL19 | -0.0546 | 2531.93 | -2.16E-05 | -0.15743 | 0.154207 | -1.02087 | NA | NA | NA | 0 | 0.434462 | 0 | -0.1048 | 0.106112 | -0.98765 | -0.05608 | 0.050769 | |
| CCNB1 | 0.37726 | 0.156356 | 2.412827 | 0.828029 | 0.223403 | 3.706436 | NA | NA | NA | -0.35808 | 0.431863 | -0.82915 | 0.611916 | 0.142007 | 4.309055 | 0.456916 | 0.062513 | |
| CDC20 | 0.059565 | 1057.7 | 5.63E-05 | 0.642601 | 0.178622 | 3.597547 | NA | NA | NA | -0.65381 | 0.404188 | -1.61759 | 0.490188 | 0.130676 | 3.751171 | 0.319134 | 0.064899 | |
| CDC25A | 0.288245 | 0.213701 | 1.348824 | 0.168571 | 0.225272 | 0.7483 | NA | NA | NA | -0.31967 | 0.397525 | -0.80414 | 0.330359 | 0.191096 | 1.728759 | 0.267201 | 0.060819 | |

| **Table 13** Official Symbol | STNO ∼Est | STNO ∼SE | STNO∼t | STOCK -Est | STOCK ∼SE | STOCK ~t | TRANS BIG∼Est | TRANS BIG∼SE | TRANS BIG~t | UCSF ∼Est | UCSF ∼SE | UCSF∼t | UPP∼Est | UPP∼SE | UPP~t | fe | sefe | |
|---|---|---|---|---|---|---|---|---|---|---|---|---|---|---|---|---|---|---|
| CDC25C | 0.420797 | 0.155926 | 2.698697 | 1.02036 | 0.337803 | 3.020577 | NA | NA | NA | -0.33774 | 0.477196 | -0.70776 | 0.827213 | 0.232669 | 3.555321 | 0.382935 | 0.077595 | |
| CDH11 | -0.05652 | 0.1231 | -0.45913 | -0.21142 | 0.211537 | -0.99942 | NA | NA | NA | -0.20567 | 0.246195 | -0.83541 | -0.22621 | 0.164541 | -1.37482 | -0.11417 | 0.053045 | |
| CDK4 | 0.279447 | 0.142472 | 1.961417 | 1.40458 | 0.463254 | 3.031987 | NA | NA | NA | -0.37577 | 0.674081 | -0.55746 | 0.814832 | 0.297251 | 2.741225 | 0.305255 | 0.069562 | |
| SCUBE2 | -0.21559 | 0.074112 | -2.90896 | -0.24679 | 0.122745 | -2.01059 | 0.016505 | 0.023486 | 0.702739 | NA | NA | NA | -0.14287 | 0.077009 | -1.8552 | -0.05439 | 0.018349 | |
| CENPA | NA | NA | NA | 0.724539 | 0.195614 | 3.703922 | 0.002888 | 0.04791 | 0.060269 | 0.679912 | 0.275146 | 2.471095 | 0.536476 | 0.157029 | 3.416414 | 0.185486 | 0.037867 | |
| CHAF1B | 0.259119 | 0.162074 | 1.59877 | 0.281358 | 0.148493 | 1.894756 | NA | NA | NA | -0.03447 | 0.352745 | -0.09773 | 0.209129 | 0.093425 | 2.238469 | 0.300765 | 0.05807 | |
| CLDN4 | 0.40922 | 0.128817 | 3.176755 | 1.20235 | 0.33711 | 3.56664 | 0.03236 | 0.053171 | 0.608591, | 0 | 1.8541 | 0 | 0.08503 | 0.258939 | 0.328378 | 0.125868 | 0.045235 | |
| CLIC1 | 0.238723 | 0.209629 | 1.138788 | 2.00024 | 0.600443 | 3.33127-1 | -0.26608 | 0.160756 | -1.65519 | 0.377361 | 0.552842 | 0.682584 | 0.999191 | 0.414232 | 2.412153 | 0.222753 | 0.088912 | |
| COL1A1 | 0.127256 | 0.081743 | 1.556791 | 0.05098 | 0.156488 | 0.325773 | 0.087944 | 0.034256 | 2.567237 | NA | NA | NA | -0.05544 | 0.13355 | -0.41509 | 0.083989 | 0.029343 | |
| COL1A2 | 0.01925 | 0.078156 | -0.24625 | -0.17504 | 0.228915 | -0.76466 | NA | NA | NA | -0.1405 | 0.184661 | -0.76085 | -0.15924 | 0.220113 | 0.72346 | -0.00069 | 0.041375 | |
| COMT | NA | NA | NA | 0.643165 | 0.360056 | 1.786292 | NA | NA | NA | 0.356582 | 0.628139 | 0.56768 | 0.404183 | 0.257299 | 1.570869 | 0.212925 | 0.092124 | |
| CRYZ | -0.38719 | 0.143353 | -2.70092 | 0.122949 | 0.340718 | 0.360853 | NA | NA | NA | -0.52792 | 0.412283 | -1.28048 | -0.37265 | 0.225119 | -1.65534 | -0.33167 | 0.071579 | |
| CSF1 | NA | NA | NA | -0.11449 | 0.197258 | -0.58042 | -0.09782 | 0.196881 | -0.49684 | NA | NA | NA | 0.120517 | 0.148659 | 0.810694 | -0.0334 | 0.090261 | |
| CTHRCl | NA | NA | NA | 0.263783 | 0.247606 | 1.065334 | NA | NA | NA | NA | NA | NA | -0.14789 | 0.176843 | -0.83626 | -0.00169 | 0.069075 | |
| CXCL12 | 0.066487 | 0.189775 | 0.350348 | -0.65036 | 0.168426 | -3.86137 | NA | NA | NA | -0.05795 | 0.270065 | -0.21456 | -0.35344 | 0.150278 | -2.35189 | -0.28998 | 0.062826 | |
| CXCL14 | -0.20969 | 0.073458 | -2.8546 | -0.14079 | 0.096118 | -1.46476 | NA | NA | NA | NA | NA | NA | -0.1861 | 0.08384 | -2.21976 | -0.14219 | 0.032611 | |
| CYR61 | NA | NA | NA | -0.38308 | 0.231645 | -1.65372 | NA | NA | NA | -0.22327 | 0.263371 | -0.84773 | -0.41188 | 0.174362 | -2.36221 | -0.04446 | 0.059831 | |
| DICER1 | NA | NA | NA | -1.06544 | 0.322204 | -3.30672 | NA | NA | NA | 0 | 0.311799 | 0 | 0.208326 | 0.307144 | 0.678268 | -0.19602 | 0.085879 | |
| DLC1 | 0.519601 | 0.221066 | 2.350434 | -0.66099 | 0.298518 | -2.21425 | NA | NA | NA | -0.31503 | 0.345828 | -0.91094 | -0.404 | 0.200673 | -2.01324 | -0.19876 | 0.076441 | |
| TNFRSF1 0B | -0.03773 | 0.174479 | -0.21623 | -0.03558 | 0.198203 | -0.1795 | NA | NA | NA | 0.932141 | 0.524911 | 1.775808 | 0.127348 | 0.157658 | 0.807748 | 0.02034 | 0.072745 | |
| DUSP1 | 0.095682 | 0.223995 | 0.42716 | -0.14883 | 0.12682 | -1.17351 | NA | NA | NA | 0.008053 | 0.779738 | 0.010327 | -0.41475 | 0.153012 | -2.71055 | -0.11225 | 0.054628 | |
| E2F1 | 0.171825 | 0.110793 | 1.550865 | 0.699408 | 0.207377 | 3.37264 | NA | NA | NA | NA | NA | NA | 0.570954 | 0.172882 | 3.302565 | 0.433836 | 0.067966 | |
| EEF1A2 | NA | NA | NA | -0.01256 | 0.130353 | -0.09633 | NA | NA | NA | 0.433528 | 0.267338 | 1.621648 | -0.04242 | 0.091692 | -0.46259 | 0.068177 | 0.041066 | |
| ELF3 | 0.406692 | 0.148275 | 2.742822 | 0.233332 | 0.357735 | 0.652248 | NA | NA | NA | 0.841389 | 0.55748 | 1.509272 | 0.096421 | 0.256911 | 0.375307 | 0.196003 | 0.066053 | |
| ENO1 | NA | NA | NA | 0.428884 | 0.194952 | 2.199947 | NA | NA | NA | 0.899319 | 0.369574 | 2.433394 | 0.288434 | 0.179833 | 1.603899 | 0.233559 | 0.058687 | |
| EPHB2 | NA | NA | NA | 0.192999 | 0.451341 | 0.427612 | NA | NA | NA | 0.355634 | 0604801 | 0.588018 | 0.211632 | 0.199057 | 1.063173 | 0.284709 | 0.094113 | |
| ERBB2 | 0.268938 | 0.074504 | 3.609693 | 0.092164 | 0.188964 | 0.487734 | NA | NA | NA | 0.301674 | 0.170749 | 1.766769 | 0.349689 | 0.107646 | 3.248509 | 0.181046 | 0.034939 | |
| ERBB4 | -0.10396 | 0.068988 | -1.50697 | -0.73759 | 0.209821 | -3.51532 | NA | NA | NA | NA | NA | NA | -0.1859 | 0.117619 | -1.58055 | -0.16266 | 0.037384 | |
| ESRRG | NA | NA | NA | -0.32843 | 0.127583 | -2.57425 | NA | NA | NA | NA | NA | NA | -0.04663 | 0.091723 | -0.50839 | -0.0602 | 0.044609 | |
| ESR1 | -0.14983 | 0.057346 | -2.61275 | -0.2159 | 0.120078 | -1.798 | -0.0019 | 0.019747 | -0.0963 | -030054 | 0.138369 | -2.17201 | -0.05096 | 0.082082 | -0.6196 | -0.04576 | 0.015905 | |
| EZH2 | 0.293772 | 0.156133 | 1.88155 | 0.79436 | 0.243012 | 3.26881 | -0.03007 | 0.04916 | -0.61166 | 0.123894 | 0.404373 | 0.306361 | 0.615257 | 0.155425 | 3.958546 | 0.134411 | 0.0393 | |
| F3 | NA | NA | NA | -0.3284 | 0.132658 | -2.47552 | NA | NA | NA | -0.08026 | 0.491948 | -0.16315 | -0.20405 | 0.109227 | -1.86809 | -0.22911 | 0.055029 | |

| **Table 13** Official Symbol | STNO ∼Est | STNO ∼SE | STNO~t | STOCK ∼Est | STOCK ∼SE | STOCK ∼t | TRANS BIG∼Est | TRANS BIG∼SE | TRANS BIG∼t | UCSF ∼Est | UCSF ∼SE | UCSF~t | UPP∼Est | UPP∼SE | UPP~t | fe | sefe | |
|---|---|---|---|---|---|---|---|---|---|---|---|---|---|---|---|---|---|---|
| FGFR4 | 0.201581 | 0.15216 | 1.324796 | -0.06118 | 0.174787 | -0.35001 | NA | NA | NA | 0.149034 | 0.333338 | 0.447096 | 0.204299 | 0.102078 | 2.001401 | 0.075374 | 0.053791 | |
| FHIT | -0.16819 | 0.17858 | -0.94184 | -0.27141 | 0.367689 | -0.73815 | NA | NA | NA | 0.225378 | 0.678656 | 0.332095 | 0.053025 | 0.245338 | 0.216132 | -0.11401 | 0.082797 | |
| FN1 | 0.049279 | 0.11577 | 0.425659 | 0.185381 | 0.202933 | 0.913508 | NA | NA | NA | 0.13258 | 0.244458 | 0.542343 | -0.15952 | 0.26761 | -0.59607 | 0.070337 | 0.045477 | |
| FOXA1 | NA | NA | NA | -0.18849 | 0.161048 | -1.17039 | NA | NA | NA | NA | NA | NA | 0.139273 | 0.160139 | 0869701 | -0.07105 | 0.037194 | |
| FUS | NA | NA | NA | 0.368833 | 0.437273 | 0.843485 | NA | NA | NA | NA | NA | NA | -0.15247 | 0.345172 | -0.44173 | 0.063142 | 0.111165 | |
| GADD45 A | 0.390085 | 0.342821 | 1.137868 | -0.24644 | 0.303688 | -0.81148 | NA | NA | NA | 0.153778 | 0.296649 | 0.518384 | -0.4297 | 0.20668 | -2.07904 | -0.18353 | 0.077839 | |
| GAPDH | NA | NA | NA | 0.907441 | 0.296513 | 3.060375 | NA | NA | NA | NA | NA | NA | 0.493907 | 0.232859 | 2.121056 | 0303991 | 0.05522 | |
| GATA3 | -0.20281 | 0.068842 | -2.94607 | -0.25592 | 0.122639 | -2.08677 | NA | NA | NA | -0.2038 | 0.135112 | -1.50836 | 0.052882 | 0.108852 | 0.485817 | -0.12484 | 0.03218 | |
| GBP2 | 0.104968 | 0.124764 | 0.841332 | -0.17667 | 0.338601 | -0.52176 | NA | NA | NA | 0.161775 | 0.235299 | 0.687529 | 0.215873 | 0.198252 | 1.088882 | 0.030811 | 0.064103 | |
| GDF15 | -0.02683 | 0.097032 | -0.27646 | 0.251857 | 0.169158 | 1.488886 | NA | NA | NA | 0.462744 | 0.465751 | 0.993544 | 0.139286 | 0.128201 | 1.086466 | 0.095577 | 0.04245 | |
| GRB7 | 0.28938 | 0.08099 | 3.573025 | 0.464983 | 0.21274 | 2.185687 | NA | NA | NA | 0.492397 | 0.361768 | 1.361085 | 0.39613 | 0.142688 | 2.776197 | 0.203411 | 0.041043 | |
| GSTM1 | NA | NA | NA | NA | NA | NA | NA | NA | NA | NA | NA | NA | NA | NA | NA | -0.18141 | 0.14912 | |
| GSTM2 | NA | NA | NA | NA | NA | NA | NA | NA | NA | -0.12675 | 0.336406 | -0.37676 | NA | NA | NA | -0.15328 | 0.111442 | |
| GSTM3 | -0.38478 | 0.15382 | -2.50148 | -0.43469 | 0.17404 | -2.49766 | 0.035771 | 0.038412 | 0.931246 | 0.11963 | 0.323329 | 0.369995 | -0.05308 | 0.123135 | -0.43107 | -0.06296 | 0.030752 | |
| HOXB13 | NA | NA | NA | 0.193 | 0.369898 | 0.521765 | NA | NA | NA | 0.540678 | 0.49567 | 1.090802 | 0.342881 | 0.212428 | 1.614105 | 0.227421 | 0.046188 | |
| OTUD4 | 0.372577 | 0.253393 | 1.470352 | -0.19372 | 0.251083 | -0.77155 | NA | NA | NA | -0.97971 | 0.713147 | -1.37378 | 0.231981 | 0.294286 | 0.788284 | 0.034041 | 0.081167 | |
| HSPA1A | NA | NA | NA | 0.765501 | 0.440826 | 1.736515 | NA | NA | NA | NA | NA | NA | 0.722677 | 0.40563 | 1.781616 | 0.243271 | 0.092738 | |
| HSPA1B | 0.033372 | 0.19398 | 0.172039 | 0.069621 | 0.248436 | 0.280237 | NA | NA | NA | NA | NA | NA | 0.187302 | 0.176407 | 1.061761 | 0.198207 | 0.083268 | |
| HSPA8 | 0.22166 | 0.199205 | 1.112723 | 0.32649 | 0.265007 | 1.232005 | NA | NA | NA | -0.30224 | 0.477926 | -0.63239 | 0.126525 | 0.166299 | 0.760828 | 0.218804 | 0.082393 | |
| IDH2 | 0.127942 | 0.255302 | 0.50114 | 0.574289 | 0.193387 | 2.969636 | NA | NA | NA | -0.009 | 0.554612 | -0.01623 | 0.659908 | 0.186426 | 3.539785 | 0.303626 | 0.056121 | |
| IGF1R | -0.16723 | 0.112062 | -1.49233 | -0.35887 | 0.141569 | -2.53498 | NA | NA | NA | 0.277384 | 0.391147 | 0.709155 | -0.04996 | 0.122321 | -0.40843 | -014872 | 0.0484 | |
| IGFBP7 | 0.121056 | 0.164973 | 0.733793 | -0.55896 | 0.34775 | -1.60736 | NA | NA | NA | -0.50275 | 0.332753 | -1.51087 | -0.16594 | 0.185086 | -0.89655 | 0.005398 | 0.068861 | |
| IL11 | NA | NA | NA | 0.086327 | 0.225669 | 0.38254 | NA | NA | NA | NA | NA | NA | 0.000507 | 0.151608 | 0.003346 | -0.05199 | 0.075711 | |
| IL17RB | NA | NA | NA | -0.01403 | 0.212781 | -0.06594 | NA | NA | NA | NA | NA | NA | -0.1861 | 0.139748 | -1.33168 | -0.16557 | 0.069337 | |
| IL6ST | NA | NA | NA | -0.65682 | 0.195937 | -3.35217 | NA | NA | NA | -0.11749 | 0.19789 | -0.5937 | -0.26213 | 0.150485 | -1.74192 | -0.31568 | 0.063376 | |
| IL8 | 0.548269 | 0.238841 | 2.29554 | 0.382317 | 0.203112 | 1.882296 | NA | NA | NA | -0.3673 | 0.460322 | -0.79791 | 0.076262 | 0.135635 | 0.562257 | 0.136391 | 0.05243 | |
| INHBA | -0.12998 | 0.113709 | -1.14313 | 0.249729 | 0.184419 | 1.354139 | NA | NA | NA | 0.094476 | 0.303634 | 0.311152 | 0.036575 | 0.162207 | 0.225485 | 0.026824 | 0.056655 | |
| IRF1 | 0.307333 | 0.166134 | 1.84991 | 0.248132 | 0.447433 | 0.554568 | NA | NA | NA | 0.380822 | 0.370842 | 1.026912 | -0.01044 | 0.283877 | -0.03676 | 0.082446 | 0.091982 | |
| ITGA4 | 0.02688 | 2341.09 | 1.15E-05 | 0.198854 | 0.302824 | 0.656665 | NA | NA | NA | -0.54938 | 0.583992 | -0.94073 | -0.01192 | 0.18086 | -0.0659 | 0.002027 | 0.059101 | |
| ITGA5 | NA | NA | NA | 0.025981 | 0.423908 | 0.061288 | NA | NA | NA | NA | NA | NA | 0.406364 | 0.36399 | 1.116415 | 0.431369 | 0.112958 | |
| ITGAV | 0 | 0.216251 | 0 | -0.403 | 0.45413 | -0.88742 | NA | NA | NA | -0.59197 | 0.499066 | -1.18615 | -0.24399 | 0.30418 | -0.80213 | -0.15415 | 0.089488 | |
| ITGB1 | 0.131284 | 0.165432 | 0.793583 | 0.195878 | 0.3192 | 0.613653 | NA | NA | NA | 0.430257 | 0.540622 | 0.795856 | -0.18009 | 0.530248 | -0.33962 | 0.026471 | 0.072949 | |

| **Table 13** Official Symbol | STNO ∼Est | STNO ∼SE | STNO∼t | STOCK ∼Est | STOCK ∼SE | STOCK ∼t | TRANS BIG∼Est | TRANS BIG∼SE | TRANS BIG∼t | UCSF ∼Est | UCSF ∼SE | UCSF∼t | UPP∼Est | UPP∼SE | UPP∼t | fe | sefe | |
|---|---|---|---|---|---|---|---|---|---|---|---|---|---|---|---|---|---|---|
| ITGB4 | 0.100533 | 0.106548 | 0.943547 | 0.035914 | 0.241068 | 0.14898 | NA | NA | NA | 0.754519 | 0.285307 | 2.644586 | 0.075057 | 0.181963 | 0.412483 | 0.132678 | 0.060938 | |
| ITGB5 | -0.19722 | 0.165947 | -1.18843 | -0.29946 | 0.281956 | -1.06207 | NA | NA | NA | -0.19391 | 0.378906 | -0.51177 | -0.21379 | 0.157719 | -1.35549 | -0.09296 | 0.063571 | |
| MKI67 | -0.07823 | 0.088982 | -0.87915 | 0.96424 | 0.257398 | 3.746105 | NA | NA | NA | -0.19193 | 0.462712 | -0.4148 | 0.597931 | 0.152281 | 3.926498 | 0.183915 | 0.058442 | |
| KIAA119 9 | NA | NA | NA | 0.293164 | 0.194272 | 1.509039 | NA | NA | NA | NA | NA | NA | 0.070065 | 0.141965 | 0.493538 | 0.153719 | 0-066186 | |
| KPNA2 | 0.328818 | 0.112579 | 2.920776 | 0.857218 | 0.267225 | 3207851 | NA | NA | NA | 0.32028 | 0.315031 | 1.016662 | 0.615022 | 0.206117 | 2.983849 | 0.374909 | 0.054897 | |
| LAMA3 | - 0.28334 | 0.120229 | - 2.3567 | -0.42291 | 0.12869 | - 3.28625 | NA | NA | NA | -0.14266 | 0.366741 | -0.38899 | -0.27285 | 0.091038 | -2.99711 | -0.26764 | 0.054897 0.050305 | |
| LAMB3 | NA | NA | NA | -0.15767 | 0.230936 | -0.68274 | NA | NA | NA | NA | NA | NA | -0.1353 | 0.168256 | -0.8041 | -0.00591 | 0.051501 | |
| LAPTM4 B | 0.405684 | 0.113287 | 3.581029 | 0.28652 | 0.19422 | 1.475234 | NA | NA | NA | NA | NA | NA | 0.095487 | 0.136338 | 0.700367 | 0.270104 | 0.051501 0.051492 | |
| LMNB1 | NA | NA | NA | 0.755925 | 0.25541 | 2.959653 | NA | NA | NA | 0.121429 | 0.384263 | 0.316005 | 0.805734 | 0.199208 | 4.044687 | 0.481816 | 0.073226 | |
| LRIG1 | -0.31422 | 0.128149 | -2.45197 | -0.95351 | 0.258142 | -3.69375 | NA | NA | NA | NA | NA | NA | -0.05954 | 0.178366 | 0.33383 | -0.37679 | 0.062401 | |
| MTDH | 0.242242 | 0.285145 | 0.84954 | 0.472647 | 0.340076 | 1389828 | 0.052038 | 0.077589 | 0.670683 | NA | NA | NA | 0.45556 | 0.239663 | 1.900836 | 0.158161 | 0.059133 | |
| MCM2 | 0.008185 | 0.084857 | 0.096455 | 0.732134 | 0.216462 | 3.382275 | NA | NA | NA | 0.138969 | 0.340074 | 0.408643 | 0.602555 | 0.182898 | 3.294487 | 0.275153 | 0.05978 | |
| MELK | NA | NA | NA | 0.749617 | 0.195032 | 3.843559 | 0.022669 | 0.036962 | 0.613293 | NA | NA | NA | 0.46629 | 0.128065 | 3.641042 | 0.132605 | 0.031744 | |
| MGMT | NA | NA | NA | 0.377527 | 0.48364 | 0.780595 | NA | NA | NA | 0.368174 | 0.453282 | 0.812241 | 0.725329 | 0.346508 | 2.093253 | 0.085317 | 0.117786 | |
| MMP1 | 0.083945 | 0.055744 | 1.505895 | 0.28871 | 0.081435 | 3.545299 | NA | NA | NA | 0.150509 | 0.33411 | 0.450477 | 0.11015 | 0.051829 | 2.12525 | 0.151235 | 0.027295 | |
| MMP7 | 0.102783 | 0.072986 | 1.408258 | -0.00343 | 0.153901 | -0.0223 | NA | NA | NA | 0.166646 | 0.143301 | 1.162909 | 0.059637 | 0.10332 | 0.57721 | 0.08418 | 0.042799 | |
| MYBL2 | 0.399355 | 0.118084 | 3.381957 | 0.579872 | 0.192026 | 3.019758 | NA | NA | NA | 0.030169 | 0.282699 | 0.106717 | 0.445705 | 0.102011 | 4.369186 | 0.479924 | 0.057205 | |
| NAT1 | -0.14333 | 0.060602 | -2.36509 | -0.26529 | 0.117131 | -2.26487 | NA | NA | NA | -0.1696 | 0.138069 | -1.22836 | -0.05668 | 0.076583 | -0.7401 | -0.14009 | 0.030446 | |
| PGF | -0.17016 | 0.153912 | -1.10557 | -0.08334 | 0.183966 | -0.45304 | 0.095422 | 0.145828 | 0.654349 | -1.00442 | 0.630097 | -1.59407 | 0.038005 | 0.124883 | 0.304328 | 0.009034 | 0.063633 | |
| PGR | NA | NA | NA | -0.18022 | 0.108941 | -1.65427 | NA | NA | NA | 0.451216 | 0.527475 | 0.855426 | -0.01652 | 0.065638 | -0.25164 | -0.12464 | 0.038764 | |
| PRDX1 | NA | NA | NA | 1.52553 | 0.420489 | 3.62799 | NA | NA | NA | 0.358079 | 0.32938 | 1.08713 | 0.706059 | 0.303105 | 2.32942 | 0.347764 | 0.10081 | |
| PTEN | 0 | 226.764 | 0 | -0.26976 | 0.225651 | -1.19546 | NA | NA | NA | NA | NA | NA | 0.110294 | 0.254356 | 0.433621 | -0.15381 | 0.092467 | |
| RPL41 | NA | NA | NA | -0.40807 | 0.786496 | -0.51884 | NA | NA | NA | NA | NA | NA | 0.24408 | 0.604521 | 0.403758 | -0.01769 | 0.094765 | |
| RPLP0 | NA | NA | NA | 0.018324 | 0.458438 | 0.039971 | NA | NA | NA | NA | NA | NA | 0.964584 | 0.554848 | 1.738465 | 0.108162 | 0.064823 | |
| RRM2 | 0305217 | 0.104337 | 2.9253 | 0.926244 | 0.22125 | 4.186414 | 0.038487 | 0.042471 | 0.906208 | -0.03281 | 0.279791 | -0.11727 | 0.674794 | 0.149386 | 4.517117 | 0.159696 | 0.03419 | |
| RUNX1 | -0.17832 | 0.165636 | -1.07657 | -0.39722 | 0.244634 | -1.62372 | NA | NA | NA | -0.58909 | 0.385997 | -1.52616 | -0.2142 | 0.105479 | -2.03071 | -0.07498 | 0.052758 | |
| S100A8 | 0.093477 | 0.04547 | 2.055818 | 0.164366 | 0.096581 | 1.701846 | NA | NA | NA | 0.123771 | 0.178963 | 0.691601 | 0.125784 | 0.065874 | 1.909478 | 0.106936 | 0.024582 | |
| S100A9 | NA | NA | NA | 0.15514 | 0.10905 | 1.42265 | NA | NA | NA | NA | NA | NA | 0.135096 | 0.074987 | 1.801592 | 0.112811 | 0.030203 | |
| S100B | 0.136825 | 0.163838 | 0.835124 | -0.11862 | 0.158461 | -0.74859 | -0.01591 | 0.034049 | -0.46712 | -0.05362 | 0.218098 | -0.24584 | -0.13315 | 0.115177 | -1.15608 | -0.01134 | 0.030069 | |
| S100P | 0.19922 | 0.078236 | 2.546395 | 0.201435 | 0.097583 | 2.064251 | NA | NA | NA | 0.416003 | 0.200351 | 2.076371 | 0.174292 | 0.063687 | 2.736705 | 0.179884 | 0.028697 | |
| SEMA3F | 0.023257 | 0.162267 | 0.143327 | 0.472655 | 0.292764 | 1.614457 | NA | NA | NA | NA | NA | NA | 0.545294 | 0.227357 | 2.398404 | 0.117569 | 0.092557 | |

| **Table 13** Official Symbol | STNO ∼Est | STNO ∼SE | STNO∼t | STOCK ∼Est | STOCK ∼SE | STOCK ∼t | TRAN S BIG∼Est | TRANS BIG∼SE | TRANS BIG∼t | UCSF ∼Est | UCSF ∼SE | UCSF∼t | UPP∼Est | UPP∼SE | UPP∼t | fe | sefe | |
|---|---|---|---|---|---|---|---|---|---|---|---|---|---|---|---|---|---|---|
| SKIL | NA | NA | NA | 0.015831 | 0.262101 | 0.060402 | NA | NA | NA | 0.141704 | 0.348326 | 0.406814 | 0.179419 | 0.152532 | 1.176271 | 0.134826 | 0.065866 | |
| SKP2 | NA | NA | NA | 0.312141 | 0.339582 | 0.919192 | NA | NA | NA | NA | NA | NA | 0.482145 | 0.194873 | 2.47415 | 0.167902 | 0.091018 | |
| SNAI1 | NA | NA | NA | 0.152799 | 0.210056 | 0.72742 | NA | NA | NA | NA | NA | NA | 0.329059 | 0.159704 | 2.060431 | 0.140674 | 0.078745 | |
| SYK | 0.21812 | 0.150626 | 1.44809 | -0.06882 | 0.155403 | -0.44285 | NA | NA | NA | 0.159029 | 0.431388 | 0.368645 | 0.066162 | 0.136668 | 0.484107 | 0.063381 | 0.072639 | |
| TAGLN | -0.00434 | 0.108525 | -0.04003 | -0.2578 | 0.197826 | -1.30316 | NA | NA | NA | NA | NA | NA | -0.06802 | 0.191196 | -0.35574 | 0.032416 | 0.049944 | |
| TFRC | 0.406546 | 0.131339 | 3.095394 | 0.178145 | 0.153331 | 1.161833 | -0.03263 | 0.051129 | -0.63826 | -0.22576 | 0.249301 | -0.90558 | 0.545839 | 0.208978 | 2.611945 | 0.062825 | 0.038345 | |
| TGFB3 | -0.07166 | 0.134442 | -0.53298 | -1.08462 | 0.322799 | -3.36005 | 0.013681 | 0.046103 | 0.296755 | -0.25719 | 0.253264 | -1.01551 | -0.49773 | 0.225603 | -2.20621 | -0.10353 | 0.03709 | |
| TNFRSF1 1B | 0 | 0.08306 | 0 | -0.10987 | 0.128194 | -0.85708 | NA | NA | NA | NA | NA | NA | -0.03866 | 0.087545 | -0.44163 | -0.09599 | 0.046815 | |
| VTN | -0.01674 | 0.109545 | -0.15278 | 0.100648 | 0.186529 | 0.539584 | 0.226938 | 0.091337 | 2.484623 | -0.22804 | 0.193542 | -1.17822 | 0.167418 | 0.152274 | 1.099452 | 0.063022 | 0.050706 | |
| WISP1 | 0.03435 | 0.194412 | 0.176685 | 0.236658 | 0.340736 | 0.694549 | -0.00282 | 0.068308 | -0.04121 | NA | NA | NA | -0.29716 | 0.212939 | -1.39552 | -0.05687 | 0.054306 | |
| WNT5A | 0.121343 | 0.108022 | 1.123317 | -0.01524 | 0.172902 | -0.08815 | NA | NA | NA | -0.96994 | 0.719267 | -1.34851 | -0.23507 | 0.152819 | -1.5382 | -0.12181 | 0.051129 | |
| C6orf66 | NA | NA | NA | 0.530409 | 0.355488 | 1.492059 | NA | NA | NA | NA | NA | NA | -0.04983 | 0.251179 | -0.19837 | 0.167784 | 0.123636 | |
| FOXO3A | NA | NA | NA | 0.087341 | 0.128833 | 0.67794 | NA | NA | NA | -0.03591 | 0.49687 | -0.07227 | -0.00291 | 0.074227 | -0.03914 | 0.007101 | 0.054798 | |
| GPR30 | NA | NA | NA | -0.36866 | 0.173755 | -2.12169 | NA | NA | NA | NA | NA | NA | -0.07779 | 0.125956 | -0.61763 | -0.02487 | 0.058543 | |
| KNTC2 | NA | NA | NA | 0.442783 | 0.170315 | 2.599789 | -0.00276 | 0.041235 | -0.06696 | -0.02041 | 0.366566 | -0.05568 | 0.347484 | 0.117596 | 2.954896 | 0.093083 | 0.034359 | |

**Table 14: Validation of Transferrin Receptor Group genes in SIB data sets.**

| | **Genes** | | | | | | |
|---|---|---|---|---|---|---|---|
| **Study data set** | TFRC | ENO1 | IDH2 | ARF1 | CLDN4 | PRDX1 | GBP1 |
| EMC2∼Est | NA | NA | NA | NA | NA | NA | NA |
| EMC2∼SE | NA | NA | NA | NA | NA | NA | NA |
| EMC2∼t | NA | NA | NA | NA | NA | NA | NA |
| JRH1∼Fst | -0.91825 | NA | -0.0525 | 0.839013 | -0.54144 | NA | 0.137268 |
| JRH1∼SE | 0.636275 | NA | 0.232201 | 0.346692 | 0.470758 | NA | 0.159849 |
| JRH1∼t | -1.44317 | NA | -0.22611 | 2.420053 | -1.15014 | NA | 0.858735 |
| JRH2∼Est | 0.162921 | 0.179739 | 0.151299 | 0.369609 | 0.33033 | -0.41082 | -0.07418 |
| JRH2∼SE | 0.352486 | 0.312848 | 0.327466 | 0.40789 | 0.351865 | 0.47383 | 0.198642 |
| JRH2∼t | 0.462206 | 0.574525 | 0.46203 | 0.906149 | 0.938798 | -0.86703 | -0.37345 |
| MGH∼Est | 0.029015 | NA | NA | 2.03958 | 0.185116 | NA | 0.15434 |
| MGH∼SE | 0.193689 | NA | NA | 0.804729 | 0.314723 | NA | 0.188083 |
| MGH∼t | 0.149803 | NA | NA | 2.534493 | 0.588187 | NA | 0.820595 |
| NCH∼Est | 0.056174 | -0.01727 | 0.265828 | -0.15337 | -0.23129 | 0.253047 | 0.095457 |
| NCH∼SE | 0.166875 | 0.097939 | 0.105592 | 0.204529 | 0.426627 | 0.182621 | 0.1323 |
| NCH∼t | 0.336622 | -0.17629 | 2.517501 | -0.74984 | -0.54213 | 1.38564 | 0.721522 |
| NKI∼Est | 0.157216 | 0.3682 | 0.284862 | 0.944168 | 0.564756 | 0.231612 | 0.13712 |
| NKI∼SE | 0.10845 | 0.094778 | 0.089145 | 0.204641 | 0.210595 | 0.161791 | 0.075391 |
| NKI∼t | 1.449663 | 3.884888 | 3.195498 | 4.613777 | 2.681716 | 1.431551 | 1.818777 |
| STNO-Est | 0.406546 | NA | 0.127942 | 0 | 0.40922 | NA | 0.298139 |
| STNO-SE | 0.131339 | NA | 0.255302 | 0.107397 | 0.128817 | NA | 0.113901 |
| STNO∼t | 3.095394 | NA | 0.50114 | 0 | 3.176755 | NA | 2.617528 |
| STOCK∼Est | 0.178145 | 0.428884 | 0.574289 | 0.862387 | 1.20235 | 1.52553 | 0.068821 |
| STOCK-SE | 0.153331 | 0.194952 | 0.193387 | 0.279535 | 0.33711 | 0.420489 | 0.183692 |
| STOCK∼t | 1.161833 | 2.199947 | 2.969636 | 3.085077 | 3.56664 | 3.62799 | 0.374652 |
| TRANSBIG∼Est | -0.03263 | NA | NA | NA | 0.03236 | NA | NA |
| TRANSBIG-SE | 0.051129 | NA | NA | NA | 0.053171 | NA | NA |
| TRANSBIG∼t | -0.63826 | NA | NA | NA | 0.608591 | NA | NA |
| UCSF∼Est | -0.22576 | 0.899319 | -0.009 | 0.304097 | 0 | 0.358079 | -0.43879 |
| UCSF∼SE | 0.249301 | 0.369574 | 0.554612 | 0.58718 | 1.8541 | 0.32938 | 0.874728 |
| UCSF∼t | -0.90558 | 2.433394 | -0.01623 | 0.517894 | 0 | 1.08713 | -0.50163 |
| UPP∼Est | 0.545839 | 0.288434 | 0.659908 | 0.751279 | 0.08503 | 0.706059 | 0.119778 |
| UPP∼SE | 0.208978 | 0.179833 | 0.186426 | 0.361093 | 0.258939 | 0.303105 | 0.117879 |
| UPP∼t | 2.611945 | 1.603899 | 3.539785 | 2.080569 | 0.328378 | 2.32942 | 1.01611 |
| Fe | 0.062825 | 0.233559 | 0.303626 | 0.281544 | 0.125868 | 0.347764 | 0.139381 |
| Sefe | 0.038345 | 0.058687 | 0.056121 | 0.07587 | 0.045235 | 0.10081 | 0.044464 |

**Table 15: Validation of Stromal Group genes in SIB data sets.**

| | | | | | | | | | | | | | |
|---|---|---|---|---|---|---|---|---|---|---|---|---|---|
| Gene | CXCL14 | TNFRSF11B | CXCL12 | C10orf116 | RUNX1 | GSTM2 | TGFB3 | BCAR3 | CAV1 | DLC1 | TNFRSF10B | F3 | DICER1 |
| EMC2∼Est | NA | NA | NA | NA | NA | NA | NA | NA | NA | NA | NA | NA | NA |
| EMC2∼SE | NA | NA | NA | NA | NA | NA | NA | NA | NA | NA | NA | NA | NA |
| EMC2∼t | NA | NA | NA | NA | NA | NA | NA | NA | NA | NA | NA | NA | NA |
| JRH1∼Est | -0.23692 | NA | -0.36476 | -0.1418 | -0.22834 | NA | -1.0219 | NA | -0.20701 | 0.13581 | -0.09001 | 0.719395 | NA |
| JRH1∼SE | 0.333761 | NA | 0.372499 | 0.261554 | 0.318666 | NA | 0.358953 | NA | 0.254401 | 0.37927 | 0.619057 | 0.524742 | NA |
| JRH1∼t | -0.70985 | NA | -0.97921 | -0.54216 | -0.71656 | NA | -2.84689 | NA | -0.81372 | 0.358083 | -0.1454 | 1.37095 | NA |
| JRH2∼Est | 0.361375 | -0.10399 | -04566 | 0.036378 | 0.302803 | NA | -0.39774 | -0.29238 | -0.19588 | -0.4102 | 0.80742 | -0.21237 | -0.33943 |
| JRH2∼SE | 0.159544 | 0.440721 | 0.219587 | 0.182183 | 0.420043 | NA | 0.470041 | 0.522706 | 0.289251 | 0.387258 | 0.544479 | 0.363632 | 0.39364 |
| JRH2∼t | 2.265049 | -0.23595 | -2.07935 | 0.19968 | 0720886 | NA | -0.84619 | -0.55935 | -0.67721 | -1.05923 | 1.482922 | -0.58402 | -0.8623 |
| MGH∼Est | NA | -1.15976 | NA | NA | 0.277566 | NA | 0.046498 | -0.41595 | -0.06896 | -0.09793 | 0.159018 | -0.00167 | 0.038811 |
| MGH∼SE | NA | 0.400921 | NA | NA | 0.267511 | NA | 0.2296 | 0.216837 | 0.2269 | 0.247069 | 0.456205 | 0.448211 | 0.409835 |
| MGH∼t | NA | -2.89274 | NA | NA | 1.037587 | NA | 0.202518 | -1.91825 | -0.30391 | -0.39638 | 0.348567 | -0.00372 | 0.0947 |
| NCH∼Est | -0.06592 | -02492 | -0.08863 | 0.064337 | 0.124568 | NA | -0.30473 | 0.072246 | 0.078825 | -0.03473 | -0.19927 | -0.13187 | 0.086141 |
| NCH-SE | 0.093353 | 0.289075 | 0.138097 | 0.14087 | 0.088457 | NA | 0.247338 | 0.304443 | 0.340843 | 0.238947 | 0.160381 | 0.134218 | 0.143687 |
| NCH∼t | -0.70609 | -0.86207 | -0.64183 | 0.456713 | 1.408231 | NA | -1.23202 | 0.237306 | 0.231265 | -0.14533 | -1.24248 | -0.98248 | 0.599504 |
| NKI∼Est | -0.16877 | -0.22072 | -0.36944 | -0.22589 | -0.18878 | -0.15655 | -0.36531 | -0.26067 | -0.30885 | -0.35001 | 0.053214 | -0.29217 | -0.46887 |
| NKI∼SE | 0.054117 | 0.10171 | 0.138735 | 0.082836 | 0.138365 | 0.118111 | 0.09592 | 0.114992 | 0.133788 | 0.130472 | 0.164091 | 0.093753 | 0.150367 |
| NKI∼t | -3.11866 | -2.17005 | -2.66293 | -2.72696 | -1.36435 | -1.32547 | -3.80851 | -2.26685 | -2.30848 | -2.68262 | 0.324294 | -3.11637 | -3.11814 |
| STNO-Est | -0.20969 | 0 | 0.066487 | -0.09621 | -0.17832 | NA | -0.07166 | NA | 0.135002 | 0.519601 | -0.03773 | NA | NA |
| STNO∼SE | 0.073458 | 0.08306 | 0.189775 | 0.085948 | 0.165636 | NA | 0.134442 | NA | 0.093948 | 0.221066 | 0.174479 | NA | NA |
| STNO∼t | -2.8546 | 0 | 0.350348 | -1.11936 | -1.07657 | NA | -0.53298 | NA | 1.436991 | 2.350434 | -0.21623 | NA | NA |
| STOCK∼Est | -0.14079 | -0.10987 | -0.65036 | -0.34745 | -0.39722 | NA | -1.08462 | -0.49692 | -0.65852 | -0.66099 | -0.03558 | -0.3284 | -1.06544 |
| STOCK∼SE | 0.096118 | 0.128194 | 0.168426 | 0.112777 | 0.244634 | NA | 0.322799 | 0.265837 | 0.275751 | 0.298518 | 0.198203 | 0.132658 | 0.322204 |
| STOCK∼t | -1.46476 | -0.85708 | -3.86137 | -3.08087 | -1.62372 | NA | -3.36005 | -1.86927 | -2.38811 | -2.21425 | -0.1795 | -2.47552 | -3.30672 |

| Gene | CXXL14 | TNFRSF11B | CXCL12 | C10orf116 | RUNX1 | GSTM2 | TGFB3 | BCAR3 | CAV1 | DLC1 | TNFRSF10B | F3 | DICER1 |
|---|---|---|---|---|---|---|---|---|---|---|---|---|---|
| TRANSBIG∼Est | NA | NA | NA | NA | NA | NA | 0.013681 | NA | NA | NA | NA | NA | N/A |
| TRANSBIG∼SE | NA | NA | NA | NA | NA | NA | 0.046103 | NA | NA | NA | NA | NA | N/A |
| TRANSBIG∼t | NA | NA | NA | NA | NA | NA | 0.296755 | NA | NA | NA | NA | NA | N/A |
| UCSF∼Est | NA | NA | -0.05795 | 0.013111 | -0.58909 | -0.12675 | -0.25719 | NA | -0.54391 | -0.31503 | 0.932141 | -0.08026 | 0 |
| UCSF∼SE | NA | NA | 0.270065 | 156.117 | 0.385997 | 0.336406 | 0.253264 | NA | 0.428883 | 0.345828 | 0.524911 | 0.491948 | 0.311799 |
| UCSF∼t | NA | NA | -0.21456 | 8.40E-05 | -1.52616 | -0.37676 | -1.01551 | NA | -1.2682 | -0.91094 | 1.775808 | -0.16315 | 0 |
| UPP∼Est | -0.1861 | -0.03866 | -0.35344 | -0.00923 | -0.2142 | NA | -0.49773 | -0.29435 | -0.31503 | -0.404 | 0.127348 | -0.20405 | 0.208326 |
| UPP∼SE | 0.08384 | 0.087545 | 0.150278 | 0.100902 | 0.105479 | NA | 0.225603 | 0.182614 | 0.150431 | 0.200673 | 0.157658 | 0.109227 | 0.307144 |
| UPP∼t | -2.21976 | -0.44163 | -2.35189 | -0.09148 | -2.03071 | NA | -2.20621 | -1.61186 | -2.09415 | -2.01324 | 0.807748 | -1.86809 | 0.678268 |
| Fe | -0.14219 | -0.09599 | -0.28998 | -0.13 | -0.07498 | -0.15328 | -0.10353 | -0.28755 | -0.11726 | -0.19876 | 0.02034 | -0.22911 | -0.19602 |
| Sefe | 0.032611 | 0.046815 | 0.062826 | 0.042521 | 0.052758 | 0.111442 | 0.03709 | 0.080198 | 0.058989 | 0.076441 | 0.072745 | 0.055029 | 0.085879 |

**Table 16: Genes that co-express with Prognostic genes in ER+ breast cancer tumors (Spearman corr. coef. ≥ 0.7)**

| **Prognostic Gene** | **Table 16 Co-expressed Genes** | | | | |
|---|---|---|---|---|---|
| INHBA | AEBP1 | CDH11 | COL10A1 | COL11A1 | COL1A2 |
| | COL5A1 | COL5A2 | COL8A2 | ENTPD4 | LOXL2 |
| | LRRC15 | MMP11 | NOX4 | PLAU | THBS2 |
| | THY1 | VCAN | | | |
| CAV1 | ANK2 | ANXA1 | AQP1 | C10orf56 | CAV2 |
| | CFH | COL14A1 | CRYAB | CXCL12 | DAB2 |
| | DCN | ECM2 | FHL1 | FLRT2 | GNG11 |
| | GSN | IGF1 | JAM2 | LDB2 | NDN |
| | NRN1 | PCSK5 | PLSCR4 | PROS1 | TGFBR2 |
| NAT1 | PSD3 | | | | |
| GSTM1 | GSTM2 | | | | |
| GSTM2 | GSTM1 | | | | |
| ITGA4 | ARHGAP15 | ARHGAP25 | CCL5 | CD3D | CD48 |
| | CD53 | CORO1A | EVI2B | FGL2 | GIMAP4 |
| | IRF8 | LCK | PTPRC | TFEC | TRAC |
| | TRAF3IP3 | TRBC1 | EVI2A | FLI1 | GPR65 |
| | IL2RB | LCP2 | LOC100133233 | MNDA | PLAC8 |
| | PLEK | TNFAIP8 | | | |
| CCL19 | ARHGAP15 | ARHGAP25 | CCL5 | CCR2 | CCR7 |
| | CD2 | CD247 | CD3D | CD3E | CD48 |
| | CD53 | FLJ78302 | GPR171 | IL10RA | IL7R |
| | IRF8 | LAMP3 | LCK | LTB | PLAC8 |
| | PRKCB 1 | PTPRC | PTPRCAP | SASH3 | SPOCK2 |
| | TRA@ | TRBC1 | TRD@ | PPP1R16B | TRAC |
| CDH11 | TAGLN | ADAM12 | AEBP1 | ANGPTL2 | ASPN |
| | BGN | BICC1 | C10orf56 | C1R | C1S |
| | C20orf39 | CALD1 | COL10A1 | COL11A1 | COL1A1 |
| | COL1A2 | COL3A1 | COL5A1 | COL5A2 | COL6A1 |
| | COL6A2 | COL6A3 | COL8A2 | COMP | COPZ2 |
| | CRISPLD2 | CTSK | DACT1 | DCN | DPYSL3 |
| | ECM2 | EFEMP2 | ENTPD4 | FAP | FBLN1 |
| | FBLN2 | FBN1 | FERMT2 | FLRT2 | FN1 |
| | FSTL1 | GAS1 | GLT8D2 | HEPH | HTRA1 |
| | ISLR | ITGBL1 | JAM3 | KDELC1 | LAMA4 |
| | LAMB1 | LOC100133502 | LOX | LOXL2 | LRRC15 |
| | LRRC17 | LUM | MFAP2 | MFAP5 | MMP2 |
| | MRC2 | MXRA5 | MXRA8 | MYL9 | NDN |
| | NIDI | NID2 | NINJ2 | NOX4 | OLFML2B |
| | OMD | PALLD | PCOLCE | PDGFRA | PDGFRB |
| | PDGFRL | POSTN | PRKCDBP | PRKD1 | PTRF |
| | RARRES2 | RCN3 | SFRPINF1 | SERPINH1 | SFRP4 |
| | SNAI2 | SPARC | SPOCK1 | SPON1 | SRPX2 |
| | SSPN | TCF4 | THBS2 | THY1 | TNFAIP6 |
| | VCAN | WWTR1 | ZEB1 | ZFPM2 | INHBA |
| | PLS3 | SEC23A | WISP1 | | |
| TAGLN | CDH11 | ADAM12 | AEBP1 | ANGPTL2 | ASPN |
| | BGN | BICC1 | C10orf56 | C1R | C1S |
| | C20orf39 | CALD1 | COL10A1 | COL11A1 | COL1A1 |
| | COL1A2 | COL3A1 | COL5A1 | COL5A2 | COL6A1 |
| | COL6A2 | COL6A3 | COL8A2 | COMP | COPZ2 |
| | CRISPLD2 | CTSK | DACT1 | DCN | DPYSL3 |
| | ECM2 | EFEMP2 | ENTPD4 | FAP | FBLN1 |
| | FBLN2 | FBN1 | FERMT2 | FLRT2 | FN1 |
| | FSTL1 | GAS1 | GLT8D2 | HEPH | HTRA1 |
| | ISLR | ITGBL1 | JAM3 | KDELC1 | LAMA4 |
| | LAMB1 | LOC100133502 | LOX | LOXL2 | LRRC15 |
| | LRRC17 | LUM | MFAP2 | MFAP5 | MMP2 |
| | MRC2 | MXRA5 | MXRA8 | MYL9 | NDN |
| | NID1 | NID2 | NINJ2 | NOX4 | OLFML2B |
| | OMD | PALLD | PCOLCE | PDGFRA | PDGFRB |
| | PDGFRL | POSTN | PRKCDBP | PRKD 1 | PTRF |
| | RARRES2 | RCN3 | SERPINF1 | SERPINH1 | SFRP4 |
| | SNAI2 | SPARC | SPOCK1 | SPON1 | SRPX2 |
| | SSPN | TCF4 | THBS2 | THY1 | TNFAIP6 |
| | VCAN | WWTR1 | ZEB1 | ZFPM2 | ACTA2 |
| | CNN1 | DZIP1 | EMILIN 1 | | |
| ENO1 | ATP5J2 | C10orf10 | CLDN15 | CNGB1 | DET1 |
| | EIF3CL | HS2ST1 | IGHG4 | KIAA0195 | KIR2DS5 |
| | PARP6 | PRH1 | RAD1 | RIN3 | RPL10 |
| | SGCG | SLC16A2 | SLC9A3R1 | SYNPO2L | THBS1 |
| | ZNF230 | | | | |
| IDH2 | AEBP1 | HIST1 H2BN | PCDHAC1 | | |
| ARF1 | CRIM1 | | | | |
| DICER1 | ADM | LOC100133583 | | | |
| AKT3 | AKAP12 | ECM2 | FERMT2 | FLRT2 | JAM3 |
| | LOC100133502 | PROS1 | TCF4 | WWTR1 | ZEB1 |
| CXCL12 | ANXA1 | C1R | C1S | CAV1 | DCN |
| | FLRT2 | SRPX | | | |
| CYR61 | CTGF | | | | |
| IGFBP7 | VIM | | | | |
| KIAA1199 | COL11A1 | PLAU | | | |
| SPC25 | ASPM | BUB1 | BUB1B | CCNA2 | CCNE2 |
| | CDC2 | CDC25C | CENPA | CEP55 | FANCI |
| | GINS1 | HJURP | KIAA0101 | KIF11 | KIF14 |
| | KIF15 | KIF18A | KIF20A | KIF4A | MAD2L1 |
| | MELK | NCAPG | NEK2 | NUSAP1 | PRC1 |
| | STIL | ZWINT | | | |
| WISP1 | CDH11 | COL5A2 | | | |

**Table 17: Genes that co-express with Prognostic Genes in ER- breast cancer tumors (Spearman corr. coef. ≥ 0.7)**

| **Prognostic Gene** | **Table 17** | | | | |
|---|---|---|---|---|---|
| | **Co-expressed Genes** | | | | |
| IRF1 | APOL6 | CXCL10 | GABBR1 | GBP1 | HCP5 |
| | HLA-E | HLA-F | HLA-G | HLA-J | INDO |
| | PSMB8 | PSMB9 | STAT1 | TAP1 | UBD |
| | UBE2L6 | WARS | APOBEC3F | APOBEC3G | APOL1 |
| | APOL3 | ARHGAP25 | BTN3A1 | BTN3A2 | BTN3A3 |
| | C1QB | CCL5 | CD2 | CD38 | CD40 |
| | CD53 | CD74 | CD86 | CSF2RB | CTSS |
| | CYBB | FGL2 | GIMAP5 | GZMA | hCG_1998957 |
| | HCLS1 | HLA-C | HLA-DMA | HLA-DMB | HLA-DPA1 |
| | HLA-DQB1 | HLA-DQB2 | HLA-DRA | HLA-DRB1 | HLA-DRB2 |
| | HLA-DRB3 | HLA-DRB4 | HLA-DRB5 | HLA-DRB6 | IL10RA |
| | IL2RB | LAP3 | LAPTM5 | LOC100133484 | LOC100133583 |
| | LOC100133661 | LOC100133811 | LOC730415 | NKG7 | PLEK |
| | PSMB10 | PTPRC | RNASE2 | SLAMF8 | TFEC |
| | TNFRSF1B | TRA@ | TRAC | TRAJ17 | TRAV20 |
| | ZNF749 | | | | |
| CDH11 | ADAM12 | AEBP1 | ANGPTL2 | ASPN | CFH |
| | CFHR1 | COL10A1 | COL11A1 | COL1A1 | COL1A2 |
| | COL3A1 | COL5A1 | COL5A2 | COL6A3 | CRISPLD2 |
| | CTSK | DACT1 | DCN | FAP | FBN1 |
| | FN1 | HTRA1 | LOX | LRRC15 | LUM |
| | NID2 | PCOLCE | PDGFRB | POSTN | SERPINF1 |
| | SPARC | THBS2 | THY1 | VCAN | DAB2 |
| | GLT8D2 | ITGB5 | JAM3 | LOC100133502 | MMP2 |
| | PRSS23 | TIMP3 | ZEB1 | | |
| CCL19 | ITGA4 | ADAM28 | AIF1 | APOBEC3F | APOBEC3G |
| | APOL3 | ARIIGAP15 | ARIIGAP25 | CASP1 | CCDC69 |
| | CCR2 | CCR7 | CD2 | CD247 | CD27 |
| | CD37 | CD3D | CD3G | CD48 | CD52 |
| | CD53 | CD74 | CD86 | CD8A | CLEC4A |
| | CORO1A | CTSS | CXCL13 | DOCK10 | EVI2A |
| | EVI2B | FGL2 | FLJ78302 (CCR2) | FYB | GIMAP4 |
| | GIMAP5 | GIMAP6 | GMFG | GPR171 | GPR 18 |
| | GPR65 | GZMA | GZMB | GZMK | hCG_1998957 |
| | HCLS1 | HLA-DMA | HLA-DMB | HLA-DPA1 | HLA-DQA1 |
| | HLA-DQA2 | HLA-DQB1 | HLA-DQB2 | HLA-DRB1 1 | HLA-DRB2 |
| | HLA-DRB3 | HLA-DRB4 | HLA-DRB5 | HLA-E | IGHM |
| | IGSF6 | IL10RA | IL2RG | IL7R | IRF8 |
| | KLRB1 | KLRK1 | LAPTM5 | LAT2 | LCK |
| | LCP2 | LOC100133484 | LOC100133583 | LOC100133661 | LOC100133811 |
| | LOC730415 | LPXN | LRMP | LST1 | LTB |
| | LY96 | LYZ | MFNG | MNDA | MS4A4A |
| | NCKAP1L | PLAC8 | PLEK | PRKCB1 | PSCDBP |
| | PTPRC | PTPRCAP | RAC2 | RNASE2 | RNASE6 |
| | SAMHD1 | SAMSN1 | SASH3 | SELL | SELPLG |
| | SLA | SLAMF1 | SLC7A7 | SP140 | SRGN |
| | TCL1A | TFEC | TNFAIP8 | TNFRSF1B | TRA@ |
| | TRAC | TRAJ17 | TRAT1 | TRAV20 | TRBC1 |
| | TYROBP | ZNF749 | ITM2A | LTB | P2RY13 |
| | PRKCB1 | PTPRCAP | SELL | TRBC1 | |
| ITGA4 | CCL19 | ADAM28 | AIF1 | APOBEC3F | APOBEC3G |
| | APOL3 | ARHGAP15 | ARHGAP25 | CASP1 | CCDC69 |
| | CCR2 | CCR7 | CD2 | CD247 | CD27 |
| | CD37 | CD3D | CD3G | CD48 | CD52 |
| | CD53 | CD74 | CD86 | CD8A | CLEC4A |
| | CORO1A | CTSS | CXCL13 | DOCK10 | EVI2A |
| | EVI2B | FGL2 | FLJ78302 (CCR2) | FYB | GIMAP4 |
| | GIMAP5 | GIMAP6 | GMFG | GPR171 | GPR18 |
| | GPR65 | GZMA | GZMB | GZMK | hCG_1998957 |
| | HCLS1 | HLA-DMA | HLA-DMB | HLA-DPA1 | HLA-DQA1 |
| | HLA-DQA2 | HLA-DQB 1 | HLA-DQB2 | HLA-DRB 1 | HLA-DRB2 |
| | HLA-DRB3 | HLA-DRB4 | HLA-DRB5 | HLA-E | IGHM |
| | IGSF6 | IL10RA | IL2RG | IL7R | IRF8 |
| | KLRB1 | KLRK1 | LAPTM5 | LAT2 | LCK |
| | LCP2 | LOC100133484 | LOC100133583 | LOC100133661 | LOC100133811 |
| | LOC730415 | LPXN | LRMP | LST1 | LTB |
| | LY96 | LYZ | MFNG | MNDA | MS4A4A |
| | NCKAP1L | PLAC8 | PLEK | PRKCB1 | PSCDBP |
| | PTPRC | PTPRCAP | RAC2 | RNASE2 | RNASE6 |
| | SAMHD1 | SAMSN1 | SASH3 | SELL | SELPLG |
| | SLA | SLAMF1 | SLC7A7 | SP140 | SRGN |
| | TCL1A | TFEC | TNFAIP8 | TNFRSF1B | TRA@ |
| | TRAC | TRAJ17 | TRAT1 | TRAV20 | TRBC1 |
| | TYROBP | ZNF749 | MARCH1 | C17orf60 | CSF1R |
| | FLI1 | FLJ78302 | FYN | IKZF1 | INPP5D |
| | NCF4 | NR3C1 | P2RY13 | PLXNC1 | PSCD4 |
| | PTPN22 | SERPINB9 | SLCO2B1 | VAMP3 | WIPF1 |
| IDH2 | AEBP1 | DSG3 | HIST1H2BN | PCDHAC1 | |
| ARF1 | FABP5L2 | FLNB | IL1RN | PAX6 | |
| DICER1 | ARS2 | IGHA 1 | VDAC3 | | |
| TFRC | RGS20 | | | | |
| ADAM17 | TFDP3 | GPR107 | | | |
| CAV1 | CAV2 | CXCL12 | IGF1 | | |
| CYR61 | CTGF | | | | |
| ESR1 | CBLN1 | SLC45A2 | | | |
| GSTM1 | GSTM2 | | | | |
| GSTM2 | GSTM1 | | | | |
| IL11 | FAM135A | | | | |
| IL6ST | P2RY5 | | | | |
| IGFBP7 | SPARCL1 | TMEM204 | | | |
| INHBA | COL10A1 | FN1 | SULF1 | | |
| SPC25 | KIF4A | KIF20A | NCAPG | | |
| TAGLN | ACTA2 | MYL9 | NNMT | PTRF | |
| TGFB3 | GALNT10 | HTRA 1 | LIMA1 | | |
| TNFRSF10B | BIN3 | | | | |
| FOXA1 | CLCA2 | TFAP2B | AGR2 | MLPH | SPDEF |
| CXCL12 | DCN | CAV1 | IGF1 | CFH | |
| GBP2 | APOL1 | APOL3 | CD2 | CTSS | CXCL9 |
| | CXCR6 | GBP1 | GZMA | IILA-DMA | IILA-DMB |
| | IL2RB | PTPRC | TRBC1 | | |

**Table 18: Genes that co-express with Prognostic Genes in all breast cancer tumors (Spearman corr. coef. ≥ 0.7)**

| **Prognostic Gene** | **Table 18** | | | | |
|---|---|---|---|---|---|
| | **Co-expressed Genes** | | | | |
| S100A8 | S100A9 | | | | |
| S100A9 | S100A8 | | | | |
| MKI67 | BIRC5 | KIF20A | MCM10 | | |
| MTDH | ARMC1 | AZIN1 | ENY2 | MTERFD1 | POLR2K |
| | PTDSS1 | RAD54B | SLC25A32 | TMEM70 | UBE2V2 |
| GSTM1 | GSTM2 | | | | |
| GSTM2 | GSTM1 | | | | |
| CXCL12 | AKAP12 | DCN | F13A1 | | |
| TGFB3 | C10orf56 | JAM3 | | | |
| TAGLN | ACTA2 | CALD1 | COPZ2 | FERMT2 | HEPH |
| | MYL9 | NNMT | PTRF | TPM2 | |
| PGF | ALMS1 | ATP8B1 | CEP27 | DBT | FAM128B |
| | FBXW12 | FGFR1 | FLJ12151 | FLJ42627 | GTF2H3 |
| | HCG2P7 | KIAA0894 | KLHL24 | LOC152719 | PDE4C |
| | PODNL1 | POLR1B | PRDX2 | PRR11 | RIOK3 |
| | RP5-886K2.1 | SLC35E1 | SPN | USP34 | ZC3H7B |
| | ZNF160 | ZNF611 | | | |
| CCL19 | ARHGAP15 | ARHGAP25 | CCL5 | CCR2 | CCR7 |
| | CD2 | CD37 | CD3D | CD48 | CD52 |
| | CSF2RB | FLJ78302 | GIMAP5 | GIMAP6 | GPR171 |
| | GZMK | IGHM | IRF8 | LCK | LTB |
| | PLAC8 | PRKCB1 | PTGDS | PTPRC | PTPRCAP |
| | SASH3 | TNFRSF1B | TRA@ | TRAC | TRAJ17 |
| | TRAV20 | TRBC1 | | | |
| IRF1 | ITGA4 | MARCH1 | AIF1 | APOBEC3F | APOBEC3G |
| | APOL1 | APOL3 | ARHGAP15 | ARHGAP25 | BTN3A2 |
| | BTN3A3 | CASP1 | CCL4 | CCL5 | CD2 |
| | CD37 | CD3D | CD48 | CD53 | CD69 |
| | CD8A | CORO1A | CSF2RB | CST7 | CYBB |
| | EVI2A | EVI2B | FGL2 | FLI1 | GBP1 |
| | GIMAP4 | GIMAP5 | GIMAP6 | GMFG | GPR65 |
| | GZMA | GZMK | hCG_1998957 | HCLS1 | HLA-DMA |
| | HLA-DMB | HLA-DPA1 | HLA-DQB1 | HLA-DQB2 | HLA-DRA |
| | HLA-DRB1 | HLA-DRB2 | HLA-DRB3 | HLA-DRB4 | HLA-DRB5 |
| | HLA-E | HLA-F | IGSF6 | IL10RA | IL2RB |
| | IRF8 | KLRK1 | LCK | LCP2 | LOC100133583 |
| | LOC100133661 | LOC100133811 | LST1 | LTB | LY86 |
| | MFNG | MNDA | NKG7 | PLEK | PRKCB 1 |
| | PSCDBP | PSMB10 | PSMB8 | PSMB9 | PTPRC |
| | PTPRCAP | RAC2 | RNASE2 | RNASE6 | SAMSN1 |
| | SLA | SRGN | TAP1 | TFEC | TNFAIP3 |
| | TNFRSF1B | TRA@ | TRAC | TRAJ17 | TRAV20 |
| | TRBC1 | TRIM22 | ZNF749 | | |
| ITGA4 | IRF1 | MARCH1 | AIF1 | APOBEC3F | APOBEC3G |
| | APOL1 | APOL3 | ARIIGAP15 | ARHGAP25 | BTN3A2 |
| | BTN3A3 | CASP1 | CCL4 | CCL5 | CD2 |
| | CD37 | CD3D | CD48 | CD53 | CD69 |
| | CD8A | CORO1A | CSF2RB | CST7 | CYBB |
| | EVI2A | EVI2B | FGL2 | FLI1 | GBP1 |
| | GIMAP4 | GIMAP5 | GIMAP6 | GMFG | GPR65 |
| | GZMA | GZMK | hCG_1998957 | HCLS1 | HLA-DMA |
| | HLA-DMB | HLA-DPA1 | HLA-DQB1 | HLA-DQB2 | HLA-DRA |
| | HLA-DRB 1 | HLA-DRB2 | HLA-DRB3 | HLA-DRB4 | HLA-DRB5 |
| | HLA-E | HLA-F | IGSF6 | IL10RA | IL2RB |
| | IRF8 | KLRK1 | LCK | LCP2 | LOC100133583 |
| | LOC100133661 | LOC100133811 | LST1 | LTB | LY86 |
| | MFNG | MNDA | NKG7 | PLEK | PRKCB 1 |
| | PSCDBP | PSMB10 | PSMB8 | PSMB9 | PTPRC |
| | PTPRCAP | RAC2 | RNASE2 | RNASE6 | SAMSN1 |
| | SLA | SRGN | TAP1 | TFEC | TNFAIP3 |
| | TNFRSF1B | TRA@ | TRAC | TRAJ17 | TRAV20 |
| | TRBC1 | TRIM22 | ZNF749 | CTSS | |
| SPC25 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DRPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | MCM2 | MELK |
| | NDC80 | TPX2 | | | |
| AURKA | ASPM | ATAD2 | AURKB | BTTB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | SPC25 | BIRC5 | BUB1 | CCNB1 |
| | CENPA | KPNA2 | LMNB 1 | MCM2 | MELK |
| | NDC80 | TPX2 | PSMA7 | CSE1L | |
| BIRC5 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | K1F11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | SPC25 | BUB 1 | CCNB1 |
| | CENPA | KPNA2 | LMNB1 | MCM2 | MELK |
| | NDC80 | TPX2 | MKI67 | | |
| BUB1 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHFK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP13 | TTK | TYMS | UBE2C |
| | UBE2S | AURKA | BIRC5 | SPC25 | CCNB1 |
| | CENPA | KPNA2 | LMNB1 | MCM2 | MELK |
| | NDC80 | TPX2 | | | |
| CCNB1 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP13 | TTK | TYMS | UBE2C |
| | UBE2S | AURKA | BIRC5 | BUB1 | SPC25 |
| | CENPA | KPNA2 | LMNB1 | MCM2 | MELK |
| | NDC80 | TPX2 | | | |
| CENPA | ASPM | ATAD2 | AURKB | BUB 1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP13 | TTK | TYMS | UBE2C |
| | UBE2S | AURKA | BIRC5 | BUB1 | CCNB1 |
| | SPC25 | KPNA2 | LMNB1 | MCM2 | MELK |
| | NDC80 | TPX2 | | | |
| KPNA2 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DLPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP13 | TTK | TYMS | UBE2C |
| | UBE2S | AURKA | BIRC5 | BUB1 | CCNB1 |
| | CENPA | SPC25 | LMNB1 | MCM2 | MELK |
| | NDC80 | TPX2 | NOL11 | PSMD12 | |
| LMNB1 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CRP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP13 | TTK | TYMS | UBE2C |
| | UBE2S | AURKA | BIRC5 | BUB1 | CCNB1 |
| | CENPA | KPNA2 | SPC25 | MCM2 | MELK |
| | NDC80 | TPX2 | | | |
| MCM2 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOPXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP13 | TTK | TYMS | UBE2C |
| | UBE2S | AURKA | BIRC5 | BUB 1 | CCNB1 |
| | CENPA | KPNA2 | LMNB 1 | SPC25 | MELK |
| | NDC80 | TPX2 | | | |
| MELK | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | MCM2 | SPC25 |
| | NDC80 | TPX2 | | | |
| NDC80 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIP4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP13 | TTK | TYMS | UBE2C |
| | UBE2S | AURKA | BIRC5 | BUB1 | CCNB1 |
| | CENPA | KPNA2 | LMNB1 | MCM2 | MELK |
| | SPC25 | TPX2 | | | |
| TPX2 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | MCM2 | MELK |
| | NDC80 | SPC25 | | | |
| CDH11 | INHB A | WISP 1 | COL1A1 | COL1 A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN 1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| INHBA | CDH11 | WISP1 | COL1A1 | COL1A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| WISP1 | INHBA | CDH11 | COL1A1 | COL1A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN 1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| COL1A1 | INHBA | WISP1 | CDH11 | COL1A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| COL1A2 | INHBA | WISP1 | COL1A1 | CDH11 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| FN1 | INHBA | WISP1 | COL1A1 | COL1A2 | CDH11 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN 1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC 17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |

The present invention will now be described by way of reference to the following clauses:
1. A method for predicting the clinical outcome of a patient diagnosed with cancer comprising:
   (a) obtaining an expression level of an expression product of at least one prognostic gene from a tissue sample obtained from a tumor of the patient, wherein the at least one prognostic gene is selected from GSTM2, IL6ST, GSTM3, C8orf4, TNFRSF11B, NAT1, RUNX1, CSF1, ACTR2, LMNB1, TFRC, LAPTM4B, ENO1, CDC20, and IDH2, or a gene listed in Tables 1, 2, 7, or 8;
   (b) normalizing the expression level of the expression product of the at least one prognostics gene to obtain a normalized expression level; and
   (c) calculating a risk score based on the normalized expression value, wherein increased expression of a prognostic gene selected from GSTM2, IL6ST, GSTM3, C8orf4, TNFRSF11B, NAT1, RUNX1, and CSF1, or a prognostic gene listed in Tables 1 and 7, is positively correlated with good prognosis, and wherein increased expression of a prognostic gene selected from ACTR2, LMNB1, TFRC, LAPTM4B, ENO1, CDC20, and IDH2, or a prognostic gene in Tables 2 and 8, is negatively associated with good prognosis.
2. The method of clause 1, further comprising: generating a report based on the risk score.
3. The method of clause 1, wherein the patient is a human patient.
4. The method of clause 1, wherein the tumor is a breast cancer tumor.
5. The method of clause 1, wherein the tissue sample is a fixed paraffin-embedded tissue.
6. The method of clause 1, wherein the expression level is obtained using a PCR-based method.
7. The method of clause 1, wherein an expression level is obtained from at least two of the genes in any of the stromal, metabolic, immune, proliferation, or metabolic groups, or their gene products.
8. The method of clause 1, wherein an expression level is obtained from at least four genes in any two of the stromal, metabolic, immune, proliferation, or metabolic groups, or their gene products.
9. The method of clause 1, further comprising obtaining an expression level of at least one co-expressed gene from those listed in Table 18.
10. A method for predicting the clinical outcome of a patient diagnosed with estrogen receptor-negative (ER-) breast cancer comprising:
   (a) obtaining an expression level of an expression product of at least one prognostic gene listed in Tables 3, 4, 9 or 10 from a tissue sample obtained from a tumor of the patient, wherein the tumor is estrogen receptor negative;
   (b) normalizing the expression level of the expression product of the at least one prognostic gene to obtain a normalized expression level; and
   (c) calculating a risk score based on the normalized expression value, wherein increased expression of prognostic genes in Table 3 and Table 9 are positively correlated with good prognosis, and wherein increased expression of prognostic genes in Table 4 and Table 10 are negatively associated with good prognosis.
11. The method of clause 10, further comprising: generating a report based on the risk score.
12. The method of clause 10, wherein the patient is a human patient.
13. The method of clause 10, wherein the tumor is a breast cancer tumor is fixed paraffin-embedded tissue.
14. The method of clause 10, wherein the expression level is obtained using a PCR-based method.
15. The method of clause 10, wherein an expression level is obtained from at least two of the genes in any of the stromal, metabolic, immune, proliferation, or metabolic groups, or their gene products.
16. The method of clause 10, wherein an expression level is obtained from at least four genes in any two of the stromal, metabolic, immune, proliferation, or metabolic groups, or their gene products.
17. The method of clause 10, further comprising obtaining an expression level of at least one co-expressed gene from those listed in Table 17.
18. A computer program product for classifying a cancer patient according to prognosis, the computer program product for use in conjunction with a computer having a memory and a processor, the computer program product comprising a computer readable storage medium having a computer program encoded thereon, wherein said computer program product can be loaded into the one or more memory units of a computer and causes the one or more processor units of the computer to execute the steps of:
   (a) receiving a first data structure comprising the respective levels of an expression product of each of at least three different prognostic genes listed in any of Tables 1-12 in a tissue samples obtained from tumor in said patient;
   (b) normalizing said at least three expression values to obtain normalized expression values;
   (c) determining the similarity of the normalized expression values of each of said at least three prognostic genes to respective control levels of expression of the at least three prognostic genes obtained from a second data structure to obtain a patient similarity value, wherein the second data structure is based on levels of expression from a plurality of cancer tumors;
   (d) comparing said patient similarity value to a selected threshold value of similarity of said respective normalized expression values of each of said at least three prognostic genes to said respective control levels of expression of said at least three prognostic genes; and
   (e) classifying said patient as having a first prognosis if said patient similarity value exceeds said threshold similarity value, and a second prognosis if said patient similarity value does not exceed said threshold similarity value.

## Claims

1. A method of predicting the clinical outcome of a patient diagnosed with breast cancer comprising:
extracting RNA from a breast tumor tissue sample obtained from the patient;
determining, by digital gene expression DNA sequencing, levels of each of GRB7, ERBB2, ESR1, PGR, BCL2, SCUBE2 (CEGP1), BIRC5 (SURV), Ki67 (MKI67), CCNB1, STK15 (AURKA), GSTM1, and BAG1, wherein at least one of the following primer and probe sequence sets are used:
GRB7, SEQ ID Nos: 210, 594, and 978;
ERBB2, SEQ ID Nos: 224, 608, and 992;
ESR1, SEQ ID Nos: 163, 547, and 931;
PGR, SEQ ID Nos: 329, 713, and 1097;
BCL2, SEQ ID Nos: 43, 427, and 811;
SCUBE2 (CEGP1), SEQ ID Nos: 92, 476, and 860;
BIRC5 (SURV), SEQ ID Nos: 362, 746, and 1130;
Ki67 (MKI67), SEQ ID Nos: 275, 659, and 1043;
CCNB1, SEQ ID Nos: 66, 450, and 834;
STK15 (AURKA), SEQ ID Nos: 359, 743, and 1127;
GSTM1, SEQ ID Nos: 215, 599, and 983; or
BAG1: SEQ ID Nos: 35, 419, and 803;
wherein increased expression of a gene correlates with a good prognosis.

2. A method of predicting the clinical outcome of a patient diagnosed with breast cancer comprising:
extracting RNA from a breast tumor tissue sample obtained from the patient;
reverse transcribing RNA transcripts from the extracted RNA to produce cDNA amplicons; and
determining, by digital gene expression DNA sequencing, levels of cDNA amplicons of each of GRB7, ERBB2, ESR1, PGR, BCL2, SCUBE2 (CEGP1), BIRC5 (SURV), Ki67 (MKI67), CCNB1, STK15 (AURKA), GSTM1, and BAG1, wherein the cDNA amplicons comprise at least one of the following polynucleotides:
SEQ ID NO: 1362 (for GRB7);
SEQ ID NO: 1376 (for ERBB2);
SEQ ID NO: 1323 (for ESR1);
SEQ ID NO: 1481 (for PGR);
SEQ ID NO: 1196 (for BCL2);
SEQ ID NO: 1514 (for BIRC5 (SURV));
SEQ ID NO: 1427 (for Ki67 (MKI67));
SEQ ID NO: 1218 (for CCNB1);
SEQ ID NO: 1511 (for STK15 (AURKA));
SEQ ID NO: 1367 (for GSTM1); or
SEQ ID NO: 1187 (for BAG1);
wherein increased expression of a gene correlates with a good prognosis.

3. The method of claim 1 or 2, wherein the sample is a fixed, paraffin-embedded tissue sample.

4. The method of any preceding claim, wherein the patient is an ER positive breast cancer patient.

5. The method of any preceding claim, wherein the cDNA levels are normalized based on either the total RNA level in the sample or the cDNA level of at least one reference RNA transcript.

6. The method of claim 5, wherein the cDNA levels are normalized based on the cDNA level of one or more of AAMP, ARF1, EEF1A1, ESD, GPS1, H3F3A, HNRPC, RPL13A, RPL41, RPS23, RPS27, SDHA, TCEA1, UBB, YWHAZ, B-actin, GUS, GAPDH, RPLPO, or TFRC.

7. The method of any preceding claim, further comprising providing a report based on the digital gene expression data from each of GRB7, ERBB2, ESR1, PGR, BCL2, SCUBE2 (CEGP1), BIRC5 (SURV), Ki67 (MKI67), CCNB1, STK15 (AURKA), MMP11 (STMY3), CD68, GSTM1, and BAG1.


SEQUENCE LISTING

<110> Genomic Health, Inc.
      BAKER, JOFFRE B.
      Cronin, Maureen T.
      Collin, Francois
      Liu, Mei-Lan

<120> Methods To Predict Clinical Outcome Of
  Cancer

<130> GHDX-040WO

<150> US 61/263,763
<151> 2009-11-23

<160> 1536

<170> FastSEQ for Windows Version 4.0

<210> 1
<211> 23
<212> DNA
<213> Artificial Sequence

<220>
<223> Synthetic primer

<400> 1
cgttccgatc ctctatactg cat                                              23

<210> 2
<211> 20
<212> DNA
<213> Artificial Sequence

<220>
<223> Synthetic primer

<400> 2
gtgtggcagg tggacactaa                                                  20

<210> 3
<211> 20
<212> DNA
<213> Artificial Sequence

<220>
<223> Synthetic primer

<400> 3
aaacaccact ggagcattga                                                  20

<210> 4
<211> 18
<212> DNA
<213> Artificial Sequence

<220>
<223> Synthetic primer

<400> 4
accagtgcca caatgcag                                                    18

<210> 5
<211> 20
<212> DNA
<213> Artificial Sequence

<220>
<223> Synthetic primer

<400> 5
tgcagactgt accatgctga                                                  20

<210> 6
<211> 20
<212> DNA
<213> Artificial Sequence

<220>
<223> Synthetic primer

<400> 6
acacgtctgt caccatggaa                                                  20

<210> 7
<211> 18
<212> DNA
<213> Artificial Sequence

<220>
<223> Synthetic primer

<400> 7
atccgcattg aagaccca                                                    18

<210> 8
<211> 20
<212> DNA
<213> Artificial Sequence

<220>
<223> Synthetic primer

<400> 8
gactgtctcg tttccctggt                                                  20

<210> 9
<211> 21
<212> DNA
<213> Artificial Sequence

<220>
<223> Synthetic primer

<400> 9
tcaaaagtac ggacacctcc t                                                21

<210> 10
<211> 19
<212> DNA
<213> Artificial Sequence

<220>
<223> Synthetic primer

<400> 10
gagcatgcgt ctactgcct                                                    19

<210> 11
<211> 20
<212> DNA
<213> Artificial Sequence

<220>
<223> Synthetic primer

<400> 11
gaagtgccag gaggcgatta                                                   20

<210> 12
<211> 19
<212> DNA
<213> Artificial Sequence

<220>
<223> Synthetic primer

<400> 12
caaggcccca tctgaatca                                                    19

<210> 13
<211> 20
<212> DNA
<213> Artificial Sequence

<220>
<223> Synthetic primer

<400> 13
gcgagttcaa agtgttcgag                                                   20

<210> 14
<211> 18
<212> DNA
<213> Artificial Sequence

<220>
<223> Synthetic primer

<400> 14
taagccacaa gcacacgg                                                     18

<210> 15
<211> 21
<212> DNA
<213> Artificial Sequence

<220>
<223> Synthetic primer

<400> 15
acgagatgtc ctacggcttg a                                                 21

<210> 16
<211> 22

<212> DNA
<213> Artificial Sequence

<220>
<223> Synthetic primer

<400> 16
agccaacatg tgactaattg ga                                                22

<210> 17
<211> 27
<212> DNA
<213> Artificial Sequence

<220>
<223> Synthetic primer

<400> 17
ctgcatgtga ttgaataaga aacaaga                                           27

<210> 18
<211> 19
<212> DNA
<213> Artificial Sequence

<220>
<223> Synthetic primer

<400> 18
gtggaaacgg agctcttcc                                                    19

<210> 19
<211> 25
<212> DNA
<213> Artificial Sequence

<220>
<223> Synthetic primer

<400> 19
ttgtctctgc cttggactat ctaca                                             25

<210> 20
<211> 21
<212> DNA
<213> Artificial Sequence

<220>
<223> Synthetic primer

<400> 20
gaggaatatg gaatccaagg g                                                 21

<210> 21
<211> 20
<212> DNA
<213> Artificial Sequence

<220>
<223> Synthetic primer

<400> 21
ggacagggta agaccgtgat                                                   20

<210> 22
<211> 20
<212> DNA
<213> Artificial Sequence

<220>
<223> Synthetic primer

<400> 22
ccgtgaaagc tgctctgtaa                                                    20

<210> 23
<211> 23
<212> DNA
<213> Artificial Sequence

<220>
<223> Synthetic primer

<400> 23
caagacacta agggcgacta cca                                                23

<210> 24
<211> 20
<212> DNA
<213> Artificial Sequence

<220>
<223> Synthetic primer

<400> 24
gactgcaaag atggaaacga                                                    20

<210> 25
<211> 20
<212> DNA
<213> Artificial Sequence

<220>
<223> Synthetic primer

<400> 25
gatgaagcct ttcgcaagtt                                                    20

<210> 26
<211> 18
<212> DNA
<213> Artificial Sequence

<220>
<223> Synthetic primer

<400> 26
gtttatgcca tcggcacc                                                      18

<210> 27
<211> 20
<212> DNA
<213> Artificial Sequence

<220>
<223> Synthetic primer

<400> 27
cagtagagat ccccgcaact                                                    20

<210> 28
<211> 20
<212> DNA
<213> Artificial Sequence

<220>
<223> Synthetic primer

<400> 28
atcagagatt accgcgtcgt                                                    20

<210> 29
<211> 19
<212> DNA
<213> Artificial Sequence

<220>
<223> Synthetic primer

<400> 29
gactgggtca gtgatggca                                                     19

<210> 30
<211> 20
<212> DNA
<213> Artificial Sequence

<220>
<223> Synthetic primer

<400> 30
tccctgagaa cgaaaccact                                                    20

<210> 31
<211> 21
<212> DNA
<213> Artificial Sequence

<220>
<223> Synthetic primer

<400> 31
agctgcagaa gagctgcaca t                                                  21

<210> 32
<211> 21
<212> DNA
<213> Artificial Sequence

<220>
<223> Synthetic primer

<400> 32
cagcagatgt ggatcagcaa g                                                  21

<210> 33
<211> 22
<212> DNA
<213> Artificial Sequence

<220>
<223> Synthetic primer

<400> 33
ggctcttgtg cgtactgtcc tt                                                22

<210> 34
<211> 19
<212> DNA
<213> Artificial Sequence

<220>
<223> Synthetic primer

<400> 34
gggtcaggtg cctcgagat                                                    19

<210> 35
<211> 23
<212> DNA
<213> Artificial Sequence

<220>
<223> Synthetic primer

<400> 35
cgttgtcagc acttggaata caa                                               23

<210> 36
<211> 18
<212> DNA
<213> Artificial Sequence

<220>
<223> Synthetic primer

<400> 36
cctacggccg ctactacg                                                     18

<210> 37
<211> 23
<212> DNA
<213> Artificial Sequence

<220>
<223> Synthetic primer

<400> 37
gactcctcag ggcagacttt ctt                                               23

<210> 38
<211> 18
<212> DNA
<213> Artificial Sequence

<220>
<223> Synthetic primer

<400> 38
ccgccgtgga cacagact                                                     18

<210> 39

<211> 20
<212> DNA
<213> Artificial Sequence

<220>
<223> Synthetic primer

<400> 39
cctggagggt cctgtacaat                                                    20

<210> 40
<211> 20
<212> DNA
<213> Artificial Sequence

<220>
<223> Synthetic primer

<400> 40
actgacaaga ccagcagcat                                                    20

<210> 41
<211> 26
<212> DNA
<213> Artificial Sequence

<220>
<223> Synthetic primer

<400> 41
tgacttccta gttcgtgact ctctgt                                             26

<210> 42
<211> 20
<212> DNA
<213> Artificial Sequence

<220>
<223> Synthetic primer

<400> 42
ccccgagaca acggagataa                                                    20

<210> 43
<211> 25
<212> DNA
<213> Artificial Sequence

<220>
<223> Synthetic primer

<400> 43
cagatggacc tagtacccac tgaga                                              25

<210> 44
<211> 18
<212> DNA
<213> Artificial Sequence

<220>
<223> Synthetic primer

<400> 44

aacccacccc tgtcttgg 18

<210> 45
<211> 18
<212> DNA
<213> Artificial Sequence

<220>
<223> Synthetic primer

<400> 45
gagctccgca aggatgac 18

<210> 46
<211> 23
<212> DNA
<213> Artificial Sequence

<220>
<223> Synthetic primer

<400> 46
attcctatgg ctctgcaatt gtc 23

<210> 47
<211> 20
<212> DNA
<213> Artificial Sequence

<220>
<223> Synthetic primer

<400> 47
ctggacggag tagctccaag 20

<210> 48
<211> 19
<212> DNA
<213> Artificial Sequence

<220>
<223> Synthetic primer

<400> 48
aattttatga gggccacgg 19

<210> 49
<211> 20
<212> DNA
<213> Artificial Sequence

<220>
<223> Synthetic primer

<400> 49
gttgggacac agttggtctg 20

<210> 50
<211> 21
<212> DNA
<213> Artificial Sequence

<220>

<223> Synthetic primer

<400> 50
ccgaggttaa tccagcacgt a                                                21

<210> 51
<211> 24
<212> DNA
<213> Artificial Sequence

<220>
<223> Synthetic primer

<400> 51
tcaacagaag gctgaaccac taga                                             24

<210> 52
<211> 22
<212> DNA
<213> Artificial Sequence

<220>
<223> Synthetic primer

<400> 52
ctgaagcaga tggttcatca tt                                               22

<210> 53
<211> 25
<212> DNA
<213> Artificial Sequence

<220>
<223> Synthetic primer

<400> 53
gaggcaactg cttatggctt aatta                                            25

<210> 54
<211> 18
<212> DNA
<213> Artificial Sequence

<220>
<223> Synthetic primer

<400> 54
caagagcaga gccaccgt                                                    18

<210> 55
<211> 20
<212> DNA
<213> Artificial Sequence

<220>
<223> Synthetic primer

<400> 55
gtgactgcac aggactctgg                                                  20

<210> 56
<211> 20
<212> DNA

<213> Artificial Sequence

<220>
<223> Synthetic primer

<400> 56
tcagctgtga gctgcggata                                                    20

<210> 57
<211> 22
<212> DNA
<213> Artificial Sequence

<220>
<223> Synthetic primer

<400> 57
gcggtatcag gaatttcaac ct                                                 22

<210> 58
<211> 20
<212> DNA
<213> Artificial Sequence

<220>
<223> Synthetic primer

<400> 58
ctacgagtca gcccatccat                                                    20

<210> 59
<211> 20
<212> DNA
<213> Artificial Sequence

<220>
<223> Synthetic primer

<400> 59
tgatgctgca gagaacttcc                                                    20

<210> 60
<211> 22
<212> DNA
<213> Artificial Sequence

<220>
<223> Synthetic primer

<400> 60
atccattcga tctcaccaag gt                                                 22

<210> 61
<211> 20
<212> DNA
<213> Artificial Sequence

<220>
<223> Synthetic primer

<400> 61
gtggctcaac attgtgttcc                                                    20

<210> 62
<211> 20
<212> DNA
<213> Artificial Sequence

<220>
<223> Synthetic primer

<400> 62
aggggatggt ctctgtcatt 20

<210> 63
<211> 21
<212> DNA
<213> Artificial Sequence

<220>
<223> Synthetic primer

<400> 63
gaacgcatca tccagagact g 21

<210> 64
<211> 21
<212> DNA
<213> Artificial Sequence

<220>
<223> Synthetic primer

<400> 64
agcagacagt ggtcagtcct t 21

<210> 65
<211> 20
<212> DNA
<213> Artificial Sequence

<220>
<223> Synthetic primer

<400> 65
aggttctgag ctctggcttt 20

<210> 66
<211> 20
<212> DNA
<213> Artificial Sequence

<220>
<223> Synthetic primer

<400> 66
ttcaggttgt tgcaggagac 20

<210> 67
<211> 27
<212> DNA
<213> Artificial Sequence

<220>
<223> Synthetic primer

<400> 67
cctctgtgct acagattata cctttgc                                          27

<210> 68
<211> 25
<212> DNA
<213> Artificial Sequence

<220>
<223> Synthetic primer

<400> 68
ggtcaccaag aaacatcagt atgaa                                            25

<210> 69
<211> 18
<212> DNA
<213> Artificial Sequence

<220>
<223> Synthetic primer

<400> 69
cagactgaat gggggtgg                                                    18

<210> 70
<211> 20
<212> DNA
<213> Artificial Sequence

<220>
<223> Synthetic primer

<400> 70
ggatgacatg cactcagctc                                                  20

<210> 71
<211> 20
<212> DNA
<213> Artificial Sequence

<220>
<223> Synthetic primer

<400> 71
ggagtggaag gaactggaaa                                                  20

<210> 72
<211> 21
<212> DNA
<213> Artificial Sequence

<220>
<223> Synthetic primer

<400> 72
tccaactaat gccaccacca a                                                21

<210> 73
<211> 20
<212> DNA
<213> Artificial Sequence

<220>
<223> Synthetic primer

<400> 73
gtgctggagt cgggactaac                                                   20

<210> 74
<211> 19
<212> DNA
<213> Artificial Sequence

<220>
<223> Synthetic primer

<400> 74
atcaccgaca gcacagaca                                                    19

<210> 75
<211> 20
<212> DNA
<213> Artificial Sequence

<220>
<223> Synthetic primer

<400> 75
gacgaagaca gtccctggat                                                   20

<210> 76
<211> 20
<212> DNA
<213> Artificial Sequence

<220>
<223> Synthetic primer

<400> 76
ctcataccag ccatccaatg                                                   20

<210> 77
<211> 18
<212> DNA
<213> Artificial Sequence

<220>
<223> Synthetic primer

<400> 77
tggttcccag ccctgtgt                                                     18

<210> 78
<211> 20
<212> DNA
<213> Artificial Sequence

<220>
<223> Synthetic primer

<400> 78
gtgcaggctc aggtgaagtg                                                   20

<210> 79
<211> 21

<212> DNA
<213> Artificial Sequence

<220>
<223> Synthetic primer

<400> 79
tggattggag ttctgggaat g                                                21

<210> 80
<211> 20
<212> DNA
<213> Artificial Sequence

<220>
<223> Synthetic primer

<400> 80
tcttgctggc tacgcctctt                                                  20

<210> 81
<211> 20
<212> DNA
<213> Artificial Sequence

<220>
<223> Synthetic primer

<400> 81
ggtgagcaga agtggcctat                                                  20

<210> 82
<211> 18
<212> DNA
<213> Artificial Sequence

<220>
<223> Synthetic primer

<400> 82
gcagtccgct gtgttcaa                                                    18

<210> 83
<211> 20
<212> DNA
<213> Artificial Sequence

<220>
<223> Synthetic primer

<400> 83
gagctgaaag acgcacactg                                                  20

<210> 84
<211> 18
<212> DNA
<213> Artificial Sequence

<220>
<223> Synthetic primer

<400> 84
cggagaaggg caccagta                                                    18

<210> 85
<211> 18
<212> DNA
<213> Artificial Sequence

<220>
<223> Synthetic primer

<400> 85
gtcggcagaa gcaggact                                                    18

<210> 86
<211> 18
<212> DNA
<213> Artificial Sequence

<220>
<223> Synthetic primer

<400> 86
acccatgtac cgtcctcg                                                    18

<210> 87
<211> 20
<212> DNA
<213> Artificial Sequence

<220>
<223> Synthetic primer

<400> 87
ccttcccatc agcacagttc                                                  20

<210> 88
<211> 20
<212> DNA
<213> Artificial Sequence

<220>
<223> Synthetic primer

<400> 88
aagccctatc cgatgtaccc                                                  20

<210> 89
<211> 21
<212> DNA
<213> Artificial Sequence

<220>
<223> Synthetic primer

<400> 89
tggatctcta ccagcaatgt g                                                21

<210> 90
<211> 22
<212> DNA
<213> Artificial Sequence

<220>
<223> Synthetic primer

<400> 90
acttgcctgt tcagagcact ca                                                22

<210> 91
<211> 21
<212> DNA
<213> Artificial Sequence

<220>
<223> Synthetic primer

<400> 91
ttggttttgc tcggatactt g                                                 21

<210> 92
<211> 21
<212> DNA
<213> Artificial Sequence

<220>
<223> Synthetic primer

<400> 92
tgacaatcag cacacctgca t                                                 21

<210> 93
<211> 21
<212> DNA
<213> Artificial Sequence

<220>
<223> Synthetic primer

<400> 93
taaattcact cgtggtgtgg a                                                 21

<210> 94
<211> 20
<212> DNA
<213> Artificial Sequence

<220>
<223> Synthetic primer

<400> 94
ctgaaggagc tccaagacct                                                   20

<210> 95
<211> 21
<212> DNA
<213> Artificial Sequence

<220>
<223> Synthetic primer

<400> 95
ccagaatgca cgctacagga a                                                 21

<210> 96
<211> 18
<212> DNA
<213> Artificial Sequence

<220>
<223> Synthetic primer

<400> 96
ccaccatagg cagaggca                                                    18

<210> 97
<211> 20
<212> DNA
<213> Artificial Sequence

<220>
<223> Synthetic primer

<400> 97
gaggccagtg gtggaaacag                                                  20

<210> 98
<211> 20
<212> DNA
<213> Artificial Sequence

<220>
<223> Synthetic primer

<400> 98
aaggaagtgg tccctctgtg                                                  20

<210> 99
<211> 18
<212> DNA
<213> Artificial Sequence

<220>
<223> Synthetic primer

<400> 99
agaatgggtg tgaaggcg                                                    18

<210> 100
<211> 20
<212> DNA
<213> Artificial Sequence

<220>
<223> Synthetic primer

<400> 100
ggctggacgt ggttttgtct                                                  20

<210> 101
<211> 19
<212> DNA
<213> Artificial Sequence

<220>
<223> Synthetic primer

<400> 101
ggctgctttg ctgcaactg                                                   19

<210> 102

<211> 20
<212> DNA
<213> Artificial Sequence

<220>
<223> Synthetic primer

<400> 102
cggtacttga gcaatgccta                                                    20

<210> 103
<211> 23
<212> DNA
<213> Artificial Sequence

<220>
<223> Synthetic primer

<400> 103
ccccaggata cctaccacta cct                                                23

<210> 104
<211> 21
<212> DNA
<213> Artificial Sequence

<220>
<223> Synthetic primer

<400> 104
aaatcgcagc ttatcacaag g                                                  21

<210> 105
<211> 18
<212> DNA
<213> Artificial Sequence

<220>
<223> Synthetic primer

<400> 105
gtggccatcc agctgacc                                                      18

<210> 106
<211> 24
<212> DNA
<213> Artificial Sequence

<220>
<223> Synthetic primer

<400> 106
cagccaagaa ctggtatagg agct                                               24

<210> 107
<211> 21
<212> DNA
<213> Artificial Sequence

<220>
<223> Synthetic primer

<400> 107

ccttatcggc tggaacgagt t                                                21

<210> 108
<211> 24
<212> DNA
<213> Artificial Sequence

<220>
<223> Synthetic primer

<400> 108
cgacagttgc gatgaaagtt ctaa                                            24

<210> 109
<211> 20
<212> DNA
<213> Artificial Sequence

<220>
<223> Synthetic primer

<400> 109
atgcccagtg ttcctgactt                                                 20

<210> 110
<211> 20
<212> DNA
<213> Artificial Sequence

<220>
<223> Synthetic primer

<400> 110
gatgtgattg aggtgcatgg                                                 20

<210> 111
<211> 20
<212> DNA
<213> Artificial Sequence

<220>
<223> Synthetic primer

<400> 111
aagtcctgaa attgcgatca                                                 20

<210> 112
<211> 20
<212> DNA
<213> Artificial Sequence

<220>
<223> Synthetic primer

<400> 112
cagcaagaac tgcaacaaca                                                 20

<210> 113
<211> 18
<212> DNA
<213> Artificial Sequence

<220>

<223> Synthetic primer

<400> 113
tgcagcggct gattgaca                                                    18

<210> 114
<211> 20
<212> DNA
<213> Artificial Sequence

<220>
<223> Synthetic primer

<400> 114
gagcacaacc aaacctacga                                                  20

<210> 115
<211> 20
<212> DNA
<213> Artificial Sequence

<220>
<223> Synthetic primer

<400> 115
taccacaccc agcattcctc                                                  20

<210> 116
<211> 20
<212> DNA
<213> Artificial Sequence

<220>
<223> Synthetic primer

<400> 116
cctgaacatg aaggagctga                                                  20

<210> 117
<211> 19
<212> DNA
<213> Artificial Sequence

<220>
<223> Synthetic primer

<400> 117
gagttcaagt gccctgacg                                                   19

<210> 118
<211> 20
<212> DNA
<213> Artificial Sequence

<220>
<223> Synthetic primer

<400> 118
gctcacttcg gctaaaatgc                                                  20

<210> 119
<211> 24
<212> DNA

<213> Artificial Sequence

<220>
<223> Synthetic primer

<400> 119
gtacatgatc ccctgtgaga aggt                                              24

<210> 120
<211> 21
<212> DNA
<213> Artificial Sequence

<220>
<223> Synthetic primer

<400> 120
tgtctcactg agcgagcaga a                                                 21

<210> 121
<211> 21
<212> DNA
<213> Artificial Sequence

<220>
<223> Synthetic primer

<400> 121
accaggcaat aacctaacag c                                                 21

<210> 122
<211> 19
<212> DNA
<213> Artificial Sequence

<220>
<223> Synthetic primer

<400> 122
ggagcaaaat cgatgcagt                                                    19

<210> 123
<211> 19
<212> DNA
<213> Artificial Sequence

<220>
<223> Synthetic primer

<400> 123
gagctacaga tgcccatgc                                                    19

<210> 124
<211> 18
<212> DNA
<213> Artificial Sequence

<220>
<223> Synthetic primer

<400> 124
tgcgcccttt cctctgta                                                     18

<210> 125
<211> 20
<212> DNA
<213> Artificial Sequence

<220>
<223> Synthetic primer

<400> 125
tgaccgcttc taccccaatg                                                    20

<210> 126
<211> 20
<212> DNA
<213> Artificial Sequence

<220>
<223> Synthetic primer

<400> 126
ccggagtgac tctatcacca                                                    20

<210> 127
<211> 26
<212> DNA
<213> Artificial Sequence

<220>
<223> Synthetic primer

<400> 127
tccttatagg tactttcagc catttg                                             26

<210> 128
<211> 22
<212> DNA
<213> Artificial Sequence

<220>
<223> Synthetic primer

<400> 128
ccagctttgt gcctgtcact at                                                 22

<210> 129
<211> 20
<212> DNA
<213> Artificial Sequence

<220>
<223> Synthetic primer

<400> 129
tgctcattct tgaggagcat                                                    20

<210> 130
<211> 20
<212> DNA
<213> Artificial Sequence

<220>
<223> Synthetic primer

<400> 130
tggtgggtct aggtggtgta                                                    20

<210> 131
<211> 20
<212> DNA
<213> Artificial Sequence

<220>
<223> Synthetic primer

<400> 131
aaatgtcctc ctcgactgct                                                    20

<210> 132
<211> 20
<212> DNA
<213> Artificial Sequence

<220>
<223> Synthetic primer

<400> 132
ggtcaccgtt ggtgtcatca                                                    20

<210> 133
<211> 24
<212> DNA
<213> Artificial Sequence

<220>
<223> Synthetic primer

<400> 133
atggagatgt ggtcattcct agtg                                               24

<210> 134
<211> 21
<212> DNA
<213> Artificial Sequence

<220>
<223> Synthetic primer

<400> 134
gccgccacaa gactaaggaa t                                                  21

<210> 135
<211> 20
<212> DNA
<213> Artificial Sequence

<220>
<223> Synthetic primer

<400> 135
tccaattcca gcatcactgt                                                    20

<210> 136
<211> 20
<212> DNA
<213> Artificial Sequence

<220>
<223> Synthetic primer

<400> 136
gattcagacg aggatgagcc                                                    20

<210> 137
<211> 21
<212> DNA
<213> Artificial Sequence

<220>
<223> Synthetic primer

<400> 137
cacggaggta taaggcagga g                                                  21

<210> 138
<211> 24
<212> DNA
<213> Artificial Sequence

<220>
<223> Synthetic primer

<400> 138
ctctgagaca gtgcttcgat gact                                               24

<210> 139
<211> 22
<212> DNA
<213> Artificial Sequence

<220>
<223> Synthetic primer

<400> 139
tggcactact gcatgattga ca                                                 22

<210> 140
<211> 19
<212> DNA
<213> Artificial Sequence

<220>
<223> Synthetic primer

<400> 140
aaatcgctgg gaacaagtg                                                     19

<210> 141
<211> 20
<212> DNA
<213> Artificial Sequence

<220>
<223> Synthetic primer

<400> 141
agacatcagc tcctggttca                                                    20

<210> 142
<211> 18

<212> DNA
<213> Artificial Sequence

<220>
<223> Synthetic primer

<400> 142
tggtgacgat ggaggagc                                                    18

<210> 143
<211> 20
<212> DNA
<213> Artificial Sequence

<220>
<223> Synthetic primer

<400> 143
actccctcta cccttgagca                                                  20

<210> 144
<211> 19
<212> DNA
<213> Artificial Sequence

<220>
<223> Synthetic primer

<400> 144
ctgctggatg accttcctc                                                   19

<210> 145
<211> 20
<212> DNA
<213> Artificial Sequence

<220>
<223> Synthetic primer

<400> 145
tgccacctgg acatcatttg                                                  20

<210> 146
<211> 23
<212> DNA
<213> Artificial Sequence

<220>
<223> Synthetic primer

<400> 146
cgacaaggag tgcgtctact tct                                              23

<210> 147
<211> 21
<212> DNA
<213> Artificial Sequence

<220>
<223> Synthetic primer

<400> 147
tttcctcaaa tttgcctcaa g                                                21

<210> 148
<211> 19
<212> DNA
<213> Artificial Sequence

<220>
<223> Synthetic primer

<400> 148
actgtgaact gcctggtgc                                                    19

<210> 149
<211> 21
<212> DNA
<213> Artificial Sequence

<220>
<223> Synthetic primer

<400> 149
cgagtggaga ctggtgttct c                                                 21

<210> 150
<211> 18
<212> DNA
<213> Artificial Sequence

<220>
<223> Synthetic primer

<400> 150
atggactcca cagagccg                                                     18

<210> 151
<211> 20
<212> DNA
<213> Artificial Sequence

<220>
<223> Synthetic primer

<400> 151
ttgaacagag cctgaccaag                                                   20

<210> 152
<211> 19
<212> DNA
<213> Artificial Sequence

<220>
<223> Synthetic primer

<400> 152
gtccccgctg cagatctct                                                    19

<210> 153
<211> 20
<212> DNA
<213> Artificial Sequence

<220>
<223> Synthetic primer

<400> 153
ccatgtggat gaatgaggtg                                                    20

<210> 154
<211> 19
<212> DNA
<213> Artificial Sequence

<220>
<223> Synthetic primer

<400> 154
ggcggtgaag agtcacagt                                                     19

<210> 155
<211> 19
<212> DNA
<213> Artificial Sequence

<220>
<223> Synthetic primer

<400> 155
tcgagggcaa gaagagcaa                                                     19

<210> 156
<211> 25
<212> DNA
<213> Artificial Sequence

<220>
<223> Synthetic primer

<400> 156
gctagtactt tgatgctccc ttgat                                              25

<210> 157
<211> 20
<212> DNA
<213> Artificial Sequence

<220>
<223> Synthetic primer

<400> 157
caaggccgtg aacgagaagt                                                    20

<210> 158
<211> 20
<212> DNA
<213> Artificial Sequence

<220>
<223> Synthetic primer

<400> 158
agccccagca actacagtct                                                    20

<210> 159
<211> 20
<212> DNA
<213> Artificial Sequence

<220>
<223> Synthetic primer

<400> 159
gggccctcca gaacaatgat                                                    20

<210> 160
<211> 21
<212> DNA
<213> Artificial Sequence

<220>
<223> Synthetic primer

<400> 160
cgcctgttca ccaagattga c                                                  21

<210> 161
<211> 18
<212> DNA
<213> Artificial Sequence

<220>
<223> Synthetic primer

<400> 161
caaccaggca gctccatc                                                      18

<210> 162
<211> 20
<212> DNA
<213> Artificial Sequence

<220>
<223> Synthetic primer

<400> 162
tgaacggggt atcctcctta                                                    20

<210> 163
<211> 20
<212> DNA
<213> Artificial Sequence

<220>
<223> Synthetic primer

<400> 163
tggtccatcg ccagttatca                                                    20

<210> 164
<211> 25
<212> DNA
<213> Artificial Sequence

<220>
<223> Synthetic primer

<400> 164
tggctcttaa tcagtttcgt tacct                                              25

<210> 165

<211> 20
<212> DNA
<213> Artificial Sequence

<220>
<223> Synthetic primer

<400> 165
gtccaggtgg atgtgaaaga                                                    20

<210> 166
<211> 18
<212> DNA
<213> Artificial Sequence

<220>
<223> Synthetic primer

<400> 166
ccaacactag gctcccca                                                      18

<210> 167
<211> 22
<212> DNA
<213> Artificial Sequence

<220>
<223> Synthetic primer

<400> 167
ggcattgagc ctctctacat ca                                                 22

<210> 168
<211> 18
<212> DNA
<213> Artificial Sequence

<220>
<223> Synthetic primer

<400> 168
gtcactccgc caccgtag                                                      18

<210> 169
<211> 18
<212> DNA
<213> Artificial Sequence

<220>
<223> Synthetic primer

<400> 169
acccccagac cggatcag                                                      18

<210> 170
<211> 20
<212> DNA
<213> Artificial Sequence

<220>
<223> Synthetic primer

<400> 170

ccagcaccat tgttgaagat                                                    20

<210> 171
<211> 19
<212> DNA
<213> Artificial Sequence

<220>
<223> Synthetic primer

<400> 171
cgtggtgccc ctctatgac                                                     19

<210> 172
<211> 19
<212> DNA
<213> Artificial Sequence

<220>
<223> Synthetic primer

<400> 172
accatgtatc gagaggggc                                                     19

<210> 173
<211> 20
<212> DNA
<213> Artificial Sequence

<220>
<223> Synthetic primer

<400> 173
tggaaacagc gaaggataca                                                    20

<210> 174
<211> 23
<212> DNA
<213> Artificial Sequence

<220>
<223> Synthetic primer

<400> 174
gtgaaggatg tgaagcagac gta                                                23

<210> 175
<211> 18
<212> DNA
<213> Artificial Sequence

<220>
<223> Synthetic primer

<400> 175
ctgaccagaa ccacggct                                                      18

<210> 176
<211> 18
<212> DNA
<213> Artificial Sequence

<220>

<223> Synthetic primer

<400> 176
gcctcttcct gttcgacg                                                    18

<210> 177
<211> 19
<212> DNA
<213> Artificial Sequence

<220>
<223> Synthetic primer

<400> 177
gagggactgt tggcatgca                                                   19

<210> 178
<211> 20
<212> DNA
<213> Artificial Sequence

<220>
<223> Synthetic primer

<400> 178
ctggcttaag gatggacagg                                                  20

<210> 179
<211> 18
<212> DNA
<213> Artificial Sequence

<220>
<223> Synthetic primer

<400> 179
ccagtggagc gcttccat                                                    18

<210> 180
<211> 18
<212> DNA
<213> Artificial Sequence

<220>
<223> Synthetic primer

<400> 180
gacatctgcg ctccatcc                                                    18

<210> 181
<211> 21
<212> DNA
<213> Artificial Sequence

<220>
<223> Synthetic primer

<400> 181
ggaagtgaca gacgtgaagg t                                                21

<210> 182
<211> 21
<212> DNA

<213> Artificial Sequence

<220>
<223> Synthetic primer

<400> 182
cgagcccttt gatgacttcc t                                                21

<210> 183
<211> 18
<212> DNA
<213> Artificial Sequence

<220>
<223> Synthetic primer

<400> 183
gagaacaagc agggctgg                                                    18

<210> 184
<211> 20
<212> DNA
<213> Artificial Sequence

<220>
<223> Synthetic primer

<400> 184
tgaagtccag gacgatgatg                                                  20

<210> 185
<211> 19
<212> DNA
<213> Artificial Sequence

<220>
<223> Synthetic primer

<400> 185
cgacagagct tgtgcacct                                                   19

<210> 186
<211> 18
<212> DNA
<213> Artificial Sequence

<220>
<223> Synthetic primer

<400> 186
ctgtttgctg tccggagg                                                    18

<210> 187
<211> 22
<212> DNA
<213> Artificial Sequence

<220>
<223> Synthetic primer

<400> 187
ccagctgcta ctttgacatc ga                                               22

<210> 188
<211> 26
<212> DNA
<213> Artificial Sequence

<220>
<223> Synthetic primer

<400> 188
ggataattca gacaacaaca ccatct                                          26

<210> 189
<211> 20
<212> DNA
<213> Artificial Sequence

<220>
<223> Synthetic primer

<400> 189
gaagcgcaga tcatgaagaa                                                  20

<210> 190
<211> 19
<212> DNA
<213> Artificial Sequence

<220>
<223> Synthetic primer

<400> 190
tcagcagcaa gggcatcat                                                   19

<210> 191
<211> 18
<212> DNA
<213> Artificial Sequence

<220>
<223> Synthetic primer

<400> 191
tgtttggagg gaagggct                                                    18

<210> 192
<211> 18
<212> DNA
<213> Artificial Sequence

<220>
<223> Synthetic primer

<400> 192
gtgctggtga cgaatcca                                                    18

<210> 193
<211> 20
<212> DNA
<213> Artificial Sequence

<220>
<223> Synthetic primer

<400> 193
accctcgaca agaccacact                                                    20

<210> 194
<211> 20
<212> DNA
<213> Artificial Sequence

<220>
<223> Synthetic primer

<400> 194
attccaccca tggcaaattc                                                    20

<210> 195
<211> 23
<212> DNA
<213> Artificial Sequence

<220>
<223> Synthetic primer

<400> 195
caaaggagct cactgtggtg tct                                                23

<210> 196
<211> 20
<212> DNA
<213> Artificial Sequence

<220>
<223> Synthetic primer

<400> 196
ttgggaaata tttgggcatt                                                    20

<210> 197
<211> 19
<212> DNA
<213> Artificial Sequence

<220>
<223> Synthetic primer

<400> 197
gcatgggaac catcaacca                                                     19

<210> 198
<211> 30
<212> DNA
<213> Artificial Sequence

<220>
<223> Synthetic primer

<400> 198
tgtagaatca aactcttcat catcaactag                                         30

<210> 199
<211> 21
<212> DNA
<213> Artificial Sequence

<220>
<223> Synthetic primer

<400> 199
cgctccagac ctatgatgac t                                                21

<210> 200
<211> 18
<212> DNA
<213> Artificial Sequence

<220>
<223> Synthetic primer

<400> 200
gatcccaagg cccaactc                                                    18

<210> 201
<211> 19
<212> DNA
<213> Artificial Sequence

<220>
<223> Synthetic primer

<400> 201
gttcactggg ggtgtatgg                                                   19

<210> 202
<211> 20
<212> DNA
<213> Artificial Sequence

<220>
<223> Synthetic primer

<400> 202
tgtcatgtac gacggcttct                                                  20

<210> 203
<211> 20
<212> DNA
<213> Artificial Sequence

<220>
<223> Synthetic primer

<400> 203
gttcgctacg aggattgagc                                                  20

<210> 204
<211> 20
<212> DNA
<213> Artificial Sequence

<220>
<223> Synthetic primer

<400> 204
ttctggacct gggaccttag                                                  20

<210> 205
<211> 20

<212> DNA
<213> Artificial Sequence

<220>
<223> Synthetic primer

<400> 205
cgtgcctcta caccatcttc 20

<210> 206
<211> 20
<212> DNA
<213> Artificial Sequence

<220>
<223> Synthetic primer

<400> 206
agtacaagca ggctgccaag 20

<210> 207
<211> 18
<212> DNA
<213> Artificial Sequence

<220>
<223> Synthetic primer

<400> 207
gcttatgacc gaccccaa 18

<210> 208
<211> 24
<212> DNA
<213> Artificial Sequence

<220>
<223> Synthetic primer

<400> 208
cacacagatc tcctactcca tcca 24

<210> 209
<211> 19
<212> DNA
<213> Artificial Sequence

<220>
<223> Synthetic primer

<400> 209
ctgagtgtgg tttgcggat 19

<210> 210
<211> 20
<212> DNA
<213> Artificial Sequence

<220>
<223> Synthetic primer

<400> 210
ccatctgcat ccatcttgtt 20

<210> 211
<211> 20
<212> DNA
<213> Artificial Sequence

<220>
<223> Synthetic primer

<400> 211
cagatgacaa tggccacaat                                                    20

<210> 212
<211> 21
<212> DNA
<213> Artificial Sequence

<220>
<223> Synthetic primer

<400> 212
tgcttaggtg cggtaaaacc a                                                  21

<210> 213
<211> 18
<212> DNA
<213> Artificial Sequence

<220>
<223> Synthetic primer

<400> 213
ccccaggcac cagcttta                                                      18

<210> 214
<211> 19
<212> DNA
<213> Artificial Sequence

<220>
<223> Synthetic primer

<400> 214
tgcccccaag acactgtgt                                                     19

<210> 215
<211> 27
<212> DNA
<213> Artificial Sequence

<220>
<223> Synthetic primer

<400> 215
aagctatgag gaaaagaagt acacgat                                            27

<210> 216
<211> 19
<212> DNA
<213> Artificial Sequence

<220>
<223> Synthetic primer

<400> 216
ctgggctgtg aggctgaga                                                    19

<210> 217
<211> 20
<212> DNA
<213> Artificial Sequence

<220>
<223> Synthetic primer

<400> 217
ctgcaggcac tccctgaaat                                                   20

<210> 218
<211> 21
<212> DNA
<213> Artificial Sequence

<220>
<223> Synthetic primer

<400> 218
caatgccatc ttgcgctaca t                                                 21

<210> 219
<211> 19
<212> DNA
<213> Artificial Sequence

<220>
<223> Synthetic primer

<400> 219
caccatcccc accctgtct                                                    19

<210> 220
<211> 20
<212> DNA
<213> Artificial Sequence

<220>
<223> Synthetic primer

<400> 220
cccactcagt agccaagtca                                                   20

<210> 221
<211> 22
<212> DNA
<213> Artificial Sequence

<220>
<223> Synthetic primer

<400> 221
ccaaacgtgt aacaattatg cc                                                22

<210> 222
<211> 27
<212> DNA
<213> Artificial Sequence

<220>
<223> Synthetic primer

<400> 222
caagtaccac agcgatgact acattaa                                          27

<210> 223
<211> 18
<212> DNA
<213> Artificial Sequence

<220>
<223> Synthetic primer

<400> 223
tcctgtgctc tggaagcc                                                    18

<210> 224
<211> 20
<212> DNA
<213> Artificial Sequence

<220>
<223> Synthetic primer

<400> 224
cggtgtgaga agtgcagcaa                                                  20

<210> 225
<211> 18
<212> DNA
<213> Artificial Sequence

<220>
<223> Synthetic primer

<400> 225
gaaagatagc tcgcggca                                                    18

<210> 226
<211> 20
<212> DNA
<213> Artificial Sequence

<220>
<223> Synthetic primer

<400> 226
caggacacaa gtgccagatt                                                  20

<210> 227
<211> 21
<212> DNA
<213> Artificial Sequence

<220>
<223> Synthetic primer

<400> 227
tccatgatgg ttctgcaggt t                                                21

<210> 228

<211> 19
<212> DNA
<213> Artificial Sequence

<220>
<223> Synthetic primer

<400> 228
tggcctgtcc attggtgat                                                    19

<210> 229
<211> 24
<212> DNA
<213> Artificial Sequence

<220>
<223> Synthetic primer

<400> 229
tccaggatgt taggaactgt gaag                                              24

<210> 230
<211> 19
<212> DNA
<213> Artificial Sequence

<220>
<223> Synthetic primer

<400> 230
agcaggagcg accaactga                                                    19

<210> 231
<211> 18
<212> DNA
<213> Artificial Sequence

<220>
<223> Synthetic primer

<400> 231
gcagcagtcg gcttctct                                                     18

<210> 232
<211> 23
<212> DNA
<213> Artificial Sequence

<220>
<223> Synthetic primer

<400> 232
agtgacagat ggacaatgca aga                                               23

<210> 233
<211> 22
<212> DNA
<213> Artificial Sequence

<220>
<223> Synthetic primer

<400> 233

tcccttgtgt tccttctgtg aa                                                22

<210> 234
<211> 21
<212> DNA
<213> Artificial Sequence

<220>
<223> Synthetic primer

<400> 234
cgtgccttat ggttactttg g                                                 21

<210> 235
<211> 20
<212> DNA
<213> Artificial Sequence

<220>
<223> Synthetic primer

<400> 235
cagcctcaag ttcggttttc                                                   20

<210> 236
<211> 18
<212> DNA
<213> Artificial Sequence

<220>
<223> Synthetic primer

<400> 236
ctggaccgca cggacatc                                                     18

<210> 237
<211> 20
<212> DNA
<213> Artificial Sequence

<220>
<223> Synthetic primer

<400> 237
gctttccaag tggggaatta                                                   20

<210> 238
<211> 20
<212> DNA
<213> Artificial Sequence

<220>
<223> Synthetic primer

<400> 238
cagtctcgcc atgttgaagt                                                   20

<210> 239
<211> 19
<212> DNA
<213> Artificial Sequence

<220>

<223> Synthetic primer

<400> 239
ctgctgcgac agtccacta                                                    19

<210> 240
<211> 19
<212> DNA
<213> Artificial Sequence

<220>
<223> Synthetic primer

<400> 240
ggtccgcttc gtctttcga                                                    19

<210> 241
<211> 20
<212> DNA
<213> Artificial Sequence

<220>
<223> Synthetic primer

<400> 241
ttcagtgtgt ccagtgcatc                                                   20

<210> 242
<211> 24
<212> DNA
<213> Artificial Sequence

<220>
<223> Synthetic primer

<400> 242
ggctagtaga actggatccc aaca                                              24

<210> 243
<211> 19
<212> DNA
<213> Artificial Sequence

<220>
<223> Synthetic primer

<400> 243
cctccctctg gtggtgctt                                                    19

<210> 244
<211> 19
<212> DNA
<213> Artificial Sequence

<220>
<223> Synthetic primer

<400> 244
ccgactggag gagcataaa                                                    19

<210> 245
<211> 27
<212> DNA

<213> Artificial Sequence

<220>
<223> Synthetic primer

<400> 245
gaataccaca ctttctgcta caacact                                          27

<210> 246
<211> 24
<212> DNA
<213> Artificial Sequence

<220>
<223> Synthetic primer

<400> 246
gcagacagtg accatctaca gctt                                             24

<210> 247
<211> 19
<212> DNA
<213> Artificial Sequence

<220>
<223> Synthetic primer

<400> 247
agaaccgcaa ggtgagcaa                                                   19

<210> 248
<211> 19
<212> DNA
<213> Artificial Sequence

<220>
<223> Synthetic primer

<400> 248
tggcctggct cttaatttg                                                   19

<210> 249
<211> 20
<212> DNA
<213> Artificial Sequence

<220>
<223> Synthetic primer

<400> 249
ggtggagagt ggagccatga                                                  20

<210> 250
<211> 21
<212> DNA
<213> Artificial Sequence

<220>
<223> Synthetic primer

<400> 250
gcatggtagc cgaagatttc a                                                21

<210> 251
<211> 19
<212> DNA
<213> Artificial Sequence

<220>
<223> Synthetic primer

<400> 251
ccgtgcttcc ggacaactt                                                    19

<210> 252
<211> 20
<212> DNA
<213> Artificial Sequence

<220>
<223> Synthetic primer

<400> 252
tgaaccgcag agaccaacag                                                   20

<210> 253
<211> 23
<212> DNA
<213> Artificial Sequence

<220>
<223> Synthetic primer

<400> 253
gggtcactat ggagttcaaa gga                                               23

<210> 254
<211> 20
<212> DNA
<213> Artificial Sequence

<220>
<223> Synthetic primer

<400> 254
gcctcccata gctccttacc                                                   20

<210> 255
<211> 26
<212> DNA
<213> Artificial Sequence

<220>
<223> Synthetic primer

<400> 255
aaggaaccat ctcactgtgt gtaaac                                            26

<210> 256
<211> 20
<212> DNA
<213> Artificial Sequence

<220>
<223> Synthetic primer

<400> 256
ggcgctgtca tcgatttctt                                                    20

<210> 257
<211> 20
<212> DNA
<213> Artificial Sequence

<220>
<223> Synthetic primer

<400> 257
tggaaggttc cacaagtcac                                                    20

<210> 258
<211> 20
<212> DNA
<213> Artificial Sequence

<220>
<223> Synthetic primer

<400> 258
accctctggt ggtaaatgga                                                    20

<210> 259
<211> 20
<212> DNA
<213> Artificial Sequence

<220>
<223> Synthetic primer

<400> 259
ggcctaatgt tccagatcct                                                    20

<210> 260
<211> 20
<212> DNA
<213> Artificial Sequence

<220>
<223> Synthetic primer

<400> 260
actttcctgc gaggtcagtc                                                    20

<210> 261
<211> 19
<212> DNA
<213> Artificial Sequence

<220>
<223> Synthetic primer

<400> 261
gtgcccgagc catatagca                                                     19

<210> 262
<211> 20
<212> DNA
<213> Artificial Sequence

<220>
<223> Synthetic primer

<400> 262
agtccagccg agatgctaag                                                    20

<210> 263
<211> 20
<212> DNA
<213> Artificial Sequence

<220>
<223> Synthetic primer

<400> 263
ccacagctca ccttctgtca                                                    20

<210> 264
<211> 20
<212> DNA
<213> Artificial Sequence

<220>
<223> Synthetic primer

<400> 264
ccatgatcct cactctgctg                                                    20

<210> 265
<211> 21
<212> DNA
<213> Artificial Sequence

<220>
<223> Synthetic primer

<400> 265
caacgcttca gtgatcaatc c                                                  21

<210> 266
<211> 20
<212> DNA
<213> Artificial Sequence

<220>
<223> Synthetic primer

<400> 266
aggccagccc tacattatca                                                    20

<210> 267
<211> 20
<212> DNA
<213> Artificial Sequence

<220>
<223> Synthetic primer

<400> 267
cagtgacaaa cagcccttcc                                                    20

<210> 268
<211> 21

<212> DNA
<213> Artificial Sequence

<220>
<223> Synthetic primer

<400> 268
actcggactg cacaagctat t                                                21

<210> 269
<211> 20
<212> DNA
<213> Artificial Sequence

<220>
<223> Synthetic primer

<400> 269
tcagaattgg atttggctca                                                  20

<210> 270
<211> 19
<212> DNA
<213> Artificial Sequence

<220>
<223> Synthetic primer

<400> 270
accggggagc cctacatga                                                   19

<210> 271
<211> 18
<212> DNA
<213> Artificial Sequence

<220>
<223> Synthetic primer

<400> 271
caaggtgccc tcagtgga                                                    18

<210> 272
<211> 20
<212> DNA
<213> Artificial Sequence

<220>
<223> Synthetic primer

<400> 272
tcgtgaaaga tgaccaggag                                                  20

<210> 273
<211> 19
<212> DNA
<213> Artificial Sequence

<220>
<223> Synthetic primer

<400> 273
tggcttacac tggcaatgg                                                   19

<210> 274
<211> 18
<212> DNA
<213> Artificial Sequence

<220>
<223> Synthetic primer

<400> 274
ctgtcagctg ctgcttgg                                                    18

<210> 275
<211> 19
<212> DNA
<213> Artificial Sequence

<220>
<223> Synthetic primer

<400> 275
cggactttgg gtgcgactt                                                   19

<210> 276
<211> 18
<212> DNA
<213> Artificial Sequence

<220>
<223> Synthetic primer

<400> 276
aagcccgagg cactcatt                                                    18

<210> 277
<211> 18
<212> DNA
<213> Artificial Sequence

<220>
<223> Synthetic primer

<400> 277
gctgggaggc aggacttc                                                    18

<210> 278
<211> 18
<212> DNA
<213> Artificial Sequence

<220>
<223> Synthetic primer

<400> 278
gagctccatg gctcatcc                                                    18

<210> 279
<211> 19
<212> DNA
<213> Artificial Sequence

<220>
<223> Synthetic primer

<400> 279
tctcttgcag gaagccaga                                                    19

<210> 280
<211> 25
<212> DNA
<213> Artificial Sequence

<220>
<223> Synthetic primer

<400> 280
aattcctgct ccaaaagaaa gtctt                                             25

<210> 281
<211> 18
<212> DNA
<213> Artificial Sequence

<220>
<223> Synthetic primer

<400> 281
caccccggct tcaacaac                                                     18

<210> 282
<211> 20
<212> DNA
<213> Artificial Sequence

<220>
<223> Synthetic primer

<400> 282
gacgtgaggg tcctgattct                                                   20

<210> 283
<211> 20
<212> DNA
<213> Artificial Sequence

<220>
<223> Synthetic primer

<400> 283
catcctcatg gattggtgtg                                                   20

<210> 284
<211> 20
<212> DNA
<213> Artificial Sequence

<220>
<223> Synthetic primer

<400> 284
ccacctcgcc atgattttc                                                    20

<210> 285
<211> 20
<212> DNA
<213> Artificial Sequence

<220>
<223> Synthetic primer

<400> 285
atgtgccagt gagcttgagt                                                    20

<210> 286
<211> 20
<212> DNA
<213> Artificial Sequence

<220>
<223> Synthetic primer

<400> 286
tgatggtcca aatgaacgaa                                                    20

<210> 287
<211> 18
<212> DNA
<213> Artificial Sequence

<220>
<223> Synthetic primer

<400> 287
cttgctggcc aatgccta                                                      18

<210> 288
<211> 20
<212> DNA
<213> Artificial Sequence

<220>
<223> Synthetic primer

<400> 288
cagatgaggc acatggagac                                                    20

<210> 289
<211> 18
<212> DNA
<213> Artificial Sequence

<220>
<223> Synthetic primer

<400> 289
ctcctggcca acagcact                                                      18

<210> 290
<211> 20
<212> DNA
<213> Artificial Sequence

<220>
<223> Synthetic primer

<400> 290
caaggagact gggaggtgtc                                                    20

<210> 291

<211> 20
<212> DNA
<213> Artificial Sequence

<220>
<223> Synthetic primer

<400> 291
actgaccaag cctgagacct                                                    20

<210> 292
<211> 21
<212> DNA
<213> Artificial Sequence

<220>
<223> Synthetic primer

<400> 292
actcaagcgg aaattgaagc a                                                  21

<210> 293
<211> 18
<212> DNA
<213> Artificial Sequence

<220>
<223> Synthetic primer

<400> 293
agcgatgaag atggtcgc                                                      18

<210> 294
<211> 20
<212> DNA
<213> Artificial Sequence

<220>
<223> Synthetic primer

<400> 294
agcggaaaat ggcagacaat                                                    20

<210> 295
<211> 20
<212> DNA
<213> Artificial Sequence

<220>
<223> Synthetic primer

<400> 295
gcttcaggtg ttgtgactgc                                                    20

<210> 296
<211> 20
<212> DNA
<213> Artificial Sequence

<220>
<223> Synthetic primer

<400> 296

tgaacagtaa tggggagctg                                                    20

<210> 297
<211> 19
<212> DNA
<213> Artificial Sequence

<220>
<223> Synthetic primer

<400> 297
tgcaaacgct ggtgtcaca                                                     19

<210> 298
<211> 18
<212> DNA
<213> Artificial Sequence

<220>
<223> Synthetic primer

<400> 298
ccaatgggag aacaacgg                                                      18

<210> 299
<211> 19
<212> DNA
<213> Artificial Sequence

<220>
<223> Synthetic primer

<400> 299
ctgcaacacc gaagtggac                                                     19

<210> 300
<211> 20
<212> DNA
<213> Artificial Sequence

<220>
<223> Synthetic primer

<400> 300
agaccaagct ggaagcagag                                                    20

<210> 301
<211> 18
<212> DNA
<213> Artificial Sequence

<220>
<223> Synthetic primer

<400> 301
acatccaggg ctctgtgg                                                      18

<210> 302
<211> 18
<212> DNA
<213> Artificial Sequence

<220>

<223> Synthetic primer

<400> 302
gacctggcct tgctgaag                                                    18

<210> 303
<211> 20
<212> DNA
<213> Artificial Sequence

<220>
<223> Synthetic primer

<400> 303
agaagctgtc cctgcaagag                                                  20

<210> 304
<211> 21
<212> DNA
<213> Artificial Sequence

<220>
<223> Synthetic primer

<400> 304
gacttttgcc cgctaccttt c                                                21

<210> 305
<211> 20
<212> DNA
<213> Artificial Sequence

<220>
<223> Synthetic primer

<400> 305
aggatcgcct gtcagaagag                                                  20

<210> 306
<211> 20
<212> DNA
<213> Artificial Sequence

<220>
<223> Synthetic primer

<400> 306
gtgaaatgaa acgcaccaca                                                  20

<210> 307
<211> 21
<212> DNA
<213> Artificial Sequence

<220>
<223> Synthetic primer

<400> 307
acggatctac cacaccattg c                                                21

<210> 308
<211> 21
<212> DNA

<213> Artificial Sequence

<220>
<223> Synthetic primer

<400> 308
gggagatcat cgggacaact c                                                21

<210> 309
<211> 19
<212> DNA
<213> Artificial Sequence

<220>
<223> Synthetic primer

<400> 309
ccaacgcttg ccaaatcct                                                   19

<210> 310
<211> 20
<212> DNA
<213> Artificial Sequence

<220>
<223> Synthetic primer

<400> 310
ccatgatgga gaggcagaca                                                  20

<210> 311
<211> 26
<212> DNA
<213> Artificial Sequence

<220>
<223> Synthetic primer

<400> 311
ggatggtagc agtctaggga ttaact                                           26

<210> 312
<211> 23
<212> DNA
<213> Artificial Sequence

<220>
<223> Synthetic primer

<400> 312
tcacctctca tcttcaccag gat                                              23

<210> 313
<211> 20
<212> DNA
<213> Artificial Sequence

<220>
<223> Synthetic primer

<400> 313
ccatacgtgc tgctacctgt                                                  20

<210> 314
<211> 23
<212> DNA
<213> Artificial Sequence

<220>
<223> Synthetic primer

<400> 314
cgagagtctg taggagggaa acc                                               23

<210> 315
<211> 18
<212> DNA
<213> Artificial Sequence

<220>
<223> Synthetic primer

<400> 315
tcatggtgcc cgtcaatg                                                     18

<210> 316
<211> 22
<212> DNA
<213> Artificial Sequence

<220>
<223> Synthetic primer

<400> 316
tcatcctggc gatctacttc ct                                                22

<210> 317
<211> 20
<212> DNA
<213> Artificial Sequence

<220>
<223> Synthetic primer

<400> 317
tgagaaacaa actgcaccca                                                   20

<210> 318
<211> 20
<212> DNA
<213> Artificial Sequence

<220>
<223> Synthetic primer

<400> 318
gatgcagaat tgaggcagac                                                   20

<210> 319
<211> 20
<212> DNA
<213> Artificial Sequence

<220>
<223> Synthetic primer

<400> 319
gctcgtggtt ctgtagtcca                                                    20

<210> 320
<211> 23
<212> DNA
<213> Artificial Sequence

<220>
<223> Synthetic primer

<400> 320
gaaggaatgg gaatcagtca tga                                                23

<210> 321
<211> 18
<212> DNA
<213> Artificial Sequence

<220>
<223> Synthetic primer

<400> 321
gccgagatcg ccaagatg                                                      18

<210> 322
<211> 20
<212> DNA
<213> Artificial Sequence

<220>
<223> Synthetic primer

<400> 322
tggttttgag accacgatgt                                                    20

<210> 323
<211> 25
<212> DNA
<213> Artificial Sequence

<220>
<223> Synthetic primer

<400> 323
taactgacat tcttgagcac cagat                                              25

<210> 324
<211> 27
<212> DNA
<213> Artificial Sequence

<220>
<223> Synthetic primer

<400> 324
cgagactctc ctcatagtga aaggtat                                            27

<210> 325
<211> 23
<212> DNA
<213> Artificial Sequence

<220>
<223> Synthetic primer

<400> 325
caaccgaagt tttcactcca gtt                                               23

<210> 326
<211> 19
<212> DNA
<213> Artificial Sequence

<220>
<223> Synthetic primer

<400> 326
gcggaaggtc cctcagaca                                                    19

<210> 327
<211> 19
<212> DNA
<213> Artificial Sequence

<220>
<223> Synthetic primer

<400> 327
ccgcaacgtg gttttctca                                                    19

<210> 328
<211> 18
<212> DNA
<213> Artificial Sequence

<220>
<223> Synthetic primer

<400> 328
gtggttttcc ctcggagc                                                     18

<210> 329
<211> 20
<212> DNA
<213> Artificial Sequence

<220>
<223> Synthetic primer

<400> 329
gcatcaggct gtcattatgg                                                   20

<210> 330
<211> 19
<212> DNA
<213> Artificial Sequence

<220>
<223> Synthetic primer

<400> 330
aggactggga cccatgaac                                                    19

<210> 331
<211> 25

<212> DNA
<213> Artificial Sequence

<220>
<223> Synthetic primer

<400> 331
tggctaagtg aagatgacaa tcatg 25

<210> 332
<211> 20
<212> DNA
<213> Artificial Sequence

<220>
<223> Synthetic primer

<400> 332
aatatttgtg cggggtatgg 20

<210> 333
<211> 21
<212> DNA
<213> Artificial Sequence

<220>
<223> Synthetic primer

<400> 333
aagcatgaac aggacttgac c 21

<210> 334
<211> 22
<212> DNA
<213> Artificial Sequence

<220>
<223> Synthetic primer

<400> 334
gcaaggaaag ggtcttagtc ac 22

<210> 335
<211> 18
<212> DNA
<213> Artificial Sequence

<220>
<223> Synthetic primer

<400> 335
gaaacctctg cgccatga 18

<210> 336
<211> 24
<212> DNA
<213> Artificial Sequence

<220>
<223> Synthetic primer

<400> 336
ccattctatc atcaacgggt acaa 24

<210> 337
<211> 20
<212> DNA
<213> Artificial Sequence

<220>
<223> Synthetic primer

<400> 337
gttctggttg ctggatttgg                                                   20

<210> 338
<211> 19
<212> DNA
<213> Artificial Sequence

<220>
<223> Synthetic primer

<400> 338
tcaccacggt ctttagcca                                                    19

<210> 339
<211> 20
<212> DNA
<213> Artificial Sequence

<220>
<223> Synthetic primer

<400> 339
gggctactgg cagctacatt                                                   20

<210> 340
<211> 20
<212> DNA
<213> Artificial Sequence

<220>
<223> Synthetic primer

<400> 340
cagcgggatt aaacagtcct                                                   20

<210> 341
<211> 20
<212> DNA
<213> Artificial Sequence

<220>
<223> Synthetic primer

<400> 341
aacagagaca ttgccaacca                                                   20

<210> 342
<211> 20
<212> DNA
<213> Artificial Sequence

<220>
<223> Synthetic primer

<400> 342
acaccaaaat gccatctcaa                                                    20

<210> 343
<211> 21
<212> DNA
<213> Artificial Sequence

<220>
<223> Synthetic primer

<400> 343
tggctgtgct ggtcactacc t                                                  21

<210> 344
<211> 18
<212> DNA
<213> Artificial Sequence

<220>
<223> Synthetic primer

<400> 344
gactgctgtc atggcgtg                                                      18

<210> 345
<211> 21
<212> DNA
<213> Artificial Sequence

<220>
<223> Synthetic primer

<400> 345
cctgctgacg atgatgaagg a                                                  21

<210> 346
<211> 22
<212> DNA
<213> Artificial Sequence

<220>
<223> Synthetic primer

<400> 346
actccctgat aaaggggaat tt                                                 22

<210> 347
<211> 19
<212> DNA
<213> Artificial Sequence

<220>
<223> Synthetic primer

<400> 347
caccctgcct ctacccaac                                                     19

<210> 348
<211> 20
<212> DNA
<213> Artificial Sequence

<220>
<223> Synthetic primer

<400> 348
catggccgtg tagaccctaa                                                    20

<210> 349
<211> 19
<212> DNA
<213> Artificial Sequence

<220>
<223> Synthetic primer

<400> 349
accctgagca ctggaggaa                                                     19

<210> 350
<211> 21
<212> DNA
<213> Artificial Sequence

<220>
<223> Synthetic primer

<400> 350
agacaaggat gccgtggata a                                                  21

<210> 351
<211> 22
<212> DNA
<213> Artificial Sequence

<220>
<223> Synthetic primer

<400> 351
gcagaactga agatgggaag at                                                 22

<210> 352
<211> 20
<212> DNA
<213> Artificial Sequence

<220>
<223> Synthetic primer

<400> 352
cgcgagcccc tcattataca                                                    20

<210> 353
<211> 19
<212> DNA
<213> Artificial Sequence

<220>
<223> Synthetic primer

<400> 353
caagctgaac ggtgtgtcc                                                     19

<210> 354

<211> 20
<212> DNA
<213> Artificial Sequence

<220>
<223> Synthetic primer

<400> 354
agctggggtg tctgtttcat                                                   20

<210> 355
<211> 21
<212> DNA
<213> Artificial Sequence

<220>
<223> Synthetic primer

<400> 355
agaggctgaa tatgcaggac a                                                 21

<210> 356
<211> 23
<212> DNA
<213> Artificial Sequence

<220>
<223> Synthetic primer

<400> 356
agttgcagaa tctaagcctg gaa                                               23

<210> 357
<211> 19
<212> DNA
<213> Artificial Sequence

<220>
<223> Synthetic primer

<400> 357
atggccaatg tttgatgct                                                    19

<210> 358
<211> 20
<212> DNA
<213> Artificial Sequence

<220>
<223> Synthetic primer

<400> 358
cccaatcgga agcctaacta                                                   20

<210> 359
<211> 20
<212> DNA
<213> Artificial Sequence

<220>
<223> Synthetic primer

<400> 359

catcttccag gaggaccact                                                    20

<210> 360
<211> 20
<212> DNA
<213> Artificial Sequence

<220>
<223> Synthetic primer

<400> 360
aatacccaac gcacaaatga                                                    20

<210> 361
<211> 20
<212> DNA
<213> Artificial Sequence

<220>
<223> Synthetic primer

<400> 361
cctggaggct gcaacatacc                                                    20

<210> 362
<211> 20
<212> DNA
<213> Artificial Sequence

<220>
<223> Synthetic primer

<400> 362
tgttttgatt cccgggctta                                                    20

<210> 363
<211> 21
<212> DNA
<213> Artificial Sequence

<220>
<223> Synthetic primer

<400> 363
tctccagcaa aagcgatgtc t                                                  21

<210> 364
<211> 20
<212> DNA
<213> Artificial Sequence

<220>
<223> Synthetic primer

<400> 364
gatggagcag gtggctcagt                                                    20

<210> 365
<211> 18
<212> DNA
<213> Artificial Sequence

<220>

<223> Synthetic primer

<400> 365
cagccctgag gcaagaga                                                    18

<210> 366
<211> 20
<212> DNA
<213> Artificial Sequence

<220>
<223> Synthetic primer

<400> 366
gccaactgct ttcatttgtg                                                  20

<210> 367
<211> 20
<212> DNA
<213> Artificial Sequence

<220>
<223> Synthetic primer

<400> 367
accagtcccc cagaagacta                                                  20

<210> 368
<211> 22
<212> DNA
<213> Artificial Sequence

<220>
<223> Synthetic primer

<400> 368
ggatcgagct cttccagatc ct                                               22

<210> 369
<211> 20
<212> DNA
<213> Artificial Sequence

<220>
<223> Synthetic primer

<400> 369
aacaccaatg ggttccatct                                                  20

<210> 370
<211> 20
<212> DNA
<213> Artificial Sequence

<220>
<223> Synthetic primer

<400> 370
ctacctgcct tgctttgtga                                                  20

<210> 371
<211> 19
<212> DNA

<213> Artificial Sequence

<220>
<223> Synthetic primer

<400> 371
ccagcccaca gaccagtta                                                    19

<210> 372
<211> 18
<212> DNA
<213> Artificial Sequence

<220>
<223> Synthetic primer

<400> 372
tggcgaccaa gacacctt                                                     18

<210> 373
<211> 21
<212> DNA
<213> Artificial Sequence

<220>
<223> Synthetic primer

<400> 373
catatcgttg gatcacagca c                                                 21

<210> 374
<211> 18
<212> DNA
<213> Artificial Sequence

<220>
<223> Synthetic primer

<400> 374
gcgctgcgga agatcatc                                                     18

<210> 375
<211> 18
<212> DNA
<213> Artificial Sequence

<220>
<223> Synthetic primer

<400> 375
gagtcgaccc tgcacctg                                                     18

<210> 376
<211> 21
<212> DNA
<213> Artificial Sequence

<220>
<223> Synthetic primer

<400> 376
tggcttcagg agctgaatac c                                                 21

<210> 377
<211> 21
<212> DNA
<213> Artificial Sequence

<220>
<223> Synthetic primer

<400> 377
tgcccttaaa ggaaccaatg a                                                21

<210> 378
<211> 19
<212> DNA
<213> Artificial Sequence

<220>
<223> Synthetic primer

<400> 378
agtcaatctt cgcacacgg                                                   19

<210> 379
<211> 18
<212> DNA
<213> Artificial Sequence

<220>
<223> Synthetic primer

<400> 379
ctctccagtg tgggcacc                                                    18

<210> 380
<211> 22
<212> DNA
<213> Artificial Sequence

<220>
<223> Synthetic primer

<400> 380
agaggcatcc atgaacttca ca                                               22

<210> 381
<211> 25
<212> DNA
<213> Artificial Sequence

<220>
<223> Synthetic primer

<400> 381
gtatcaggac cacatgcagt acatc                                            25

<210> 382
<211> 20
<212> DNA
<213> Artificial Sequence

<220>
<223> Synthetic primer

<400> 382
tgtcttcagg gtcttgtcca                                                    20

<210> 383
<211> 20
<212> DNA
<213> Artificial Sequence

<220>
<223> Synthetic primer

<400> 383
atcgcagctg gtgggtgtac                                                    20

<210> 384
<211> 20
<212> DNA
<213> Artificial Sequence

<220>
<223> Synthetic primer

<400> 384
gtggacatcg gatacccaag                                                    20

<210> 385
<211> 22
<212> DNA
<213> Artificial Sequence

<220>
<223> Synthetic reverse primer

<400> 385
aggtccctgt tggccttata gg                                                 22

<210> 386
<211> 20
<212> DNA
<213> Artificial Sequence

<220>
<223> Synthetic reverse primer

<400> 386
ctccatccac tccaggtctc                                                    20

<210> 387
<211> 20
<212> DNA
<213> Artificial Sequence

<220>
<223> Synthetic reverse primer

<400> 387
caagcctgga acctatagcc                                                    20

<210> 388
<211> 18
<212> DNA
<213> Artificial Sequence

<220>
<223> Synthetic reverse primer

<400> 388
atagcgctga ccactgcc                                                    18

<210> 389
<211> 20
<212> DNA
<213> Artificial Sequence

<220>
<223> Synthetic reverse primer

<400> 389
ggccagcacc ataatcctat                                                  20

<210> 390
<211> 20
<212> DNA
<213> Artificial Sequence

<220>
<223> Synthetic reverse primer

<400> 390
actagggtgc tccgagtgac                                                  20

<210> 391
<211> 20
<212> DNA
<213> Artificial Sequence

<220>
<223> Synthetic reverse primer

<400> 391
atccgctaga actgcaccac                                                  20

<210> 392
<211> 20
<212> DNA
<213> Artificial Sequence

<220>
<223> Synthetic reverse primer

<400> 392
tgggcttaga tgcttgactc                                                  20

<210> 393
<211> 20
<212> DNA
<213> Artificial Sequence

<220>
<223> Synthetic reverse primer

<400> 393
tgcaaatgct ttgatggaat                                                  20

<210> 394
<211> 19

<212> DNA
<213> Artificial Sequence

<220>
<223> Synthetic reverse primer

<400> 394
ctggtcacgg tctccatgt                                                    19

<210> 395
<211> 21
<212> DNA
<213> Artificial Sequence

<220>
<223> Synthetic reverse primer

<400> 395
cgggcactca ctgctattac c                                                 21

<210> 396
<211> 21
<212> DNA
<213> Artificial Sequence

<220>
<223> Synthetic reverse primer

<400> 396
acccagaatc caacagtgca a                                                 21

<210> 397
<211> 20
<212> DNA
<213> Artificial Sequence

<220>
<223> Synthetic reverse primer

<400> 397
cacagatggc cagtgtttct                                                   20

<210> 398
<211> 18
<212> DNA
<213> Artificial Sequence

<220>
<223> Synthetic reverse primer

<400> 398
tgggcgccta aatcctaa                                                     18

<210> 399
<211> 19
<212> DNA
<213> Artificial Sequence

<220>
<223> Synthetic reverse primer

<400> 399
gggcacaaat cccgttcag                                                    19

<210> 400
<211> 20
<212> DNA
<213> Artificial Sequence

<220>
<223> Synthetic reverse primer

<400> 400
tctgatctcc atctgcctca                                                    20

<210> 401
<211> 22
<212> DNA
<213> Artificial Sequence

<220>
<223> Synthetic reverse primer

<400> 401
tgtggacctg atccctgtac ac                                                 22

<210> 402
<211> 20
<212> DNA
<213> Artificial Sequence

<220>
<223> Synthetic reverse primer

<400> 402
ccagagggtt gaaggcatag                                                    20

<210> 403
<211> 24
<212> DNA
<213> Artificial Sequence

<220>
<223> Synthetic reverse primer

<400> 403
ccagcattag attctccaac ttga                                               24

<210> 404
<211> 18
<212> DNA
<213> Artificial Sequence

<220>
<223> Synthetic reverse primer

<400> 404
gtggcggaga tcaagagg                                                      18

<210> 405
<211> 20
<212> DNA
<213> Artificial Sequence

<220>
<223> Synthetic reverse primer

<400> 405
aaccggaaga agtcgatgag                                                    20

<210> 406
<211> 20
<212> DNA
<213> Artificial Sequence

<220>
<223> Synthetic reverse primer

<400> 406
ttgcagtggg aagaacagtc                                                    20

<210> 407
<211> 19
<212> DNA
<213> Artificial Sequence

<220>
<223> Synthetic reverse primer

<400> 407
cgtgtcgggc ttcagtcat                                                     19

<210> 408
<211> 20
<212> DNA
<213> Artificial Sequence

<220>
<223> Synthetic reverse primer

<400> 408
tagccataag gtccgctctc                                                    20

<210> 409
<211> 20
<212> DNA
<213> Artificial Sequence

<220>
<223> Synthetic reverse primer

<400> 409
aggtctccac acagcacaag                                                    20

<210> 410
<211> 22
<212> DNA
<213> Artificial Sequence

<220>
<223> Synthetic reverse primer

<400> 410
ggaatacacg agggcatagt tc                                                 22

<210> 411
<211> 20
<212> DNA
<213> Artificial Sequence

<220>
<223> Synthetic reverse primer

<400> 411
acaagcacat ggctatggaa                                                    20

<210> 412
<211> 20
<212> DNA
<213> Artificial Sequence

<220>
<223> Synthetic reverse primer

<400> 412
acttgtgcag cagcgtactt                                                    20

<210> 413
<211> 19
<212> DNA
<213> Artificial Sequence

<220>
<223> Synthetic reverse primer

<400> 413
ggagtgacgc atggacaga                                                     19

<210> 414
<211> 20
<212> DNA
<213> Artificial Sequence

<220>
<223> Synthetic reverse primer

<400> 414
tggtgccatt ttcctatgag                                                    20

<210> 415
<211> 20
<212> DNA
<213> Artificial Sequence

<220>
<223> Synthetic reverse primer

<400> 415
gcatctgcca actcctccat                                                    20

<210> 416
<211> 18
<212> DNA
<213> Artificial Sequence

<220>
<223> Synthetic reverse primer

<400> 416
gcatttgcgg tggacgat                                                      18

<210> 417

<211> 23
<212> DNA
<213> Artificial Sequence

<220>
<223> Synthetic reverse primer

<400> 417
tcagatgacg aagagcacag atg                                              23

<210> 418
<211> 21
<212> DNA
<213> Artificial Sequence

<220>
<223> Synthetic reverse primer

<400> 418
ctgctcactc ggctcaaact c                                                21

<210> 419
<211> 24
<212> DNA
<213> Artificial Sequence

<220>
<223> Synthetic reverse primer

<400> 419
gttcaacctc ttcctgtgga ctgt                                             24

<210> 420
<211> 20
<212> DNA
<213> Artificial Sequence

<220>
<223> Synthetic reverse primer

<400> 420
gggcgaagag gatataaggg                                                  20

<210> 421
<211> 23
<212> DNA
<213> Artificial Sequence

<220>
<223> Synthetic reverse primer

<400> 421
cgaaggcact actcaatggt ttc                                              23

<210> 422
<211> 21
<212> DNA
<213> Artificial Sequence

<220>
<223> Synthetic reverse primer

<400> 422

ttgccgtcag aaaacatgtc a                                                21

<210> 423
<211> 19
<212> DNA
<213> Artificial Sequence

<220>
<223> Synthetic reverse primer

<400> 423
ctaattgggc tccatctcg                                                   19

<210> 424
<211> 20
<212> DNA
<213> Artificial Sequence

<220>
<223> Synthetic reverse primer

<400> 424
tcctgggagg tgaacttagg                                                  20

<210> 425
<211> 21
<212> DNA
<213> Artificial Sequence

<220>
<223> Synthetic reverse primer

<400> 425
tgagcgaggt tcttccactg a                                                21

<210> 426
<211> 19
<212> DNA
<213> Artificial Sequence

<220>
<223> Synthetic reverse primer

<400> 426
ctcgggtttg gcctctttc                                                   19

<210> 427
<211> 24
<212> DNA
<213> Artificial Sequence

<220>
<223> Synthetic reverse primer

<400> 427
cctatgattt aagggcattt ttcc                                             24

<210> 428
<211> 18
<212> DNA
<213> Artificial Sequence

<220>

<223> Synthetic reverse primer

<400> 428
ctcagctgac gggaaagg                                                    18

<210> 429
<211> 20
<212> DNA
<213> Artificial Sequence

<220>
<223> Synthetic reverse primer

<400> 429
cttgttgttc accaggacga                                                  20

<210> 430
<211> 21
<212> DNA
<213> Artificial Sequence

<220>
<223> Synthetic reverse primer

<400> 430
ggcaggagtg aatggctctt c                                                21

<210> 431
<211> 20
<212> DNA
<213> Artificial Sequence

<220>
<223> Synthetic reverse primer

<400> 431
ggtatcttgt ggtgtctgcg                                                  20

<210> 432
<211> 20
<212> DNA
<213> Artificial Sequence

<220>
<223> Synthetic reverse primer

<400> 432
gtggccaaga ggtcagagtc                                                  20

<210> 433
<211> 20
<212> DNA
<213> Artificial Sequence

<220>
<223> Synthetic reverse primer

<400> 433
tgaagcagtc agttgtgctg                                                  20

<210> 434
<211> 21
<212> DNA

<213> Artificial Sequence

<220>
<223> Synthetic reverse primer

<400> 434
aagacatggc gctctcagtt c                                                21

<210> 435
<211> 22
<212> DNA
<213> Artificial Sequence

<220>
<223> Synthetic reverse primer

<400> 435
caacagagtt tgccgagaca ct                                               22

<210> 436
<211> 22
<212> DNA
<213> Artificial Sequence

<220>
<223> Synthetic reverse primer

<400> 436
gctgattccc aagagtctaa cc                                               22

<210> 437
<211> 18
<212> DNA
<213> Artificial Sequence

<220>
<223> Synthetic reverse primer

<400> 437
ggcactcggc ttgagcat                                                    18

<210> 438
<211> 20
<212> DNA
<213> Artificial Sequence

<220>
<223> Synthetic reverse primer

<400> 438
tgagaccgtt ggattggatt                                                  20

<210> 439
<211> 19
<212> DNA
<213> Artificial Sequence

<220>
<223> Synthetic reverse primer

<400> 439
aggaccaaag ggagaccaa                                                   19

<210> 440
<211> 24
<212> DNA
<213> Artificial Sequence

<220>
<223> Synthetic reverse primer

<400> 440
acggtcctag gtttgaggtt aaga                                              24

<210> 441
<211> 20
<212> DNA
<213> Artificial Sequence

<220>
<223> Synthetic reverse primer

<400> 441
gcgacagagg gcttcatctt                                                   20

<210> 442
<211> 18
<212> DNA
<213> Artificial Sequence

<220>
<223> Synthetic reverse primer

<400> 442
tgcccacggc tttcttac                                                     18

<210> 443
<211> 20
<212> DNA
<213> Artificial Sequence

<220>
<223> Synthetic reverse primer

<400> 443
cacgatgtct tcctccttga                                                   20

<210> 444
<211> 23
<212> DNA
<213> Artificial Sequence

<220>
<223> Synthetic reverse primer

<400> 444
tccggtttaa gaccagttta cca                                               23

<210> 445
<211> 20
<212> DNA
<213> Artificial Sequence

<220>
<223> Synthetic reverse primer

<400> 445
caatggcctc cattttacag                                                    20

<210> 446
<211> 20
<212> DNA
<213> Artificial Sequence

<220>
<223> Synthetic reverse primer

<400> 446
aaaggggtgg gtagaaagga                                                    20

<210> 447
<211> 20
<212> DNA
<213> Artificial Sequence

<220>
<223> Synthetic reverse primer

<400> 447
cctctgcacg gtcataggtt                                                    20

<210> 448
<211> 20
<212> DNA
<213> Artificial Sequence

<220>
<223> Synthetic reverse primer

<400> 448
ctgcatgatt ctgagcaggt                                                    20

<210> 449
<211> 20
<212> DNA
<213> Artificial Sequence

<220>
<223> Synthetic reverse primer

<400> 449
atgctgactt ccttcctggt                                                    20

<210> 450
<211> 20
<212> DNA
<213> Artificial Sequence

<220>
<223> Synthetic reverse primer

<400> 450
catcttcttg ggcacacaat                                                    20

<210> 451
<211> 18
<212> DNA
<213> Artificial Sequence

<220>
<223> Synthetic reverse primer

<400> 451
cactgcagcc ccaatgct                                                    18

<210> 452
<211> 25
<212> DNA
<213> Artificial Sequence

<220>
<223> Synthetic reverse primer

<400> 452
ttcaatgata atgcaaggac tgatc                                            25

<210> 453
<211> 20
<212> DNA
<213> Artificial Sequence

<220>
<223> Synthetic reverse primer

<400> 453
ctggtttgtc tggagaaggc                                                  20

<210> 454
<211> 20
<212> DNA
<213> Artificial Sequence

<220>
<223> Synthetic reverse primer

<400> 454
cctgacattt cccttgtcct                                                  20

<210> 455
<211> 20
<212> DNA
<213> Artificial Sequence

<220>
<223> Synthetic reverse primer

<400> 455
tcatgggcgt atctacgaat                                                  20

<210> 456
<211> 25
<212> DNA
<213> Artificial Sequence

<220>
<223> Synthetic reverse primer

<400> 456
gagagagtga gaccacgaag agact                                            25

<210> 457
<211> 18

<212> DNA
<213> Artificial Sequence

<220>
<223> Synthetic reverse primer

<400> 457
tccctgcatt caagaggc                                                    18

<210> 458
<211> 20
<212> DNA
<213> Artificial Sequence

<220>
<223> Synthetic reverse primer

<400> 458
acctgtgttt ggatttgcag                                                  20

<210> 459
<211> 20
<212> DNA
<213> Artificial Sequence

<220>
<223> Synthetic reverse primer

<400> 459
actggggtgg aatgtgtctt                                                  20

<210> 460
<211> 21
<212> DNA
<213> Artificial Sequence

<220>
<223> Synthetic reverse primer

<400> 460
ttgggttgaa gaaatcagtc c                                                21

<210> 461
<211> 19
<212> DNA
<213> Artificial Sequence

<220>
<223> Synthetic reverse primer

<400> 461
ctcctccacc ctgggttgt                                                   19

<210> 462
<211> 20
<212> DNA
<213> Artificial Sequence

<220>
<223> Synthetic reverse primer

<400> 462
gacctcaggg cgattcatga                                                  20

<210> 463
<211> 22
<212> DNA
<213> Artificial Sequence

<220>
<223> Synthetic reverse primer

<400> 463
gcttgcactc cacaggtaca ca                                                22

<210> 464
<211> 21
<212> DNA
<213> Artificial Sequence

<220>
<223> Synthetic reverse primer

<400> 464
ctgcattgtg gcacagttct g                                                 21

<210> 465
<211> 20
<212> DNA
<213> Artificial Sequence

<220>
<223> Synthetic reverse primer

<400> 465
cttcagtctt ggcctgttca                                                   20

<210> 466
<211> 22
<212> DNA
<213> Artificial Sequence

<220>
<223> Synthetic reverse primer

<400> 466
ggatcccaca cctttaccat aa                                                22

<210> 467
<211> 18
<212> DNA
<213> Artificial Sequence

<220>
<223> Synthetic reverse primer

<400> 467
gccgctcatt gatctcca                                                     18

<210> 468
<211> 18
<212> DNA
<213> Artificial Sequence

<220>
<223> Synthetic reverse primer

<400> 468
ccgtcattgg ccttcttc                                                    18

<210> 469
<211> 18
<212> DNA
<213> Artificial Sequence

<220>
<223> Synthetic reverse primer

<400> 469
ctactcatgg gcgggatg                                                    18

<210> 470
<211> 19
<212> DNA
<213> Artificial Sequence

<220>
<223> Synthetic reverse primer

<400> 470
ccgccttcag gttctcaat                                                   19

<210> 471
<211> 18
<212> DNA
<213> Artificial Sequence

<220>
<223> Synthetic reverse primer

<400> 471
ttgggatgct caaaagcc                                                    18

<210> 472
<211> 19
<212> DNA
<213> Artificial Sequence

<220>
<223> Synthetic reverse primer

<400> 472
ctgtggcatt gagtttggg                                                   19

<210> 473
<211> 20
<212> DNA
<213> Artificial Sequence

<220>
<223> Synthetic reverse primer

<400> 473
atgtcaggag tccctccatc                                                  20

<210> 474
<211> 22
<212> DNA
<213> Artificial Sequence

<220>
<223> Synthetic reverse primer

<400> 474
tggcaaatcc gaattagagt ga                                                22

<210> 475
<211> 18
<212> DNA
<213> Artificial Sequence

<220>
<223> Synthetic reverse primer

<400> 475
gtctcagacc cttccccc                                                     18

<210> 476
<211> 23
<212> DNA
<213> Artificial Sequence

<220>
<223> Synthetic reverse primer

<400> 476
tgtgactaca gccgtgatcc tta                                               23

<210> 477
<211> 20
<212> DNA
<213> Artificial Sequence

<220>
<223> Synthetic reverse primer

<400> 477
gcctcttgta gggccaatag                                                   20

<210> 478
<211> 20
<212> DNA
<213> Artificial Sequence

<220>
<223> Synthetic reverse primer

<400> 478
caaaaccgct gtgtttcttc                                                   20

<210> 479
<211> 21
<212> DNA
<213> Artificial Sequence

<220>
<223> Synthetic reverse primer

<400> 479
gcccatgcac tgaagtattg g                                                 21

<210> 480

<211> 20
<212> DNA
<213> Artificial Sequence

<220>
<223> Synthetic reverse primer

<400> 480
agtcgtcgag tgctagggac                                                   20

<210> 481
<211> 18
<212> DNA
<213> Artificial Sequence

<220>
<223> Synthetic reverse primer

<400> 481
tccgaggcca cagcaaac                                                     18

<210> 482
<211> 20
<212> DNA
<213> Artificial Sequence

<220>
<223> Synthetic reverse primer

<400> 482
gacgcagtct ttctgtctgg                                                   20

<210> 483
<211> 18
<212> DNA
<213> Artificial Sequence

<220>
<223> Synthetic reverse primer

<400> 483
tgcagagcag cactggag                                                     18

<210> 484
<211> 20
<212> DNA
<213> Artificial Sequence

<220>
<223> Synthetic reverse primer

<400> 484
cgctgcagaa aatgaaacga                                                   20

<210> 485
<211> 19
<212> DNA
<213> Artificial Sequence

<220>
<223> Synthetic reverse primer

<400> 485

cagagcgggc agcagaata                                                    19

<210> 486
<211> 20
<212> DNA
<213> Artificial Sequence

<220>
<223> Synthetic reverse primer

<400> 486
tcgatctcct catcatctgg                                                   20

<210> 487
<211> 18
<212> DNA
<213> Artificial Sequence

<220>
<223> Synthetic reverse primer

<400> 487
tgcgggactt gggaaaga                                                     18

<210> 488
<211> 20
<212> DNA
<213> Artificial Sequence

<220>
<223> Synthetic reverse primer

<400> 488
tgttggtacc cctgttgttg                                                   20

<210> 489
<211> 23
<212> DNA
<213> Artificial Sequence

<220>
<223> Synthetic reverse primer

<400> 489
cagtggtagg tgatgttctg gga                                               23

<210> 490
<211> 19
<212> DNA
<213> Artificial Sequence

<220>
<223> Synthetic reverse primer

<400> 490
aaactggctg ccagcattg                                                    19

<210> 491
<211> 21
<212> DNA
<213> Artificial Sequence

<220>

<223> Synthetic reverse primer

<400> 491
ctccttggtg tcacccatga g                                                21

<210> 492
<211> 22
<212> DNA
<213> Artificial Sequence

<220>
<223> Synthetic reverse primer

<400> 492
ggctgctaga gaccatggac at                                               22

<210> 493
<211> 20
<212> DNA
<213> Artificial Sequence

<220>
<223> Synthetic reverse primer

<400> 493
ctccccatta caagtgctga                                                  20

<210> 494
<211> 21
<212> DNA
<213> Artificial Sequence

<220>
<223> Synthetic reverse primer

<400> 494
gaactccctg gagatgaaac c                                                21

<210> 495
<211> 20
<212> DNA
<213> Artificial Sequence

<220>
<223> Synthetic reverse primer

<400> 495
cacatgcatg gaccttgatt                                                  20

<210> 496
<211> 18
<212> DNA
<213> Artificial Sequence

<220>
<223> Synthetic reverse primer

<400> 496
atccctcgga ctgcctct                                                    18

<210> 497
<211> 25
<212> DNA

<213> Artificial Sequence

<220>
<223> Synthetic reverse primer

<400> 497
caactgttcc tggtctacaa actca                                          25

<210> 498
<211> 20
<212> DNA
<213> Artificial Sequence

<220>
<223> Synthetic reverse primer

<400> 498
cctgcagaga tgggtatgaa                                                20

<210> 499
<211> 20
<212> DNA
<213> Artificial Sequence

<220>
<223> Synthetic reverse primer

<400> 499
ctagaggctg gtgccactgt                                                20

<210> 500
<211> 19
<212> DNA
<213> Artificial Sequence

<220>
<223> Synthetic reverse primer

<400> 500
catcacgtct ccgaactcc                                                 19

<210> 501
<211> 20
<212> DNA
<213> Artificial Sequence

<220>
<223> Synthetic reverse primer

<400> 501
agttgtaatg gcaggcacag                                                20

<210> 502
<211> 21
<212> DNA
<213> Artificial Sequence

<220>
<223> Synthetic reverse primer

<400> 502
tcagctccat tgaatgtgaa a                                              21

<210> 503
<211> 21
<212> DNA
<213> Artificial Sequence

<220>
<223> Synthetic reverse primer

<400> 503
gggacagctt gtagcctttg c                                                21

<210> 504
<211> 19
<212> DNA
<213> Artificial Sequence

<220>
<223> Synthetic reverse primer

<400> 504
accattgcag ccctgattg                                                   19

<210> 505
<211> 20
<212> DNA
<213> Artificial Sequence

<220>
<223> Synthetic reverse primer

<400> 505
ctgttctcca agccaagaca                                                  20

<210> 506
<211> 20
<212> DNA
<213> Artificial Sequence

<220>
<223> Synthetic reverse primer

<400> 506
tagggaagtg atgggagagg                                                  20

<210> 507
<211> 20
<212> DNA
<213> Artificial Sequence

<220>
<223> Synthetic reverse primer

<400> 507
tttgagatgc ttgacgttgg                                                  20

<210> 508
<211> 19
<212> DNA
<213> Artificial Sequence

<220>
<223> Synthetic reverse primer

<400> 508
caatgcggca tatactggg                                                    19

<210> 509
<211> 22
<212> DNA
<213> Artificial Sequence

<220>
<223> Synthetic reverse primer

<400> 509
aggataaggc caaccatgat gt                                                22

<210> 510
<211> 19
<212> DNA
<213> Artificial Sequence

<220>
<223> Synthetic reverse primer

<400> 510
gccagcattg ccattatct                                                    19

<210> 511
<211> 21
<212> DNA
<213> Artificial Sequence

<220>
<223> Synthetic reverse primer

<400> 511
caccatggcg atgtactttc c                                                 21

<210> 512
<211> 20
<212> DNA
<213> Artificial Sequence

<220>
<223> Synthetic reverse primer

<400> 512
gggaatgtgg tagcccaaga                                                   20

<210> 513
<211> 20
<212> DNA
<213> Artificial Sequence

<220>
<223> Synthetic reverse primer

<400> 513
gtggctgcat tagtgtccat                                                   20

<210> 514
<211> 20
<212> DNA
<213> Artificial Sequence

<220>
<223> Synthetic reverse primer

<400> 514
accaaagatg ctgtgttcca                                                    20

<210> 515
<211> 20
<212> DNA
<213> Artificial Sequence

<220>
<223> Synthetic reverse primer

<400> 515
tgaatgccat ctttcttcca                                                    20

<210> 516
<211> 18
<212> DNA
<213> Artificial Sequence

<220>
<223> Synthetic reverse primer

<400> 516
gagcgtcggg tgcaaatc                                                      18

<210> 517
<211> 22
<212> DNA
<213> Artificial Sequence

<220>
<223> Synthetic reverse primer

<400> 517
caccacccca agtatccgta ag                                                 22

<210> 518
<211> 20
<212> DNA
<213> Artificial Sequence

<220>
<223> Synthetic reverse primer

<400> 518
cgatgttccc ttcgatggag                                                    20

<210> 519
<211> 20
<212> DNA
<213> Artificial Sequence

<220>
<223> Synthetic reverse primer

<400> 519
ggcagtgaag gcgataaagt                                                    20

<210> 520
<211> 20

<212> DNA
<213> Artificial Sequence

<220>
<223> Synthetic reverse primer

<400> 520
cacctcttgc tgtccctttg                                                   20

<210> 521
<211> 18
<212> DNA
<213> Artificial Sequence

<220>
<223> Synthetic reverse primer

<400> 521
agaaggaagg tccagccg                                                     18

<210> 522
<211> 19
<212> DNA
<213> Artificial Sequence

<220>
<223> Synthetic reverse primer

<400> 522
ccatgaggcc caacttcct                                                    19

<210> 523
<211> 19
<212> DNA
<213> Artificial Sequence

<220>
<223> Synthetic reverse primer

<400> 523
cctgccgcat tgttttcag                                                    19

<210> 524
<211> 20
<212> DNA
<213> Artificial Sequence

<220>
<223> Synthetic reverse primer

<400> 524
aatgcgtatc tgtccacgac                                                   20

<210> 525
<211> 20
<212> DNA
<213> Artificial Sequence

<220>
<223> Synthetic reverse primer

<400> 525
gacaaacacc cttcctccag                                                   20

<210> 526
<211> 18
<212> DNA
<213> Artificial Sequence

<220>
<223> Synthetic reverse primer

<400> 526
ctcgtcccgg ttcatcag                                                    18

<210> 527
<211> 20
<212> DNA
<213> Artificial Sequence

<220>
<223> Synthetic reverse primer

<400> 527
caggcctcag ttccttcagt                                                  20

<210> 528
<211> 20
<212> DNA
<213> Artificial Sequence

<220>
<223> Synthetic reverse primer

<400> 528
ccaacagtac agccagttgc                                                  20

<210> 529
<211> 21
<212> DNA
<213> Artificial Sequence

<220>
<223> Synthetic reverse primer

<400> 529
tggacctagg gcttccaagt c                                                21

<210> 530
<211> 20
<212> DNA
<213> Artificial Sequence

<220>
<223> Synthetic reverse primer

<400> 530
caggccgtaa ggagctgtct                                                  20

<210> 531
<211> 20
<212> DNA
<213> Artificial Sequence

<220>
<223> Synthetic reverse primer

<400> 531
ttacacatcc aaccagtgcc                                                    20

<210> 532
<211> 19
<212> DNA
<213> Artificial Sequence

<220>
<223> Synthetic reverse primer

<400> 532
accacagcat gggtgagag                                                     19

<210> 533
<211> 20
<212> DNA
<213> Artificial Sequence

<220>
<223> Synthetic reverse primer

<400> 533
ccgttgtaac gttgactgga                                                    20

<210> 534
<211> 18
<212> DNA
<213> Artificial Sequence

<220>
<223> Synthetic reverse primer

<400> 534
ggcgctgact tccttgac                                                      18

<210> 535
<211> 20
<212> DNA
<213> Artificial Sequence

<220>
<223> Synthetic reverse primer

<400> 535
tgttgagatt cctcgcagtt                                                    20

<210> 536
<211> 24
<212> DNA
<213> Artificial Sequence

<220>
<223> Synthetic reverse primer

<400> 536
ctccagctta gggtagttgt ccat                                               24

<210> 537
<211> 20
<212> DNA
<213> Artificial Sequence

<220>
<223> Synthetic reverse primer

<400> 537
tgcctgagaa gaggtgaggt                                                    20

<210> 538
<211> 20
<212> DNA
<213> Artificial Sequence

<220>
<223> Synthetic reverse primer

<400> 538
ttggtagtgc tccacacgat                                                    20

<210> 539
<211> 20
<212> DNA
<213> Artificial Sequence

<220>
<223> Synthetic reverse primer

<400> 539
gatgaggatg tcccggatga                                                    20

<210> 540
<211> 20
<212> DNA
<213> Artificial Sequence

<220>
<223> Synthetic reverse primer

<400> 540
gaacagctgg aggccaagtc                                                    20

<210> 541
<211> 18
<212> DNA
<213> Artificial Sequence

<220>
<223> Synthetic reverse primer

<400> 541
cggtcacgga gccaatct                                                      18

<210> 542
<211> 20
<212> DNA
<213> Artificial Sequence

<220>
<223> Synthetic reverse primer

<400> 542
tgttcaaagg ttgaccatgc                                                    20

<210> 543

<211> 21
<212> DNA
<213> Artificial Sequence

<220>
<223> Synthetic reverse primer

<400> 543
tgcactgctt ggccttaaag a                                                21

<210> 544
<211> 18
<212> DNA
<213> Artificial Sequence

<220>
<223> Synthetic reverse primer

<400> 544
gtggcgtgcc tcgaagtc                                                    18

<210> 545
<211> 19
<212> DNA
<213> Artificial Sequence

<220>
<223> Synthetic reverse primer

<400> 545
gtaatgctgt ccacggtgc                                                   19

<210> 546
<211> 20
<212> DNA
<213> Artificial Sequence

<220>
<223> Synthetic reverse primer

<400> 546
aggtacctct cggtcagtgg                                                  20

<210> 547
<211> 23
<212> DNA
<213> Artificial Sequence

<220>
<223> Synthetic reverse primer

<400> 547
tgttctagcg atcttgcttc aca                                              23

<210> 548
<211> 25
<212> DNA
<213> Artificial Sequence

<220>
<223> Synthetic reverse primer

<400> 548

caaggcatat cgatcctcat aaagt                                          25

<210> 549
<211> 20
<212> DNA
<213> Artificial Sequence

<220>
<223> Synthetic reverse primer

<400> 549
cggccaggat acacatctta                                                20

<210> 550
<211> 19
<212> DNA
<213> Artificial Sequence

<220>
<223> Synthetic reverse primer

<400> 550
cctccgccag gtctttagt                                                 19

<210> 551
<211> 19
<212> DNA
<213> Artificial Sequence

<220>
<223> Synthetic reverse primer

<400> 551
tctccgagga accctttgg                                                 19

<210> 552
<211> 21
<212> DNA
<213> Artificial Sequence

<220>
<223> Synthetic reverse primer

<400> 552
ctgtccaatt gctgattgct t                                              21

<210> 553
<211> 23
<212> DNA
<213> Artificial Sequence

<220>
<223> Synthetic reverse primer

<400> 553
tgtagggcag acttcctcaa aca                                            23

<210> 554
<211> 20
<212> DNA
<213> Artificial Sequence

<220>

<223> Synthetic reverse primer

<400> 554
agtctcttgg gcatcgagtt                                                    20

<210> 555
<211> 19
<212> DNA
<213> Artificial Sequence

<220>
<223> Synthetic reverse primer

<400> 555
ggctagtggg cgcatgtag                                                     19

<210> 556
<211> 19
<212> DNA
<213> Artificial Sequence

<220>
<223> Synthetic reverse primer

<400> 556
tgaccaggaa ctgccacag                                                     19

<210> 557
<211> 20
<212> DNA
<213> Artificial Sequence

<220>
<223> Synthetic reverse primer

<400> 557
caccgaacac tccctagtcc                                                    20

<210> 558
<211> 20
<212> DNA
<213> Artificial Sequence

<220>
<223> Synthetic reverse primer

<400> 558
aaccggtgct ctccacattc                                                    20

<210> 559
<211> 18
<212> DNA
<213> Artificial Sequence

<220>
<223> Synthetic reverse primer

<400> 559
ggaagtgggt catgtggg                                                      18

<210> 560
<211> 18
<212> DNA

<213> Artificial Sequence

<220>
<223> Synthetic reverse primer

<400> 560
gctttgcccg gtagctct                                                    18

<210> 561
<211> 25
<212> DNA
<213> Artificial Sequence

<220>
<223> Synthetic reverse primer

<400> 561
gagtgagaat tcgatccaag tcttc                                            25

<210> 562
<211> 20
<212> DNA
<213> Artificial Sequence

<220>
<223> Synthetic reverse primer

<400> 562
acgagactcc agtgctgatg                                                  20

<210> 563
<211> 22
<212> DNA
<213> Artificial Sequence

<220>
<223> Synthetic reverse primer

<400> 563
ctctctgggt cgtctgaaac aa                                               22

<210> 564
<211> 18
<212> DNA
<213> Artificial Sequence

<220>
<223> Synthetic reverse primer

<400> 564
caaactggtc ccggtcct                                                    18

<210> 565
<211> 18
<212> DNA
<213> Artificial Sequence

<220>
<223> Synthetic reverse primer

<400> 565
acacggtagc cggtcact                                                    18

<210> 566
<211> 18
<212> DNA
<213> Artificial Sequence

<220>
<223> Synthetic reverse primer

<400> 566
ggagcgggct gtctcaga                                                    18

<210> 567
<211> 20
<212> DNA
<213> Artificial Sequence

<220>
<223> Synthetic reverse primer

<400> 567
cttgacgaag cactcgttga                                                  20

<210> 568
<211> 20
<212> DNA
<213> Artificial Sequence

<220>
<223> Synthetic reverse primer

<400> 568
acggcttgct tactgaaggt                                                  20

<210> 569
<211> 19
<212> DNA
<213> Artificial Sequence

<220>
<223> Synthetic reverse primer

<400> 569
ggtcgtccat tggaatcct                                                   19

<210> 570
<211> 20
<212> DNA
<213> Artificial Sequence

<220>
<223> Synthetic reverse primer

<400> 570
gtggaggaac tctgggaatg                                                  20

<210> 571
<211> 21
<212> DNA
<213> Artificial Sequence

<220>
<223> Synthetic reverse primer

<400> 571
ggtcacaaac ttgccattgg a                                                21

<210> 572
<211> 25
<212> DNA
<213> Artificial Sequence

<220>
<223> Synthetic reverse primer

<400> 572
tgaagtaatc agccacagac tcaat                                            25

<210> 573
<211> 20
<212> DNA
<213> Artificial Sequence

<220>
<223> Synthetic reverse primer

<400> 573
ctcctcagac accactgcat                                                  20

<210> 574
<211> 23
<212> DNA
<213> Artificial Sequence

<220>
<223> Synthetic reverse primer

<400> 574
ggtggtttc ttgagcgtgt act                                               23

<210> 575
<211> 22
<212> DNA
<213> Artificial Sequence

<220>
<223> Synthetic reverse primer

<400> 575
gaagatagct gagggctgtg ac                                               22

<210> 576
<211> 20
<212> DNA
<213> Artificial Sequence

<220>
<223> Synthetic reverse primer

<400> 576
cccggcaaaa acaaataagt                                                  20

<210> 577
<211> 20
<212> DNA
<213> Artificial Sequence

<220>
<223> Synthetic reverse primer

<400> 577
tgggagttca tgggtacaga                                                    20

<210> 578
<211> 22
<212> DNA
<213> Artificial Sequence

<220>
<223> Synthetic reverse primer

<400> 578
gatgggattt ccattgatga ca                                                 22

<210> 579
<211> 26
<212> DNA
<213> Artificial Sequence

<220>
<223> Synthetic reverse primer

<400> 579
gagtcagaat ggcttattca cagatg                                             26

<210> 580
<211> 20
<212> DNA
<213> Artificial Sequence

<220>
<223> Synthetic reverse primer

<400> 580
agaagctagg gtggttgtcc                                                    20

<210> 581
<211> 21
<212> DNA
<213> Artificial Sequence

<220>
<223> Synthetic reverse primer

<400> 581
tgaggagttt gccttgattc g                                                  21

<210> 582
<211> 22
<212> DNA
<213> Artificial Sequence

<220>
<223> Synthetic reverse primer

<400> 582
cacagaatcc agctgtgcaa ct                                                 22

<210> 583
<211> 20

<212> DNA
<213> Artificial Sequence

<220>
<223> Synthetic reverse primer

<400> 583
acagtggaag gaccaggact                                                   20

<210> 584
<211> 20
<212> DNA
<213> Artificial Sequence

<220>
<223> Synthetic reverse primer

<400> 584
agccattgca gctaggtgag                                                   20

<210> 585
<211> 23
<212> DNA
<213> Artificial Sequence

<220>
<223> Synthetic reverse primer

<400> 585
aaataccaac atgcacctct ctt                                               23

<210> 586
<211> 20
<212> DNA
<213> Artificial Sequence

<220>
<223> Synthetic reverse primer

<400> 586
agtccacagt gttgggacaa                                                   20

<210> 587
<211> 18
<212> DNA
<213> Artificial Sequence

<220>
<223> Synthetic reverse primer

<400> 587
tgcgtaccca cttcctgc                                                     18

<210> 588
<211> 20
<212> DNA
<213> Artificial Sequence

<220>
<223> Synthetic reverse primer

<400> 588
aaagagctgt gagtggctgg                                                   20

<210> 589
<211> 20
<212> DNA
<213> Artificial Sequence

<220>
<223> Synthetic reverse primer

<400> 589
atgttcacca ccaggatcag                                                   20

<210> 590
<211> 19
<212> DNA
<213> Artificial Sequence

<220>
<223> Synthetic reverse primer

<400> 590
gcagctcagg gaagtcaca                                                    19

<210> 591
<211> 20
<212> DNA
<213> Artificial Sequence

<220>
<223> Synthetic reverse primer

<400> 591
aaagttccag gcaacatcgt                                                   20

<210> 592
<211> 21
<212> DNA
<213> Artificial Sequence

<220>
<223> Synthetic reverse primer

<400> 592
ggtccagcag tgtctcctga a                                                 21

<210> 593
<211> 19
<212> DNA
<213> Artificial Sequence

<220>
<223> Synthetic reverse primer

<400> 593
tactccctgg ctcctgctt                                                    19

<210> 594
<211> 20
<212> DNA
<213> Artificial Sequence

<220>
<223> Synthetic reverse primer

<400> 594
ggccaccagg gtattatctg                                                    20

<210> 595
<211> 20
<212> DNA
<213> Artificial Sequence

<220>
<223> Synthetic reverse primer

<400> 595
gaagcctttc tttccacagc                                                    20

<210> 596
<211> 21
<212> DNA
<213> Artificial Sequence

<220>
<223> Synthetic reverse primer

<400> 596
caagagcctg aatgcgtcag t                                                  21

<210> 597
<211> 23
<212> DNA
<213> Artificial Sequence

<220>
<223> Synthetic reverse primer

<400> 597
acttcggctg tgtgttatat gca                                                23

<210> 598
<211> 21
<212> DNA
<213> Artificial Sequence

<220>
<223> Synthetic reverse primer

<400> 598
gaggtccgtg gtagcgttct c                                                  21

<210> 599
<211> 20
<212> DNA
<213> Artificial Sequence

<220>
<223> Synthetic reverse primer

<400> 599
ggcccagctt gaatttttca                                                    20

<210> 600
<211> 21
<212> DNA
<213> Artificial Sequence

<220>
<223> Synthetic reverse primer

<400> 600
gcgaatctgc tccttttctg a                                                21

<210> 601
<211> 20
<212> DNA
<213> Artificial Sequence

<220>
<223> Synthetic reverse primer

<400> 601
ccaagaaacc atggctgctt                                                  20

<210> 602
<211> 25
<212> DNA
<213> Artificial Sequence

<220>
<223> Synthetic reverse primer

<400> 602
gtccactcga atcttttctt cttca                                            25

<210> 603
<211> 21
<212> DNA
<213> Artificial Sequence

<220>
<223> Synthetic reverse primer

<400> 603
ggcctcagtg tgcatcattc t                                                21

<210> 604
<211> 20
<212> DNA
<213> Artificial Sequence

<220>
<223> Synthetic reverse primer

<400> 604
cacgcaggtg gtatcagtct                                                  20

<210> 605
<211> 20
<212> DNA
<213> Artificial Sequence

<220>
<223> Synthetic reverse primer

<400> 605
tcttaagcac gttctccacg                                                  20

<210> 606

<211> 22
<212> DNA
<213> Artificial Sequence

<220>
<223> Synthetic reverse primer

<400> 606
gcttgctgta ctccgacatg tt                                                22

<210> 607
<211> 21
<212> DNA
<213> Artificial Sequence

<220>
<223> Synthetic reverse primer

<400> 607
ctccacggtc tcagttgatc t                                                 21

<210> 608
<211> 19
<212> DNA
<213> Artificial Sequence

<220>
<223> Synthetic reverse primer

<400> 608
cctctcgcaa gtgctccat                                                    19

<210> 609
<211> 21
<212> DNA
<213> Artificial Sequence

<220>
<223> Synthetic reverse primer

<400> 609
ggaggtgctt cactgtcatt t                                                 21

<210> 610
<211> 18
<212> DNA
<213> Artificial Sequence

<220>
<223> Synthetic reverse primer

<400> 610
gcagggagct ggagtagc                                                     18

<210> 611
<211> 21
<212> DNA
<213> Artificial Sequence

<220>
<223> Synthetic reverse primer

<400> 611

tgagcagcac catcagtaac g                                                21

<210> 612
<211> 22
<212> DNA
<213> Artificial Sequence

<220>
<223> Synthetic reverse primer

<400> 612
gcttgtcatc tgcagcagtg tt                                               22

<210> 613
<211> 22
<212> DNA
<213> Artificial Sequence

<220>
<223> Synthetic reverse primer

<400> 613
gcgtgtctgc gtagtagctg tt                                               22

<210> 614
<211> 28
<212> DNA
<213> Artificial Sequence

<220>
<223> Synthetic reverse primer

<400> 614
gtttgccaag ttaaatttgg tacataat                                         28

<210> 615
<211> 21
<212> DNA
<213> Artificial Sequence

<220>
<223> Synthetic reverse primer

<400> 615
gggagggaga agagattcga t                                                21

<210> 616
<211> 20
<212> DNA
<213> Artificial Sequence

<220>
<223> Synthetic reverse primer

<400> 616
ccgagtcgcc actgctaagt                                                  20

<210> 617
<211> 24
<212> DNA
<213> Artificial Sequence

<220>

<223> Synthetic reverse primer

<400> 617
ggcaataaac aggctcatga ttaa                                              24

<210> 618
<211> 18
<212> DNA
<213> Artificial Sequence

<220>
<223> Synthetic reverse primer

<400> 618
cacagggttt cagcgagc                                                     18

<210> 619
<211> 18
<212> DNA
<213> Artificial Sequence

<220>
<223> Synthetic reverse primer

<400> 619
gttggaagca aacgcaca                                                     18

<210> 620
<211> 18
<212> DNA
<213> Artificial Sequence

<220>
<223> Synthetic reverse primer

<400> 620
cgcctcgcga aagacttg                                                     18

<210> 621
<211> 20
<212> DNA
<213> Artificial Sequence

<220>
<223> Synthetic reverse primer

<400> 621
tgcctgcgat atttgttagg                                                   20

<210> 622
<211> 24
<212> DNA
<213> Artificial Sequence

<220>
<223> Synthetic reverse primer

<400> 622
ataaacgctt caaatttctc tctg                                              24

<210> 623
<211> 18
<212> DNA

<213> Artificial Sequence

<220>
<223> Synthetic reverse primer

<400> 623
caggttcgct ctgggaag                                                    18

<210> 624
<211> 19
<212> DNA
<213> Artificial Sequence

<220>
<223> Synthetic reverse primer

<400> 624
gcacaggttc gctctggaa                                                   19

<210> 625
<211> 20
<212> DNA
<213> Artificial Sequence

<220>
<223> Synthetic reverse primer

<400> 625
atctgtttcc attggctcct                                                  20

<210> 626
<211> 20
<212> DNA
<213> Artificial Sequence

<220>
<223> Synthetic reverse primer

<400> 626
ggtctgccca aatgcttttc                                                  20

<210> 627
<211> 26
<212> DNA
<213> Artificial Sequence

<220>
<223> Synthetic reverse primer

<400> 627
gctacatcta cacttggttg gcttaa                                           26

<210> 628
<211> 19
<212> DNA
<213> Artificial Sequence

<220>
<223> Synthetic reverse primer

<400> 628
atgctggctg actctgctc                                                   19

<210> 629
<211> 24
<212> DNA
<213> Artificial Sequence

<220>
<223> Synthetic reverse primer

<400> 629
ggattgcagc taaccctgta tacc                                              24

<210> 630
<211> 22
<212> DNA
<213> Artificial Sequence

<220>
<223> Synthetic reverse primer

<400> 630
cttctgagac ctctggcttc gt                                                22

<210> 631
<211> 21
<212> DNA
<213> Artificial Sequence

<220>
<223> Synthetic reverse primer

<400> 631
tccaactgaa ggtccctgat g                                                 21

<210> 632
<211> 19
<212> DNA
<213> Artificial Sequence

<220>
<223> Synthetic reverse primer

<400> 632
tgcaatcatg caagaccac                                                    19

<210> 633
<211> 21
<212> DNA
<213> Artificial Sequence

<220>
<223> Synthetic reverse primer

<400> 633
gctcgttcag cttcacattg c                                                 21

<210> 634
<211> 30
<212> DNA
<213> Artificial Sequence

<220>
<223> Synthetic reverse primer

<400> 634
tttccggtaa tagtctgtct catagatatc                                        30

<210> 635
<211> 20
<212> DNA
<213> Artificial Sequence

<220>
<223> Synthetic reverse primer

<400> 635
tggactgctt ccaggtgtca                                                   20

<210> 636
<211> 20
<212> DNA
<213> Artificial Sequence

<220>
<223> Synthetic reverse primer

<400> 636
gtcttggaca cccgcagaat                                                   20

<210> 637
<211> 19
<212> DNA
<213> Artificial Sequence

<220>
<223> Synthetic reverse primer

<400> 637
gggtctgaat ggccaggtt                                                    19

<210> 638
<211> 20
<212> DNA
<213> Artificial Sequence

<220>
<223> Synthetic reverse primer

<400> 638
cagagctctt gcatgtggag                                                   20

<210> 639
<211> 20
<212> DNA
<213> Artificial Sequence

<220>
<223> Synthetic reverse primer

<400> 639
atcaggaagg ctgccaagag                                                   20

<210> 640
<211> 25
<212> DNA
<213> Artificial Sequence

<220>
<223> Synthetic reverse primer

<400> 640
tggagcttat taaaggcatt cttca                                          25

<210> 641
<211> 20
<212> DNA
<213> Artificial Sequence

<220>
<223> Synthetic reverse primer

<400> 641
tcttgacctt gcagctttgt                                                20

<210> 642
<211> 20
<212> DNA
<213> Artificial Sequence

<220>
<223> Synthetic reverse primer

<400> 642
ggccccaatg aaatagactg                                                20

<210> 643
<211> 20
<212> DNA
<213> Artificial Sequence

<220>
<223> Synthetic reverse primer

<400> 643
aaaattgtgc cttggaggag                                                20

<210> 644
<211> 19
<212> DNA
<213> Artificial Sequence

<220>
<223> Synthetic reverse primer

<400> 644
aactccgagt ggtgatcca                                                 19

<210> 645
<211> 23
<212> DNA
<213> Artificial Sequence

<220>
<223> Synthetic reverse primer

<400> 645
cggtagtggt tgatgactgt tga                                            23

<210> 646
<211> 20

<212> DNA
<213> Artificial Sequence

<220>
<223> Synthetic reverse primer

<400> 646
agaaggtatc agggctggaa                                                   20

<210> 647
<211> 20
<212> DNA
<213> Artificial Sequence

<220>
<223> Synthetic reverse primer

<400> 647
cctcagtgcc agtctcttcc                                                   20

<210> 648
<211> 20
<212> DNA
<213> Artificial Sequence

<220>
<223> Synthetic reverse primer

<400> 648
gaagctttgt agccggtgat                                                   20

<210> 649
<211> 18
<212> DNA
<213> Artificial Sequence

<220>
<223> Synthetic reverse primer

<400> 649
gtctggccgg gattcttt                                                     18

<210> 650
<211> 20
<212> DNA
<213> Artificial Sequence

<220>
<223> Synthetic reverse primer

<400> 650
gtcttctcca cagtccagca                                                   20

<210> 651
<211> 19
<212> DNA
<213> Artificial Sequence

<220>
<223> Synthetic reverse primer

<400> 651
gtttagcctc atgggcgtc                                                    19

<210> 652
<211> 20
<212> DNA
<213> Artificial Sequence

<220>
<223> Synthetic reverse primer

<400> 652
tgccatcacc attgaaatct                                                    20

<210> 653
<211> 20
<212> DNA
<213> Artificial Sequence

<220>
<223> Synthetic reverse primer

<400> 653
cctgagctta gctggtgttg                                                    20

<210> 654
<211> 27
<212> DNA
<213> Artificial Sequence

<220>
<223> Synthetic reverse primer

<400> 654
ccttaagctc tttcactgac tcaatct                                            27

<210> 655
<211> 19
<212> DNA
<213> Artificial Sequence

<220>
<223> Synthetic reverse primer

<400> 655
gcgcacacct tcatctcat                                                     19

<210> 656
<211> 20
<212> DNA
<213> Artificial Sequence

<220>
<223> Synthetic reverse primer

<400> 656
ggtgaacatc atgacgcagt                                                    20

<210> 657
<211> 19
<212> DNA
<213> Artificial Sequence

<220>
<223> Synthetic reverse primer

<400> 657
gcatagctgt gagatgcgg                                                    19

<210> 658
<211> 18
<212> DNA
<213> Artificial Sequence

<220>
<223> Synthetic reverse primer

<400> 658
   agggggtgtc cgtaaagg                                                      18

<210> 659
<211> 24
<212> DNA
<213> Artificial Sequence

<220>
<223> Synthetic reverse primer

<400> 659
ttacaactct tccactggga cgat                                              24

<210> 660
<211> 20
<212> DNA
<213> Artificial Sequence

<220>
<223> Synthetic reverse primer

<400> 660
tgtctgtgag cttggtcctg                                                   20

<210> 661
<211> 20
<212> DNA
<213> Artificial Sequence

<220>
<223> Synthetic reverse primer

<400> 661
gaagcaggtc agagtgagcc                                                   20

<210> 662
<211> 22
<212> DNA
<213> Artificial Sequence

<220>
<223> Synthetic reverse primer

<400> 662
tcacacccac tgaatcctac tg                                                22

<210> 663
<211> 19
<212> DNA
<213> Artificial Sequence

<220>
<223> Synthetic reverse primer

<400> 663
ccgtagggcc aattcagac                                                    19

<210> 664
<211> 20
<212> DNA
<213> Artificial Sequence

<220>
<223> Synthetic reverse primer

<400> 664
cgtgatgcga agctctgaga                                                   20

<210> 665
<211> 22
<212> DNA
<213> Artificial Sequence

<220>
<223> Synthetic reverse primer

<400> 665
catcttcacc agcatgatgt ca                                                22

<210> 666
<211> 20
<212> DNA
<213> Artificial Sequence

<220>
<223> Synthetic reverse primer

<400> 666
tcctcactca tcacgtcctc                                                   20

<210> 667
<211> 20
<212> DNA
<213> Artificial Sequence

<220>
<223> Synthetic reverse primer

<400> 667
gccaaaccat tcattgtcac                                                   20

<210> 668
<211> 25
<212> DNA
<213> Artificial Sequence

<220>
<223> Synthetic reverse primer

<400> 668
gcaatctctt caaacacttc atcct                                             25

<210> 669

<211> 20
<212> DNA
<213> Artificial Sequence

<220>
<223> Synthetic reverse primer

<400> 669
   tgagcccctg gttaacagta                                                20

<210> 670
<211> 19
<212> DNA
<213> Artificial Sequence

<220>
<223> Synthetic reverse primer

<400> 670
aagcttcaca agttggggc                                                  19

<210> 671
<211> 18
<212> DNA
<213> Artificial Sequence

<220>
<223> Synthetic reverse primer

<400> 671
tgattgtccg cagtcagg                                                   18

<210> 672
<211> 20
<212> DNA
<213> Artificial Sequence

<220>
<223> Synthetic reverse primer

<400> 672
ttgaaatggc agaacggtag                                                 20

<210> 673
<211> 18
<212> DNA
<213> Artificial Sequence

<220>
<223> Synthetic reverse primer

<400> 673
acacaaggcc cagcctct                                                   18

<210> 674
<211> 20
<212> DNA
<213> Artificial Sequence

<220>
<223> Synthetic reverse primer

<400> 674

cggcagaact gacagtgttc                                                    20

<210> 675
<211> 20
<212> DNA
<213> Artificial Sequence

<220>
<223> Synthetic reverse primer

<400> 675
gtcacacttg cagcatttca                                                    20

<210> 676
<211> 21
<212> DNA
<213> Artificial Sequence

<220>
<223> Synthetic reverse primer

<400> 676
actccctgaa gccgagacac t                                                  21

<210> 677
<211> 18
<212> DNA
<213> Artificial Sequence

<220>
<223> Synthetic reverse primer

<400> 677
gacatggcag cacaagca                                                      18

<210> 678
<211> 20
<212> DNA
<213> Artificial Sequence

<220>
<223> Synthetic reverse primer

<400> 678
cttgagggtt tgggtttcca                                                    20

<210> 679
<211> 20
<212> DNA
<213> Artificial Sequence

<220>
<223> Synthetic reverse primer

<400> 679
aagagctgcc catccttctc                                                    20

<210> 680
<211> 19
<212> DNA
<213> Artificial Sequence

<220>

<223> Synthetic reverse primer

<400> 680
ttctgggaac tgctggaag                                                    19

<210> 681
<211> 21
<212> DNA
<213> Artificial Sequence

<220>
<223> Synthetic reverse primer

<400> 681
ccccacgagt tctggttctt c                                                 21

<210> 682
<211> 19
<212> DNA
<213> Artificial Sequence

<220>
<223> Synthetic reverse primer

<400> 682
cgctgaggct ggtactgtg                                                    19

<210> 683
<211> 19
<212> DNA
<213> Artificial Sequence

<220>
<223> Synthetic reverse primer

<400> 683
gtctctggac acaggctgg                                                    19

<210> 684
<211> 20
<212> DNA
<213> Artificial Sequence

<220>
<223> Synthetic reverse primer

<400> 684
gaggaatgga aagacctcgg                                                   20

<210> 685
<211> 21
<212> DNA
<213> Artificial Sequence

<220>
<223> Synthetic reverse primer

<400> 685
gcagacacaa tggaaagaac c                                                 21

<210> 686
<211> 19
<212> DNA

<213> Artificial Sequence

<220>
<223> Synthetic reverse primer

<400> 686
cggacagttt cttccggtt                                                    19

<210> 687
<211> 20
<212> DNA
<213> Artificial Sequence

<220>
<223> Synthetic reverse primer

<400> 687
agccgtacca gctcagactt                                                   20

<210> 688
<211> 26
<212> DNA
<213> Artificial Sequence

<220>
<223> Synthetic reverse primer

<400> 688
gccactaact gcttcagtat gaagag                                            26

<210> 689
<211> 20
<212> DNA
<213> Artificial Sequence

<220>
<223> Synthetic reverse primer

<400> 689
tgcacataag caacagcaga                                                   20

<210> 690
<211> 20
<212> DNA
<213> Artificial Sequence

<220>
<223> Synthetic reverse primer

<400> 690
gaccctgctc acaaccagac                                                   20

<210> 691
<211> 26
<212> DNA
<213> Artificial Sequence

<220>
<223> Synthetic reverse primer

<400> 691
tccatatcca acaaaaaaac tcaaag                                            26

<210> 692
<211> 19
<212> DNA
<213> Artificial Sequence

<220>
<223> Synthetic reverse primer

<400> 692
gggcctggtt gaaaagcat                                                    19

<210> 693
<211> 24
<212> DNA
<213> Artificial Sequence

<220>
<223> Synthetic reverse primer

<400> 693
acggtagtga cagcatcaaa actc                                              24

<210> 694
<211> 21
<212> DNA
<213> Artificial Sequence

<220>
<223> Synthetic reverse primer

<400> 694
ggagtccgtc cttaccgtca a                                                 21

<210> 695
<211> 22
<212> DNA
<213> Artificial Sequence

<220>
<223> Synthetic reverse primer

<400> 695
ggaatgtccc atacccaaag aa                                                22

<210> 696
<211> 23
<212> DNA
<213> Artificial Sequence

<220>
<223> Synthetic reverse primer

<400> 696
tgtcaccgtg atctctttgg taa                                               23

<210> 697
<211> 21
<212> DNA
<213> Artificial Sequence

<220>
<223> Synthetic reverse primer

<400> 697
cctaaaggtt tgaatggcag a                                                21

<210> 698
<211> 24
<212> DNA
<213> Artificial Sequence

<220>
<223> Synthetic reverse primer

<400> 698
ggttccgata tttggtggtc ttac                                             24

<210> 699
<211> 23
<212> DNA
<213> Artificial Sequence

<220>
<223> Synthetic reverse primer

<400> 699
cgattgtctt tgctcttcat gtg                                              23

<210> 700
<211> 20
<212> DNA
<213> Artificial Sequence

<220>
<223> Synthetic reverse primer

<400> 700
ccgttgagtg gaatcagcaa                                                  20

<210> 701
<211> 20
<212> DNA
<213> Artificial Sequence

<220>
<223> Synthetic reverse primer

<400> 701
caaggcctca aatctcaagg                                                  20

<210> 702
<211> 20
<212> DNA
<213> Artificial Sequence

<220>
<223> Synthetic reverse primer

<400> 702
tcttggcaag tcggttaaga                                                  20

<210> 703
<211> 20
<212> DNA
<213> Artificial Sequence

<220>
<223> Synthetic reverse primer

<400> 703
acaaagggag agcgtgaagt                                                    20

<210> 704
<211> 26
<212> DNA
<213> Artificial Sequence

<220>
<223> Synthetic reverse primer

<400> 704
gtctattaga gtcagatccg ggacat                                             26

<210> 705
<211> 27
<212> DNA
<213> Artificial Sequence

<220>
<223> Synthetic reverse primer

<400> 705
cttttgatgg tagagttcca gtgattc                                            27

<210> 706
<211> 20
<212> DNA
<213> Artificial Sequence

<220>
<223> Synthetic reverse primer

<400> 706
tgaatcatgc cagtgctgta                                                    20

<210> 707
<211> 18
<212> DNA
<213> Artificial Sequence

<220>
<223> Synthetic reverse primer

<400> 707
atggcttgcc cacaatgc                                                      18

<210> 708
<211> 22
<212> DNA
<213> Artificial Sequence

<220>
<223> Synthetic reverse primer

<400> 708
cttggcgtgt ggaaatctac ag                                                 22

<210> 709
<211> 25

<212> DNA
<213> Artificial Sequence

<220>
<223> Synthetic reverse primer

<400> 709
cctcagtcca taaaccacac tatca                                          25

<210> 710
<211> 24
<212> DNA
<213> Artificial Sequence

<220>
<223> Synthetic reverse primer

<400> 710
tgatgatcta agtttcccga ggtt                                           24

<210> 711
<211> 21
<212> DNA
<213> Artificial Sequence

<220>
<223> Synthetic reverse primer

<400> 711
tgctgggttt ctcctcctgt t                                              21

<210> 712
<211> 19
<212> DNA
<213> Artificial Sequence

<220>
<223> Synthetic reverse primer

<400> 712
agcaagggaa cagcctcat                                                 19

<210> 713
<211> 20
<212> DNA
<213> Artificial Sequence

<220>
<223> Synthetic reverse primer

<400> 713
agtagttgtg ctgcccttcc                                                20

<210> 714
<211> 20
<212> DNA
<213> Artificial Sequence

<220>
<223> Synthetic reverse primer

<400> 714
cccataatcc tgagcaatgg                                                20

<210> 715
<211> 25
<212> DNA
<213> Artificial Sequence

<220>
<223> Synthetic reverse primer

<400> 715
tgcacatatc attacaccag ttcgt                                          25

<210> 716
<211> 20
<212> DNA
<213> Artificial Sequence

<220>
<223> Synthetic reverse primer

<400> 716
aacgagatcc ctgtgcttgt                                                20

<210> 717
<211> 18
<212> DNA
<213> Artificial Sequence

<220>
<223> Synthetic reverse primer

<400> 717
cctccccaag tcagttgc                                                  18

<210> 718
<211> 19
<212> DNA
<213> Artificial Sequence

<220>
<223> Synthetic reverse primer

<400> 718
acacctgcac aattctccg                                                 19

<210> 719
<211> 18
<212> DNA
<213> Artificial Sequence

<220>
<223> Synthetic reverse primer

<400> 719
ttcttttgcg cttcagcc                                                  18

<210> 720
<211> 23
<212> DNA
<213> Artificial Sequence

<220>
<223> Synthetic reverse primer

<400> 720
tcagcaagtg ggaaggtgta atc                                               23

<210> 721
<211> 19
<212> DNA
<213> Artificial Sequence

<220>
<223> Synthetic reverse primer

<400> 721
ccttaaagcg gactccagg                                                    19

<210> 722
<211> 18
<212> DNA
<213> Artificial Sequence

<220>
<223> Synthetic reverse primer

<400> 722
tcctcctgta ggctggca                                                     18

<210> 723
<211> 20
<212> DNA
<213> Artificial Sequence

<220>
<223> Synthetic reverse primer

<400> 723
ctctcagcat cggtacaagg                                                   20

<210> 724
<211> 20
<212> DNA
<213> Artificial Sequence

<220>
<223> Synthetic reverse primer

<400> 724
atctgcgttg aagcagtgag                                                   20

<210> 725
<211> 21
<212> DNA
<213> Artificial Sequence

<220>
<223> Synthetic reverse primer

<400> 725
gtgatttgcc caggaaagtt t                                                 21

<210> 726
<211> 20
<212> DNA
<213> Artificial Sequence

<220>
<223> Synthetic reverse primer

<400> 726
tttatcccca gcgaatttgt                                                    20

<210> 727
<211> 22
<212> DNA
<213> Artificial Sequence

<220>
<223> Synthetic reverse primer

<400> 727
   tcccccttac tcagcttgaa ct                                                  22

<210> 728
<211> 22
<212> DNA
<213> Artificial Sequence

<220>
<223> Synthetic reverse primer

<400> 728
cgagtacttg tggaaggtgg ac                                                 22

<210> 729
<211> 21
<212> DNA
<213> Artificial Sequence

<220>
<223> Synthetic reverse primer

<400> 729
gcgaggtaat ttgtgccctt t                                                  21

<210> 730
<211> 21
<212> DNA
<213> Artificial Sequence

<220>
<223> Synthetic reverse primer

<400> 730
tgaggacact cggtctctag c                                                  21

<210> 731
<211> 18
<212> DNA
<213> Artificial Sequence

<220>
<223> Synthetic reverse primer

<400> 731
ctagccccac agccaaga                                                      18

<210> 732

<211> 18
<212> DNA
<213> Artificial Sequence

<220>
<223> Synthetic reverse primer

<400> 732
agttttaagg gtgccccg                                                    18

<210> 733
<211> 19
<212> DNA
<213> Artificial Sequence

<220>
<223> Synthetic reverse primer

<400> 733
gagactttgg gggattcca                                                   19

<210> 734
<211> 20
<212> DNA
<213> Artificial Sequence

<220>
<223> Synthetic reverse primer

<400> 734
gaagtccacc tgggcatctc                                                  20

<210> 735
<211> 19
<212> DNA
<213> Artificial Sequence

<220>
<223> Synthetic reverse primer

<400> 735
ccctttccaa acttgaggc                                                   19

<210> 736
<211> 19
<212> DNA
<213> Artificial Sequence

<220>
<223> Synthetic reverse primer

<400> 736
cactcgccgt tgacatcct                                                   19

<210> 737
<211> 19
<212> DNA
<213> Artificial Sequence

<220>
<223> Synthetic reverse primer

<400> 737

tgcaagctgt ctttgagcc                                                    19

<210> 738
<211> 20
<212> DNA
<213> Artificial Sequence

<220>
<223> Synthetic reverse primer

<400> 738
acagcaaggc gagcataaat                                                   20

<210> 739
<211> 18
<212> DNA
<213> Artificial Sequence

<220>
<223> Synthetic reverse primer

<400> 739
ctatcggcct cagcatgg                                                     18

<210> 740
<211> 25
<212> DNA
<213> Artificial Sequence

<220>
<223> Synthetic reverse primer

<400> 740
tgagtttttt gcgagagtat tgaca                                             25

<210> 741
<211> 20
<212> DNA
<213> Artificial Sequence

<220>
<223> Synthetic reverse primer

<400> 741
acacttcaag tcacgcttgc                                                   20

<210> 742
<211> 20
<212> DNA
<213> Artificial Sequence

<220>
<223> Synthetic reverse primer

<400> 742
gtagggctgc tggaaggtaa                                                   20

<210> 743
<211> 20
<212> DNA
<213> Artificial Sequence

<220>

<223> Synthetic reverse primer

<400> 743
tccgaccttc aatcatttca                                                    20

<210> 744
<211> 20
<212> DNA
<213> Artificial Sequence

<220>
<223> Synthetic reverse primer

<400> 744
ggagacaatg caaaccacac                                                    20

<210> 745
<211> 23
<212> DNA
<213> Artificial Sequence

<220>
<223> Synthetic reverse primer

<400> 745
tacaatggct ttggaggata gca                                                23

<210> 746
<211> 24
<212> DNA
<213> Artificial Sequence

<220>
<223> Synthetic reverse primer

<400> 746
caaagctgtc agctctagca aaag                                               24

<210> 747
<211> 22
<212> DNA
<213> Artificial Sequence

<220>
<223> Synthetic reverse primer

<400> 747
ttcatccctc gatatggctt ct                                                 22

<210> 748
<211> 25
<212> DNA
<213> Artificial Sequence

<220>
<223> Synthetic reverse primer

<400> 748
agtctggaac atgtcagtct tgatg                                              25

<210> 749
<211> 21
<212> DNA

<213> Artificial Sequence

<220>
<223> Synthetic reverse primer

<400> 749
cgagcatttg tctcatcctt t                                                21

<210> 750
<211> 20
<212> DNA
<213> Artificial Sequence

<220>
<223> Synthetic reverse primer

<400> 750
actcaggccc atttccttta                                                  20

<210> 751
<211> 20
<212> DNA
<213> Artificial Sequence

<220>
<223> Synthetic reverse primer

<400> 751
cctggtgctg ttgtagatgg                                                  20

<210> 752
<211> 20
<212> DNA
<213> Artificial Sequence

<220>
<223> Synthetic reverse primer

<400> 752
gccaccgata tagcgctgtt                                                  20

<210> 753
<211> 20
<212> DNA
<213> Artificial Sequence

<220>
<223> Synthetic reverse primer

<400> 753
cctcttcatc aggccaaact                                                  20

<210> 754
<211> 20
<212> DNA
<213> Artificial Sequence

<220>
<223> Synthetic reverse primer

<400> 754
accgaaattg gagagcatgt                                                  20

<210> 755
<211> 21
<212> DNA
<213> Artificial Sequence

<220>
<223> Synthetic reverse primer

<400> 755
ttcagagaaa ggaggtgtgg a                                                21

<210> 756
<211> 22
<212> DNA
<213> Artificial Sequence

<220>
<223> Synthetic reverse primer

<400> 756
gggaaagtgg tacgtctttg ag                                               22

<210> 757
<211> 20
<212> DNA
<213> Artificial Sequence

<220>
<223> Synthetic reverse primer

<400> 757
ttggccagat ctaaccatga                                                  20

<210> 758
<211> 22
<212> DNA
<213> Artificial Sequence

<220>
<223> Synthetic reverse primer

<400> 758
gcttgagggt ctgaatcttg ct                                               22

<210> 759
<211> 18
<212> DNA
<213> Artificial Sequence

<220>
<223> Synthetic reverse primer

<400> 759
gcgaatgcca tgactgaa                                                    18

<210> 760
<211> 24
<212> DNA
<213> Artificial Sequence

<220>
<223> Synthetic reverse primer

<400> 760
tgctgtcgtg atgagaaaat agtg                                              24

<210> 761
<211> 22
<212> DNA
<213> Artificial Sequence

<220>
<223> Synthetic reverse primer

<400> 761
gcttcaacgg caaagttctc tt                                                22

<210> 762
<211> 19
<212> DNA
<213> Artificial Sequence

<220>
<223> Synthetic reverse primer

<400> 762
gtactgagcg atggagcgt                                                    19

<210> 763
<211> 19
<212> DNA
<213> Artificial Sequence

<220>
<223> Synthetic reverse primer

<400> 763
gcggtgtagc tcccagagt                                                    19

<210> 764
<211> 24
<212> DNA
<213> Artificial Sequence

<220>
<223> Synthetic reverse primer

<400> 764
caaactccac agtacttggg ttga                                              24

<210> 765
<211> 22
<212> DNA
<213> Artificial Sequence

<220>
<223> Synthetic reverse primer

<400> 765
tgtcggaatt gatactggca tt                                                22

<210> 766
<211> 20
<212> DNA
<213> Artificial Sequence

<220>
<223> Synthetic reverse primer

<400> 766
gtgcacgtgg atgaaagagt                                                    20

<210> 767
<211> 21
<212> DNA
<213> Artificial Sequence

<220>
<223> Synthetic reverse primer

<400> 767
agctccctgt tgcatggact t                                                  21

<210> 768
<211> 20
<212> DNA
<213> Artificial Sequence

<220>
<223> Synthetic reverse primer

<400> 768
gcagacaaaa gttggaaggc                                                    20

<210> 769
<211> 25
<212> DNA
<213> Artificial Sequence

<220>
<223> Synthetic probe

<400> 769
atgcctacag caccctgatg tcgca                                              25

<210> 770
<211> 24
<212> DNA
<213> Artificial Sequence

<220>
<223> Synthetic probe

<400> 770
cgcttcaaag gaccagacct cctc                                               24

<210> 771
<211> 24
<212> DNA
<213> Artificial Sequence

<220>
<223> Synthetic probe

<400> 771
ctcgccaatg atgctgctca agtt                                               24

<210> 772
<211> 24

<212> DNA
<213> Artificial Sequence

<220>
<223> Synthetic probe

<400> 772
ccatgagctg tagccgaatg tcca                                              24

<210> 773
<211> 22
<212> DNA
<213> Artificial Sequence

<220>
<223> Synthetic probe

<400> 773
ctgcacacgg ttctaggctc cg                                                22

<210> 774
<211> 24
<212> DNA
<213> Artificial Sequence

<220>
<223> Synthetic probe

<400> 774
tctgctctac aagcccattg accg                                              24

<210> 775
<211> 24
<212> DNA
<213> Artificial Sequence

<220>
<223> Synthetic probe

<400> 775
cccgcagaaa gcacatggta ttcc                                              24

<210> 776
<211> 24
<212> DNA
<213> Artificial Sequence

<220>
<223> Synthetic probe

<400> 776
ctctgtcacc aatgtggacc tgcc                                              24

<210> 777
<211> 29
<212> DNA
<213> Artificial Sequence

<220>
<223> Synthetic probe

<400> 777
tgtaggtatc tcttagtccc gccatctga                                         29

<210> 778
<211> 24
<212> DNA
<213> Artificial Sequence

<220>
<223> Synthetic probe

<400> 778
ctgacactca tctgagccct ccca                                              24

<210> 779
<211> 28
<212> DNA
<213> Artificial Sequence

<220>
<223> Synthetic probe

<400> 779
tgctacttgc aaaggcgtgt cctactgc                                          28

<210> 780
<211> 20
<212> DNA
<213> Artificial Sequence

<220>
<223> Synthetic probe

<400> 780
ctgcgctgga tggacaccgc                                                   20

<210> 781
<211> 24
<212> DNA
<213> Artificial Sequence

<220>
<223> Synthetic probe

<400> 781
cacacagggt gccatcaatc acct                                              24

<210> 782
<211> 24
<212> DNA
<213> Artificial Sequence

<220>
<223> Synthetic probe

<400> 782
cgagtggaag tgctccccac tttc                                              24

<210> 783
<211> 28
<212> DNA
<213> Artificial Sequence

<220>
<223> Synthetic probe

<400> 783
cgatctcagc ctgtttgtgc atctcgat                                          28

<210> 784
<211> 24
<212> DNA
<213> Artificial Sequence

<220>
<223> Synthetic probe

<400> 784
caacacgtca ccaccctttg ctct                                              24

<210> 785
<211> 23
<212> DNA
<213> Artificial Sequence

<220>
<223> Synthetic probe

<400> 785
tgaccacacc aaagcctccc tgg                                               23

<210> 786
<211> 24
<212> DNA
<213> Artificial Sequence

<220>
<223> Synthetic probe

<400> 786
acctcagtcc aaagtgcctg aggc                                              24

<210> 787
<211> 23
<212> DNA
<213> Artificial Sequence

<220>
<223> Synthetic probe

<400> 787
tcacggtaca caatctttcc gga                                               23

<210> 788
<211> 24
<212> DNA
<213> Artificial Sequence

<220>
<223> Synthetic probe

<400> 788
ccagttcctg ccgtctgctc ttct                                              24

<210> 789
<211> 22
<212> DNA
<213> Artificial Sequence

<220>
<223> Synthetic probe

<400> 789
ctgcagcgtc aatctccgct tg                                                22

<210> 790
<211> 24
<212> DNA
<213> Artificial Sequence

<220>
<223> Synthetic probe

<400> 790
aagctgacac agccctccca agtg                                              24

<210> 791
<211> 24
<212> DNA
<213> Artificial Sequence

<220>
<223> Synthetic probe

<400> 791
ccaccacaca ggtacagcag cgct                                              24

<210> 792
<211> 24
<212> DNA
<213> Artificial Sequence

<220>
<223> Synthetic probe

<400> 792
ctatgacgat gccctcaacg cctc                                              24

<210> 793
<211> 21
<212> DNA
<213> Artificial Sequence

<220>
<223> Synthetic probe

<400> 793
ctttcgggaa gccaggccct t                                                 21

<210> 794
<211> 24
<212> DNA
<213> Artificial Sequence

<220>
<223> Synthetic probe

<400> 794
actggatcct ggccaccgac tatg                                              24

<210> 795

<211> 22
<212> DNA
<213> Artificial Sequence

<220>
<223> Synthetic probe

<400> 795
cttgtccttg ggtcaccctg ca                                                22

<210> 796
<211> 21
<212> DNA
<213> Artificial Sequence

<220>
<223> Synthetic probe

<400> 796
acaccagcgg tgccgactac c                                                 21

<210> 797
<211> 24
<212> DNA
<213> Artificial Sequence

<220>
<223> Synthetic probe

<400> 797
ctagagccat ccttggccat cctg                                              24

<210> 798
<211> 24
<212> DNA
<213> Artificial Sequence

<220>
<223> Synthetic probe

<400> 798
caagaatctt gcagcagcat ggct                                              24

<210> 799
<211> 22
<212> DNA
<213> Artificial Sequence

<220>
<223> Synthetic probe

<400> 799
tgacgagcag cgaacagcca cg                                                22

<210> 800
<211> 23
<212> DNA
<213> Artificial Sequence

<220>
<223> Synthetic probe

<400> 800

aggagtatga cgagtccggc ccc                                              23

<210> 801
<211> 29
<212> DNA
<213> Artificial Sequence

<220>
<223> Synthetic probe

<400> 801
aggctcagtg atgtcttccc tgtcaccag                                        29

<210> 802
<211> 24
<212> DNA
<213> Artificial Sequence

<220>
<223> Synthetic probe

<400> 802
tgggcccaga gcatgttcca gatc                                             24

<210> 803
<211> 26
<212> DNA
<213> Artificial Sequence

<220>
<223> Synthetic probe

<400> 803
cccaattaac atgacccggc aaccat                                           26

<210> 804
<211> 23
<212> DNA
<213> Artificial Sequence

<220>
<223> Synthetic probe

<400> 804
agatgtgccg gtacacccac ctc                                              23

<210> 805
<211> 23
<212> DNA
<213> Artificial Sequence

<220>
<223> Synthetic probe

<400> 805
ccagcctgca gacaactggc ctc                                              23

<210> 806
<211> 25
<212> DNA
<213> Artificial Sequence

<220>

<223> Synthetic probe

<400> 806
tgccactcgg aaaaagacct ctcgg                                          25

<210> 807
<211> 24
<212> DNA
<213> Artificial Sequence

<220>
<223> Synthetic probe

<400> 807
catcatggga ctcctgccct tacc                                           24

<210> 808
<211> 21
<212> DNA
<213> Artificial Sequence

<220>
<223> Synthetic probe

<400> 808
agtcacgacc cctgccctca c                                              21

<210> 809
<211> 25
<212> DNA
<213> Artificial Sequence

<220>
<223> Synthetic probe

<400> 809
cagccctggg aactttgtcc tgacc                                          25

<210> 810
<211> 23
<212> DNA
<213> Artificial Sequence

<220>
<223> Synthetic probe

<400> 810
ctttccgttg gcatccgcaa cag                                            23

<210> 811
<211> 22
<212> DNA
<213> Artificial Sequence

<220>
<223> Synthetic probe

<400> 811
ttccacgccg aaggacagcg at                                             22

<210> 812
<211> 24
<212> DNA

<213> Artificial Sequence

<220>
<223> Synthetic probe

<400> 812
tccgggtagc tctcaaactc gagg                                              24

<210> 813
<211> 24
<212> DNA
<213> Artificial Sequence

<220>
<223> Synthetic probe

<400> 813
caagggtctc cagcacctct acgc                                              24

<210> 814
<211> 23
<212> DNA
<213> Artificial Sequence

<220>
<223> Synthetic probe

<400> 814
ccggttaact gtggcctgtg ccc                                               23

<210> 815
<211> 24
<212> DNA
<213> Artificial Sequence

<220>
<223> Synthetic probe

<400> 815
ctctcactgt gacagcccac ctcg                                              24

<210> 816
<211> 24
<212> DNA
<213> Artificial Sequence

<220>
<223> Synthetic probe

<400> 816
ctgtgttcga ctcagcctca ggga                                              24

<210> 817
<211> 23
<212> DNA
<213> Artificial Sequence

<220>
<223> Synthetic probe

<400> 817
cagtcggccc aggacggtct act                                               23

<210> 818
<211> 23
<212> DNA
<213> Artificial Sequence

<220>
<223> Synthetic probe

<400> 818
tgctgggagc ctacacttgg ccc                                              23

<210> 819
<211> 24
<212> DNA
<213> Artificial Sequence

<220>
<223> Synthetic probe

<400> 819
tacagtccca gcaccgacaa ttcc                                             24

<210> 820
<211> 24
<212> DNA
<213> Artificial Sequence

<220>
<223> Synthetic probe

<400> 820
cctcgctttg tttaacagcc cagg                                             24

<210> 821
<211> 24
<212> DNA
<213> Artificial Sequence

<220>
<223> Synthetic probe

<400> 821
ttacagcgac agtcatggcc gcat                                             24

<210> 822
<211> 24
<212> DNA
<213> Artificial Sequence

<220>
<223> Synthetic probe

<400> 822
ccggagtcct agcctcccaa attc                                             24

<210> 823
<211> 24
<212> DNA
<213> Artificial Sequence

<220>
<223> Synthetic probe

<400> 823
cctgctctgt tctggggtcc aaac                                              24

<210> 824
<211> 19
<212> DNA
<213> Artificial Sequence

<220>
<223> Synthetic probe

<400> 824
caggtcccat tgccgggcg                                                    19

<210> 825
<211> 24
<212> DNA
<213> Artificial Sequence

<220>
<223> Synthetic probe

<400> 825
tgatttcccg ttccgctcgg ttct                                              24

<210> 826
<211> 24
<212> DNA
<213> Artificial Sequence

<220>
<223> Synthetic probe

<400> 826
catggctacc acttcgacac agcc                                              24

<210> 827
<211> 21
<212> DNA
<213> Artificial Sequence

<220>
<223> Synthetic probe

<400> 827
aaagcacacc gctggcagga c                                                 21

<210> 828
<211> 28
<212> DNA
<213> Artificial Sequence

<220>
<223> Synthetic probe

<400> 828
tggcctcaca aggactaccc tctcatcc                                          28

<210> 829
<211> 24
<212> DNA
<213> Artificial Sequence

<220>
<223> Synthetic probe

<400> 829
atttcagctg atcagtgggc ctcc                                              24

<210> 830
<211> 28
<212> DNA
<213> Artificial Sequence

<220>
<223> Synthetic probe

<400> 830
cataatacat tcacctccct gcctcctc                                          28

<210> 831
<211> 24
<212> DNA
<213> Artificial Sequence

<220>
<223> Synthetic probe

<400> 831
cgcttcatct tggctgaggt cctc                                              24

<210> 832
<211> 24
<212> DNA
<213> Artificial Sequence

<220>
<223> Synthetic probe

<400> 832
ctctgctgac actcgagccc acat                                              24

<210> 833
<211> 22
<212> DNA
<213> Artificial Sequence

<220>
<223> Synthetic probe

<400> 833
acagagccct ggcaaagcca ag                                                22

<210> 834
<211> 27
<212> DNA
<213> Artificial Sequence

<220>
<223> Synthetic probe

<400> 834
tgtctccatt attgatcggt tcatgca                                           27

<210> 835
<211> 22

<212> DNA
<213> Artificial Sequence

<220>
<223> Synthetic probe

<400> 835
tacccgccat ccatgatcgc ca                                                22

<210> 836
<211> 30
<212> DNA
<213> Artificial Sequence

<220>
<223> Synthetic probe

<400> 836
cccagataat acaggtggcc aacaattcct                                        30

<210> 837
<211> 25
<212> DNA
<213> Artificial Sequence

<220>
<223> Synthetic probe

<400> 837
tggaataagt acctaaggcg ccccc                                             25

<210> 838
<211> 21
<212> DNA
<213> Artificial Sequence

<220>
<223> Synthetic probe

<400> 838
ctcccatccc agtggagcca a                                                 21

<210> 839
<211> 22
<212> DNA
<213> Artificial Sequence

<220>
<223> Synthetic probe

<400> 839
cgcaccattc ggtcatttga gg                                                22

<210> 840
<211> 23
<212> DNA
<213> Artificial Sequence

<220>
<223> Synthetic probe

<400> 840
ctgttgactg cagggcacca cca                                               23

<210> 841
<211> 24
<212> DNA
<213> Artificial Sequence

<220>
<223> Synthetic probe

<400> 841
caggtccctt gtcccaagtt ccac                                              24

<210> 842
<211> 27
<212> DNA
<213> Artificial Sequence

<220>
<223> Synthetic probe

<400> 842
ccctgctacc aatatggact ccagtca                                           27

<210> 843
<211> 24
<212> DNA
<213> Artificial Sequence

<220>
<223> Synthetic probe

<400> 843
caccgacagc acagacagaa tccc                                              24

<210> 844
<211> 24
<212> DNA
<213> Artificial Sequence

<220>
<223> Synthetic probe

<400> 844
caccaagccc agaggacagt tcct                                              24

<210> 845
<211> 28
<212> DNA
<213> Artificial Sequence

<220>
<223> Synthetic probe

<400> 845
ctccaagccc agattcagat tcgagtca                                          28

<210> 846
<211> 30
<212> DNA
<213> Artificial Sequence

<220>
<223> Synthetic probe

<400> 846
tcagcttcta caactggaca gacaacgctg                                        30

<210> 847
<211> 23
<212> DNA
<213> Artificial Sequence

<220>
<223> Synthetic probe

<400> 847
actggccgtg gcactggaca aca                                               23

<210> 848
<211> 28
<212> DNA
<213> Artificial Sequence

<220>
<223> Synthetic probe

<400> 848
tgtccctgtt agacgtcctc cgtccata                                          28

<210> 849
<211> 23
<212> DNA
<213> Artificial Sequence

<220>
<223> Synthetic probe

<400> 849
ctccccgtcg atgccagaga act                                               23

<210> 850
<211> 24
<212> DNA
<213> Artificial Sequence

<220>
<223> Synthetic probe

<400> 850
tgctccacta acaaccctcc tgcc                                              24

<210> 851
<211> 24
<212> DNA
<213> Artificial Sequence

<220>
<223> Synthetic probe

<400> 851
aattcctgca tggccagttt cctc                                              24

<210> 852
<211> 23
<212> DNA
<213> Artificial Sequence

<220>
<223> Synthetic probe

<400> 852
ctgcccaaga gcctgtcatc cag                                                23

<210> 853
<211> 24
<212> DNA
<213> Artificial Sequence

<220>
<223> Synthetic probe

<400> 853
ccttctgccc atagtgatca gcga                                               24

<210> 854
<211> 24
<212> DNA
<213> Artificial Sequence

<220>
<223> Synthetic probe

<400> 854
ccaacccaga tgaaatcggc aact                                               24

<210> 855
<211> 24
<212> DNA
<213> Artificial Sequence

<220>
<223> Synthetic probe

<400> 855
ccagtcgcct cagtaaagcc acct                                               24

<210> 856
<211> 24
<212> DNA
<213> Artificial Sequence

<220>
<223> Synthetic probe

<400> 856
cacaacatcc ctggtgaacg tcgt                                               24

<210> 857
<211> 24
<212> DNA
<213> Artificial Sequence

<220>
<223> Synthetic probe

<400> 857
atcacccatc atcatccaat cgca                                               24

<210> 858

<211> 23
<212> DNA
<213> Artificial Sequence

<220>
<223> Synthetic probe

<400> 858
tccttcccac ccccagtcct gtc                                                23

<210> 859
<211> 24
<212> DNA
<213> Artificial Sequence

<220>
<223> Synthetic probe

<400> 859
aaaatgagac tctccgtcgg cagc                                               24

<210> 860
<211> 20
<212> DNA
<213> Artificial Sequence

<220>
<223> Synthetic probe

<400> 860
caggccctct tccgagcggt                                                    20

<210> 861
<211> 21
<212> DNA
<213> Artificial Sequence

<220>
<223> Synthetic probe

<400> 861
cttcaattgg caagcccagg c                                                  21

<210> 862
<211> 22
<212> DNA
<213> Artificial Sequence

<220>
<223> Synthetic probe

<400> 862
tgctgatgtg ccctctcctt gg                                                 22

<210> 863
<211> 22
<212> DNA
<213> Artificial Sequence

<220>
<223> Synthetic probe

<400> 863

acccattctt ctcccagccg gg                                                22

<210> 864
<211> 24
<212> DNA
<213> Artificial Sequence

<220>
<223> Synthetic probe

<400> 864
acaccctact ccctgtgcct ccag                                              24

<210> 865
<211> 26
<212> DNA
<213> Artificial Sequence

<220>
<223> Synthetic probe

<400> 865
agctgatgag tctgccctac cgcctg                                            26

<210> 866
<211> 24
<212> DNA
<213> Artificial Sequence

<220>
<223> Synthetic probe

<400> 866
tgaagtctcc agctttgcct cagc                                              24

<210> 867
<211> 24
<212> DNA
<213> Artificial Sequence

<220>
<223> Synthetic probe

<400> 867
caccaggacc acaaagcctg tttg                                              24

<210> 868
<211> 18
<212> DNA
<213> Artificial Sequence

<220>
<223> Synthetic probe

<400> 868
ctgcgcccgc tcttcgcg                                                     18

<210> 869
<211> 24
<212> DNA
<213> Artificial Sequence

<220>

<223> Synthetic probe

<400> 869
cgcacagaca agccttactc cgcc                                              24

<210> 870
<211> 23
<212> DNA
<213> Artificial Sequence

<220>
<223> Synthetic probe

<400> 870
cgggaagaat tcgcttccac ctg                                               23

<210> 871
<211> 20
<212> DNA
<213> Artificial Sequence

<220>
<223> Synthetic probe

<400> 871
cccttcagcc tgccccaccg                                                   20

<210> 872
<211> 24
<212> DNA
<213> Artificial Sequence

<220>
<223> Synthetic probe

<400> 872
actcagttac cgagccacgt cacg                                              24

<210> 873
<211> 21
<212> DNA
<213> Artificial Sequence

<220>
<223> Synthetic probe

<400> 873
tcctgcgcct gatgtccacc g                                                 21

<210> 874
<211> 30
<212> DNA
<213> Artificial Sequence

<220>
<223> Synthetic probe

<400> 874
tctcctagcc agacgtgttt cttgtccttg                                        30

<210> 875
<211> 21
<212> DNA

<213> Artificial Sequence

<220>
<223> Synthetic probe

<400> 875
cctgcagccc atccacaacc t                                                21

<210> 876
<211> 27
<212> DNA
<213> Artificial Sequence

<220>
<223> Synthetic probe

<400> 876
cctcctcctg ttgctgccac taatgct                                          27

<210> 877
<211> 26
<212> DNA
<213> Artificial Sequence

<220>
<223> Synthetic probe

<400> 877
cgaaacgcta ttctcacagg ttcagc                                           26

<210> 878
<211> 24
<212> DNA
<213> Artificial Sequence

<220>
<223> Synthetic probe

<400> 878
tgttcatcct ggcgctcttc atgt                                             24

<210> 879
<211> 26
<212> DNA
<213> Artificial Sequence

<220>
<223> Synthetic probe

<400> 879
ccgattccaa aagaccatca ggttct                                           26

<210> 880
<211> 23
<212> DNA
<213> Artificial Sequence

<220>
<223> Synthetic probe

<400> 880
tttgctgaat gctccagcca agg                                              23

<210> 881
<211> 28
<212> DNA
<213> Artificial Sequence

<220>
<223> Synthetic probe

<400> 881
tcagatggag acctcgtgcc aaattaca                                          28

<210> 882
<211> 21
<212> DNA
<213> Artificial Sequence

<220>
<223> Synthetic probe

<400> 882
agccactccc cacgctgttg t                                                 21

<210> 883
<211> 24
<212> DNA
<213> Artificial Sequence

<220>
<223> Synthetic probe

<400> 883
cgcagatccg atttctctgg gatc                                              24

<210> 884
<211> 21
<212> DNA
<213> Artificial Sequence

<220>
<223> Synthetic probe

<400> 884
tcccgatggt ctgcagcagc t                                                 21

<210> 885
<211> 28
<212> DNA
<213> Artificial Sequence

<220>
<223> Synthetic probe

<400> 885
aacatcatgt tcttcttcat gacctcgc                                          28

<210> 886
<211> 24
<212> DNA
<213> Artificial Sequence

<220>
<223> Synthetic probe

<400> 886
accaacgctg acagcatgca tttc                                              24

<210> 887
<211> 22
<212> DNA
<213> Artificial Sequence

<220>
<223> Synthetic probe

<400> 887
accctgcccg cgatcacact ga                                                22

<210> 888
<211> 24
<212> DNA
<213> Artificial Sequence

<220>
<223> Synthetic probe

<400> 888
cttgaggacg cgaacagtcc acca                                              24

<210> 889
<211> 29
<212> DNA
<213> Artificial Sequence

<220>
<223> Synthetic probe

<400> 889
aggtgcaata tgggcatata tctccattg                                         29

<210> 890
<211> 24
<212> DNA
<213> Artificial Sequence

<220>
<223> Synthetic probe

<400> 890
tctgtgtggt ccatccttgg aagc                                              24

<210> 891
<211> 24
<212> DNA
<213> Artificial Sequence

<220>
<223> Synthetic probe

<400> 891
ttcttcgaaa gccatgttgc caga                                              24

<210> 892
<211> 24
<212> DNA
<213> Artificial Sequence

<220>
<223> Synthetic probe

<400> 892
   tacccttaag aacgccccct ccac 24

<210> 893
<211> 28
<212> DNA
<213> Artificial Sequence

<220>
<223> Synthetic probe

<400> 893
ctgaaactgg aacacaacca cccacaag                                          28

<210> 894
<211> 24
<212> DNA
<213> Artificial Sequence

<220>
<223> Synthetic probe

<400> 894
tggacacact gatgcaagcc aaga                                              24

<210> 895
<211> 19
<212> DNA
<213> Artificial Sequence

<220>
<223> Synthetic probe

<400> 895
cacagccacg ggcccaaa                                                     19

<210> 896
<211> 25
<212> DNA
<213> Artificial Sequence

<220>
<223> Synthetic probe

<400> 896
ctcatgccac cactgccaac acctc                                             25

<210> 897
<211> 24
<212> DNA
<213> Artificial Sequence

<220>
<223> Synthetic probe

<400> 897
cagcaccctt ggcagtttcg aaat                                              24

<210> 898
<211> 24

<212> DNA
<213> Artificial Sequence

<220>
<223> Synthetic probe

<400> 898
ctgtcacact ccctcaggca ggac                                              24

<210> 899
<211> 24
<212> DNA
<213> Artificial Sequence

<220>
<223> Synthetic probe

<400> 899
atcactggaa ctcctcggtc ggac                                              24

<210> 900
<211> 26
<212> DNA
<213> Artificial Sequence

<220>
<223> Synthetic probe

<400> 900
cagccacgat gaccactacc agcact                                            26

<210> 901
<211> 26
<212> DNA
<213> Artificial Sequence

<220>
<223> Synthetic probe

<400> 901
tgcttcctcc cactatctga aaataa                                            26

<210> 902
<211> 24
<212> DNA
<213> Artificial Sequence

<220>
<223> Synthetic probe

<400> 902
agctgcccgt ctttctcagc cagc                                              24

<210> 903
<211> 25
<212> DNA
<213> Artificial Sequence

<220>
<223> Synthetic probe

<400> 903
agaaaagctg tttgtctccc cagca                                             25

<210> 904
<211> 24
<212> DNA
<213> Artificial Sequence

<220>
<223> Synthetic probe

<400> 904
aaagtccatt tgccactgat ggca                                              24

<210> 905
<211> 24
<212> DNA
<213> Artificial Sequence

<220>
<223> Synthetic probe

<400> 905
ctacctggac atccctgctc agcc                                              24

<210> 906
<211> 23
<212> DNA
<213> Artificial Sequence

<220>
<223> Synthetic probe

<400> 906
cagacttggt gccctttgac tcc                                               23

<210> 907
<211> 21
<212> DNA
<213> Artificial Sequence

<220>
<223> Synthetic probe

<400> 907
cagggccatg acaatcgcca a                                                 21

<210> 908
<211> 24
<212> DNA
<213> Artificial Sequence

<220>
<223> Synthetic probe

<400> 908
cgccctggct caacttttcc ttaa                                              24

<210> 909
<211> 26
<212> DNA
<213> Artificial Sequence

<220>
<223> Synthetic probe

<400> 909
cgaggccatt gacttcatag actcca                                          26

<210> 910
<211> 24
<212> DNA
<213> Artificial Sequence

<220>
<223> Synthetic probe

<400> 910
ttgagcacac tgcagtccat ctcc                                            24

<210> 911
<211> 24
<212> DNA
<213> Artificial Sequence

<220>
<223> Synthetic probe

<400> 911
cagaagaaca gctcagggac ccct                                            24

<210> 912
<211> 24
<212> DNA
<213> Artificial Sequence

<220>
<223> Synthetic probe

<400> 912
ctcaccagaa gccccaacct caac                                            24

<210> 913
<211> 23
<212> DNA
<213> Artificial Sequence

<220>
<223> Synthetic probe

<400> 913
cactcccgag cacgttgttc cgt                                             23

<210> 914
<211> 29
<212> DNA
<213> Artificial Sequence

<220>
<223> Synthetic probe

<400> 914
ccacttggac atcatctggg tgaacactc                                       29

<210> 915
<211> 23
<212> DNA
<213> Artificial Sequence

<220>
<223> Synthetic probe

<400> 915
cctttgcctc agggcatcct ttt                                              23

<210> 916
<211> 24
<212> DNA
<213> Artificial Sequence

<220>
<223> Synthetic probe

<400> 916
tgctacctgc ccctttgtca tgtg                                             24

<210> 917
<211> 24
<212> DNA
<213> Artificial Sequence

<220>
<223> Synthetic probe

<400> 917
caaaggtgac caccataccg ggtt                                             24

<210> 918
<211> 24
<212> DNA
<213> Artificial Sequence

<220>
<223> Synthetic probe

<400> 918
ctcgtcgtag cgcttctcgc tgta                                             24

<210> 919
<211> 28
<212> DNA
<213> Artificial Sequence

<220>
<223> Synthetic probe

<400> 919
tgatgctttc tccagaaact cgaactca                                         28

<210> 920
<211> 23
<212> DNA
<213> Artificial Sequence

<220>
<223> Synthetic probe

<400> 920
cggatccttt cctcactcgc cca                                              23

<210> 921

<211> 24
<212> DNA
<213> Artificial Sequence

<220>
<223> Synthetic probe

<400> 921
acccagtctc accttctccc cacc                                              24

<210> 922
<211> 26
<212> DNA
<213> Artificial Sequence

<220>
<223> Synthetic probe

<400> 922
tgagatggac atttaaagca ccagcc                                            26

<210> 923
<211> 20
<212> DNA
<213> Artificial Sequence

<220>
<223> Synthetic probe

<400> 923
cgcccagagg cacccacctg                                                   20

<210> 924
<211> 22
<212> DNA
<213> Artificial Sequence

<220>
<223> Synthetic probe

<400> 924
ccagagagcc tccctgcagc ca                                                22

<210> 925
<211> 26
<212> DNA
<213> Artificial Sequence

<220>
<223> Synthetic probe

<400> 925
ctgcaactgc ctcctgctca aagtca                                            26

<210> 926
<211> 23
<212> DNA
<213> Artificial Sequence

<220>
<223> Synthetic probe

<400> 926

cactgacatc atggctggcc ttg                                              23

<210> 927
<211> 25
<212> DNA
<213> Artificial Sequence

<220>
<223> Synthetic probe

<400> 927
ccgctctcat cgcagtcagg atcat                                            25

<210> 928
<211> 20
<212> DNA
<213> Artificial Sequence

<220>
<223> Synthetic probe

<400> 928
tgcgcccgat gagatcaccg                                                  20

<210> 929
<211> 24
<212> DNA
<213> Artificial Sequence

<220>
<223> Synthetic probe

<400> 929
cacctgatgc atgatggaca ctgc                                             24

<210> 930
<211> 24
<212> DNA
<213> Artificial Sequence

<220>
<223> Synthetic probe

<400> 930
cgtcccattt gagcctgtca atgt                                             24

<210> 931
<211> 30
<212> DNA
<213> Artificial Sequence

<220>
<223> Synthetic probe

<400> 931
atctgtatgc ggaacctcaa aagagtccct                                       30

<210> 932
<211> 30
<212> DNA
<213> Artificial Sequence

<220>

<223> Synthetic probe

<400> 932
tgtcccacga ataatgcgta aattctccag                                        30

<210> 933
<211> 21
<212> DNA
<213> Artificial Sequence

<220>
<223> Synthetic probe

<400> 933
cagcaggccc tcaaggagct g                                                 21

<210> 934
<211> 24
<212> DNA
<213> Artificial Sequence

<220>
<223> Synthetic probe

<400> 934
agccatatgc cttctcatct gggc                                              24

<210> 935
<211> 21
<212> DNA
<213> Artificial Sequence

<220>
<223> Synthetic probe

<400> 935
agagccggcc agccctgaca g                                                 21

<210> 936
<211> 24
<212> DNA
<213> Artificial Sequence

<220>
<223> Synthetic probe

<400> 936
tcgcctacca tttggtgcaa gcaa                                              24

<210> 937
<211> 23
<212> DNA
<213> Artificial Sequence

<220>
<223> Synthetic probe

<400> 937
ctggccctca tgtccccttc acg                                               23

<210> 938
<211> 26
<212> DNA

<213> Artificial Sequence

<220>
<223> Synthetic probe

<400> 938
ccccagacca agtgtgaata catgct                                            26

<210> 939
<211> 19
<212> DNA
<213> Artificial Sequence

<220>
<223> Synthetic probe

<400> 939
ctggagatgc tggacgccc                                                    19

<210> 940
<211> 24
<212> DNA
<213> Artificial Sequence

<220>
<223> Synthetic probe

<400> 940
ttaccagagg cgaggttccc ttca                                              24

<210> 941
<211> 25
<212> DNA
<213> Artificial Sequence

<220>
<223> Synthetic probe

<400> 941
tcctgacttc tgtgagctca ttgcg                                             25

<210> 942
<211> 21
<212> DNA
<213> Artificial Sequence

<220>
<223> Synthetic probe

<400> 942
tggcacgggt cttctcctac c                                                 21

<210> 943
<211> 24
<212> DNA
<213> Artificial Sequence

<220>
<223> Synthetic probe

<400> 943
cggcctgtcc acgaaccact tata                                              24

<210> 944
<211> 24
<212> DNA
<213> Artificial Sequence

<220>
<223> Synthetic probe

<400> 944
tcgcccacct acgtactggc ctac                                              24

<210> 945
<211> 27
<212> DNA
<213> Artificial Sequence

<220>
<223> Synthetic probe

<400> 945
tcccagagac caacgttcaa gcagttg                                           27

<210> 946
<211> 23
<212> DNA
<213> Artificial Sequence

<220>
<223> Synthetic probe

<400> 946
cctttcatgg ggagaaccgc att                                               23

<210> 947
<211> 23
<212> DNA
<213> Artificial Sequence

<220>
<223> Synthetic probe

<400> 947
tcggccactt catcaggacg cag                                               23

<210> 948
<211> 24
<212> DNA
<213> Artificial Sequence

<220>
<223> Synthetic probe

<400> 948
aatctgctcc actgtcaggg tccc                                              24

<210> 949
<211> 24
<212> DNA
<213> Artificial Sequence

<220>
<223> Synthetic probe

<400> 949
actctcaggc ggtgtccaca tgat                                              24

<210> 950
<211> 22
<212> DNA
<213> Artificial Sequence

<220>
<223> Synthetic probe

<400> 950
tcccagcatc atccaggccc ag                                                22

<210> 951
<211> 24
<212> DNA
<213> Artificial Sequence

<220>
<223> Synthetic probe

<400> 951
agaacagcat ccgccacaac ctct                                              24

<210> 952
<211> 24
<212> DNA
<213> Artificial Sequence

<220>
<223> Synthetic probe

<400> 952
ctctacagca gctcagccag cctg                                              24

<210> 953
<211> 24
<212> DNA
<213> Artificial Sequence

<220>
<223> Synthetic probe

<400> 953
cagaccaagc ctttgcccag aatt                                              24

<210> 954
<211> 24
<212> DNA
<213> Artificial Sequence

<220>
<223> Synthetic probe

<400> 954
tgtttccatg gctaccccac aggt                                              24

<210> 955
<211> 22
<212> DNA
<213> Artificial Sequence

<220>
<223> Synthetic probe

<400> 955
tgaccggcgc atcacactga gg                                                22

<210> 956
<211> 29
<212> DNA
<213> Artificial Sequence

<220>
<223> Synthetic probe

<400> 956
tcaattgtaa cattctcacc caggccttg                                         29

<210> 957
<211> 24
<212> DNA
<213> Artificial Sequence

<220>
<223> Synthetic probe

<400> 957
ctgaagcacg acaagctggt ccag                                              24

<210> 958
<211> 19
<212> DNA
<213> Artificial Sequence

<220>
<223> Synthetic probe

<400> 958
   cgcccgcagg cctcatcct                                                   19

<210> 959
<211> 24
<212> DNA
<213> Artificial Sequence

<220>
<223> Synthetic probe

<400> 959
tgagccagat tccacacctc acgt                                              24

<210> 960
<211> 25
<212> DNA
<213> Artificial Sequence

<220>
<223> Synthetic probe

<400> 960
ttcatctcaa tggaaggatc ctgcc                                             25

<210> 961
<211> 24

<212> DNA
<213> Artificial Sequence

<220>
<223> Synthetic probe

<400> 961
aacttcagcc ccagctccca agtc                                              24

<210> 962
<211> 22
<212> DNA
<213> Artificial Sequence

<220>
<223> Synthetic probe

<400> 962
ccgttctcag ccttgacggt gc                                                22

<210> 963
<211> 24
<212> DNA
<213> Artificial Sequence

<220>
<223> Synthetic probe

<400> 963
tgttccaacc actgaatctg gacc                                              24

<210> 964
<211> 26
<212> DNA
<213> Artificial Sequence

<220>
<223> Synthetic probe

<400> 964
ttgggacatt gtagacttgg ccagac                                            26

<210> 965
<211> 30
<212> DNA
<213> Artificial Sequence

<220>
<223> Synthetic probe

<400> 965
ccatggacca acttcactat gtgacagagc                                        30

<210> 966
<211> 26
<212> DNA
<213> Artificial Sequence

<220>
<223> Synthetic probe

<400> 966
tgcagttgac atggcctgtt cagtcc                                            26

<210> 967
<211> 24
<212> DNA
<213> Artificial Sequence

<220>
<223> Synthetic probe

<400> 967
tgttagccaa agactgccac tgca                                              24

<210> 968
<211> 24
<212> DNA
<213> Artificial Sequence

<220>
<223> Synthetic probe

<400> 968
tgtccacagg accctgagtg gttc                                              24

<210> 969
<211> 24
<212> DNA
<213> Artificial Sequence

<220>
<223> Synthetic probe

<400> 969
atcccctccc tctccaccca tcta                                              24

<210> 970
<211> 21
<212> DNA
<213> Artificial Sequence

<220>
<223> Synthetic probe

<400> 970
aggcgttgca cttcaccagc c                                                 21

<210> 971
<211> 24
<212> DNA
<213> Artificial Sequence

<220>
<223> Synthetic probe

<400> 971
cctcttgccc acttactggg tgga                                              24

<210> 972
<211> 24
<212> DNA
<213> Artificial Sequence

<220>
<223> Synthetic probe

<400> 972
ccgggtgaca gcactaacca gacc                                              24

<210> 973
<211> 22
<212> DNA
<213> Artificial Sequence

<220>
<223> Synthetic probe

<400> 973
ctcttcccca tcggctttgt gg                                                22

<210> 974
<211> 24
<212> DNA
<213> Artificial Sequence

<220>
<223> Synthetic probe

<400> 974
cctcctgctg gcttcctttg atca                                              24

<210> 975
<211> 24
<212> DNA
<213> Artificial Sequence

<220>
<223> Synthetic probe

<400> 975
ctcatcacct ggtctccggt gtgt                                              24

<210> 976
<211> 26
<212> DNA
<213> Artificial Sequence

<220>
<223> Synthetic probe

<400> 976
catgctgcat cctaaggctc ctcagg                                            26

<210> 977
<211> 24
<212> DNA
<213> Artificial Sequence

<220>
<223> Synthetic probe

<400> 977
ctggccttcc cgtgtaacca gttc                                              24

<210> 978
<211> 23
<212> DNA
<213> Artificial Sequence

<220>
<223> Synthetic probe

<400> 978
ctccccaccc ttgagaagtg cct                                              23

<210> 979
<211> 28
<212> DNA
<213> Artificial Sequence

<220>
<223> Synthetic probe

<400> 979
cacaattccc agagaaacca agaagagc                                         28

<210> 980
<211> 20
<212> DNA
<213> Artificial Sequence

<220>
<223> Synthetic probe

<400> 980
accacgcgaa cggtgcatcg                                                  20

<210> 981
<211> 20
<212> DNA
<213> Artificial Sequence

<220>
<223> Synthetic probe

<400> 981
tccccgagcc cagcaggaca                                                  20

<210> 982
<211> 29
<212> DNA
<213> Artificial Sequence

<220>
<223> Synthetic probe

<400> 982
tgacctgatc cagagtaagt gcctctcca                                        29

<210> 983
<211> 30
<212> DNA
<213> Artificial Sequence

<220>
<223> Synthetic probe

<400> 983
tcagccactg gcttctgtca taatcaggag                                       30

<210> 984

<211> 27
<212> DNA
<213> Artificial Sequence

<220>
<223> Synthetic probe

<400> 984
cccgcctacc ctcgtaaagc agattca                                          27

<210> 985
<211> 26
<212> DNA
<213> Artificial Sequence

<220>
<223> Synthetic probe

<400> 985
ctgaagctct actcacagtt tctggg                                           26

<210> 986
<211> 27
<212> DNA
<213> Artificial Sequence

<220>
<223> Synthetic probe

<400> 986
ctcgcaagca caacatgtgt ggtgaga                                          27

<210> 987
<211> 23
<212> DNA
<213> Artificial Sequence

<220>
<223> Synthetic probe

<400> 987
cacagccgcc tgaaagccac aat                                              23

<210> 988
<211> 27
<212> DNA
<213> Artificial Sequence

<220>
<223> Synthetic probe

<400> 988
tcaagtaaac gggctgtttt ccaaaca                                          27

<210> 989
<211> 23
<212> DNA
<213> Artificial Sequence

<220>
<223> Synthetic probe

<400> 989

aaagacatcc agctagcacg ccg                                                23

<210> 990
<211> 23
<212> DNA
<213> Artificial Sequence

<220>
<223> Synthetic probe

<400> 990
ttcttgcgct ccatccgtcc aga                                                23

<210> 991
<211> 24
<212> DNA
<213> Artificial Sequence

<220>
<223> Synthetic probe

<400> 991
caagaacctc ccagaagggc tcaa                                               24

<210> 992
<211> 24
<212> DNA
<213> Artificial Sequence

<220>
<223> Synthetic probe

<400> 992
ccagaccata gcacactcgg gcac                                               24

<210> 993
<211> 24
<212> DNA
<213> Artificial Sequence

<220>
<223> Synthetic probe

<400> 993
cagaatgtcc gccttctcca gctt                                               24

<210> 994
<211> 23
<212> DNA
<213> Artificial Sequence

<220>
<223> Synthetic probe

<400> 994
cgctcacgtt ctcatccaag tgg                                                23

<210> 995
<211> 21
<212> DNA
<213> Artificial Sequence

<220>

<223> Synthetic probe

<400> 995
ccccggacag tggctctgac g                                                21

<210> 996
<211> 28
<212> DNA
<213> Artificial Sequence

<220>
<223> Synthetic probe

<400> 996
ttccacatct ctcccagttt cttcgcaa                                         28

<210> 997
<211> 24
<212> DNA
<213> Artificial Sequence

<220>
<223> Synthetic probe

<400> 997
agtcgctggt ttcatgccct tcca                                             24

<210> 998
<211> 28
<212> DNA
<213> Artificial Sequence

<220>
<223> Synthetic probe

<400> 998
ctccatatcc aaacaaagca tgtgtgcg                                         28

<210> 999
<211> 24
<212> DNA
<213> Artificial Sequence

<220>
<223> Synthetic probe

<400> 999
agtctcctac tcccgggttc tgcg                                             24

<210> 1000
<211> 24
<212> DNA
<213> Artificial Sequence

<220>
<223> Synthetic probe

<400> 1000
tgaactcctt cctggaatac ccca                                             24

<210> 1001
<211> 28
<212> DNA

<213> Artificial Sequence

<220>
<223> Synthetic probe

<400> 1001
agccctgttc tcgttgccct aattcatc                                          28

<210> 1002
<211> 24
<212> DNA
<213> Artificial Sequence

<220>
<223> Synthetic probe

<400> 1002
acactcggca ggagtagtac ccgc                                              24

<210> 1003
<211> 24
<212> DNA
<213> Artificial Sequence

<220>
<223> Synthetic probe

<400> 1003
accggagcct tcccagaaca aact                                              24

<210> 1004
<211> 25
<212> DNA
<213> Artificial Sequence

<220>
<223> Synthetic probe

<400> 1004
accgcttcta ccaatacctc gccca                                             25

<210> 1005
<211> 24
<212> DNA
<213> Artificial Sequence

<220>
<223> Synthetic probe

<400> 1005
agttgcttcc atccaacctg gagg                                              24

<210> 1006
<211> 30
<212> DNA
<213> Artificial Sequence

<220>
<223> Synthetic probe

<400> 1006
cagaatggcc tgtattcact atcttcgaga                                        30

<210> 1007
<211> 24
<212> DNA
<213> Artificial Sequence

<220>
<223> Synthetic probe

<400> 1007
agagtgactc ccgttgtccc aagg                                              24

<210> 1008
<211> 20
<212> DNA
<213> Artificial Sequence

<220>
<223> Synthetic probe

<400> 1008
tgactcccgc ggtcccaagg                                                   20

<210> 1009
<211> 29
<212> DNA
<213> Artificial Sequence

<220>
<223> Synthetic probe

<400> 1009
cattttcctc agacttgtga acctccact                                         29

<210> 1010
<211> 30
<212> DNA
<213> Artificial Sequence

<220>
<223> Synthetic probe

<400> 1010
taattagacc taggcctcag ctgcactgcc                                        30

<210> 1011
<211> 26
<212> DNA
<213> Artificial Sequence

<220>
<223> Synthetic probe

<400> 1011
ctcagggccc accattgaag aggttg                                            26

<210> 1012
<211> 24
<212> DNA
<213> Artificial Sequence

<220>
<223> Synthetic probe

<400> 1012
cgcacttttc tgagcagacg tcca                                              24

<210> 1013
<211> 23
<212> DNA
<213> Artificial Sequence

<220>
<223> Synthetic probe

<400> 1013
ccaggcgtgg cgtcctctcc ata                                               23

<210> 1014
<211> 20
<212> DNA
<213> Artificial Sequence

<220>
<223> Synthetic probe

<400> 1014
ccggcgccca acgtgattct                                                   20

<210> 1015
<211> 26
<212> DNA
<213> Artificial Sequence

<220>
<223> Synthetic probe

<400> 1015
tggagattct ccagcacgtc atcgac                                            26

<210> 1016
<211> 30
<212> DNA
<213> Artificial Sequence

<220>
<223> Synthetic probe

<400> 1016
cttttgtttt gcccagtata gactcggaag                                        30

<210> 1017
<211> 20
<212> DNA
<213> Artificial Sequence

<220>
<223> Synthetic probe

<400> 1017
ccgtgaatgc agcccgccag                                                   20

<210> 1018
<211> 28
<212> DNA
<213> Artificial Sequence

<220>
<223> Synthetic probe

<400> 1018
cgcgtcatac caaaatctcc gattttga                                          28

<210> 1019
<211> 24
<212> DNA
<213> Artificial Sequence

<220>
<223> Synthetic probe

<400> 1019
taccccgtgg gcaagttctt ccaa                                              24

<210> 1020
<211> 25
<212> DNA
<213> Artificial Sequence

<220>
<223> Synthetic probe

<400> 1020
atccaggcac ctctaccacg ccctc                                             25

<210> 1021
<211> 23
<212> DNA
<213> Artificial Sequence

<220>
<223> Synthetic probe

<400> 1021
cccggtcacc aggcaggagt tct                                               23

<210> 1022
<211> 24
<212> DNA
<213> Artificial Sequence

<220>
<223> Synthetic probe

<400> 1022
cagccctaca cgaaaggacc tgct                                              24

<210> 1023
<211> 22
<212> DNA
<213> Artificial Sequence

<220>
<223> Synthetic probe

<400> 1023
tgacttccaa gctggccgtg gc                                                22

<210> 1024
<211> 22

<212> DNA
<213> Artificial Sequence

<220>
<223> Synthetic probe

<400> 1024
ctgctccacg gccttgctct tg                                                22

<210> 1025
<211> 26
<212> DNA
<213> Artificial Sequence

<220>
<223> Synthetic probe

<400> 1025
cctgtgatca acagtacccg tatggg                                            26

<210> 1026
<211> 24
<212> DNA
<213> Artificial Sequence

<220>
<223> Synthetic probe

<400> 1026
tcggcttccc tgtagagctg aaca                                              24

<210> 1027
<211> 25
<212> DNA
<213> Artificial Sequence

<220>
<223> Synthetic probe

<400> 1027
catattgccc agtggtcacc tcaca                                             25

<210> 1028
<211> 24
<212> DNA
<213> Artificial Sequence

<220>
<223> Synthetic probe

<400> 1028
attcaaaaca gagcccccaa agcc                                              24

<210> 1029
<211> 25
<212> DNA
<213> Artificial Sequence

<220>
<223> Synthetic probe

<400> 1029
acgtccgggt cctcactgtc cttcc                                             25

<210> 1030
<211> 24
<212> DNA
<213> Artificial Sequence

<220>
<223> Synthetic probe

<400> 1030
cccacatgac ttcctcttgg cctt                                              24

<210> 1031
<211> 20
<212> DNA
<213> Artificial Sequence

<220>
<223> Synthetic probe

<400> 1031
tccatcccag ctccagccag                                                   20

<210> 1032
<211> 24
<212> DNA
<213> Artificial Sequence

<220>
<223> Synthetic probe

<400> 1032
cactccagac ctcgcttagc atgg                                              24

<210> 1033
<211> 24
<212> DNA
<213> Artificial Sequence

<220>
<223> Synthetic probe

<400> 1033
cgatcctgca tctgtaaatc gccc                                              24

<210> 1034
<211> 24
<212> DNA
<213> Artificial Sequence

<220>
<223> Synthetic probe

<400> 1034
tctgagcctt gtcctctatc cggc                                              24

<210> 1035
<211> 24
<212> DNA
<213> Artificial Sequence

<220>
<223> Synthetic probe

<400> 1035
tcgccatctt ttgtgggatt cctt                                              24

<210> 1036
<211> 24
<212> DNA
<213> Artificial Sequence

<220>
<223> Synthetic probe

<400> 1036
ccgacagcca cagaataacc caaa                                              24

<210> 1037
<211> 30
<212> DNA
<213> Artificial Sequence

<220>
<223> Synthetic probe

<400> 1037
tgctaatgta aggcatcaca gtcttttcca                                        30

<210> 1038
<211> 28
<212> DNA
<213> Artificial Sequence

<220>
<223> Synthetic probe

<400> 1038
aaatacctgc aaccgttact gccgtgac                                          28

<210> 1039
<211> 24
<212> DNA
<213> Artificial Sequence

<220>
<223> Synthetic probe

<400> 1039
caccaacctg tacccgtatt gcga                                              24

<210> 1040
<211> 27
<212> DNA
<213> Artificial Sequence

<220>
<223> Synthetic probe

<400> 1040
tgctatgttt ctacaaaacc gccaagg                                           27

<210> 1041
<211> 24
<212> DNA
<213> Artificial Sequence

<220>
<223> Synthetic probe

<400> 1041
actcgatttc ccagccaacc acag                                              24

<210> 1042
<211> 23
<212> DNA
<213> Artificial Sequence

<220>
<223> Synthetic probe

<400> 1042
caagggacac gccttctgaa cgt                                               23

<210> 1043
<211> 23
<212> DNA
<213> Artificial Sequence

<220>
<223> Synthetic probe

<400> 1043
ccacttgtcg aaccaccgct cgt                                               23

<210> 1044
<211> 24
<212> DNA
<213> Artificial Sequence

<220>
<223> Synthetic probe

<400> 1044
cccttcaagc tgccaatgaa gacc                                              24

<210> 1045
<211> 24
<212> DNA
<213> Artificial Sequence

<220>
<223> Synthetic probe

<400> 1045
cttcaaggcc atgctgacca tcag                                              24

<210> 1046
<211> 24
<212> DNA
<213> Artificial Sequence

<220>
<223> Synthetic probe

<400> 1046
tgcattcctc tgagctcact gctg                                              24

<210> 1047

<211> 24
<212> DNA
<213> Artificial Sequence

<220>
<223> Synthetic probe

<400> 1047
agtcagtggc ccatcagcaa tcag                                              24

<210> 1048
<211> 22
<212> DNA
<213> Artificial Sequence

<220>
<223> Synthetic probe

<400> 1048
aagccgctcc actcgcatgt cc                                                22

<210> 1049
<211> 23
<212> DNA
<213> Artificial Sequence

<220>
<223> Synthetic probe

<400> 1049
cctccccaac aaagaccacc gca                                               23

<210> 1050
<211> 24
<212> DNA
<213> Artificial Sequence

<220>
<223> Synthetic probe

<400> 1050
ttaccccagc tccatccttg catc                                              24

<210> 1051
<211> 26
<212> DNA
<213> Artificial Sequence

<220>
<223> Synthetic probe

<400> 1051
ttcgtaacag cagtcatcat ccatgg                                            26

<210> 1052
<211> 25
<212> DNA
<213> Artificial Sequence

<220>
<223> Synthetic probe

<400> 1052

tttgaccggg tattcccacc aggaa                                          25

<210> 1053
<211> 24
<212> DNA
<213> Artificial Sequence

<220>
<223> Synthetic probe

<400> 1053
ccttggagaa acacaagcac ctgc                                           24

<210> 1054
<211> 24
<212> DNA
<213> Artificial Sequence

<220>
<223> Synthetic probe

<400> 1054
actcctgttt tcaccaccat gcca                                           24

<210> 1055
<211> 24
<212> DNA
<213> Artificial Sequence

<220>
<223> Synthetic probe

<400> 1055
atctacgttg tccagctgcc agcc                                           24

<210> 1056
<211> 24
<212> DNA
<213> Artificial Sequence

<220>
<223> Synthetic probe

<400> 1056
ctgattcctc aggtccttgg cctg                                           24

<210> 1057
<211> 24
<212> DNA
<213> Artificial Sequence

<220>
<223> Synthetic probe

<400> 1057
ctgttcctgg agcatggcct cttc                                           24

<210> 1058
<211> 24
<212> DNA
<213> Artificial Sequence

<220>

<223> Synthetic probe

<400> 1058
caagtgcctg taccacacgg aagg                                              24

<210> 1059
<211> 23
<212> DNA
<213> Artificial Sequence

<220>
<223> Synthetic probe

<400> 1059
ccactcgcca tactgggtgc agt                                               23

<210> 1060
<211> 28
<212> DNA
<213> Artificial Sequence

<220>
<223> Synthetic probe

<400> 1060
aggtcttatc agcacagtct ccgcctcc                                          28

<210> 1061
<211> 24
<212> DNA
<213> Artificial Sequence

<220>
<223> Synthetic probe

<400> 1061
ctggacgcgg ttctactcca acag                                              24

<210> 1062
<211> 25
<212> DNA
<213> Artificial Sequence

<220>
<223> Synthetic probe

<400> 1062
acccagataa cgcatcatgg agcga                                             25

<210> 1063
<211> 24
<212> DNA
<213> Artificial Sequence

<220>
<223> Synthetic probe

<400> 1063
tgcctccctg tcgcaccagt acta                                              24

<210> 1064
<211> 24
<212> DNA

<213> Artificial Sequence

<220>
<223> Synthetic probe

<400> 1064
actgagcgca cacgaaacac tgct                                              24

<210> 1065
<211> 22
<212> DNA
<213> Artificial Sequence

<220>
<223> Synthetic probe

<400> 1065
cagcccccca actgacctca tc                                                22

<210> 1066
<211> 24
<212> DNA
<213> Artificial Sequence

<220>
<223> Synthetic probe

<400> 1066
caggctcagc aagctgaaca cctg                                              24

<210> 1067
<211> 24
<212> DNA
<213> Artificial Sequence

<220>
<223> Synthetic probe

<400> 1067
ttactccagg ggacaagcct tcca                                              24

<210> 1068
<211> 24
<212> DNA
<213> Artificial Sequence

<220>
<223> Synthetic probe

<400> 1068
ccttcagggc ctgcactttc aact                                              24

<210> 1069
<211> 24
<212> DNA
<213> Artificial Sequence

<220>
<223> Synthetic probe

<400> 1069
ctgtgtttag gcactcccct tgcg                                              24

<210> 1070
<211> 24
<212> DNA
<213> Artificial Sequence

<220>
<223> Synthetic probe

<400> 1070
ttcttcttct gttcctcgct ccgg                                              24

<210> 1071
<211> 25
<212> DNA
<213> Artificial Sequence

<220>
<223> Synthetic probe

<400> 1071
catgttcttc acaatcgctg catcc                                             25

<210> 1072
<211> 24
<212> DNA
<213> Artificial Sequence

<220>
<223> Synthetic probe

<400> 1072
acagctcatt gttgtcacgc cgga                                              24

<210> 1073
<211> 24
<212> DNA
<213> Artificial Sequence

<220>
<223> Synthetic probe

<400> 1073
cccgggttgt cttccgtcag atag                                              24

<210> 1074
<211> 20
<212> DNA
<213> Artificial Sequence

<220>
<223> Synthetic probe

<400> 1074
cagccctttg gggaagctgg                                                   20

<210> 1075
<211> 22
<212> DNA
<213> Artificial Sequence

<220>
<223> Synthetic probe

<400> 1075
tttgacaccc cttccccagc ca                                                22

<210> 1076
<211> 30
<212> DNA
<213> Artificial Sequence

<220>
<223> Synthetic probe

<400> 1076
agcaagattt cctccaggtc catcaaaagg                                        30

<210> 1077
<211> 24
<212> DNA
<213> Artificial Sequence

<220>
<223> Synthetic probe

<400> 1077
aaccagctct ctgtgacccc aatt                                              24

<210> 1078
<211> 24
<212> DNA
<213> Artificial Sequence

<220>
<223> Synthetic probe

<400> 1078
ctgggagcat ggcgatggat accc                                              24

<210> 1079
<211> 28
<212> DNA
<213> Artificial Sequence

<220>
<223> Synthetic probe

<400> 1079
cctgtatgct gcaactcatg aacttggc                                          28

<210> 1080
<211> 30
<212> DNA
<213> Artificial Sequence

<220>
<223> Synthetic probe

<400> 1080
aagcaatgtt gatatctgcc tctccctgtg                                        30

<210> 1081
<211> 29
<212> DNA
<213> Artificial Sequence

<220>
<223> Synthetic probe

<400> 1081
tcatcaaacc atggttcatc accaatatc                                         29

<210> 1082
<211> 22
<212> DNA
<213> Artificial Sequence

<220>
<223> Synthetic probe

<400> 1082
cgagggcaac cctgatcgtc ca                                                22

<210> 1083
<211> 29
<212> DNA
<213> Artificial Sequence

<220>
<223> Synthetic probe

<400> 1083
acctgatacg tcttggtctt catcgccat                                         29

<210> 1084
<211> 26
<212> DNA
<213> Artificial Sequence

<220>
<223> Synthetic probe

<400> 1084
tctgtcctgg ctggagtcgc tttcat                                            26

<210> 1085
<211> 24
<212> DNA
<213> Artificial Sequence

<220>
<223> Synthetic probe

<400> 1085
tgaactccgc agctagcatc caaa                                              24

<210> 1086
<211> 29
<212> DNA
<213> Artificial Sequence

<220>
<223> Synthetic probe

<400> 1086
caagaagatt tacttcgtcg attcccaga                                         29

<210> 1087
<211> 27

<212> DNA
<213> Artificial Sequence

<220>
<223> Synthetic probe

<400> 1087
tcagtcaaca tcaccctcct aggatga                                          27

<210> 1088
<211> 23
<212> DNA
<213> Artificial Sequence

<220>
<223> Synthetic probe

<400> 1088
tcaccctgga gatcagctcc cga                                              23

<210> 1089
<211> 24
<212> DNA
<213> Artificial Sequence

<220>
<223> Synthetic probe

<400> 1089
cagcattgtc tgtcctccct ggca                                             24

<210> 1090
<211> 26
<212> DNA
<213> Artificial Sequence

<220>
<223> Synthetic probe

<400> 1090
tggagtgctg taaacatacc ctccca                                           26

<210> 1091
<211> 28
<212> DNA
<213> Artificial Sequence

<220>
<223> Synthetic probe

<400> 1091
cgggctgttc cctttgagaa ccttaaca                                         28

<210> 1092
<211> 24
<212> DNA
<213> Artificial Sequence

<220>
<223> Synthetic probe

<400> 1092
atgaccaccc cggctcgtat gtca                                             24

<210> 1093
<211> 28
<212> DNA
<213> Artificial Sequence

<220>
<223> Synthetic probe

<400> 1093
tccccacagt agacacatat gatggccg                                          28

<210> 1094
<211> 29
<212> DNA
<213> Artificial Sequence

<220>
<223> Synthetic probe

<400> 1094
ctcagagcct ctctggttct ttcaatcgg                                         29

<210> 1095
<211> 22
<212> DNA
<213> Artificial Sequence

<220>
<223> Synthetic probe

<400> 1095
ctcggtgttg gccatgctcc ag                                                22

<210> 1096
<211> 24
<212> DNA
<213> Artificial Sequence

<220>
<223> Synthetic probe

<400> 1096
atcttctcag acgtcccgag ccag                                              24

<210> 1097
<211> 28
<212> DNA
<213> Artificial Sequence

<220>
<223> Synthetic probe

<400> 1097
tgtccttacc tgtgggagct gtaaggtc                                          28

<210> 1098
<211> 24
<212> DNA
<213> Artificial Sequence

<220>
<223> Synthetic probe

<400> 1098
tcctttggta tcagacccga agcg                                              24

<210> 1099
<211> 29
<212> DNA
<213> Artificial Sequence

<220>
<223> Synthetic probe

<400> 1099
cctttccagc tttacagtga attgctgca                                         29

<210> 1100
<211> 29
<212> DNA
<213> Artificial Sequence

<220>
<223> Synthetic probe

<400> 1100
ccaagagaaa cgagatttaa aaacccacc                                         29

<210> 1101
<211> 24
<212> DNA
<213> Artificial Sequence

<220>
<223> Synthetic probe

<400> 1101
ctttccaacc cctggggaag acat                                              24

<210> 1102
<211> 24
<212> DNA
<213> Artificial Sequence

<220>
<223> Synthetic probe

<400> 1102
cctcccgaag ttgcttgaaa gcac                                              24

<210> 1103
<211> 24
<212> DNA
<213> Artificial Sequence

<220>
<223> Synthetic probe

<400> 1103
cattcgcttc ttcctccact tggc                                              24

<210> 1104
<211> 25
<212> DNA
<213> Artificial Sequence

<220>
<223> Synthetic probe

<400> 1104
tctccacaga caaggccagg actcg                                          25

<210> 1105
<211> 24
<212> DNA
<213> Artificial Sequence

<220>
<223> Synthetic probe

<400> 1105
atcaccaaca gcatgacctt tgcg                                           24

<210> 1106
<211> 24
<212> DNA
<213> Artificial Sequence

<220>
<223> Synthetic probe

<400> 1106
aggacagtgg agcagccaac acac                                           24

<210> 1107
<211> 25
<212> DNA
<213> Artificial Sequence

<220>
<223> Synthetic probe

<400> 1107
cattggaatt gccattagtc ccagc                                          25

<210> 1108
<211> 25
<212> DNA
<213> Artificial Sequence

<220>
<223> Synthetic probe

<400> 1108
ccagcacagc cagttaaaag atgca                                          25

<210> 1109
<211> 24
<212> DNA
<213> Artificial Sequence

<220>
<223> Synthetic probe

<400> 1109
ttggatctgc ttgctgtcca aacc                                           24

<210> 1110

<211> 24
<212> DNA
<213> Artificial Sequence

<220>
<223> Synthetic probe

<400> 1110
cacgccatgg aaaccatgat gttt                                              24

<210> 1111
<211> 27
<212> DNA
<213> Artificial Sequence

<220>
<223> Synthetic probe

<400> 1111
cacaagtact cctgccaaga gggcgac                                           27

<210> 1112
<211> 24
<212> DNA
<213> Artificial Sequence

<220>
<223> Synthetic probe

<400> 1112
atcacatcca gggccttctc caga                                              24

<210> 1113
<211> 28
<212> DNA
<213> Artificial Sequence

<220>
<223> Synthetic probe

<400> 1113
ttccccaact tccttagtgc ctgtgaca                                          28

<210> 1114
<211> 24
<212> DNA
<213> Artificial Sequence

<220>
<223> Synthetic probe

<400> 1114
catgccgtct acaggatgga cctg                                              24

<210> 1115
<211> 23
<212> DNA
<213> Artificial Sequence

<220>
<223> Synthetic probe

<400> 1115

cccggggcct gttatgtcaa act                                              23

<210> 1116
<211> 24
<212> DNA
<213> Artificial Sequence

<220>
<223> Synthetic probe

<400> 1116
ccggagggaa ccctgactac agaa                                             24

<210> 1117
<211> 24
<212> DNA
<213> Artificial Sequence

<220>
<223> Synthetic probe

<400> 1117
aggataagac cacagcacag gcgc                                             24

<210> 1118
<211> 23
<212> DNA
<213> Artificial Sequence

<220>
<223> Synthetic probe

<400> 1118
ttgctcaagg acctggacgc caa                                              23

<210> 1119
<211> 24
<212> DNA
<213> Artificial Sequence

<220>
<223> Synthetic probe

<400> 1119
ctgtccacca aatgcacgct gata                                             24

<210> 1120
<211> 22
<212> DNA
<213> Artificial Sequence

<220>
<223> Synthetic probe

<400> 1120
ctccccacag cgcatcgagg aa                                               22

<210> 1121
<211> 24
<212> DNA
<213> Artificial Sequence

<220>

<223> Synthetic probe

<400> 1121
cagcaccgat ttcttcaggt ccct                                              24

<210> 1122
<211> 23
<212> DNA
<213> Artificial Sequence

<220>
<223> Synthetic probe

<400> 1122
cctgacttca ggtcaaggga tgg                                               23

<210> 1123
<211> 24
<212> DNA
<213> Artificial Sequence

<220>
<223> Synthetic probe

<400> 1123
ccaatctctg cctcagttct gcca                                              24

<210> 1124
<211> 20
<212> DNA
<213> Artificial Sequence

<220>
<223> Synthetic probe

<400> 1124
cctgcggctt tcggatccca                                                   20

<210> 1125
<211> 25
<212> DNA
<213> Artificial Sequence

<220>
<223> Synthetic probe

<400> 1125
tggccatcca tctcacagaa attgg                                             25

<210> 1126
<211> 25
<212> DNA
<213> Artificial Sequence

<220>
<223> Synthetic probe

<400> 1126
tctggattag agtcctgcag ctcgc                                             25

<210> 1127
<211> 24
<212> DNA

<213> Artificial Sequence

<220>
<223> Synthetic probe

<400> 1127
ctctgtggca ccctggacta cctg                                              24

<210> 1128
<211> 23
<212> DNA
<213> Artificial Sequence

<220>
<223> Synthetic probe

<400> 1128
cacgttctct gccccgtttc ttg                                               23

<210> 1129
<211> 25
<212> DNA
<213> Artificial Sequence

<220>
<223> Synthetic probe

<400> 1129
atcctcctga agccctttc gcagc                                              25

<210> 1130
<211> 28
<212> DNA
<213> Artificial Sequence

<220>
<223> Synthetic probe

<400> 1130
tgccttcttc ctccctcact tctcacct                                          28

<210> 1131
<211> 30
<212> DNA
<213> Artificial Sequence

<220>
<223> Synthetic probe

<400> 1131
ccataggaga atgcttccca catcaacact                                        30

<210> 1132
<211> 24
<212> DNA
<213> Artificial Sequence

<220>
<223> Synthetic probe

<400> 1132
cccatagtcc tcagccgcct tcag                                              24

<210> 1133
<211> 24
<212> DNA
<213> Artificial Sequence

<220>
<223> Synthetic probe

<400> 1133
cttccagcgg caatgtaagc aaca                                              24

<210> 1134
<211> 28
<212> DNA
<213> Artificial Sequence

<220>
<223> Synthetic probe

<400> 1134
agggatctga accaatacag agcagaca                                          28

<210> 1135
<211> 21
<212> DNA
<213> Artificial Sequence

<220>
<223> Synthetic probe

<400> 1135
tcctgagccc gaggaagtcc c                                                 21

<210> 1136
<211> 21
<212> DNA
<213> Artificial Sequence

<220>
<223> Synthetic probe

<400> 1136
cggccagatg agcacattgc c                                                 21

<210> 1137
<211> 24
<212> DNA
<213> Artificial Sequence

<220>
<223> Synthetic probe

<400> 1137
ttctgggctc ctgattgctc aagc                                              24

<210> 1138
<211> 24
<212> DNA
<213> Artificial Sequence

<220>
<223> Synthetic probe

<400> 1138
ccaagaacga gtgtctctgg accg                                              24

<210> 1139
<211> 24
<212> DNA
<213> Artificial Sequence

<220>
<223> Synthetic probe

<400> 1139
tgttcctcac tgagcctgga agca                                              24

<210> 1140
<211> 24
<212> DNA
<213> Artificial Sequence

<220>
<223> Synthetic probe

<400> 1140
agggcctaat gcacgcacta aagc                                              24

<210> 1141
<211> 24
<212> DNA
<213> Artificial Sequence

<220>
<223> Synthetic probe

<400> 1141
tccaccatcg ctttctctgc tctg                                              24

<210> 1142
<211> 21
<212> DNA
<213> Artificial Sequence

<220>
<223> Synthetic probe

<400> 1142
ccacgctgcc ctcggacaag c                                                 21

<210> 1143
<211> 23
<212> DNA
<213> Artificial Sequence

<220>
<223> Synthetic probe

<400> 1143
aattaacagc cacccctcag gcg                                               23

<210> 1144
<211> 26
<212> DNA
<213> Artificial Sequence

<220>
<223> Synthetic probe

<400> 1144
caggcacaca caggtgggac acaaat                                          26

<210> 1145
<211> 22
<212> DNA
<213> Artificial Sequence

<220>
<223> Synthetic probe

<400> 1145
atttcacgca tctggcgttc ca                                              22

<210> 1146
<211> 24
<212> DNA
<213> Artificial Sequence

<220>
<223> Synthetic probe

<400> 1146
tggacactgt ggaccctccc tacc                                            24

<210> 1147
<211> 24
<212> DNA
<213> Artificial Sequence

<220>
<223> Synthetic probe

<400> 1147
ccagaacaga tgcgagcagt ccat                                            24

<210> 1148
<211> 20
<212> DNA
<213> Artificial Sequence

<220>
<223> Synthetic probe

<400> 1148
cgggctgcat cagcacacgc                                                 20

<210> 1149
<211> 24
<212> DNA
<213> Artificial Sequence

<220>
<223> Synthetic probe

<400> 1149
ttgatgcctg tcttcgcgcc ttct                                            24

<210> 1150
<211> 24

<212> DNA
<213> Artificial Sequence

<220>
<223> Synthetic probe

<400> 1150
ttccgtaaga ggcctggtgc tctc                                              24

<210> 1151
<211> 27
<212> DNA
<213> Artificial Sequence

<220>
<223> Synthetic probe

<400> 1151
ctgctgttta ccttggcgag gcctttc                                           27

<210> 1152
<211> 24
<212> DNA
<213> Artificial Sequence

<220>
<223> Synthetic probe

<400> 1152
cccctccttc tcctgcttca gctt                                              24

<210> 1153
<211> 78
<212> DNA
<213> Artificial Sequence

<220>
<223> Synthetic oligonucleotide

<400> 1153
cgttccgatc ctctatactg catcccaggc atgcctacag caccctgatg tcgcagccta 60
taaggccaac agggacct                                                     78

<210> 1154
<211> 66
<212> DNA
<213> Artificial Sequence

<220>
<223> Synthetic oligonucleotide

<400> 1154
gtgtggcagg tggacactaa ggaggaggtc tggtcctttg aagcgggaga cctggagtgg 60
atggag                                                                  66

<210> 1155
<211> 77
<212> DNA
<213> Artificial Sequence

<220>
<223> Synthetic oligonucleotide

<400> 1155
aaacaccact ggagcattga ctaccaggct cgccaatgat gctgctcaag ttaaaggggc 60
tataggttcc aggcttg 77

<210> 1156
<211> 68
<212> DNA
<213> Artificial Sequence

<220>
<223> Synthetic oligonucleotide

<400> 1156
accagtgcca caatgcagtg gctggacatt cggctacagc tcatgggggc ggcagtggtc 60
agcgctat 68

<210> 1157
<211> 76
<212> DNA
<213> Artificial Sequence

<220>
<223> Synthetic oligonucleotide

<400> 1157
tgcagactgt accatgctga ccattgccca tcgcctgcac acggttctag gctccgatag 60
gattatggtg ctggcc 76

<210> 1158
<211> 67
<212> DNA
<213> Artificial Sequence

<220>
<223> Synthetic oligonucleotide

<400> 1158
acacgtctgt caccatggaa gctctgctct acaagcccat tgaccgggtc actcggagca 60
ccctagt 67

<210> 1159
<211> 66
<212> DNA
<213> Artificial Sequence

<220>
<223> Synthetic oligonucleotide

<400> 1159
atccgcattg aagacccacc ccgcagaaag cacatggtat tcctgggtgg tgcagttcta 60
gcggat 66

<210> 1160
<211> 74
<212> DNA
<213> Artificial Sequence

<220>
<223> Synthetic oligonucleotide

<400> 1160
gactgtctcg tttccctggt gacctctgtc accaatgtgg acctgccccc taaagagtca 60
agcatctaag ccca 74

<210> 1161
<211> 74
<212> DNA
<213> Artificial Sequence

<220>
<223> Synthetic oligonucleotide

<400> 1161
tcaaaagtac ggacacctcc tgtcagatgg cgggactaag agatacctac aaggattcca 60
tcaaagcatt tgca 74

<210> 1162
<211> 66
<212> DNA
<213> Artificial Sequence

<220>
<223> Synthetic oligonucleotide

<400> 1162
gagcatgcgt ctactgcctc actgacactc atctgagccc tcccatgaca tggagaccgt 60
gaccag 66

<210> 1163
<211> 73
<212> DNA
<213> Artificial Sequence

<220>
<223> Synthetic oligonucleotide

<400> 1163
gaagtgccag gaggcgatta atgctacttg caaaggcgtg tcctactgca caggtaatag 60
cagtgagtgc ccg 73

<210> 1164
<211> 62
<212> DNA
<213> Artificial Sequence

<220>
<223> Synthetic oligonucleotide

<400> 1164
caaggcccca tctgaatcag ctgcgctgga tggacaccgc cttgcactgt tggattctgg 60
gt 62

<210> 1165
<211> 72
<212> DNA
<213> Artificial Sequence

<220>
<223> Synthetic oligonucleotide

<400> 1165
gcgagttcaa agtgttcgag gccaaggtga ttgatggcac cctgtgtggg ccagaaacac 60
tggccatctg tg 72

<210> 1166
<211> 75

<212> DNA
<213> Artificial Sequence

<220>
<223> Synthetic oligonucleotide

<400> 1166
taagccacaa gcacacgggg ctccagcccc cccgagtgga agtgctcccc actttcttta 60
ggatttaggc gccca 75

<210> 1167
<211> 78
<212> DNA
<213> Artificial Sequence

<220>
<223> Synthetic oligonucleotide

<400> 1167
acgagatgtc ctacggcttg aacatcgaga tgcacaaaca ggctgagatc gtcaaaaggc 60
tgaacgggat ttgtgccc 78

<210> 1168
<211> 70
<212> DNA
<213> Artificial Sequence

<220>
<223> Synthetic oligonucleotide

<400> 1168
agccaacatg tgactaattg gaagaagagc aaagggtggt gacgtgttga tgaggcagat 60
ggagatcaga 70

<210> 1169
<211> 78
<212> DNA
<213> Artificial Sequence

<220>
<223> Synthetic oligonucleotide

<400> 1169
   ctgcatgtga ttgaataaga aacaagaaag tgaccacacc aaagcctccc tggctggtgt 60
   acagggatca ggtccaca 78

<210> 1170
<211> 67
<212> DNA
<213> Artificial Sequence

<220>
<223> Synthetic oligonucleotide

<400> 1170
gtggaaacgg agctcttccc ctgcctcagg cactttggac tgaggttcta tgccttcaac 60
cctctgg 67

<210> 1171
<211> 75
<212> DNA
<213> Artificial Sequence

<220>
<223> Synthetic oligonucleotide

<400> 1171
ttgtctctgc cttggactat ctacattccg gaaagattgt gtaccgtgat ctcaagttgg 60
agaatctaat gctgg 75

<210> 1172
<211> 66
<212> DNA
<213> Artificial Sequence

<220>
<223> Synthetic oligonucleotide

<400> 1172
gaggaatatg gaatccaagg gggccagttc ctgccgtctg ctcttctgcc tcttgatctc 60
cgccac 66

<210> 1173
<211> 68
<212> DNA
<213> Artificial Sequence

<220>
<223> Synthetic oligonucleotide

<400> 1173
ggacagggta agaccgtgat ccaagcggag attgacgctg cagcggaact catcgacttc 60
ttccggtt 68

<210> 1174
<211> 69
<212> DNA
<213> Artificial Sequence

<220>
<223> Synthetic oligonucleotide

<400> 1174
ccgtgaaagc tgctctgtaa aagctgacac agccctccca agtgagcagg actgttcttc 60
ccactgcaa 69

<210> 1175
<211> 71
<212> DNA
<213> Artificial Sequence

<220>
<223> Synthetic oligonucleotide

<400> 1175
caagacacta agggcgacta ccagaaagcg ctgctgtacc tgtgtggtgg agatgactga 60
agcccgacac g 71

<210> 1176
<211> 81
<212> DNA
<213> Artificial Sequence

<220>
<223> Synthetic oligonucleotide

<400> 1176
gactgcaaag atggaaacga ccttctatga cgatgccctc aacgcctcgt tcctcccgtc 60
cgagagcgga ccttatggct a 81

<210> 1177
<211> 68
<212> DNA
<213> Artificial Sequence

<220>
<223> Synthetic oligonucleotide

<400> 1177
gatgaagcct ttcgcaagtt cctgaagggc ctggcttccc gaaagcccct tgtgctgtgt 60
ggagacct 68

<210> 1178
<211> 67
<212> DNA
<213> Artificial Sequence

<220>
<223> Synthetic oligonucleotide

<400> 1178
gtttatgcca tcggcaccgt actggatcct ggccaccgac tatgagaact atgccctcgt 60
gtattcc 67

<210> 1179
<211> 64
<212> DNA
<213> Artificial Sequence

<220>
<223> Synthetic oligonucleotide

<400> 1179
cagtagagat ccccgcaact cgcttgtcct tgggtcaccc tgcattccat agccatgtgc 60
ttgt 64

<210> 1180
<211> 67
<212> DNA
<213> Artificial Sequence

<220>
<223> Synthetic oligonucleotide

<400> 1180
atcagagatt accgcgtcgt ggtagtcggc accgctggtg tggggaaaag tacgctgctg 60
cacaagt 67

<210> 1181
<211> 68
<212> DNA
<213> Artificial Sequence

<220>
<223> Synthetic oligonucleotide

<400> 1181
gactgggtca gtgatggcaa caggatggcc aaggatggct ctagaacact ctgtccatgc 60
gtcactcc 68

<210> 1182
<211> 79
<212> DNA
<213> Artificial Sequence

<220>
<223> Synthetic oligonucleotide

<400> 1182
tccctgagaa cgaaaccact tttgcaagaa tcttgcagca gcatggctat gcaaccggcc 60
tcataggaaa atggcacca 79

<210> 1183
<211> 67
<212> DNA
<213> Artificial Sequence

<220>
<223> Synthetic oligonucleotide

<400> 1183
agctgcagaa gagctgcaca tttgacgagc agcgaacagc cacgatcatg gaggagttgg 60
cagatgc 67

<210> 1184
<211> 66
<212> DNA
<213> Artificial Sequence

<220>
<223> Synthetic oligonucleotide

<400> 1184
cagcagatgt ggatcagcaa gcaggagtat gacgagtccg gcccctccat cgtccaccgc 60
aaatgc 66

<210> 1185
<211> 80
<212> DNA
<213> Artificial Sequence

<220>
<223> Synthetic oligonucleotide

<400> 1185
ggctcttgtg cgtactgtcc ttcgggctgg tgacagggaa gacatcactg agcctgccat 60
ctgtgctctt cgtcatctga 80

<210> 1186
<211> 73
<212> DNA
<213> Artificial Sequence

<220>
<223> Synthetic oligonucleotide

<400> 1186
gggtcaggtg cctcgagatc gggcttgggc ccagagcatg ttccagatcc cagagtttga 60
gccgagtgag cag 73

<210> 1187
<211> 81

<212> DNA
<213> Artificial Sequence

<220>
<223> Synthetic oligonucleotide

<400> 1187
cgttgtcagc acttggaata caagatggtt gccgggtcat gttaattggg aaaaagaaca 60
gtccacagga agaggttgaa c 81

<210> 1188
<211> 76
<212> DNA
<213> Artificial Sequence

<220>
<223> Synthetic oligonucleotide

<400> 1188
cctacggccg ctactacggg cctgggggtg gagatgtgcc ggtacaccca cctccaccct 60
tatatcctct tcgccc 76

<210> 1189
<211> 72
<212> DNA
<213> Artificial Sequence

<220>
<223> Synthetic oligonucleotide

<400> 1189
gactcctcag ggcagacttt cttcccagcc tgcagacaac tggcctccag aaaccattga 60
gtagtgcctt cg 72

<210> 1190
<211> 70
<212> DNA
<213> Artificial Sequence

<220>
<223> Synthetic oligonucleotide

<400> 1190
ccgccgtgga cacagactcc ccccgagagg tctttttccg agtggcagct gacatgtttt 60
ctgacggcaa 70

<210> 1191
<211> 83
<212> DNA
<213> Artificial Sequence

<220>
<223> Synthetic oligonucleotide

<400> 1191
cctggagggt cctgtacaat ctcatcatgg gactcctgcc cttacccagg ggccacagag 60
cccccgagat ggagcccaat tag 83

<210> 1192
<211> 65
<212> DNA
<213> Artificial Sequence

<220>
<223> Synthetic oligonucleotide

<400> 1192
actgacaaga ccagcagcat ccagtcacga cccctgccct caccccctaa gttcacctcc 60
cagga 65

<210> 1193
<211> 75
<212> DNA
<213> Artificial Sequence

<220>
<223> Synthetic oligonucleotide

<400> 1193
tgacttccta gttcgtgact ctctgtccag ccctgggaac tttgtcctga cctgtcagtg 60
gaagaacctc gctca 75

<210> 1194
<211> 73
<212> DNA
<213> Artificial Sequence

<220>
<223> Synthetic oligonucleotide

<400> 1194
ccccgagaca acggagataa gtgctgttgc ggatgccaac ggaaagaatc ttgggaaaga 60
ggccaaaccc gag 73

<210> 1195
<211> 73
<212> DNA
<213> Artificial Sequence

<220>
<223> Synthetic oligonucleotide

<400> 1195
cagatggacc tagtacccac tgagatttcc acgccgaagg acagcgatgg gaaaaatgcc 60
cttaaatcat agg 73

<210> 1196
<211> 73
<212> DNA
<213> Artificial Sequence

<220>
<223> Synthetic oligonucleotide

<400> 1196
aacccacccc tgtcttggag ctccgggtag ctctcaaact cgaggctgcg cacccccttt 60
cccgtcagct gag 73

<210> 1197
<211> 66
<212> DNA
<213> Artificial Sequence

<220>
<223> Synthetic oligonucleotide

<400> 1197
gagctccgca aggatgactt caagggtctc cagcacctct acgccctcgt cctggtgaac 60
aacaag 66

<210> 1198
<211> 70
<212> DNA
<213> Artificial Sequence

<220>
<223> Synthetic oligonucleotide

<400> 1198
attcctatgg ctctgcaatt gtcaccggtt aactgtggcc tgtgcccagg aagagccatt 60
cactcctgcc 70

<210> 1199
<211> 68
<212> DNA
<213> Artificial Sequence

<220>
<223> Synthetic oligonucleotide

<400> 1199
ctggacggag tagctccaag agctctcact gtgacagccc acctcgctcg cagacaccac 60
aagatacc 68

<210> 1200
<211> 66
<212> DNA
<213> Artificial Sequence

<220>
<223> Synthetic oligonucleotide

<400> 1200
aattttatga gggccacggg tctgtgttcg actcagcctc agggacgact ctgacctctt 60
ggccac 66

<210> 1201
<211> 63
<212> DNA
<213> Artificial Sequence

<220>
<223> Synthetic oligonucleotide

<400> 1201
gttgggacac agttggtctg cagtcggccc aggacggtct actcagcaca actgactgct 60
tca 63

<210> 1202
<211> 68
<212> DNA
<213> Artificial Sequence

<220>
<223> Synthetic oligonucleotide

<400> 1202
ccgaggttaa tccagcacgt atggggccaa gtgtaggctc ccagcaggaa ctgagagcgc 60
catgtctt 68

<210> 1203
<211> 82
<212> DNA
<213> Artificial Sequence

<220>
<223> Synthetic oligonucleotide

<400> 1203
tcaacagaag gctgaaccac tagaaagact acagtcccag caccgacaat tccaagctcg 60
agtgtctcgg caaactctgt tg 82

<210> 1204
<211> 73
<212> DNA
<213> Artificial Sequence

<220>
<223> Synthetic oligonucleotide

<400> 1204
ctgaagcaga tggttcatca tttcctgggc tgttaaacaa agcgaggtta aggttagact 60
cttgggaatc agc 73

<210> 1205
<211> 75
<212> DNA
<213> Artificial Sequence

<220>
<223> Synthetic oligonucleotide

<400> 1205
gaggcaactg cttatggctt aattaagtca gatgcggcca tgactgtcgc tgtaaagatg 60
ctcaagccga gtgcc 75

<210> 1206
<211> 67
<212> DNA
<213> Artificial Sequence

<220>
<223> Synthetic oligonucleotide

<400> 1206
caagagcaga gccaccgtag ccggagtcct agcctcccaa attcggaaat ccaatccaac 60
ggtctca 67

<210> 1207
<211> 67
<212> DNA
<213> Artificial Sequence

<220>
<223> Synthetic oligonucleotide

<400> 1207
gtgactgcac aggactctgg gttcctgctc tgttctgggg tccaaacctt ggtctccctt 60
tggtcct 67

<210> 1208
<211> 65

<212> DNA
<213> Artificial Sequence

<220>
<223> Synthetic oligonucleotide

<400> 1208
tcagctgtga gctgcggata ccgcccggca atgggacctg ctcttaacct caaacctagg 60
accgt 65

<210> 1209
<211> 70
<212> DNA
<213> Artificial Sequence

<220>
<223> Synthetic oligonucleotide

<400> 1209
gcggtatcag gaatttcaac ctagagaacc gagcggaacg ggaaatcagc aagatgaagc 60
cctctgtcgc 70

<210> 1210
<211> 67
<212> DNA
<213> Artificial Sequence

<220>
<223> Synthetic oligonucleotide

<400> 1210
ctacgagtca gcccatccat ccatggctac cacttcgaca cagcctctcg taagaaagcc 60
gtgggca 67

<210> 1211
<211> 67
<212> DNA
<213> Artificial Sequence

<220>
<223> Synthetic oligonucleotide

<400> 1211
tgatgctgca gagaacttcc agaaagcaca ccgctggcag gacaacatca aggaggaaga 60
catcgtg 67

<210> 1212
<211> 78
<212> DNA
<213> Artificial Sequence

<220>
<223> Synthetic oligonucleotide

<400> 1212
atccattcga tctcaccaag gtttggcctc acaaggacta ccctctcatc ccagttggta 60
aactggtctt aaaccgga 78

<210> 1213
<211> 74
<212> DNA
<213> Artificial Sequence

<220>
<223> Synthetic oligonucleotide

<400> 1213
gtggctcaac attgtgttcc catttcagct gatcagtggg cctccaagga ggggctgtaa 60
aatggaggcc attg 74

<210> 1214
<211> 78
<212> DNA
<213> Artificial Sequence

<220>
<223> Synthetic oligonucleotide

<400> 1214
aggggatggt ctctgtcatt tctctttgta cataatacat tcacctccct gcctcctctc 60
ctttctaccc accccttt 78

<210> 1215
<211> 78
<212> DNA
<213> Artificial Sequence

<220>
<223> Synthetic oligonucleotide

<400> 1215
gaacgcatca tccagagact gcagaggacc tcagccaaga tgaagcgccg cagcagttaa 60
cctatgaccg tgcagagg 78

<210> 1216
<211> 77
<212> DNA
<213> Artificial Sequence

<220>
<223> Synthetic oligonucleotide

<400> 1216
agcagacagt ggtcagtcct ttcttggctc tgctgacact cgagcccaca ttccgtcacc 60
tgctcagaat catgcag 77

<210> 1217
<211> 65
<212> DNA
<213> Artificial Sequence

<220>
<223> Synthetic oligonucleotide

<400> 1217
aggttctgag ctctggcttt gccttggctt tgccagggct ctgtgaccag gaaggaagtc 60
agcat 65

<210> 1218
<211> 84
<212> DNA
<213> Artificial Sequence

<220>
<223> Synthetic oligonucleotide

<400> 1218
ttcaggttgt tgcaggagac catgtacatg actgtctcca ttattgatcg gttcatgcag 60
aataattgtg tgcccaagaa gatg 84

<210> 1219
<211> 76
<212> DNA
<213> Artificial Sequence

<220>
<223> Synthetic oligonucleotide

<400> 1219
cctctgtgct acagattata cctttgccat gtacccgcca tccatgatcg ccacgggcag 60
cattggggct gcagtg 76

<210> 1220
<211> 85
<212> DNA
<213> Artificial Sequence

<220>
<223> Synthetic oligonucleotide

<400> 1220
ggtcaccaag aaacatcagt atgaaattag gaattgttgg ccacctgtat tatctggggg 60
gatcagtcct tgcattatca ttgaa 85

<210> 1221
<211> 67
<212> DNA
<213> Artificial Sequence

<220>
<223> Synthetic oligonucleotide

<400> 1221
cagactgaat gggggtgggg ggggcgcctt aggtacttat tccagatgcc ttctccagac 60
aaaccag 67

<210> 1222
<211> 64
<212> DNA
<213> Artificial Sequence

<220>
<223> Synthetic oligonucleotide

<400> 1222
ggatgacatg cactcagctc ttggctccac tgggatggga ggagaggaca agggaaatgt 60
cagg 64

<210> 1223
<211> 78
<212> DNA
<213> Artificial Sequence

<220>
<223> Synthetic oligonucleotide

<400> 1223
ggagtggaag gaactggaaa cattattccg tatacgcacc attcggtcat ttgagggaat 60
tcgtagatac gcccatga 78

<210> 1224
<211> 77
<212> DNA
<213> Artificial Sequence

<220>
<223> Synthetic oligonucleotide

<400> 1224
tccaactaat gccaccacca aggcggctgg tggtgccctg cagtcaacag ccagtctctt 60
cgtggtctca ctctctc 77

<210> 1225
<211> 67
<212> DNA
<213> Artificial Sequence

<220>
<223> Synthetic oligonucleotide

<400> 1225
gtgctggagt cgggactaac ccaggtccct tgtcccaagt tccactgctg cctcttgaat 60
gcaggga 67

<210> 1226
<211> 90
<212> DNA
<213> Artificial Sequence

<220>
<223> Synthetic oligonucleotide

<400> 1226
atcaccgaca gcacagacag aatccctgct accaatatgg actccagtca tagtacaacg 60
cttcagccta ctgcaaatcc aaacacaggt 90

<210> 1227
<211> 78
<212> DNA
<213> Artificial Sequence

<220>
<223> Synthetic oligonucleotide

<400> 1227
gacgaagaca gtccctggat caccgacagc acagacagaa tccctgctac cagagaccaa 60
gacacattcc accccagt 78

<210> 1228
<211> 78
<212> DNA
<213> Artificial Sequence

<220>
<223> Synthetic oligonucleotide

<400> 1228
ctcataccag ccatccaatg caaggaagga caacaccaag cccagaggac agttcctgga 60
ctgatttctt caacccaa 78

<210> 1229
<211> 74

<212> DNA
<213> Artificial Sequence

<220>
<223> Synthetic oligonucleotide

<400> 1229
tggttcccag ccctgtgtcc acctccaagc ccagattcag attcgagtca tgtacacaac 60
ccagggtgga ggag 74

<210> 1230
<211> 84
<212> DNA
<213> Artificial Sequence

<220>
<223> Synthetic oligonucleotide

<400> 1230
gtgcaggctc aggtgaagtg ctgcggctgg gtcagcttct acaactggac agacaacgct 60
gagctcatga atcgccctga ggtc 84

<210> 1231
<211> 68
<212> DNA
<213> Artificial Sequence

<220>
<223> Synthetic oligonucleotide

<400> 1231
tggattggag ttctgggaat gtactggccg tggcactgga caacagtgtg tacctgtgga 60
gtgcaagc 68

<210> 1232
<211> 71
<212> DNA
<213> Artificial Sequence

<220>
<223> Synthetic oligonucleotide

<400> 1232
tcttgctggc tacgcctctt ctgtccctgt tagacgtcct ccgtccatat cagaactgtg 60
ccacaatgca g 71

<210> 1233
<211> 67
<212> DNA
<213> Artificial Sequence

<220>
<223> Synthetic oligonucleotide

<400> 1233
ggtgagcaga agtggcctat atcgctcccc gtcgatgcca gagaacttga acaggccaag 60
actgaag 67

<210> 1234
<211> 77
<212> DNA
<213> Artificial Sequence

<220>
<223> Synthetic oligonucleotide

<400> 1234
gcagtccgct gtgttcaata tgatggcagg agggttgtta gtggagcata tgattttatg 60
gtaaaggtgt gggatcc 77

<210> 1235
<211> 67
<212> DNA
<213> Artificial Sequence

<220>
<223> Synthetic oligonucleotide

<400> 1235
gagctgaaag acgcacactg tcagaggaaa ctggccatgc aggaattcat ggagatcaat 60
gagcggc 67

<210> 1236
<211> 67
<212> DNA
<213> Artificial Sequence

<220>
<223> Synthetic oligonucleotide

<400> 1236
cggagaaggg caccagtaag ctgcccaaga gcctgtcatc cagccccgtg aagaaggcca 60
atgacgg 67

<210> 1237
<211> 70
<212> DNA
<213> Artificial Sequence

<220>
<223> Synthetic oligonucleotide

<400> 1237
gtcggcagaa gcaggacttg taccttctgc ccatagtgat cagcgatggc ggcatcccgc 60
ccatgagtag 70

<210> 1238
<211> 71
<212> DNA
<213> Artificial Sequence

<220>
<223> Synthetic oligonucleotide

<400> 1238
acccatgtac cgtcctcggc cagccaaccc agatgaaatc ggcaacttta taattgagaa 60
cctgaaggcg g 71

<210> 1239
<211> 66
<212> DNA
<213> Artificial Sequence

<220>
<223> Synthetic oligonucleotide

<400> 1239
ccttcccatc agcacagttc gtgaggtggc tttactgagg cgactggagg cttttgagca 60
tcccaa 66

<210> 1240
<211> 67
<212> DNA
<213> Artificial Sequence

<220>
<223> Synthetic oligonucleotide

<400> 1240
aagccctatc cgatgtaccc ggccacaaca tccctggtga acgtcgtgcc caaactcaat 60
gccacag 67

<210> 1241
<211> 70
<212> DNA
<213> Artificial Sequence

<220>
<223> Synthetic oligonucleotide

<400> 1241
tggatctcta ccagcaatgt ggaattatca cccatcatca tccaatcgca gatggaggga 60
ctcctgacat 70

<210> 1242
<211> 71
<212> DNA
<213> Artificial Sequence

<220>
<223> Synthetic oligonucleotide

<400> 1242
acttgcctgt tcagagcact cattccttcc cacccccagt cctgtcctat cactctaatt 60
cggatttgcc a 71

<210> 1243
<211> 66
<212> DNA
<213> Artificial Sequence

<220>
<223> Synthetic oligonucleotide

<400> 1243
ttggttttgc tcggatactt gccaaaatga gactctccgt cggcagctgg gggaagggtc 60
tgagac 66

<210> 1244
<211> 77
<212> DNA
<213> Artificial Sequence

<220>
<223> Synthetic oligonucleotide

<400> 1244
tgacaatcag cacacctgca ttcaccgctc ggaagagggc ctgagctgca tgaataagga 60
tcacggctgt agtcaca 77

<210> 1245
<211> 63
<212> DNA
<213> Artificial Sequence

<220>
<223> Synthetic oligonucleotide

<400> 1245
taaattcact cgtggtgtgg acttcaattg gcaagcccag gccctattgg ccctacaaga 60
ggc 63

<210> 1246
<211> 76
<212> DNA
<213> Artificial Sequence

<220>
<223> Synthetic oligonucleotide

<400> 1246
ctgaaggagc tccaagacct cgctctccaa ggcgccaagg agagggcaca tcagcagaag 60
aaacacagcg gttttg 76

<210> 1247
<211> 69
<212> DNA
<213> Artificial Sequence

<220>
<223> Synthetic oligonucleotide

<400> 1247
ccagaatgca cgctacagga aaacccattc ttctcccagc cgggtgcccc aatacttcag 60
tgcatgggc 69

<210> 1248
<211> 80
<212> DNA
<213> Artificial Sequence

<220>
<223> Synthetic oligonucleotide

<400> 1248
ccaccatagg cagaggcagg ccttcctaca ccctactccc tgtgcctcca gcctcgacta 60
gtccctagca ctcgacgact 80

<210> 1249
<211> 72
<212> DNA
<213> Artificial Sequence

<220>
<223> Synthetic oligonucleotide

<400> 1249
gaggccagtg gtggaaacag gtgtggagct gatgagtctg ccctaccgcc tggtgtttgc 60
tgtggcctcg ga 72

<210> 1250
<211> 76

<212> DNA
<213> Artificial Sequence

<220>
<223> Synthetic oligonucleotide

<400> 1250
aaggaagtgg tccctctgtg gcaagtgatg aagtctccag ctttgcctca gctctcccag 60
acagaaagac tgcgtc 76

<210> 1251
<211> 66
<212> DNA
<213> Artificial Sequence

<220>
<223> Synthetic oligonucleotide

<400> 1251
agaatgggtg tgaaggcgtc tcaaacaggc tttgtggtcc tggtgctgct ccagtgctgc 60
tctgca 66

<210> 1252
<211> 62
<212> DNA
<213> Artificial Sequence

<220>
<223> Synthetic oligonucleotide

<400> 1252
ggctggacgt ggttttgtct gctgcgcccg ctcttcgcgc tctcgtttca ttttctgcag 60
cg 62

<210> 1253
<211> 72
<212> DNA
<213> Artificial Sequence

<220>
<223> Synthetic oligonucleotide

<400> 1253
ggctgctttg ctgcaactgt ccaccccgca cagacaagcc ttactccgcc aagtattctg 60
ctgcccgctc tg 72

<210> 1254
<211> 68
<212> DNA
<213> Artificial Sequence

<220>
<223> Synthetic oligonucleotide

<400> 1254
cggtacttga gcaatgccta cgcccgggaa gaattcgctt ccacctgtcc agatgatgag 60
gagatcga 68

<210> 1255
<211> 76
<212> DNA
<213> Artificial Sequence

<220>
<223> Synthetic oligonucleotide

<400> 1255
ccccaggata cctaccacta cctgcccttc agcctgcccc accggaggcc tcacttcttc 60
tttcccaagt cccgca 76

<210> 1256
<211> 67
<212> DNA
<213> Artificial Sequence

<220>
<223> Synthetic oligonucleotide

<400> 1256
aaatcgcagc ttatcacaag gcactcagtt accgagccac gtcacgccaa caacaggggt 60
accaaca 67

<210> 1257
<211> 68
<212> DNA
<213> Artificial Sequence

<220>
<223> Synthetic oligonucleotide

<400> 1257
gtggccatcc agctgacctt cctgcgcctg atgtccaccg aggcctccca gaacatcacc 60
taccactg 68

<210> 1258
<211> 80
<212> DNA
<213> Artificial Sequence

<220>
<223> Synthetic oligonucleotide

<400> 1258
cagccaagaa ctggtatagg agctccaagg acaagaaaca cgtctggcta ggagaaacta 60
tcaatgctgg cagccagttt 80

<210> 1259
<211> 67
<212> DNA
<213> Artificial Sequence

<220>
<223> Synthetic oligonucleotide

<400> 1259
ccttatcggc tggaacgagt tcatcctgca gcccatccac aacctgctca tgggtgacac 60
caaggag 67

<210> 1260
<211> 81
<212> DNA
<213> Artificial Sequence

<220>
<223> Synthetic oligonucleotide

<400> 1260
cgacagttgc gatgaaagtt ctaatctctt ccctcctcct gttgctgcca ctaatgctga 60
tgtccatggt ctctagcagc c 81

<210> 1261
<211> 72
<212> DNA
<213> Artificial Sequence

<220>
<223> Synthetic oligonucleotide

<400> 1261
atgcccagtg ttcctgactt cgaaacgcta ttctcacagg ttcagctctt catcagcact 60
tgtaatgggg ag 72

<210> 1262
<211> 69
<212> DNA
<213> Artificial Sequence

<220>
<223> Synthetic oligonucleotide

<400> 1262
gatgtgattg aggtgcatgg aaaacatgaa gagcgccagg atgaacatgg tttcatctcc 60
agggagttc 69

<210> 1263
<211> 78
<212> DNA
<213> Artificial Sequence

<220>
<223> Synthetic oligonucleotide

<400> 1263
aagtcctgaa attgcgatca gatattgcag taccgattcc aaaagaccat caggttctaa 60
tcaaggtcca tgcatgtg 78

<210> 1264
<211> 68
<212> DNA
<213> Artificial Sequence

<220>
<223> Synthetic oligonucleotide

<400> 1264
cagcaagaac tgcaacaaca gctttgctga atgctccagc caaggccatg agaggcagtc 60
cgagggat 68

<210> 1265
<211> 74
<212> DNA
<213> Artificial Sequence

<220>
<223> Synthetic oligonucleotide

<400> 1265
tgcagcggct gattgacagt cagatggaga cctcgtgcca aattacattt gagtttgtag 60
accaggaaca gttg 74

<210> 1266
<211> 80
<212> DNA
<213> Artificial Sequence

<220>
<223> Synthetic oligonucleotide

<400> 1266
gagcacaacc aaacctacga gtgcagggcc cacaacagcg tggggagtgg ctcctgggcc 60
ttcataccca tctctgcagg 80

<210> 1267
<211> 67
<212> DNA
<213> Artificial Sequence

<220>
<223> Synthetic oligonucleotide

<400> 1267
taccacaccc agcattcctc ctgatcccag agaaatcgga tctgcgaaca gtggcaccag 60
cctctag 67

<210> 1268
<211> 64
<212> DNA
<213> Artificial Sequence

<220>
<223> Synthetic oligonucleotide

<400> 1268
cctgaacatg aaggagctga agctgctgca gaccatcggg aagggggagt tcggagacgt 60
gatg 64

<210> 1269
<211> 76
<212> DNA
<213> Artificial Sequence

<220>
<223> Synthetic oligonucleotide

<400> 1269
gagttcaagt gccctgacgg cgaggtcatg aagaagaaca tgatgttcat caagacctgt 60
gcctgccatt acaact 76

<210> 1270
<211> 67
<212> DNA
<213> Artificial Sequence

<220>
<223> Synthetic oligonucleotide

<400> 1270
gctcacttcg gctaaaatgc agaaatgcat gctgtcagcg ttggtatttc acattcaatg 60
gagctga 67

<210> 1271
<211> 80

<212> DNA
<213> Artificial Sequence

<220>
<223> Synthetic oligonucleotide

<400> 1271
gtacatgatc ccctgtgaga aggtgtccac cctgcccgcg atcacactga agctgggagg 60
caaaggctac aagctgtccc 80

<210> 1272
<211> 67
<212> DNA
<213> Artificial Sequence

<220>
<223> Synthetic oligonucleotide

<400> 1272
tgtctcactg agcgagcaga atctggtgga ctgttcgcgt cctcaaggca atcagggctg 60
caatggt 67

<210> 1273
<211> 79
<212> DNA
<213> Artificial Sequence

<220>
<223> Synthetic oligonucleotide

<400> 1273
accaggcaat aacctaacag cacccattat aggtgcaata tgggcatata tctccattgt 60
gtcttggctt ggagaacag 79

<210> 1274
<211> 68
<212> DNA
<213> Artificial Sequence

<220>
<223> Synthetic oligonucleotide

<400> 1274
ggagcaaaat cgatgcagtg cttccaagga tggaccacac agaggctgcc tctcccatca 60
cttcccta 68

<210> 1275
<211> 67
<212> DNA
<213> Artificial Sequence

<220>
<223> Synthetic oligonucleotide

<400> 1275
gagctacaga tgcccatgcc gattcttcga aagccatgtt gccagagcca acgtcaagca 60
tctcaaa 67

<210> 1276
<211> 74
<212> DNA
<213> Artificial Sequence

<220>
<223> Synthetic oligonucleotide

<400> 1276
tgcgcccttt cctctgtaca tataccctta agaacgcccc ctccacacac tgccccccag 60
tatatgccgc attg 74

<210> 1277
<211> 72
<212> DNA
<213> Artificial Sequence

<220>
<223> Synthetic oligonucleotide

<400> 1277
tgaccgcttc taccccaatg acttgtgggt ggttgtgttc cagtttcagc acatcatggt 60
tggccttatc ct 72

<210> 1278
<211> 76
<212> DNA
<213> Artificial Sequence

<220>
<223> Synthetic oligonucleotide

<400> 1278
ccggagtgac tctatcacca acatgctgga cacactgatg caagccaaga tgaactcaga 60
taatggcaat gctggc 76

<210> 1279
<211> 70
<212> DNA
<213> Artificial Sequence

<220>
<223> Synthetic oligonucleotide

<400> 1279
tccttatagg tactttcagc catttggctt tgggccccgt ggctgtgcag gaaagtacat 60
cgccatggtg 70

<210> 1280
<211> 71
<212> DNA
<213> Artificial Sequence

<220>
<223> Synthetic oligonucleotide

<400> 1280
ccagctttgt gcctgtcact attcctcatg ccaccactgc caacacctct gtcttgggct 60
accacattcc c 71

<210> 1281
<211> 76
<212> DNA
<213> Artificial Sequence

<220>
<223> Synthetic oligonucleotide

<400> 1281
tgctcattct tgaggagcat taaggtattt cgaaactgcc aagggtgctg gtgcggatgg 60
acactaatgc agccac 76

<210> 1282
<211> 67
<212> DNA
<213> Artificial Sequence

<220>
<223> Synthetic oligonucleotide

<400> 1282
tggtgggtct aggtggtgta actgtcacac tccctcaggc aggaccatgg aacacagcat 60
ctttggt 67

<210> 1283
<211> 75
<212> DNA
<213> Artificial Sequence

<220>
<223> Synthetic oligonucleotide

<400> 1283
aaatgtcctc ctcgactgct ccgcggagtc cgaccgagga gttccagtga tcaagtggaa 60
gaaagatggc attca 75

<210> 1284
<211> 66
<212> DNA
<213> Artificial Sequence

<220>
<223> Synthetic oligonucleotide

<400> 1284
ggtcaccgtt ggtgtcatca cagtgctggt agtggtcatc gtggctgtga tttgcacccg 60
acgctc 66

<210> 1285
<211> 74
<212> DNA
<213> Artificial Sequence

<220>
<223> Synthetic oligonucleotide

<400> 1285
atggagatgt ggtcattcct agtgattatt ttcagatagt gggaggaagc aacttacgga 60
tacttggggt ggtg 74

<210> 1286
<211> 110
<212> DNA
<213> Artificial Sequence

<220>
<223> Synthetic oligonucleotide

<400> 1286
gccgccacaa gactaaggaa tggccacccc gcccaagaga agctgcccgt ctttctcagc 60
cagctctgag gggacccgca tcaagaaaat ctccatcgaa gggaacatcg 110

<210> 1287
<211> 68
<212> DNA
<213> Artificial Sequence

<220>
<223> Synthetic oligonucleotide

<400> 1287
tccaattcca gcatcactgt ggagaaaagc tgtttgtctc cccagcatac tttatcgcct 60
tcactgcc 68

<210> 1288
<211> 68
<212> DNA
<213> Artificial Sequence

<220>
<223> Synthetic oligonucleotide

<400> 1288
gattcagacg aggatgagcc ttgtgccatc agtggcaaat ggactttcca aagggacagc 60
aagaggtg 68

<210> 1289
<211> 67
<212> DNA
<213> Artificial Sequence

<220>
<223> Synthetic oligonucleotide

<400> 1289
cacggaggta taaggcagga gcctacctgg acatccctgc tcagccccgc ggctggacct 60
tccttct 67

<210> 1290
<211> 84
<212> DNA
<213> Artificial Sequence

<220>
<223> Synthetic oligonucleotide

<400> 1290
ctctgagaca gtgcttcgat gactttgcag acttggtgcc ctttgactcc tgggagccgc 60
tcatgaggaa gttgggcctc atgg 84

<210> 1291
<211> 73
<212> DNA
<213> Artificial Sequence

<220>
<223> Synthetic oligonucleotide

<400> 1291
tggcactact gcatgattga catagagaag atcagggcca tgacaatcgc caagctgaaa 60
acaatgcggc agg 73

<210> 1292
<211> 78

<212> DNA
<213> Artificial Sequence

<220>
<223> Synthetic oligonucleotide

<400> 1292
aaatcgctgg gaacaagtgc cgttaattaa ggaaaagttg agccagggcg tgaccctcgt 60
cgtggacaga tacgcatt 78

<210> 1293
<211> 76
<212> DNA
<213> Artificial Sequence

<220>
<223> Synthetic oligonucleotide

<400> 1293
agacatcagc tcctggttca acgaggccat tgacttcata gactccatca agaatgctgg 60
aggaagggtg tttgtc 76

<210> 1294
<211> 68
<212> DNA
<213> Artificial Sequence

<220>
<223> Synthetic oligonucleotide

<400> 1294
tggtgacgat ggaggagctg cgggagatgg actgcagtgt gctcaaaagg ctgatgaacc 60
gggacgag 68

<210> 1295
<211> 75
<212> DNA
<213> Artificial Sequence

<220>
<223> Synthetic oligonucleotide

<400> 1295
actccctcta cccttgagca agggcagggg tccctgagct gttcttctgc cccatactga 60
aggaactgag gcctg 75

<210> 1296
<211> 76
<212> DNA
<213> Artificial Sequence

<220>
<223> Synthetic oligonucleotide

<400> 1296
ctgctggatg accttcctcc cagagtggct caccagaagc cccaacctca acaccagcaa 60
ctggctgtac tgttgg 76

<210> 1297
<211> 73
<212> DNA
<213> Artificial Sequence

<220>
<223> Synthetic oligonucleotide

<400> 1297
tgccacctgg acatcatttg ggtcaacact cccgagcacg ttgttccgta tggacttgga 60
agccctaggt cca 73

<210> 1298
<211> 79
<212> DNA
<213> Artificial Sequence

<220>
<223> Synthetic oligonucleotide

<400> 1298
cgacaaggag tgcgtctact tctgccactt ggacatcatc tgggtgaaca ctcctgaaca 60
gacagctcct tacggcctg 79

<210> 1299
<211> 76
<212> DNA
<213> Artificial Sequence

<220>
<223> Synthetic oligonucleotide

<400> 1299
tttcctcaaa tttgcctcaa gatggaaacc ctttgcctca gggcatcctt ttggctggca 60
ctggttggat gtgtaa 76

<210> 1300
<211> 72
<212> DNA
<213> Artificial Sequence

<220>
<223> Synthetic oligonucleotide

<400> 1300
actgtgaact gcctggtgca gtgtccacat gacaaagggg caggtagcac cctctctcac 60
ccatgctgtg gt 72

<210> 1301
<211> 67
<212> DNA
<213> Artificial Sequence

<220>
<223> Synthetic oligonucleotide

<400> 1301
cgagtggaga ctggtgttct caaacccggt atggtggtca cctttgctcc agtcaacgtt 60
acaacgg 67

<210> 1302
<211> 66
<212> DNA
<213> Artificial Sequence

<220>
<223> Synthetic oligonucleotide

<400> 1302
atggactcca cagagccggc ctacagcgag aagcgctacg acgagatcgt caaggaagtc 60
agcgcc 66

<210> 1303
<211> 74
<212> DNA
<213> Artificial Sequence

<220>
<223> Synthetic oligonucleotide

<400> 1303
ttgaacagag cctgaccaag agggatgagt tcgagtttct ggagaaagca tcaaaactgc 60
gaggaatctc aaca 74

<210> 1304
<211> 76
<212> DNA
<213> Artificial Sequence

<220>
<223> Synthetic oligonucleotide

<400> 1304
gtccccgctg cagatctctg acccgttcgg atcctttcct cactcgccca ccatggacaa 60
ctaccctaag ctggag 76

<210> 1305
<211> 78
<212> DNA
<213> Artificial Sequence

<220>
<223> Synthetic oligonucleotide

<400> 1305
ccatgtggat gaatgaggtg tctcctttcc atacccagtc tcaccttctc cccaccctac 60
ctcacctctt ctcaggca 78

<210> 1306
<211> 66
<212> DNA
<213> Artificial Sequence

<220>
<223> Synthetic oligonucleotide

<400> 1306
ggcggtgaag agtcacagtt tgagatggac atttaaagca ccagccatcg tgtggagcac 60
taccaa 66

<210> 1307
<211> 71
<212> DNA
<213> Artificial Sequence

<220>
<223> Synthetic oligonucleotide

<400> 1307
tcgagggcaa gaagagcaag cacgcgccca gaggcaccca cctgtgggag ttcatccggg 60
acatcctcat c 71

<210> 1308
<211> 75
<212> DNA
<213> Artificial Sequence

<220>
<223> Synthetic oligonucleotide

<400> 1308
gctagtactt tgatgctccc ttgatggggt ccagagagcc tccctgcagc caccagactt 60
ggcctccagc tgttc 75

<210> 1309
<211> 68
<212> DNA
<213> Artificial Sequence

<220>
<223> Synthetic oligonucleotide

<400> 1309
caaggccgtg aacgagaagt cctgcaactg cctcctgctc aaagtcaacc agattggctc 60
cgtgaccg 68

<210> 1310
<211> 75
<212> DNA
<213> Artificial Sequence

<220>
<223> Synthetic oligonucleotide

<400> 1310
agccccagca actacagtct gggatgccaa ggccagccat gatgtcagtg gcccagcatg 60
gtcaaccttt gaaca 75

<210> 1311
<211> 75
<212> DNA
<213> Artificial Sequence

<220>
<223> Synthetic oligonucleotide

<400> 1311
gggccctcca gaacaatgat gggctttatg atcctgactg cgatgagagc gggctcttta 60
aggccaagca gtgca 75

<210> 1312
<211> 72
<212> DNA
<213> Artificial Sequence

<220>
<223> Synthetic oligonucleotide

<400> 1312
cgcctgttca ccaagattga caccattgcg cccgatgaga tcaccgtcag cagcgacttc 60
gaggcacgcc ac 72

<210> 1313
<211> 66

<212> DNA
<213> Artificial Sequence

<220>
<223> Synthetic oligonucleotide

<400> 1313
caaccaggca gctccatcgg cagtgtccat catgcatcag gtgagccgca ccgtggacag 60
cattac 66

<210> 1314
<211> 77
<212> DNA
<213> Artificial Sequence

<220>
<223> Synthetic oligonucleotide

<400> 1314
tgaacggggt atcctcctta gccacggggc ccgtcccatt tgagcctgtc aatgtcacca 60
ctgaccgaga ggtacct 77

<210> 1315
<211> 76
<212> DNA
<213> Artificial Sequence

<220>
<223> Synthetic oligonucleotide

<400> 1315
tggtccatcg ccagttatca catctgtatg cggaacctca aaagagtccc tggtgtgaag 60
caagatcgct agaaca 76

<210> 1316
<211> 86
<212> DNA
<213> Artificial Sequence

<220>
<223> Synthetic oligonucleotide

<400> 1316
tggctcttaa tcagtttcgt tacctgcctc tggagaattt acgcattatt cgtgggacaa 60
aactttatga ggatcgatat gccttg 86

<210> 1317
<211> 67
<212> DNA
<213> Artificial Sequence

<220>
<223> Synthetic oligonucleotide

<400> 1317
gtccaggtgg atgtgaaaga tccccagcag gccctcaagg agctggctaa gatgtgtatc 60
ctggccg 67

<210> 1318
<211> 70
<212> DNA
<213> Artificial Sequence

<220>
<223> Synthetic oligonucleotide

<400> 1318
ccaacactag gctccccacc agccatatgc cttctcatct gggcacttac tactaaagac 60
ctggcggagg 70

<210> 1319
<211> 67
<212> DNA
<213> Artificial Sequence

<220>
<223> Synthetic oligonucleotide

<400> 1319
ggcattgagc ctctctacat caaggcagag ccggccagcc ctgacagtcc aaagggttcc 60
tcggaga 67

<210> 1320
<211> 66
<212> DNA
<213> Artificial Sequence

<220>
<223> Synthetic oligonucleotide

<400> 1320
gtcactccgc caccgtagaa tcgcctacca tttggtgcaa gcaaaaagca atcagcaatt 60
ggacag 66

<210> 1321
<211> 70
<212> DNA
<213> Artificial Sequence

<220>
<223> Synthetic oligonucleotide

<400> 1321
acccccagac cggatcaggc aagctggccc tcatgtcccc ttcacggtgt ttgaggaagt 60
ctgccctaca 70

<210> 1322
<211> 67
<212> DNA
<213> Artificial Sequence

<220>
<223> Synthetic oligonucleotide

<400> 1322
ccagcaccat tgttgaagat ccccagacca agtgtgaata catgctcaac tcgatgccca 60
agagact 67

<210> 1323
<211> 68
<212> DNA
<213> Artificial Sequence

<220>
<223> Synthetic oligonucleotide

<400> 1323
cgtggtgccc ctctatgacc tgctgctgga gatgctggac gcccaccgcc tacatgcgcc 60
cactagcc 68

<210> 1324
<211> 67
<212> DNA
<213> Artificial Sequence

<220>
<223> Synthetic oligonucleotide

<400> 1324
accatgtatc gagaggggcc cccttaccag aggcgaggtt cccttcagct gtggcagttc 60
ctggtca 67

<210> 1325
<211> 78
<212> DNA
<213> Artificial Sequence

<220>
<223> Synthetic oligonucleotide

<400> 1325
tggaaacagc gaaggataca gcctgtgcac atcctgactt ctgtgagctc attgcgcggg 60
actagggagt gttcggtg 78

<210> 1326
<211> 73
<212> DNA
<213> Artificial Sequence

<220>
<223> Synthetic oligonucleotide

<400> 1326
gtgaaggatg tgaagcagac gtacttggca cgggtcttct cctacccggc agggaatgtg 60
gagagcaccg gtt 73

<210> 1327
<211> 66
<212> DNA
<213> Artificial Sequence

<220>
<223> Synthetic oligonucleotide

<400> 1327
ctgaccagaa ccacggctta tccggcctgt ccacgaacca cttatacacc cacatgaccc 60
acttcc 66

<210> 1328
<211> 66
<212> DNA
<213> Artificial Sequence

<220>
<223> Synthetic oligonucleotide

<400> 1328
gcctcttcct gttcgacggc tcgcccacct acgtactggc ctacacccag agctaccggg 60
caaagc 66

<210> 1329
<211> 80
<212> DNA
<213> Artificial Sequence

<220>
<223> Synthetic oligonucleotide

<400> 1329
gagggactgt tggcatgcag tgccctccca gagaccaacg ttcaagcagt tggtagaaga 60
cttggatcga attctcactc 80

<210> 1330
<211> 81
<212> DNA
<213> Artificial Sequence

<220>
<223> Synthetic oligonucleotide

<400> 1330
ctggcttaag gatggacagg cctttcatgg ggagaaccgc attggaggca ttcggctgcg 60
ccatcagcac tggagtctcg t 81

<210> 1331
<211> 67
<212> DNA
<213> Artificial Sequence

<220>
<223> Synthetic oligonucleotide

<400> 1331
ccagtggagc gcttccatga cctgcgtcct gatgaagtgg ccgatttgtt tcagacgacc 60
cagagag 67

<210> 1332
<211> 66
<212> DNA
<213> Artificial Sequence

<220>
<223> Synthetic oligonucleotide

<400> 1332
gacatctgcg ctccatcctc gggaccctga cagtggagca gatttatcag gaccgggacc 60
agtttg 66

<210> 1333
<211> 69
<212> DNA
<213> Artificial Sequence

<220>
<223> Synthetic oligonucleotide

<400> 1333
ggaagtgaca gacgtgaagg tcaccatcat gtggacaccg cctgagagtg cagtgaccgg 60
ctaccgtgt 69

<210> 1334
<211> 67

<212> DNA
<213> Artificial Sequence

<220>
<223> Synthetic oligonucleotide

<400> 1334
cgagcccttt gatgacttcc tgttcccagc atcatccagg cccagtggct ctgagacagc 60
ccgctcc 67

<210> 1335
<211> 66
<212> DNA
<213> Artificial Sequence

<220>
<223> Synthetic oligonucleotide

<400> 1335
gagaacaagc agggctggca gaacagcatc cgccacaacc tctcgctcaa cgagtgcttc 60
gtcaag 66

<210> 1336
<211> 83
<212> DNA
<213> Artificial Sequence

<220>
<223> Synthetic oligonucleotide

<400> 1336
tgaagtccag gacgatgatg cgcctctctc gcccatgctc tacagcagct cagccagcct 60
gtcaccttca gtaagcaagc cgt 83

<210> 1337
<211> 70
<212> DNA
<213> Artificial Sequence

<220>
<223> Synthetic oligonucleotide

<400> 1337
cgacagagct tgtgcaccta agctgcagac caagcctttg cccagaattt aaggattcca 60
atggacgacc 70

<210> 1338
<211> 66
<212> DNA
<213> Artificial Sequence

<220>
<223> Synthetic oligonucleotide

<400> 1338
ctgtttgctg tccggaggca cctgtggggt agccatggaa acagcacatt cccagagttc 60
ctccac 66

<210> 1339
<211> 74
<212> DNA
<213> Artificial Sequence

<220>
<223> Synthetic oligonucleotide

<400> 1339
ccagctgcta ctttgacatc gagtggcgtg accggcgcat cacactgagg gcgtccaatg 60
gcaagtttgt gacc 74

<210> 1340
<211> 80
<212> DNA
<213> Artificial Sequence

<220>
<223> Synthetic oligonucleotide

<400> 1340
ggataattca gacaacaaca ccatctttgt gcaaggcctg ggtgagaatg ttacaattga 60
gtctgtggct gattacttca 80

<210> 1341
<211> 69
<212> DNA
<213> Artificial Sequence

<220>
<223> Synthetic oligonucleotide

<400> 1341
gaagcgcaga tcatgaagaa gctgaagcac gacaagctgg tccagctcta tgcagtggtg 60
tctgaggag 69

<210> 1342
<211> 68
<212> DNA
<213> Artificial Sequence

<220>
<223> Synthetic oligonucleotide

<400> 1342
tcagcagcaa gggcatcatg gaggaggatg aggcctgcgg gcgccagtac acgctcaaga 60
aaaccacc 68

<210> 1343
<211> 74
<212> DNA
<213> Artificial Sequence

<220>
<223> Synthetic oligonucleotide

<400> 1343
tgtttggagg gaagggctgg ggctctgagc cagattccac acctcacgtt cagtcacagc 60
cctcagctat cttc 74

<210> 1344
<211> 73
<212> DNA
<213> Artificial Sequence

<220>
<223> Synthetic oligonucleotide

<400> 1344
   gtgctggtga cgaatccaca ttcatctcaa tggaaggatc ctgccttaag tcaacttatt 60
   tgtttttgcc ggg 73

<210> 1345
<211> 70
<212> DNA
<213> Artificial Sequence

<220>
<223> Synthetic oligonucleotide

<400> 1345
accctcgaca agaccacact ttgggacttg ggagctgggg ctgaagttgc tctgtaccca 60
tgaactccca 70

<210> 1346
<211> 74
<212> DNA
<213> Artificial Sequence

<220>
<223> Synthetic oligonucleotide

<400> 1346
attccaccca tggcaaattc catggcaccg tcaaggctga gaacgggaag cttgtcatca 60
atggaaatcc catc 74

<210> 1347
<211> 75
<212> DNA
<213> Artificial Sequence

<220>
<223> Synthetic oligonucleotide

<400> 1347
caaaggagct cactgtggtg tctgtgttcc aaccactgaa tctggacccc atctgtgaat 60
aagccattct gactc 75

<210> 1348
<211> 73
<212> DNA
<213> Artificial Sequence

<220>
<223> Synthetic oligonucleotide

<400> 1348
ttgggaaata tttgggcatt ggtctggcca agtctacaat gtcccaatat caaggacaac 60
caccctagct tct 73

<210> 1349
<211> 83
<212> DNA
<213> Artificial Sequence

<220>
<223> Synthetic oligonucleotide

<400> 1349
gcatgggaac catcaaccag caggccatgg accaacttca ctatgtgaca gagctgacag 60
atcgaatcaa ggcaaactcc tca 83

<210> 1350
<211> 85
<212> DNA
<213> Artificial Sequence

<220>
<223> Synthetic oligonucleotide

<400> 1350
tgtagaatca aactcttcat catcaactag aagtgcagtt gacatggcct gttcagtcct 60
tggagttgca cagctggatt ctgtg 85

<210> 1351
<211> 72
<212> DNA
<213> Artificial Sequence

<220>
<223> Synthetic oligonucleotide

<400> 1351
cgctccagac ctatgatgac ttgttagcca aagactgcca ctgcatatga gcagtcctgg 60
tccttccact gt 72

<210> 1352
<211> 66
<212> DNA
<213> Artificial Sequence

<220>
<223> Synthetic oligonucleotide

<400> 1352
gatcccaagg cccaactccc cgaaccactc agggtcctgt ggacagctca cctagctgca 60
atggct 66

<210> 1353
<211> 68
<212> DNA
<213> Artificial Sequence

<220>
<223> Synthetic oligonucleotide

<400> 1353
gttcactggg ggtgtatggg gtagatgggt ggagagggag gggataagag aggtgcatgt 60
tggtattt 68

<210> 1354
<211> 74
<212> DNA
<213> Artificial Sequence

<220>
<223> Synthetic oligonucleotide

<400> 1354
tgtcatgtac gacggcttct ccatgcagcg gctggtgaag tgcaacgcct ggccttgtcc 60
caacactgtg gact 74

<210> 1355
<211> 67

<212> DNA
<213> Artificial Sequence

<220>
<223> Synthetic oligonucleotide

<400> 1355
gttcgctacg aggattgagc gtctccaccc agtaagtggg caagaggcgg caggaagtgg 60
gtacgca 67

<210> 1356
<211> 68
<212> DNA
<213> Artificial Sequence

<220>
<223> Synthetic oligonucleotide

<400> 1356
ttctggacct gggaccttag gagccgggtg acagcactaa ccagacctcc agccactcac 60
agctcttt 68

<210> 1357
<211> 70
<212> DNA
<213> Artificial Sequence

<220>
<223> Synthetic oligonucleotide

<400> 1357
cgtgcctcta caccatcttc ctcttcccca tcggctttgt gggcaacatc ctgatcctgg 60
tggtgaacat 70

<210> 1358
<211> 66
<212> DNA
<213> Artificial Sequence

<220>
<223> Synthetic oligonucleotide

<400> 1358
agtacaagca ggctgccaag tgcctcctgc tggcttcctt tgatcactgt gacttccctg 60
agctgc 66

<210> 1359
<211> 67
<212> DNA
<213> Artificial Sequence

<220>
<223> Synthetic oligonucleotide

<400> 1359
gcttatgacc gaccccaagc tcatcacctg gtctccggtg tgtcgcaacg atgttgcctg 60
gaacttt 67

<210> 1360
<211> 75
<212> DNA
<213> Artificial Sequence

<220>
<223> Synthetic oligonucleotide

<400> 1360
cacacagatc tcctactcca tccagtcctg aggagcctta ggatgcagca tgccttcagg 60
agacactgct ggacc 75

<210> 1361
<211> 66
<212> DNA
<213> Artificial Sequence

<220>
<223> Synthetic oligonucleotide

<400> 1361
ctgagtgtgg tttgcggatc ctggccttcc cgtgtaacca gttcgggaag caggagccag 60
ggagta 66

<210> 1362
<211> 67
<212> DNA
<213> Artificial Sequence

<220>
<223> Synthetic oligonucleotide

<400> 1362
ccatctgcat ccatcttgtt tgggctcccc acccttgaga agtgcctcag ataataccct 60
ggtggcc 67

<210> 1363
<211> 71
<212> DNA
<213> Artificial Sequence

<220>
<223> Synthetic oligonucleotide

<400> 1363
cagatgacaa tggccacaat gctcttcttg gtttctctgg gaattgtgtt ggctgtggaa 60
agaaaggctt c 71

<210> 1364
<211> 73
<212> DNA
<213> Artificial Sequence

<220>
<223> Synthetic oligonucleotide

<400> 1364
tgcttaggtg cggtaaaacc agcgcttgtc cgatgcaccg ttcgcgtggt aaactgacgc 60
attcaggctc ttg 73

<210> 1365
<211> 64
<212> DNA
<213> Artificial Sequence

<220>
<223> Synthetic oligonucleotide

<400> 1365
ccccaggcac cagctttact ccccgagccc agcaggacat ctgcatataa cacacagccg 60
aagt 64

<210> 1366
<211> 72
<212> DNA
<213> Artificial Sequence

<220>
<223> Synthetic oligonucleotide

<400> 1366
tgcccccaag acactgtgtg tgacctgatc cagagtaagt gcctctccaa ggagaacgct 60
accacggacc tc 72

<210> 1367
<211> 86
<212> DNA
<213> Artificial Sequence

<220>
<223> Synthetic oligonucleotide

<400> 1367
aagctatgag gaaaagaagt acacgatggg ggacgctcct gattatgaca gaagccagtg 60
gctgaatgaa aaattcaagc tgggcc 86

<210> 1368
<211> 71
<212> DNA
<213> Artificial Sequence

<220>
<223> Synthetic oligonucleotide

<400> 1368
ctgggctgtg aggctgagag tgaatctgct ttacgagggt aggcggggaa tcagaaaagg 60
agcagattcg c 71

<210> 1369
<211> 68
<212> DNA
<213> Artificial Sequence

<220>
<223> Synthetic oligonucleotide

<400> 1369
ctgcaggcac tccctgaaat gctgaagctc tactcacagt ttctggggaa gcagccatgg 60
tttcttgg 68

<210> 1370
<211> 76
<212> DNA
<213> Artificial Sequence

<220>
<223> Synthetic oligonucleotide

<400> 1370
caatgccatc ttgcgctaca tcgctcgcaa gcacaacatg tgtggtgaga ctgaagaaga 60
aaagattcga gtggac 76

<210> 1371
<211> 66
<212> DNA
<213> Artificial Sequence

<220>
<223> Synthetic oligonucleotide

<400> 1371
caccatcccc accctgtctt ccacagccgc ctgaaagcca caatgagaat gatgcacact 60
gaggcc 66

<210> 1372
<211> 73
<212> DNA
<213> Artificial Sequence

<220>
<223> Synthetic oligonucleotide

<400> 1372
cccactcagt agccaagtca caatgtttgg aaaacagccc gtttacttga gcaagactga 60
taccacctgc gtg 73

<210> 1373
<211> 70
<212> DNA
<213> Artificial Sequence

<220>
<223> Synthetic oligonucleotide

<400> 1373
ccaaacgtgt aacaattatg ccaaaagaca tccagctagc acgccgcata cgtggagaac 60
gtgcttaaga 70

<210> 1374
<211> 74
<212> DNA
<213> Artificial Sequence

<220>
<223> Synthetic oligonucleotide

<400> 1374
caagtaccac agcgatgact acattaaatt cttgcgctcc atccgtccag ataacatgtc 60
ggagtacagc aagc 74

<210> 1375
<211> 66
<212> DNA
<213> Artificial Sequence

<220>
<223> Synthetic oligonucleotide

<400> 1375
tcctgtgctc tggaagccct tgagcccttc tgggaggttc ttgtgagatc aactgagacc 60
gtggag 66

<210> 1376
<211> 70

<212> DNA
<213> Artificial Sequence

<220>
<223> Synthetic oligonucleotide

<400> 1376
cggtgtgaga agtgcagcaa gccctgtgcc cgagtgtgct atggtctggg catggagcac 60
ttgcgagagg 70

<210> 1377
<211> 68
<212> DNA
<213> Artificial Sequence

<220>
<223> Synthetic oligonucleotide

<400> 1377
gaaagatagc tcgcggcatt ccaagctgga gaaggcggac attctggaaa tgacagtgaa 60
gcacctcc 68

<210> 1378
<211> 72
<212> DNA
<213> Artificial Sequence

<220>
<223> Synthetic oligonucleotide

<400> 1378
caggacacaa gtgccagatt gcgggctggg gccacttgga tgagaacgtg agcggctact 60
ccagctccct gc 72

<210> 1379
<211> 73
<212> DNA
<213> Artificial Sequence

<220>
<223> Synthetic oligonucleotide

<400> 1379
tccatgatgg ttctgcaggt ttctgcggcc ccccggacag tggctctgac ggcgttactg 60
atggtgctgc tca 73

<210> 1380
<211> 71
<212> DNA
<213> Artificial Sequence

<220>
<223> Synthetic oligonucleotide

<400> 1380
tggcctgtcc attggtgatg ttgcgaagaa actgggagag atgtggaata acactgctgc 60
agatgacaag c 71

<210> 1381
<211> 73
<212> DNA
<213> Artificial Sequence

<220>
<223> Synthetic oligonucleotide

<400> 1381
tccaggatgt taggaactgt gaagatggaa gggcatgaaa ccagcgactg gaacagctac 60
tacgcagaca cgc 73

<210> 1382
<211> 84
<212> DNA
<213> Artificial Sequence

<220>
<223> Synthetic oligonucleotide

<400> 1382
agcaggagcg accaactgat cgcacacatg ctttgtttgg atatggagtg aacacaatta 60
tgtaccaaat ttaacttggc aaac 84

<210> 1383
<211> 68
<212> DNA
<213> Artificial Sequence

<220>
<223> Synthetic oligonucleotide

<400> 1383
gcagcagtcg gcttctctac gcagaacccg ggagtaggag actcagaatc gaatctcttc 60
tccctccc 68

<210> 1384
<211> 69
<212> DNA
<213> Artificial Sequence

<220>
<223> Synthetic oligonucleotide

<400> 1384
agtgacagat ggacaatgca agaatgaact ccttcctgga ataccccata cttagcagtg 60
gcgactcgg 69

<210> 1385
<211> 78
<212> DNA
<213> Artificial Sequence

<220>
<223> Synthetic oligonucleotide

<400> 1385
tcccttgtgt tccttctgtg aagaagccct gttctcgttg ccctaattca tcttttaatc 60
atgagcctgt ttattgcc 78

<210> 1386
<211> 71
<212> DNA
<213> Artificial Sequence

<220>
<223> Synthetic oligonucleotide

<400> 1386
cgtgccttat ggttactttg gaggcgggta ctactcctgc cgagtgtccc ggagctcgct 60
gaaaccctgt g 71

<210> 1387
<211> 68
<212> DNA
<213> Artificial Sequence

<220>
<223> Synthetic oligonucleotide

<400> 1387
cagcctcaag ttcggttttc gctaccggag ccttcccaga acaaacttct tgtgcgtttg 60
cttccaac 68

<210> 1388
<211> 78
<212> DNA
<213> Artificial Sequence

<220>
<223> Synthetic oligonucleotide

<400> 1388
ctggaccgca cggacatcca caccttccac cgcttctacc aatacctcgc ccacagcaag 60
caagtctttc gcgaggcg 78

<210> 1389
<211> 68
<212> DNA
<213> Artificial Sequence

<220>
<223> Synthetic oligonucleotide

<400> 1389
gctttccaag tggggaatta aagttgcttc catccaacct ggaggcttcc taacaaatat 60
cgcaggca 68

<210> 1390
<211> 77
<212> DNA
<213> Artificial Sequence

<220>
<223> Synthetic oligonucleotide

<400> 1390
cagtctcgcc atgttgaagt cagaatggcc tgtattcact atcttcgaga gaacagagag 60
aaatttgaag cgtttat 77

<210> 1391
<211> 70
<212> DNA
<213> Artificial Sequence

<220>
<223> Synthetic oligonucleotide

<400> 1391
ctgctgcgac agtccactac ctttttcgag agtgactccc gttgtcccaa ggcttcccag 60
agcgaacctg 70

<210> 1392
<211> 63
<212> DNA
<213> Artificial Sequence

<220>
<223> Synthetic oligonucleotide

<400> 1392
ggtccgcttc gtctttcgag agtgactccc gcggtcccaa ggctttccag agcgaacctg 60
tgc 63

<210> 1393
<211> 72
<212> DNA
<213> Artificial Sequence

<220>
<223> Synthetic oligonucleotide

<400> 1393
ttcagtgtgt ccagtgcatc tttagtggag gttcacaagt ctgaggaaaa tgaggagcca 60
atggaaacag at 72

<210> 1394
<211> 84
<212> DNA
<213> Artificial Sequence

<220>
<223> Synthetic oligonucleotide

<400> 1394
ggctagtaga actggatccc aacaccaaac tcttaattag acctaggcct cagctgcact 60
gcccgaaaag catttgggca gacc 84

<210> 1395
<211> 73
<212> DNA
<213> Artificial Sequence

<220>
<223> Synthetic oligonucleotide

<400> 1395
cctccctctg gtggtgcttc ctcagggccc accattgaag aggttgatta agccaaccaa 60
gtgtagatgt agc 73

<210> 1396
<211> 84
<212> DNA
<213> Artificial Sequence

<220>
<223> Synthetic oligonucleotide

<400> 1396
ccgactggag gagcataaaa gcgcagccga gcccagcgcc ccgcactttt ctgagcagac 60
gtccagagca gagtcagcca gcat 84

<210> 1397
<211> 83

<212> DNA
<213> Artificial Sequence

<220>
<223> Synthetic oligonucleotide

<400> 1397
gaataccaca ctttctgcta caacactggg ctatggagag gacgccacgc ctggcacagg 60
gtatacaggg ttagctgcaa tcc 83

<210> 1398
<211> 68
<212> DNA
<213> Artificial Sequence

<220>
<223> Synthetic oligonucleotide

<400> 1398
gcagacagtg accatctaca gctttccggc gcccaacgtg attctgacga agccagaggt 60
ctcagaag 68

<210> 1399
<211> 70
<212> DNA
<213> Artificial Sequence

<220>
<223> Synthetic oligonucleotide

<400> 1399
agaaccgcaa ggtgagcaag gtggagattc tccagcacgt catcgactac atcagggacc 60
ttcagttgga 70

<210> 1400
<211> 83
<212> DNA
<213> Artificial Sequence

<220>
<223> Synthetic oligonucleotide

<400> 1400
tggcctggct cttaatttgc ttttgttttg cccagtatag actcggaagt aacagttata 60
gctagtggtc ttgcatgatt gca 83

<210> 1401
<211> 74
<212> DNA
<213> Artificial Sequence

<220>
<223> Synthetic oligonucleotide

<400> 1401
ggtggagagt ggagccatga ccaaggacct ggcgggctgc attcacggcc tcagcaatgt 60
gaagctgaac gagc 74

<210> 1402
<211> 83
<212> DNA
<213> Artificial Sequence

<220>
<223> Synthetic oligonucleotide

<400> 1402
gcatggtagc cgaagatttc acagtcaaaa tcggagattt tggtatgacg cgagatatct 60
atgagacaga ctattaccgg aaa 83

<210> 1403
<211> 72
<212> DNA
<213> Artificial Sequence

<220>
<223> Synthetic oligonucleotide

<400> 1403
ccgtgcttcc ggacaacttc cccagatacc ccgtgggcaa gttcttccaa tatgacacct 60
ggaagcagtc ca 72

<210> 1404
<211> 77
<212> DNA
<213> Artificial Sequence

<220>
<223> Synthetic oligonucleotide

<400> 1404
tgaaccgcag agaccaacag aggaatccag gcacctctac cacgccctcc cagcccaatt 60
ctgcgggtgt ccaagac 77

<210> 1405
<211> 68
<212> DNA
<213> Artificial Sequence

<220>
<223> Synthetic oligonucleotide

<400> 1405
gggtcactat ggagttcaaa ggacagaact cctgcctggt gaccgggaca acctggccat 60
tcagaccc 68

<210> 1406
<211> 66
<212> DNA
<213> Artificial Sequence

<220>
<223> Synthetic oligonucleotide

<400> 1406
gcctcccata gctccttacc ccagccctac acgaaaggac ctgcttctcc acatgcaaga 60
gctctg 66

<210> 1407
<211> 70
<212> DNA
<213> Artificial Sequence

<220>
<223> Synthetic oligonucleotide

<400> 1407
aaggaaccat ctcactgtgt gtaaacatga cttccaagct ggccgtggct ctcttggcag 60
ccttcctgat 70

<210> 1408
<211> 79
<212> DNA
<213> Artificial Sequence

<220>
<223> Synthetic oligonucleotide

<400> 1408
ggcgctgtca tcgatttctt ccctgtgaaa acaagagcaa ggccgtggag caggtgaaga 60
atgcctttaa taagctcca 79

<210> 1409
<211> 66
<212> DNA
<213> Artificial Sequence

<220>
<223> Synthetic oligonucleotide

<400> 1409
tggaaggttc cacaagtcac cctgtgatca acagtacccg tatgggacaa agctgcaagg 60
tcaaga 66

<210> 1410
<211> 76
<212> DNA
<213> Artificial Sequence

<220>
<223> Synthetic oligonucleotide

<400> 1410
accctctggt ggtaaatgga cattttccta catcggcttc cctgtagagc tgaacacagt 60
ctatttcatt ggggcc 76

<210> 1411
<211> 74
<212> DNA
<213> Artificial Sequence

<220>
<223> Synthetic oligonucleotide

<400> 1411
ggcctaatgt tccagatcct tcaaagagtc atattgccca gtggtcacct cacactcctc 60
caaggcacaa tttt 74

<210> 1412
<211> 66
<212> DNA
<213> Artificial Sequence

<220>
<223> Synthetic oligonucleotide

<400> 1412
actttcctgc gaggtcagtc aaggctttgg gggctctgtt ttgaatgtgg atcaccactc 60
ggagtt 66

<210> 1413
<211> 72
<212> DNA
<213> Artificial Sequence

<220>
<223> Synthetic oligonucleotide

<400> 1413
gtgcccgagc catatagcag gcacgtccgg gtcctcactg tccttccact caacagtcat 60
caaccactac cg 72

<210> 1414
<211> 69
<212> DNA
<213> Artificial Sequence

<220>
<223> Synthetic oligonucleotide

<400> 1414
agtccagccg agatgctaag agcaaggcca agaggaagtc atgtggggat tccagccctg 60
ataccttct 69

<210> 1415
<211> 74
<212> DNA
<213> Artificial Sequence

<220>
<223> Synthetic oligonucleotide

<400> 1415
ccacagctca ccttctgtca ggtgtccatc ccagctccag ccagctccca gagaggaaga 60
gactggcact gagg 74

<210> 1416
<211> 77
<212> DNA
<213> Artificial Sequence

<220>
<223> Synthetic oligonucleotide

<400> 1416
ccatgatcct cactctgctg gtggactata cactccagac ctcgcttagc atggtaaatc 60
accggctaca aagcttc 77

<210> 1417
<211> 66
<212> DNA
<213> Artificial Sequence

<220>
<223> Synthetic oligonucleotide

<400> 1417
caacgcttca gtgatcaatc ccggggcgat ttacagatgc aggatcggaa agaatcccgg 60
ccagac 66

<210> 1418
<211> 75

<212> DNA
<213> Artificial Sequence

<220>
<223> Synthetic oligonucleotide

<400> 1418
aggccagccc tacattatca gagcaagagc cggatagagg acaaggctca gatcttgctg 60
gactgtggag aagac 75

<210> 1419
<211> 69
<212> DNA
<213> Artificial Sequence

<220>
<223> Synthetic oligonucleotide

<400> 1419
cagtgacaaa cagcccttcc aacccaagga atcccacaaa agatggcgat gacgcccatg 60
aggctaaac 69

<210> 1420
<211> 79
<212> DNA
<213> Artificial Sequence

<220>
<223> Synthetic oligonucleotide

<400> 1420
actcggactg cacaagctat ttttgatgac agctatttgg gttattctgt ggctgtcgga 60
gatttcaatg gtgatggca 79

<210> 1421
<211> 74
<212> DNA
<213> Artificial Sequence

<220>
<223> Synthetic oligonucleotide

<400> 1421
tcagaattgg atttggctca tttgtggaaa agactgtgat gccttacatt agcacaacac 60
cagctaagct cagg 74

<210> 1422
<211> 78
<212> DNA
<213> Artificial Sequence

<220>
<223> Synthetic oligonucleotide

<400> 1422
accggggagc cctacatgac gaaaatacct gcaaccgtta ctgccgtgac gagattgagt 60
cagtgaaaga gcttaagg 78

<210> 1423
<211> 66
<212> DNA
<213> Artificial Sequence

<220>
<223> Synthetic oligonucleotide

<400> 1423
caaggtgccc tcagtggagc tcaccaacct gtacccgtat tgcgactatg agatgaaggt 60
gtgcgc 66

<210> 1424
<211> 71
<212> DNA
<213> Artificial Sequence

<220>
<223> Synthetic oligonucleotide

<400> 1424
tcgtgaaaga tgaccaggag gctgtgctat gtttctacaa aaccgccaag gactgcgtca 60
tgatgttcac c 71

<210> 1425
<211> 69
<212> DNA
<213> Artificial Sequence

<220>
<223> Synthetic oligonucleotide

<400> 1425
tggcttacac tggcaatggt agtttctgtg gttggctggg aaatcgagtg ccgcatctca 60
cagctatgc 69

<210> 1426
<211> 70
<212> DNA
<213> Artificial Sequence

<220>
<223> Synthetic oligonucleotide

<400> 1426
   ctgtcagctg ctgcttgggg tcaagggaca cgccttctga acgtcccctg cccctttacg 60
   gacaccccct 70

<210> 1427
<211> 80
<212> DNA
<213> Artificial Sequence

<220>
<223> Synthetic oligonucleotide

<400> 1427
cggactttgg gtgcgacttg acgagcggtg gttcgacaag tggccttgcg ggccggatcg 60
tcccagtgga agagttgtaa 80

<210> 1428
<211> 67
<212> DNA
<213> Artificial Sequence

<220>
<223> Synthetic oligonucleotide

<400> 1428
aagcccgagg cactcattgt tgcccttcaa gctgccaatg aagacctcag gaccaagctc 60
acagaca 67

<210> 1429
<211> 66
<212> DNA
<213> Artificial Sequence

<220>
<223> Synthetic oligonucleotide

<400> 1429
gctgggaggc aggacttcct cttcaaggcc atgctgacca tcagctggct cactctgacc 60
tgcttc 66

<210> 1430
<211> 69
<212> DNA
<213> Artificial Sequence

<220>
<223> Synthetic oligonucleotide

<400> 1430
gagctccatg gctcatcccc agcagtgagc tcagaggaat gcacacccag taggattcag 60
tgggtgtga 69

<210> 1431
<211> 67
<212> DNA
<213> Artificial Sequence

<220>
<223> Synthetic oligonucleotide

<400> 1431
tctcttgcag gaagccagac aacagtcagt ggcccatcag caatcagggt ctgaattggc 60
cctacgg 67

<210> 1432
<211> 73
<212> DNA
<213> Artificial Sequence

<220>
<223> Synthetic oligonucleotide

<400> 1432
aattcctgct ccaaaagaaa gtcttcgaag ccgctccact cgcatgtcca ctgtctcaga 60
gcttcgcatc acg 73

<210> 1433
<211> 66
<212> DNA
<213> Artificial Sequence

<220>
<223> Synthetic oligonucleotide

<400> 1433
caccccggct tcaacaacag cctccccaac aaagaccacc gcaatgacat catgctggtg 60
aagatg 66

<210> 1434
<211> 78
<212> DNA
<213> Artificial Sequence

<220>
<223> Synthetic oligonucleotide

<400> 1434
gacgtgaggg tcctgattct ccctggtttt accccagctc catccttgca tcactgggga 60
ggacgtgatg agtgagga 78

<210> 1435
<211> 67
<212> DNA
<213> Artificial Sequence

<220>
<223> Synthetic oligonucleotide

<400> 1435
catcctcatg gattggtgtg tttcgtaaca gcagtcatca tccatgggtg acaatgaatg 60
gtttggc 67

<210> 1436
<211> 77
<212> DNA
<213> Artificial Sequence

<220>
<223> Synthetic oligonucleotide

<400> 1436
ccacctcgcc atgatttttc ctttgaccgg gtattcccac caggaagtgg acaggatgaa 60
gtgtttgaag agattgc 77

<210> 1437
<211> 71
<212> DNA
<213> Artificial Sequence

<220>
<223> Synthetic oligonucleotide

<400> 1437
atgtgccagt gagcttgagt ccttggagaa acacaagcac ctgctagaaa gtactgttaa 60
ccaggggctc a 71

<210> 1438
<211> 67
<212> DNA
<213> Artificial Sequence

<220>
<223> Synthetic oligonucleotide

<400> 1438
tgatggtcca aatgaacgaa ttggcatggt ggtgaaaaca ggagttgtgc cccaacttgt 60
gaagctt 67

<210> 1439
<211> 66

<212> DNA
<213> Artificial Sequence

<220>
<223> Synthetic oligonucleotide

<400> 1439
cttgctggcc aatgcctaca tctacgttgt ccagctgcca gccaagatcc tgactgcgga 60
caatca 66

<210> 1440
<211> 73
<212> DNA
<213> Artificial Sequence

<220>
<223> Synthetic oligonucleotide

<400> 1440
cagatgaggc acatggagac ccaggccaag gacctgagga atcagttgct caactaccgt 60
tctgccattt caa 73

<210> 1441
<211> 67
<212> DNA
<213> Artificial Sequence

<220>
<223> Synthetic oligonucleotide

<400> 1441
ctcctggcca acagcactgc actagaagag gccatgctcc aggaacagca gaggctgggc 60
cttgtgt 67

<210> 1442
<211> 66
<212> DNA
<213> Artificial Sequence

<220>
<223> Synthetic oligonucleotide

<400> 1442
caaggagact gggaggtgtc tcaagtgcct gtaccacacg gaaggggaac actgtcagtt 60
ctgccg 66

<210> 1443
<211> 67
<212> DNA
<213> Artificial Sequence

<220>
<223> Synthetic oligonucleotide

<400> 1443
actgaccaag cctgagacct actgcaccca gtatggcgag tggcagatga aatgctgcaa 60
gtgtgac 67

<210> 1444
<211> 80
<212> DNA
<213> Artificial Sequence

<220>
<223> Synthetic oligonucleotide

<400> 1444
actcaagcgg aaattgaagc agataggtct tatcagcaca gtctccgcct cctggattca 60
gtgtctcggc ttcagggagt 80

<210> 1445
<211> 67
<212> DNA
<213> Artificial Sequence

<220>
<223> Synthetic oligonucleotide

<400> 1445
agcgatgaag atggtcgcgc cctggacgcg gttctactcc aacagctgct gcttgtgctg 60
ccatgtc 67

<210> 1446
<211> 69
<212> DNA
<213> Artificial Sequence

<220>
<223> Synthetic oligonucleotide

<400> 1446
agcggaaaat ggcagacaat ttttcgctcc atgatgcgtt atctgggtct ggaaacccaa 60
accctcaag 69

<210> 1447
<211> 67
<212> DNA
<213> Artificial Sequence

<220>
<223> Synthetic oligonucleotide

<400> 1447
gcttcaggtg ttgtgactgc agtgcctccc tgtcgcacca gtactatgag aaggatgggc 60
agctctt 67

<210> 1448
<211> 71
<212> DNA
<213> Artificial Sequence

<220>
<223> Synthetic oligonucleotide

<400> 1448
tgaacagtaa tggggagctg taccatgagc agtgtttcgt gtgcgctcag tgcttccagc 60
agttcccaga a 71

<210> 1449
<211> 66
<212> DNA
<213> Artificial Sequence

<220>
<223> Synthetic oligonucleotide

<400> 1449
tgcaaacgct ggtgtcacag ccagcccccc aactgacctc atctggaaga accagaactc 60
gtgggg 66

<210> 1450
<211> 66
<212> DNA
<213> Artificial Sequence

<220>
<223> Synthetic oligonucleotide

<400> 1450
ccaatgggag aacaacgggc aggtgttcag cttgctgagc ctgggctcac agtaccagcc 60
tcagcg 66

<210> 1451
<211> 67
<212> DNA
<213> Artificial Sequence

<220>
<223> Synthetic oligonucleotide

<400> 1451
ctgcaacacc gaagtggact gttactccag gggacaagcc ttccaccccc agcctgtgtc 60
cagagac 67

<210> 1452
<211> 66
<212> DNA
<213> Artificial Sequence

<220>
<223> Synthetic oligonucleotide

<400> 1452
agaccaagct ggaagcagag aagttgaaag tgcaggccct gaaggaccga ggtctttcca 60
ttcctc 66

<210> 1453
<211> 67
<212> DNA
<213> Artificial Sequence

<220>
<223> Synthetic oligonucleotide

<400> 1453
acatccaggg ctctgtggtc cgcaagggga gtgcctaaac acagagggtt ctttccattg 60
tgtctgc 67

<210> 1454
<211> 67
<212> DNA
<213> Artificial Sequence

<220>
<223> Synthetic oligonucleotide

<400> 1454
gacctggcct tgctgaagaa tctccggagc gaggaacaga agaagaagaa ccggaagaaa 60
ctgtccg 67

<210> 1455
<211> 67
<212> DNA
<213> Artificial Sequence

<220>
<223> Synthetic oligonucleotide

<400> 1455
agaagctgtc cctgcaagag caggatgcag cgattgtgaa gaacatgaag tctgagctgg 60
tacggct 67

<210> 1456
<211> 75
<212> DNA
<213> Artificial Sequence

<220>
<223> Synthetic oligonucleotide

<400> 1456
gacttttgcc cgctaccttt cattccggcg tgacaacaat gagctgttgc tcttcatact 60
gaagcagtta gtggc 75

<210> 1457
<211> 70
<212> DNA
<213> Artificial Sequence

<220>
<223> Synthetic oligonucleotide

<400> 1457
aggatcgcct gtcagaagag gagacccggg ttgtcttccg tcagatagta tctgctgttg 60
cttatgtgca 70

<210> 1458
<211> 69
<212> DNA
<213> Artificial Sequence

<220>
<223> Synthetic oligonucleotide

<400> 1458
gtgaaatgaa acgcaccaca ctggacagcc ctttggggaa gctggagctg tctggttgtg 60
agcagggtc 69

<210> 1459
<211> 79
<212> DNA
<213> Artificial Sequence

<220>
<223> Synthetic oligonucleotide

<400> 1459
acggatctac cacaccattg catatttgac accccttccc cagccaaata gagctttgag 60
ttttttgtt ggatatgga 79

<210> 1460
<211> 72

<212> DNA
<213> Artificial Sequence

<220>
<223> Synthetic oligonucleotide

<400> 1460
gggagatcat cgggacaact ctccttttga tggacctgga ggaaatcttg ctcatgcttt 60
tcaaccaggc cc 72

<210> 1461
<211> 78
<212> DNA
<213> Artificial Sequence

<220>
<223> Synthetic oligonucleotide

<400> 1461
ccaacgcttg ccaaatcctg acaattcaga accagctctc tgtgacccca atttgagttt 60
tgatgctgtc actaccgt 78

<210> 1462
<211> 86
<212> DNA
<213> Artificial Sequence

<220>
<223> Synthetic oligonucleotide

<400> 1462
ccatgatgga gaggcagaca tcatgatcaa ctttggccgc tgggagcatg gcgatggata 60
cccctttgac ggtaaggacg gactcc 86

<210> 1463
<211> 79
<212> DNA
<213> Artificial Sequence

<220>
<223> Synthetic oligonucleotide

<400> 1463
ggatggtagc agtctaggga ttaacttcct gtatgctgca actcatgaac ttggccattc 60
tttgggtatg ggacattcc 79

<210> 1464
<211> 79
<212> DNA
<213> Artificial Sequence

<220>
<223> Synthetic oligonucleotide

<400> 1464
tcacctctca tcttcaccag gatctcacag ggagaggcag atatcaacat tgctttttac 60
caaagagatc acggtgaca 79

<210> 1465
<211> 72
<212> DNA
<213> Artificial Sequence

<220>
<223> Synthetic oligonucleotide

<400> 1465
ccatacgtgc tgctacctgt agatattggt gatgaaccat ggtttgatga ttctgccatt 60
caaaccttta gg 72

<210> 1466
<211> 75
<212> DNA
<213> Artificial Sequence

<220>
<223> Synthetic oligonucleotide

<400> 1466
cgagagtctg taggagggaa accgccatgg acgatcaggg ttgccctcgg tgtaagacca 60
ccaaatatcg gaacc 75

<210> 1467
<211> 79
<212> DNA
<213> Artificial Sequence

<220>
<223> Synthetic oligonucleotide

<400> 1467
tcatggtgcc cgtcaatgct gtgatggcga tgaagaccaa gacgtatcag gtggcccaca 60
tgaagagcaa agacaatcg 79

<210> 1468
<211> 91
<212> DNA
<213> Artificial Sequence

<220>
<223> Synthetic oligonucleotide

<400> 1468
tcatcctggc gatctacttc ctctggcaga acctaggtcc ctctgtcctg gctggagtcg 60
ctttcatggt cttgctgatt ccactcaacg g 91

<210> 1469
<211> 70
<212> DNA
<213> Artificial Sequence

<220>
<223> Synthetic oligonucleotide

<400> 1469
tgagaaacaa actgcaccca ctgaactccg cagctagcat ccaaatcagc ccttgagatt 60
tgaggccttg 70

<210> 1470
<211> 73
<212> DNA
<213> Artificial Sequence

<220>
<223> Synthetic oligonucleotide

<400> 1470
gatgcagaat tgaggcagac tttacaagaa gatttacttc gtcgattccc agatcttaac 60
cgacttgcca aga 73

<210> 1471
<211> 69
<212> DNA
<213> Artificial Sequence

<220>
<223> Synthetic oligonucleotide

<400> 1471
gctcgtggtt ctgtagtcca gtcatcctag gagggtgatg ttgactgaga cttcacgctc 60
tccctttgt 69

<210> 1472
<211> 78
<212> DNA
<213> Artificial Sequence

<220>
<223> Synthetic oligonucleotide

<400> 1472
gaaggaatgg gaatcagtca tgagctaatc accctggaga tcagctcccg agatgtcccg 60
gatctgactc taatagac 78

<210> 1473
<211> 74
<212> DNA
<213> Artificial Sequence

<220>
<223> Synthetic oligonucleotide

<400> 1473
gccgagatcg ccaagatgtt gccagggagg acagacaatg ctgtgaagaa tcactggaac 60
tctaccatca aaag 74

<210> 1474
<211> 75
<212> DNA
<213> Artificial Sequence

<220>
<223> Synthetic oligonucleotide

<400> 1474
tggttttgag accacgatgt tgggagggta tgtttacagc actccagcca aaaaatacag 60
cactggcatg attca 75

<210> 1475
<211> 73
<212> DNA
<213> Artificial Sequence

<220>
<223> Synthetic oligonucleotide

<400> 1475
taactgacat tcttgagcac cagatccggg ctgttccctt tgagaacctt aacatgcatt 60
gtgggcaagc cat 73

<210> 1476
<211> 83
<212> DNA
<213> Artificial Sequence

<220>
<223> Synthetic oligonucleotide

<400> 1476
cgagactctc ctcatagtga aaggtatgtg tcagccatga ccaccccggc tcgtatgtca 60
cctgtagatt tccacacgcc aag 83

<210> 1477
<211> 80
<212> DNA
<213> Artificial Sequence

<220>
<223> Synthetic oligonucleotide

<400> 1477
caaccgaagt tttcactcca gttgtcccca cagtagacac atatgatggc cgaggtgata 60
gtgtggttta tggactgagg 80

<210> 1478
<211> 76
<212> DNA
<213> Artificial Sequence

<220>
<223> Synthetic oligonucleotide

<400> 1478
gcggaaggtc cctcagacat ccccgattga aagaaccaga gaggctctga gaaacctcgg 60
gaaacttaga tcatca 76

<210> 1479
<211> 81
<212> DNA
<213> Artificial Sequence

<220>
<223> Synthetic oligonucleotide

<400> 1479
ccgcaacgtg gttttctcac cctatggggt ggcctcggtg ttggccatgc tccagctgac 60
aacaggagga gaaacccagc a 81

<210> 1480
<211> 71
<212> DNA
<213> Artificial Sequence

<220>
<223> Synthetic oligonucleotide

<400> 1480
gtggttttcc ctcggagccc cctggctcgg gacgtctgag aagatgccgg tcatgaggct 60
gttcccttgc t 71

<210> 1481
<211> 85

<212> DNA
<213> Artificial Sequence

<220>
<223> Synthetic oligonucleotide

<400> 1481
gcatcaggct gtcattatgg tgtccttacc tgtgggagct gtaaggtctt ctttaagagg 60
gcaatggaag ggcagcacaa ctact 85

<210> 1482
<211> 67
<212> DNA
<213> Artificial Sequence

<220>
<223> Synthetic oligonucleotide

<400> 1482
aggactggga cccatgaaca ttcctttggt atcagacccg aagcgcacca ttgctcagga 60
ttatggg 67

<210> 1483
<211> 81
<212> DNA
<213> Artificial Sequence

<220>
<223> Synthetic oligonucleotide

<400> 1483
   tggctaagtg aagatgacaa tcatgttgca gcaattcact gtaaagctgg aaagggacga 60
   actggtgtaa tgatatgtgc a 81

<210> 1484
<211> 70
<212> DNA
<213> Artificial Sequence

<220>
<223> Synthetic oligonucleotide

<400> 1484
aatatttgtg cggggtatgg gggtgggttt ttaaatctcg tttctcttgg acaagcacag 60
ggatctcgtt 70

<210> 1485
<211> 67
<212> DNA
<213> Artificial Sequence

<220>
<223> Synthetic oligonucleotide

<400> 1485
aagcatgaac aggacttgac catctttcca acccctgggg aagacatttg caactgactt 60
ggggagg 67

<210> 1486
<211> 68
<212> DNA
<213> Artificial Sequence

<220>
<223> Synthetic oligonucleotide

<400> 1486
gcaaggaaag ggtcttagtc actgcctccc gaagttgctt gaaagcactc ggagaattgt 60
gcaggtgt 68

<210> 1487
<211> 66
<212> DNA
<213> Artificial Sequence

<220>
<223> Synthetic oligonucleotide

<400> 1487
gaaacctctg cgccatgaga gccaagtgga ggaagaagcg aatgcgcagg ctgaagcgca 60
aaagaa 66

<210> 1488
<211> 75
<212> DNA
<213> Artificial Sequence

<220>
<223> Synthetic oligonucleotide

<400> 1488
ccattctatc atcaacgggt acaaacgagt cctggccttg tctgtggaga cggattacac 60
cttcccactt gctga 75

<210> 1489
<211> 67
<212> DNA
<213> Artificial Sequence

<220>
<223> Synthetic oligonucleotide

<400> 1489
gttctggttg ctggatttgg tcgcaaaggt catgctgttg gtgatattcc tggagtccgc 60
tttaagg 67

<210> 1490
<211> 80
<212> DNA
<213> Artificial Sequence

<220>
<223> Synthetic oligonucleotide

<400> 1490
tcaccacggt ctttagccat gcacaaacgg tagttttgtg tgttggctgc tccactgtcc 60
tctgccagcc tacaggagga 80

<210> 1491
<211> 66
<212> DNA
<213> Artificial Sequence

<220>
<223> Synthetic oligonucleotide

<400> 1491
gggctactgg cagctacatt gctgggacta atggcaattc caatggcctt gtaccgatgc 60
tgagag 66

<210> 1492
<211> 71
<212> DNA
<213> Artificial Sequence

<220>
<223> Synthetic oligonucleotide

<400> 1492
cagcgggatt aaacagtcct ttaaccagca cagccagtta aaagatgcag cctcactgct 60
tcaacgcaga t 71

<210> 1493
<211> 69
<212> DNA
<213> Artificial Sequence

<220>
<223> Synthetic oligonucleotide

<400> 1493
aacagagaca ttgccaacca tattggatct gcttgctgtc caaaccagca aacttcctgg 60
gcaaatcac 69

<210> 1494
<211> 77
<212> DNA
<213> Artificial Sequence

<220>
<223> Synthetic oligonucleotide

<400> 1494
acaccaaaat gccatctcaa atggaacacg ccatggaaac catgatgttt acatttcaca 60
aattcgctgg ggataaa 77

<210> 1495
<211> 73
<212> DNA
<213> Artificial Sequence

<220>
<223> Synthetic oligonucleotide

<400> 1495
tggctgtgct ggtcactacc ttccacaagt actcctgcca agagggcgac aagttcaagc 60
tgagtaaggg gga 73

<210> 1496
<211> 70
<212> DNA
<213> Artificial Sequence

<220>
<223> Synthetic oligonucleotide

<400> 1496
gactgctgtc atggcgtgcc ctctggagaa ggccctggat gtgatggtgt ccaccttcca 60
caagtactcg 70

<210> 1497
<211> 75
<212> DNA
<213> Artificial Sequence

<220>
<223> Synthetic oligonucleotide

<400> 1497
cctgctgacg atgatgaagg agaacttccc caacttcctt agtgcctgtg acaaaaaggg 60
cacaaattac ctcgc 75

<210> 1498
<211> 76
<212> DNA
<213> Artificial Sequence

<220>
<223> Synthetic oligonucleotide

<400> 1498
actccctgat aaaggggaat ttccatgccg tctacaggga tgacctgaag aaattgctag 60
agaccgagtg tcctca 76

<210> 1499
<211> 67
<212> DNA
<213> Artificial Sequence

<220>
<223> Synthetic oligonucleotide

<400> 1499
caccctgcct ctacccaacc agggccccgg ggcctgttat gtcaaactgt cttggctgtg 60
gggctag 67

<210> 1500
<211> 70
<212> DNA
<213> Artificial Sequence

<220>
<223> Synthetic oligonucleotide

<400> 1500
catggccgtg tagaccctaa cccggaggga accctgacta cagaaattac cccgggcac 60
ccttaaaact 70

<210> 1501
<211> 67
<212> DNA
<213> Artificial Sequence

<220>
<223> Synthetic oligonucleotide

<400> 1501
accctgagca ctggaggaag agcgcctgtg ctgtggtctt atcctatgtg gaatccccca 60
aagtctc 67

<210> 1502
<211> 67

<212> DNA
<213> Artificial Sequence

<220>
<223> Synthetic oligonucleotide

<400> 1502
agacaaggat gccgtggata aattgctcaa ggacctggac gccaatggag atgcccaggt 60
ggacttc 67

<210> 1503
<211> 67
<212> DNA
<213> Artificial Sequence

<220>
<223> Synthetic oligonucleotide

<400> 1503
gcagaactga agatgggaag atttatcagc gtgcatttgg tggacagagc ctcaagtttg 60
gaaaggg 67

<210> 1504
<211> 86
<212> DNA
<213> Artificial Sequence

<220>
<223> Synthetic oligonucleotide

<400> 1504
cgcgagcccc tcattataca ctgggcagcc tccccacagc gcatcgagga atgcgtgctc 60
tcaggcaagg atgtcaacgg cgagtg 86

<210> 1505
<211> 66
<212> DNA
<213> Artificial Sequence

<220>
<223> Synthetic oligonucleotide

<400> 1505
caagctgaac ggtgtgtccg aaagggacct gaagaaatcg gtgctgtggc tcaaagacag 60
cttgca 66

<210> 1506
<211> 72
<212> DNA
<213> Artificial Sequence

<220>
<223> Synthetic oligonucleotide

<400> 1506
agctggggtg tctgtttcat gtggaatacc tgacttcagg tcaagggatg gtatttatgc 60
tcgccttgct gt 72

<210> 1507
<211> 66
<212> DNA
<213> Artificial Sequence

<220>
<223> Synthetic oligonucleotide

<400> 1507
agaggctgaa tatgcaggac agttggcaga actgaggcag agattggacc atgctgaggc 60
cgatag 66

<210> 1508
<211> 71
<212> DNA
<213> Artificial Sequence

<220>
<223> Synthetic oligonucleotide

<400> 1508
agttgcagaa tctaagcctg gaaggcctgc ggctttcgga tcccattgtc aatactctcg 60
caaaaaactc a 71

<210> 1509
<211> 74
<212> DNA
<213> Artificial Sequence

<220>
<223> Synthetic oligonucleotide

<400> 1509
atggccaatg tttgatgctt aaccccccca atttctgtga gatggatggc cagtgcaagc 60
gtgacttgaa gtgt 74

<210> 1510
<211> 69
<212> DNA
<213> Artificial Sequence

<220>
<223> Synthetic oligonucleotide

<400> 1510
cccaatcgga agcctaacta cagcgagctg caggactcta atccagagtt taccttccag 60
cagccctac 69

<210> 1511
<211> 69
<212> DNA
<213> Artificial Sequence

<220>
<223> Synthetic oligonucleotide

<400> 1511
catcttccag gaggaccact ctctgtggca ccctggacta cctgccccct gaaatgattg 60
aaggtcgga 69

<210> 1512
<211> 71
<212> DNA
<213> Artificial Sequence

<220>
<223> Synthetic oligonucleotide

<400> 1512
aatacccaac gcacaaatga ccgcacgttc tctgccccgt ttcttgcccc agtgtggttt 60
gcattgtctc c 71

<210> 1513
<211> 90
<212> DNA
<213> Artificial Sequence

<220>
<223> Synthetic oligonucleotide

<400> 1513
cctggaggct gcaacatacc tcaatcctgt cccaggccgg atcctcctga agccctttc 60
gcagcactgc tatcctccaa agccattgta 90

<210> 1514
<211> 80
<212> DNA
<213> Artificial Sequence

<220>
<223> Synthetic oligonucleotide

<400> 1514
tgttttgatt cccgggctta ccaggtgaga agtgagggag gaagaaggca gtgtcccttt 60
tgctagagct gacagctttg 80

<210> 1515
<211> 85
<212> DNA
<213> Artificial Sequence

<220>
<223> Synthetic oligonucleotide

<400> 1515
tctccagcaa aagcgatgtc tggagctttg gagtgttgat gtgggaagca ttctcctatg 60
ggcagaagcc atatcgaggg atgaa 85

<210> 1516
<211> 73
<212> DNA
<213> Artificial Sequence

<220>
<223> Synthetic oligonucleotide

<400> 1516
gatggagcag gtggctcagt tcctgaaggc ggctgaggac tctggggtca tcaagactga 60
catgttccag act 73

<210> 1517
<211> 72
<212> DNA
<213> Artificial Sequence

<220>
<223> Synthetic oligonucleotide

<400> 1517
cagccctgag gcaagagaag aaagtacttc cagcggcaat gtaagcaaca gaaaggatga 60
gacaaatgct cg 72

<210> 1518
<211> 68
<212> DNA
<213> Artificial Sequence

<220>
<223> Synthetic oligonucleotide

<400> 1518
gccaactgct ttcatttgtg agggatctga accaatacag agcagacata aaggaaatgg 60
gcctgagt 68

<210> 1519
<211> 75
<212> DNA
<213> Artificial Sequence

<220>
<223> Synthetic oligonucleotide

<400> 1519
accagtcccc cagaagacta tcctgagccc gaggaagtcc ccccggaggt gatttccatc 60
tacaacagca ccagg 75

<210> 1520
<211> 65
<212> DNA
<213> Artificial Sequence

<220>
<223> Synthetic oligonucleotide

<400> 1520
ggatcgagct cttccagatc cttcggccag atgagcacat tgccaaacag cgctatatcg 60
gtggc 65

<210> 1521
<211> 66
<212> DNA
<213> Artificial Sequence

<220>
<223> Synthetic oligonucleotide

<400> 1521
aacaccaatg ggttccatct ttctgggctc ctgattgctc aagcacagtt tggcctgatg 60
aagagg 66

<210> 1522
<211> 67
<212> DNA
<213> Artificial Sequence

<220>
<223> Synthetic oligonucleotide

<400> 1522
ctacctgcct tgctttgtga cttccaagaa cgagtgtctc tggaccgaca tgctctccaa 60
tttcggt 67

<210> 1523
<211> 67

<212> DNA
<213> Artificial Sequence

<220>
<223> Synthetic oligonucleotide

<400> 1523
ccagcccaca gaccagttac tgttcctcac tgagcctgga agcaaatcca cacctccttt 60
ctctgaa 67

<210> 1524
<211> 67
<212> DNA
<213> Artificial Sequence

<220>
<223> Synthetic oligonucleotide

<400> 1524
tggcgaccaa gacaccttga agggcctaat gcacgcacta aagcactcaa agacgtacca 60
ctttccc 67

<210> 1525
<211> 71
<212> DNA
<213> Artificial Sequence

<220>
<223> Synthetic oligonucleotide

<400> 1525
catatcgttg gatcacagca catcagagca gagaaagcga tggtggatgg ctcatggtta 60
gatctggcca a 71

<210> 1526
<211> 64
<212> DNA
<213> Artificial Sequence

<220>
<223> Synthetic oligonucleotide

<400> 1526
gcgctgcgga agatcatccc cacgctgccc tcggacaagc tgagcaagat tcagaccctc 60
aagc 64

<210> 1527
<211> 522
<212> DNA
<213> Artificial Sequence

<220>
<223> Synthetic oligonucleotide

<400> 1527
gagtcgaccc tgcacctggt cctgcgtctg agaggtggta tgcagatctt cgtgaagacc 60
ctgaccggca agaccatcac cctggaagtg gagcccagtg acaccatcga aaatgtgaag 120
gccaagatcc aggataaaga aggcatccct cccgaccagc agaggctcat ctttgcaggc 180
aagcagctgg aagatggccg cactctttct gactacaaca tccagaagga gtcgaccctg 240
cacctggtcc tgcgtctgag aggtggtatg cagatcttcg tgaagaccct gaccggcaag 300
accatcactc tggaagtgga gcccagtgac accatcgaaa atgtgaaggc caagatccaa 360
gataaagaag gcatccctcc cgaccagcag aggctcatct ttgcaggcaa gcagctggaa 420
gatggccgca ctctttctga ctacaacatc cagaaggagt cgaccctgca cctggtcctg 480

cgcctgaggg gtggctgtta attcttcagt catggcattc gc                    522

<210> 1528
<211> 89
<212> DNA
<213> Artificial Sequence

<220>
<223> Synthetic oligonucleotide

<400> 1528
tggcttcagg agctgaatac cctcccaggc acacacaggt gggacacaaa taagggtttt 60
ggaaccacta ttttctcatc acgacagca                                   89

<210> 1529
<211> 72
<212> DNA
<213> Artificial Sequence

<220>
<223> Synthetic oligonucleotide

<400> 1529
tgcccttaaa ggaaccaatg agtccctgga acgccagatg cgtgaaatgg aagagaactt 60
tgccgttgaa gc                                                     72

<210> 1530
<211> 67
<212> DNA
<213> Artificial Sequence

<220>
<223> Synthetic oligonucleotide

<400> 1530
agtcaatctt cgcacacggc gagtggacac tgtggaccct ccctacccac gctccatcgc 60
tcagtac                                                           67

<210> 1531
<211> 68
<212> DNA
<213> Artificial Sequence

<220>
<223> Synthetic oligonucleotide

<400> 1531
ctctccagtg tgggcaccag ccggccagaa cagatgcgag cagtccatga ctctgggagc 60
tacaccgc                                                          68

<210> 1532
<211> 75
<212> DNA
<213> Artificial Sequence

<220>
<223> Synthetic oligonucleotide

<400> 1532
agaggcatcc atgaacttca cacttgcggg ctgcatcagc acacgctcct atcaacccaa 60
gtactgtgga gtttg                                                  75

<210> 1533

<211> 75
<212> DNA
<213> Artificial Sequence

<220>
<223> Synthetic oligonucleotide

<400> 1533
gtatcaggac cacatgcagt acatcggaga aggcgcgaag acaggcatca aagaatgcca 60
gtatcaattc cgaca 75

<210> 1534
<211> 79
<212> DNA
<213> Artificial Sequence

<220>
<223> Synthetic oligonucleotide

<400> 1534
tgtcttcagg gtcttgtcca gaatgtagat gggttccgta agaggcctgg tgctctctta 60
ctctttcatc cacgtgcac 79

<210> 1535
<211> 74
<212> DNA
<213> Artificial Sequence

<220>
<223> Synthetic oligonucleotide

<400> 1535
atcgcagctg gtgggtgtac acactgctgt ttaccttggc gaggcctttc accaagtcca 60
tgcaacaggg agct 74

<210> 1536
<211> 81
<212> DNA
<213> Artificial Sequence

<220>
<223> Synthetic oligonucleotide

<400> 1536
gtggacatcg gatacccaag gagacgaagc tgaagcagga gaaggagggg aaaattaacc 60
ggccttccaa cttttgtctg c 81